# EUROPEAN PATENT APPLICATION

(11) **EP 3 323 897 A1**
(43) Date of publication of application: **23.05.2018**
(21) Application number: 17205295.3
(22) Date of filing: 02.10.2012
(51) Int. Cl.: C12Q 1/6883, G01N 33/48, G01N 33/53

(54) **METHODS AND DEVICES FOR ASSESSING RISK TO A PUTATIVE OFFSPRING OF DEVELOPING A CONDITION**

(30) Priority: 03.10.2011 US 201161542324 P
(62) Divisional of application: 12838826.1
(71) Applicant: Celmatix, Inc., New York, NY 10014 (US)
(72) Inventor: BEIM, Piraye, Yurttas, New York, NY 02111 (US)
(74) Representative: Kirkham, Nicholas Andrew

(57) **Abstract**

The invention generally relates to methods and devices for assessing risk to a putative offspring of developing a condition. In certain embodiments, the invention provides methods for assessing risk to a putative offspring of developing a condition that involve obtaining a maternal sample, conducting an assay on at least one infertility- associated biomarker, and assessing risk to a putative offspring of developing a condition based upon results of the assay.

## Description

### Related Application

The present application claims the benefit of and priority to U.S. provisional application serial number 61/542,324, filed October 3, 2011, the content of which is incorporated by reference herein in its entirety.

### Field of the Invention

The invention generally relates to methods and devices for assessing risk to a putative offspring of developing a condition.

### Background

Approximately one in seven couples have difficulty conceiving. Infertility may be due to a single cause in either partner, or a combination of factors (e.g., genetic factors, diseases, or environmental factors) that may prevent a pregnancy from occurring or continuing. Every woman will become infertile in her lifetime due to menopause. On average, egg quality and number begins to decline precipitously at 35. However, a number of women are fertile well into their 40's, and some women experience this decline much earlier in life. Though, generally, advanced maternal age (35 and above) is associated with poorer fertility outcomes, there is no way of diagnosing egg quality issues in younger women or knowing when a particular woman will start to experience decline in her egg quality or reserve.

There are many different assisted reproductive technologies available to women who have difficulty conceiving. For example, *in vitro* fertilization (IVF), a process in which egg cells are fertilized by sperm outside a woman's womb and then transferred into the womb, is a common procedure to assist women who have difficulty conceiving. Those technologies are focused on helping a woman to achieve a live birth outcome and do not address risks to the putative offspring that are potentially inherent in bypassing the infertility defect. Since assisted reproductive technologies provide procedures for women to overcome their infertility, a child is born that otherwise would not be born, and that child may be born with a risk of developing a genetic condition, which risk was not assessed or presented to the woman utilizing the assisted reproductive technology. Women would benefit greatly from information about whether reproducing at a particular age or through certain or all assisted reproductive technologies increases her risk of having an offspring with a genetic condition. That information may influence whether a woman decides to reproduce with her own oocytes and womb or with the help of donor oocytes and/or a gestational carrier (surrogate).

### Summary

The invention recognizes that underlying maternal genetic variations (e.g., mutations, polymporphisms, structural variants, expression levels) that may give rise to infertility issues are the same genetic variations that are also indicative of conditions that a putative offspring may develop, i.e., the same genetic variations that predispose a women to have infertility also create a risk for her offspring to be diagnosed with a genetic condition. Thus, even if the infertility issues may be overcome by use of assisted reproductive technologies (e.g., *in vitro* fertilization) there is still a risk that the putative offspring will develop a condition related to the genetic variations. The invention provides methods that are able to assess the risk to the putative offspring of developing a genetic condition based upon the maternal genetic variations. In this manner, methods of the invention provide information to women that allow them to make infertility related decisions while also providing them with the risk that would be present for her putative offspring.

In certain aspects, methods of the invention involve obtaining a maternal sample, conducting an assay on at least one infertility-associated biomarker, and assessing risk to a putative offspring of developing a condition based upon results of the assay. As discussed below, an array of genetic information concerning the status of various maternal effect genes and/or other infertility-related genetic regions is used in order to assess risk of an offspring developing a condition. The genetic information may include one or more polymorphisms in one or more maternal effect genes and/or other infertility-related genetic regions, mutations in one or more of those genetic regions, or epigenetic factors affecting the expression of those genetic regions. The molecular consequence of these genetic region mutations could be one or a combination of the following: alternative splicing, lowered or increased RNA expression, and/or alterations in protein expression. These alterations could also include a different protein product being produced, such as one with reduced or increased activity, or a protein that elicits an abnormal immunological reaction. All of this information is significant in terms of informing a patient of the likelihood having an offspring with a disease condition.

In addition to looking exclusively at genomic information, by combining genetic information (e.g., polymorphisms, mutations, etc.) with phenotypic and/or environmental data, methods of the invention provide an additional level of clinical clarity. For example, polymorphisms in genes discussed below may provide information about a disposition toward an offspring developing a condition. However, in certain cases, the clinical outcome may not be determinative unless combined with certain phenotypic and/or environmental information. Thus, methods of the invention provide for a combination of genetic predispositional analysis in combination with phenotypic and environmental exposure data in order to assess the potential for an offspring to develop a condition. Thus, in certain cases, genetic predisposition may be sufficient to make a diagnosis, but in other cases, the clinical outcome may not be clear based upon genetic analysis alone and the combination of genetic and phenotypic or environmental data must be used in order to assess the likelihood of having an offspring with a disease condition.

In addition to providing information to women related to the risk that her putative offspring will develop a condition if she chooses to try for a child at a particular maternal age or by using assisted reproductive technologies, methods of the invention may also be used by a physician for treatment purposes, e.g., allowing a physician to make vitamin / drug recommendations to help reduce or eliminate the risk to the putative offspring of developing the condition. For example, data herein show that a mutation in the CBS gene affects infertility and is also associated with development of an autism spectrum disorder in the putative offspring. This data may be used by a physician to generate a treatment plan that may help remediate the autism risk in the offspring. For example, the physician may advise the woman to take a high dose of folic acid or other vitamin supplements / drugs and that the same be administered to the child when born. Such a treatment plan may reduce or eliminate the autism risk in the putative offspring.

A biomarker generally refers to a molecule that acts as an indicator of a biological state. In certain embodiments, the biomarker is a genetic region. In particular embodiments, the genetic region is a maternal effect gene and/or other infertility-related genetic regions. Any assay known in the art may be used to analyze the genetic region. In certain embodiments, the assay includes sequencing at least a portion of the genetic region to determine presence or absence of a mutation that is associated with infertility. Mutations detected according to the invention may be any type of genetic mutation. Exemplary mutations include a single nucleotide polymorphism, a deletion, an insertion, an inversion, other rearrangements, a copy number variation, or a combination thereof. Any method of detecting genetic mutations is useful with methods of the invention, and numerous methods are known in the art. In certain embodiments, sequencing is used to determine the presence of a mutation in the infertility-associated genetic region. In particularly-preferred embodiments, the sequencing is sequencing-by-synthesis.

In other embodiments, the biomarker is a gene product. In particular embodiments, the gene product is a product of a maternal effect and/or other infertility-related genes. The gene product may be RNA or protein. Any assay known in the art may be used to analyze the gene product. In certain embodiments, the assay involves determining an amount of the gene product and comparing the determined amount to a reference.

Methods of the invention may further involve obtaining a sample from the mammal that includes the infertility-associated biomarker. The sample may be a human tissue or body fluid. In particular embodiments, the sample is maternal blood or maternal saliva. Methods of the invention may also involve enriching the sample for the infertility-associated biomarker.

Methods of the invention may be used to assess the risk of a putative offspring developing any condition that is linked to an infertility-associated biomarker. In certain embodiments, the condition is a neurodevelopmental, neuropsychological or neuro-genetic disorder, e.g. neural tube defects, an autism spectrum disorder (including, but not limited to classical autism, asperger syndrome, rett syndrome, childhood disintegrative disorder, and pervasive developmental disorder not otherwise specified (PDD-NOS)), Bardet-Beidl syndrome, Attention Deficit Hyperactivity Disorder (ADHD), Angelman Syndrome, Prader-Willi Syndrome, Bipolar Disorder, Charcot Marie Tooth Syndrome, or Schizophrenia. In other embodiments, the condition is a metabolic disorder, e.g. obesity and Diabetes Mellitus (Type I or II). In other embodiments, the condition is a gynecological and/or infertility disorder, e.g. Endometriosis and Premature ovarian failure (POF). In other embodiments, the condition is an autoimmune disorder, e.g. asthma, juvenile idiopathic arthritis, allergies, Addison's disease, Crohn's disease, and Celiac disease. In other embodiments, the condition is a muscular dystrophy or a cancer. In other embodiments, the condition is a cardiovascular disease such as early onset coronary heart disease.

Another aspect of the invention provides methods for assessing infertility and risk to a putative offspring of developing a condition that involve obtaining a maternal sample, conducting an assay on at least one infertility-associated biomarker, and assessing infertility and risk to a putative offspring of developing a condition based on results of the assay.

### Brief Description of the Drawings

Fig. 1 depicts three maternal effect genes (PADI6, NLRP5, and OOEP; see Table 1) that are highly conserved, both at the protein, genetic, and gene expression level in humans (H.s.) and mice (M.m.). Fig. 1 also depicts putative fertility-related genes that are clustered with each of the three maternal effect genes.
Fig. 2 depicts important mammalian egg structures that infertility-associated genes localize to and regulate.
Figs. 3-7 depicts the gene expression profile of a number of maternal effect genes in mice and humans.
Fig. 8 depicts the gene expression profile of the MTHFR gene in embryonic and adult human tissues.
Fig. 9 depicts the gene expression profile of the COMT gene in embryonic and adult human tissues.
Fig. 10 depicts the gene expression profile of the MTRR gene in embryonic and adult human tissues.
Fig. 11 depicts the gene expression profile of the BHMT gene in embryonic and adult human tissues.
Fig. 12 depicts the gene expression profile of the FOLR2 gene in embryonic and adult human tissues.
Fig. 13 depicts the gene expression profile of the FOLR1 gene in embryonic and adult human tissues.
Fig. 14 depicts the gene expression profile of the TCN2 gene in embryonic and adult human tissues.
Fig. 15 depicts the gene expression profile of the CBS gene in embryonic and adult human tissues.
Fig. 16 is a schematic depicting clustering of autism genes with fertility genes.
Fig. 17 is a schematic depicting the CBS gene as visualized in a genome browser. The red bar corresponds to a region of the gene that is deleted in a patient experiencing infertility, who with the help of IVF, was able to become pregnant. Also labeled for reference are the exons (showing that the deletion covers exons and is therefore likely to affect gene function) and the location of a SNP that has been associated with autism risk in offspring of women carrying this variation.
Fig. 18 is a schematic depicting the CBS gene with the relative size and location of the 5,000bp deletion that has been detected in a woman who was able to overcome infertility with IVF.
Fig. 19 is a schematic depicting the folate-mediated one carbon metabolism pathway. Infertility-associated genes that have also been linked to a developmental disorder are highlighted in blue.

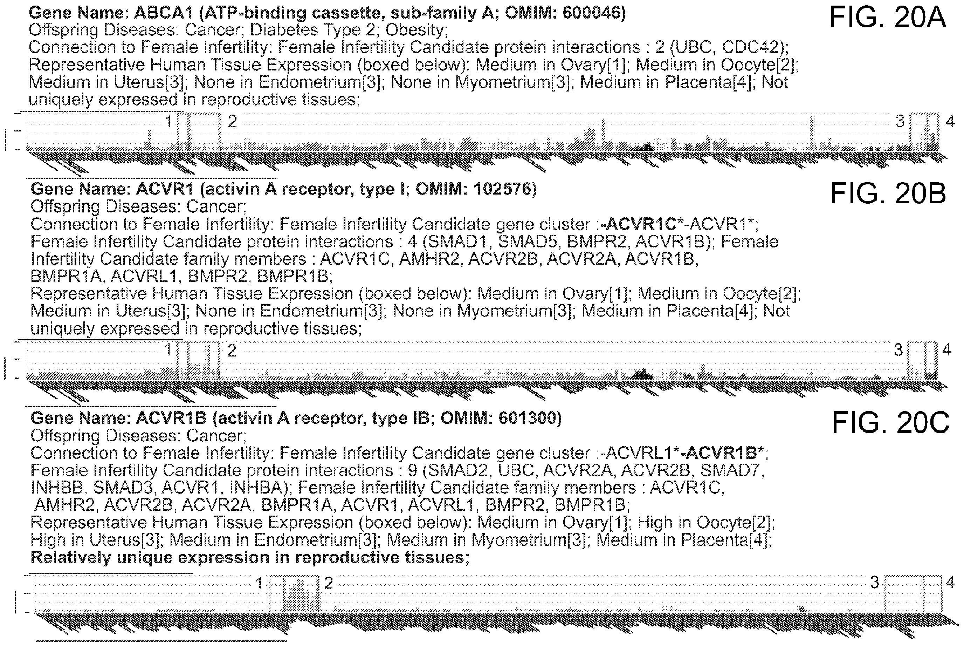

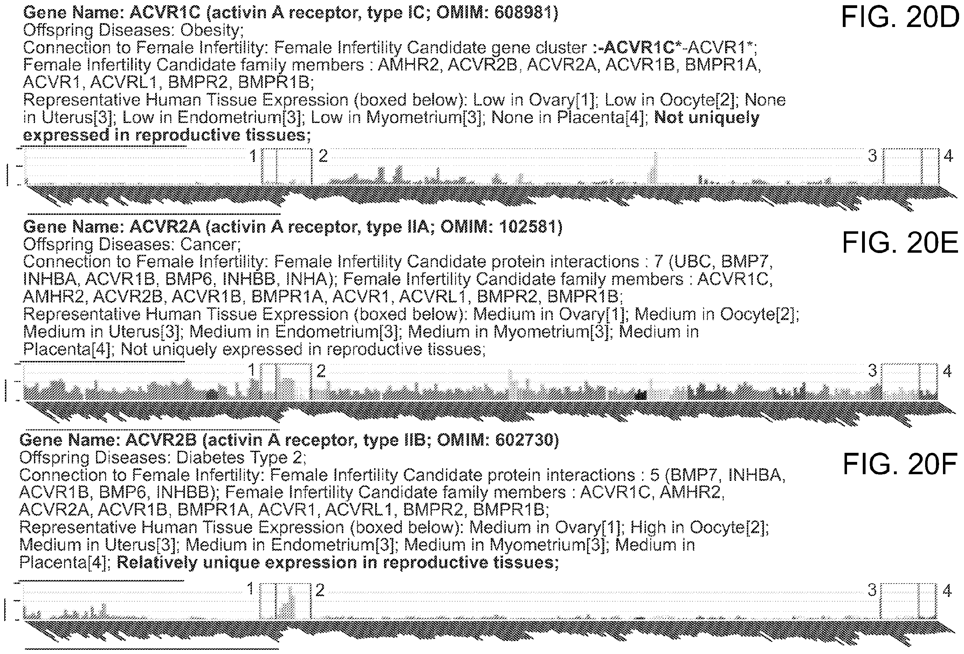

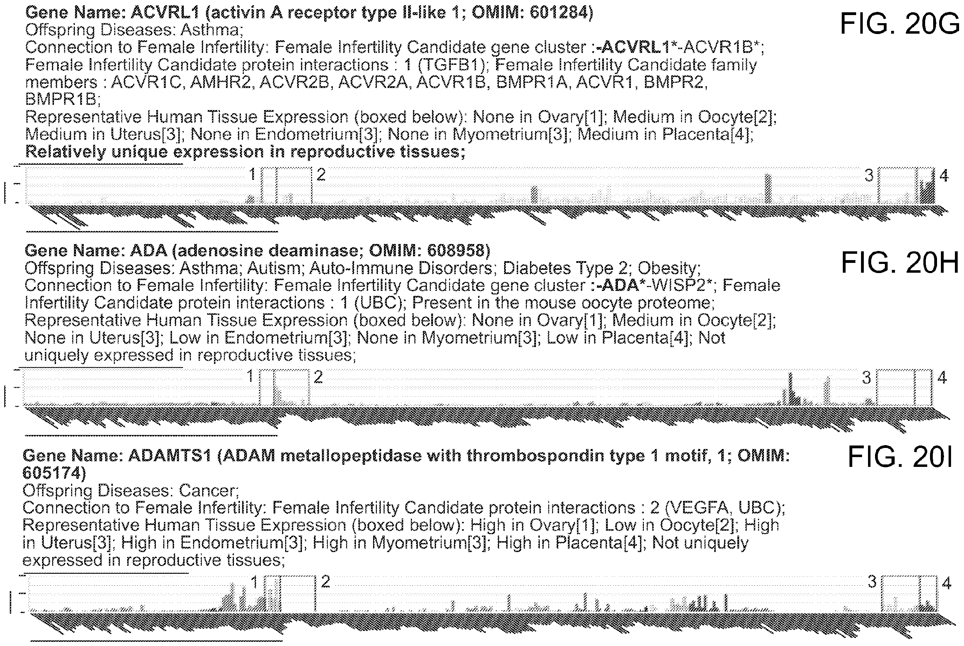

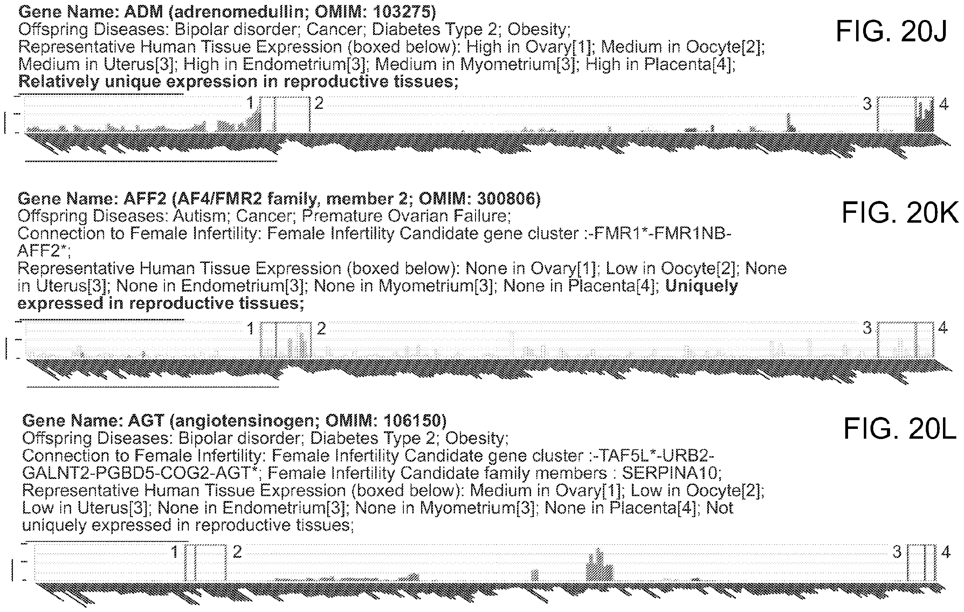

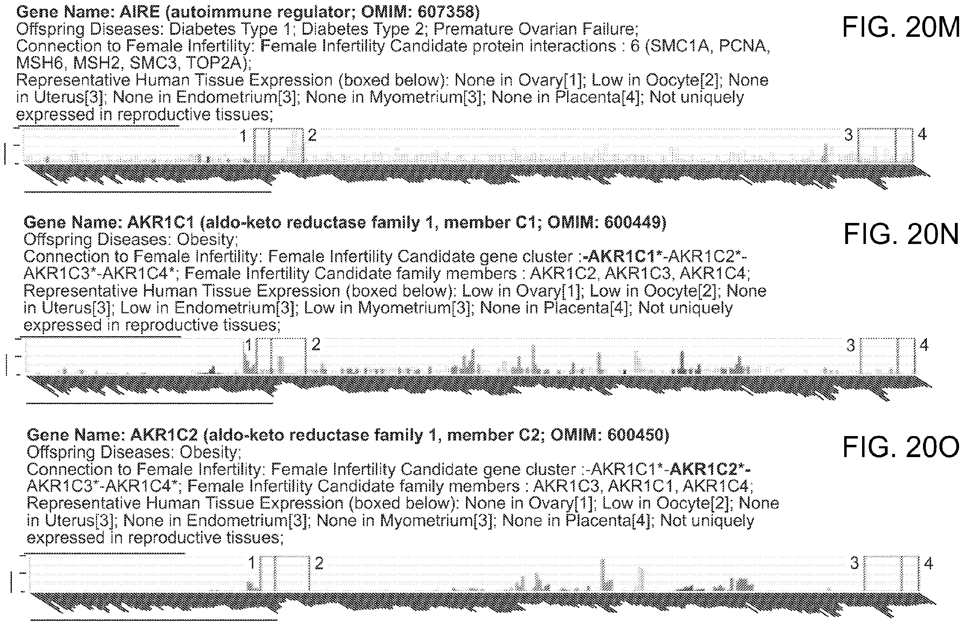

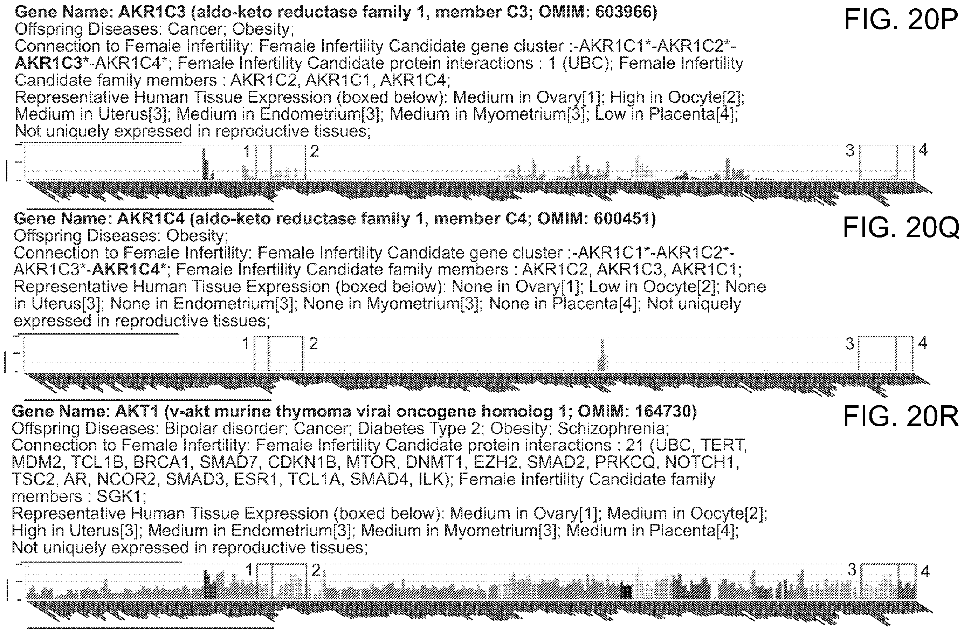

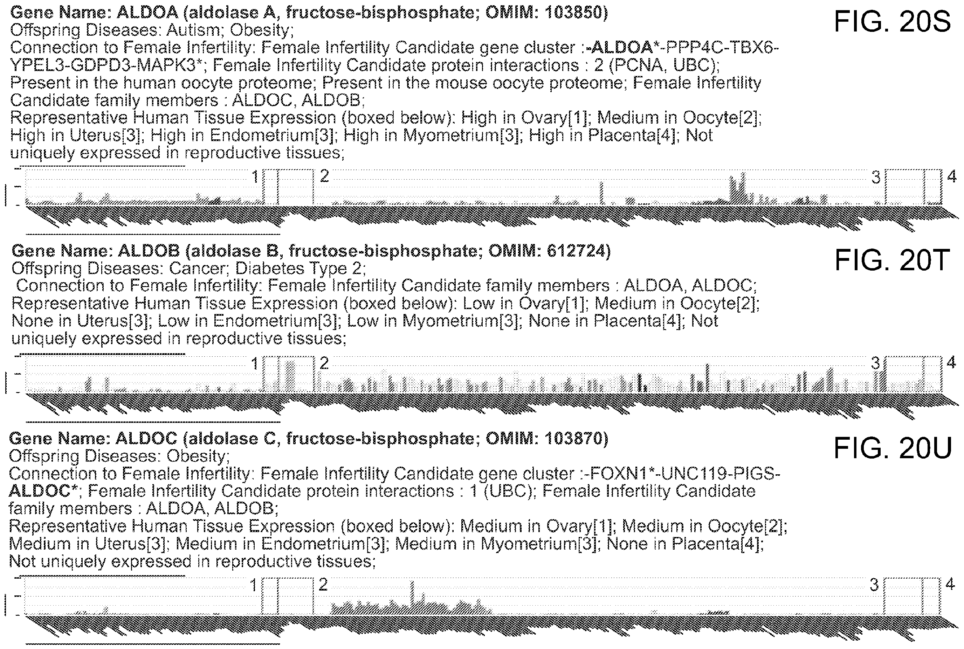

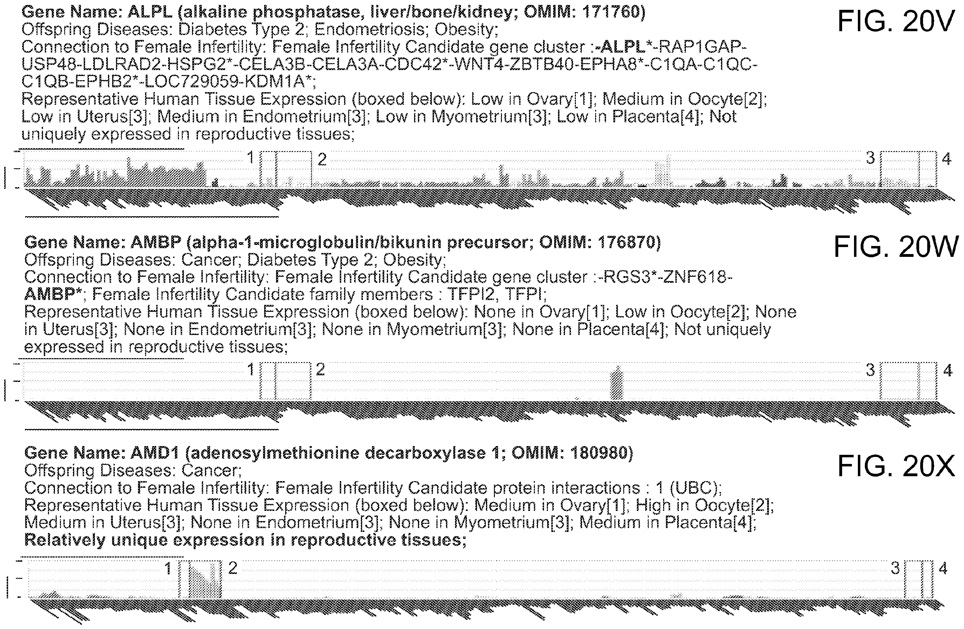

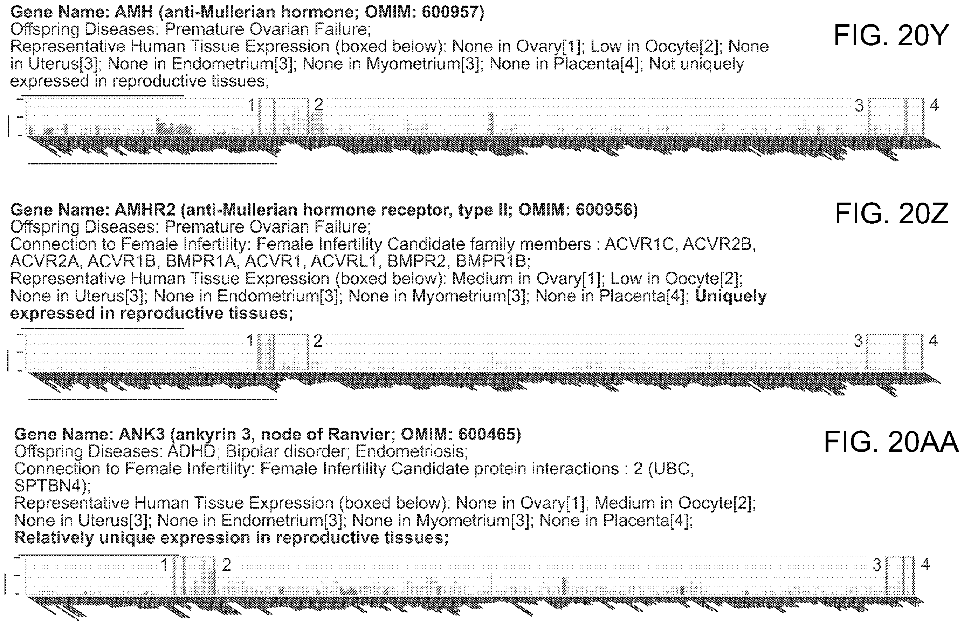

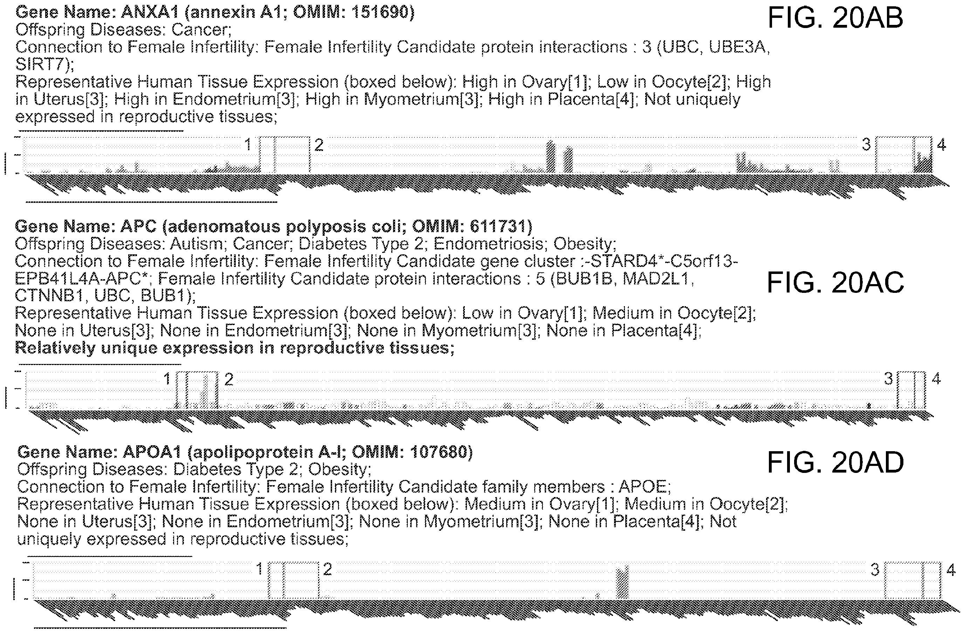

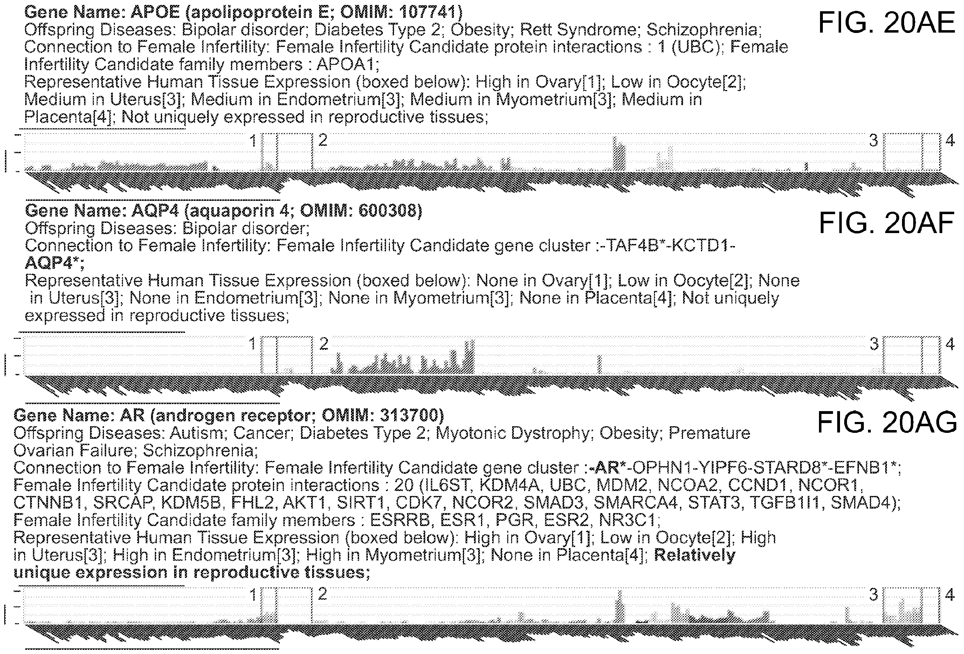

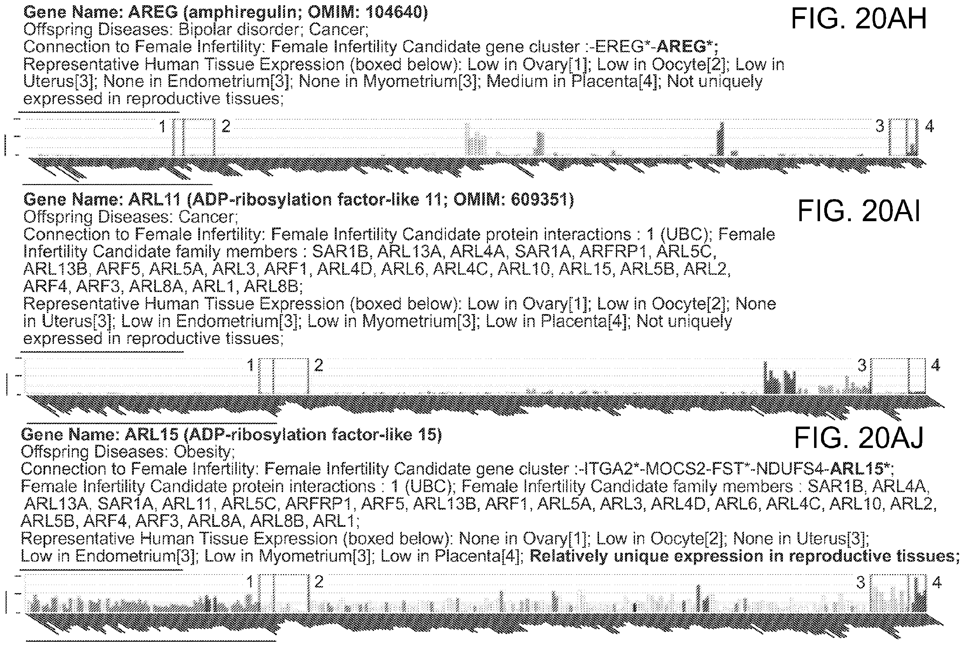

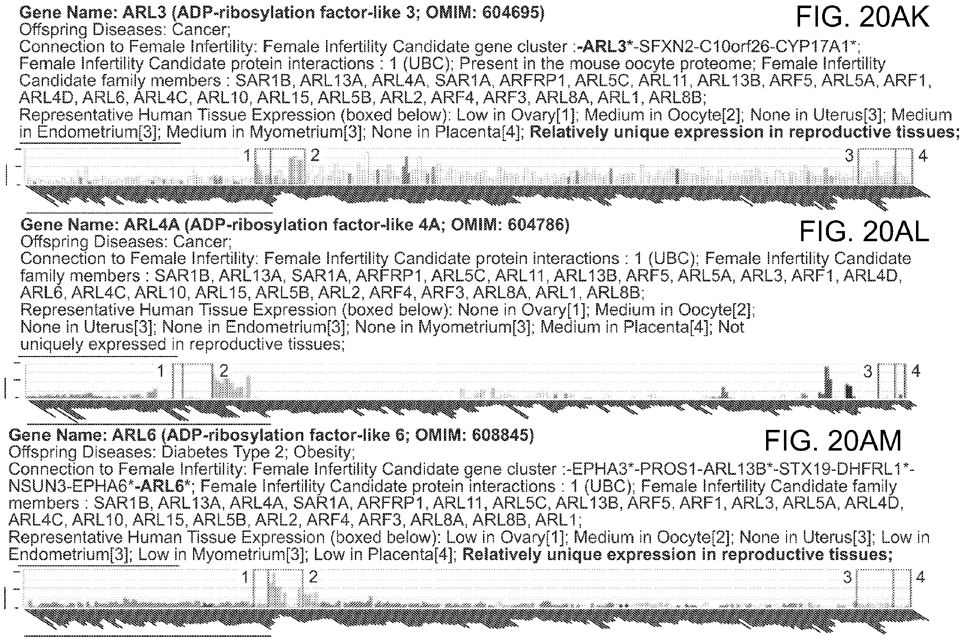

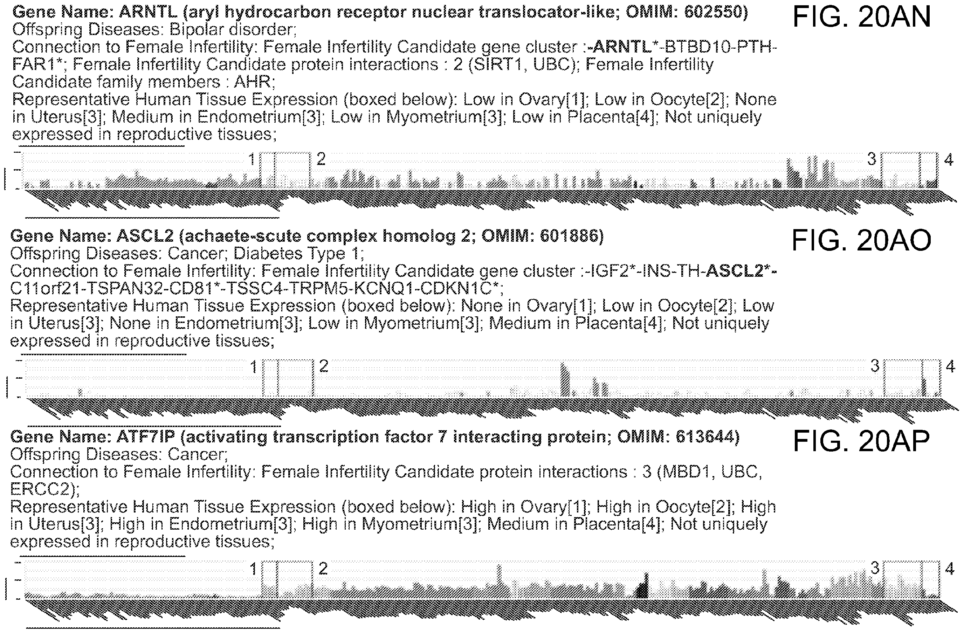

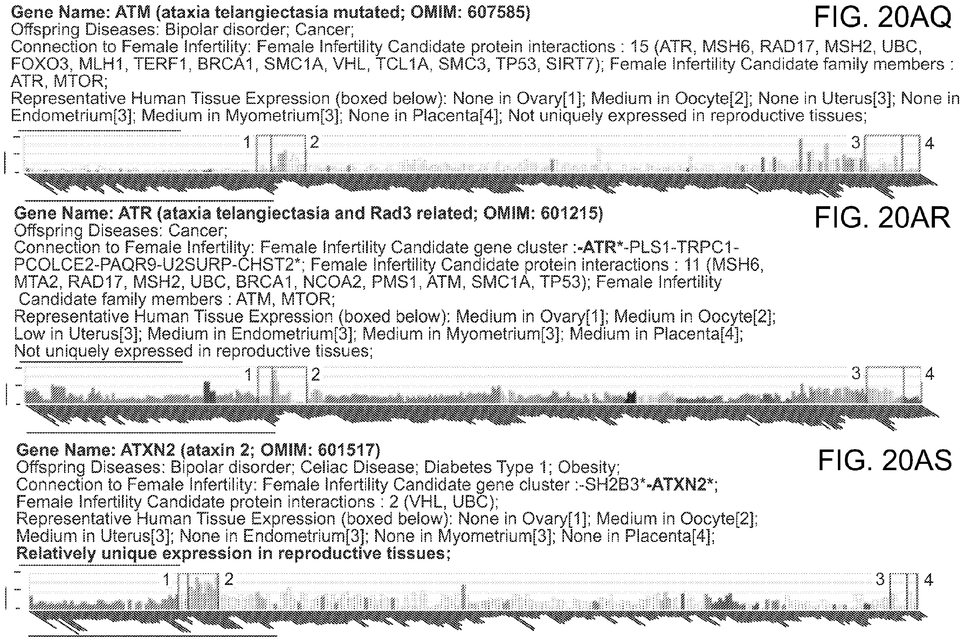

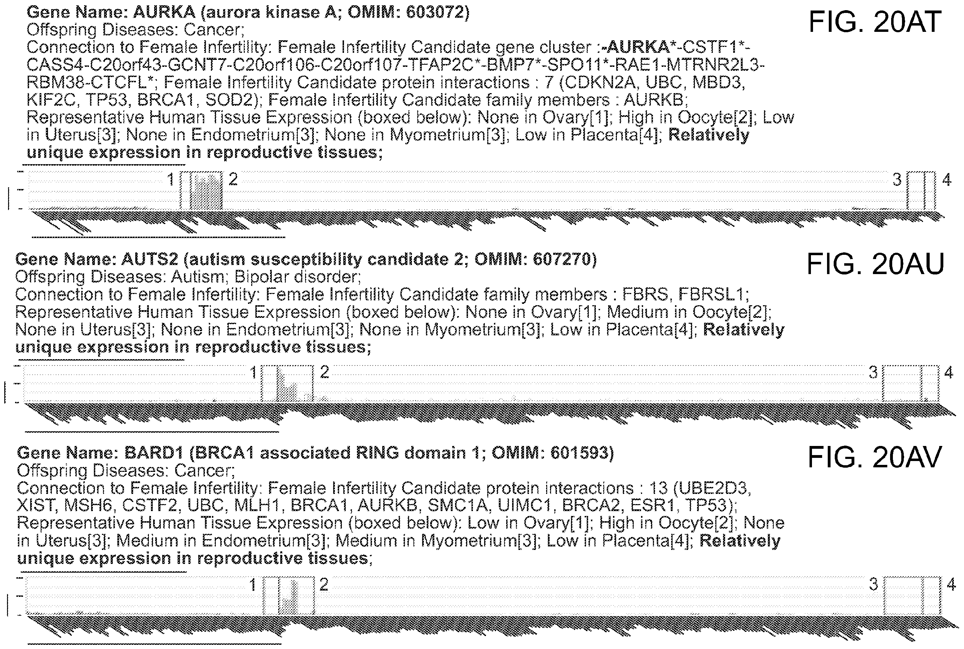

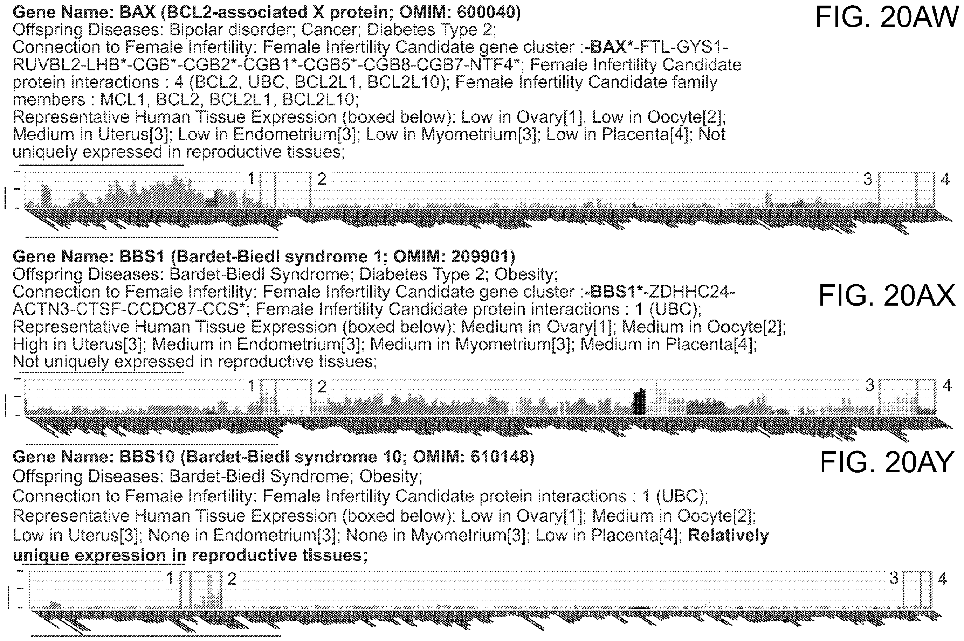

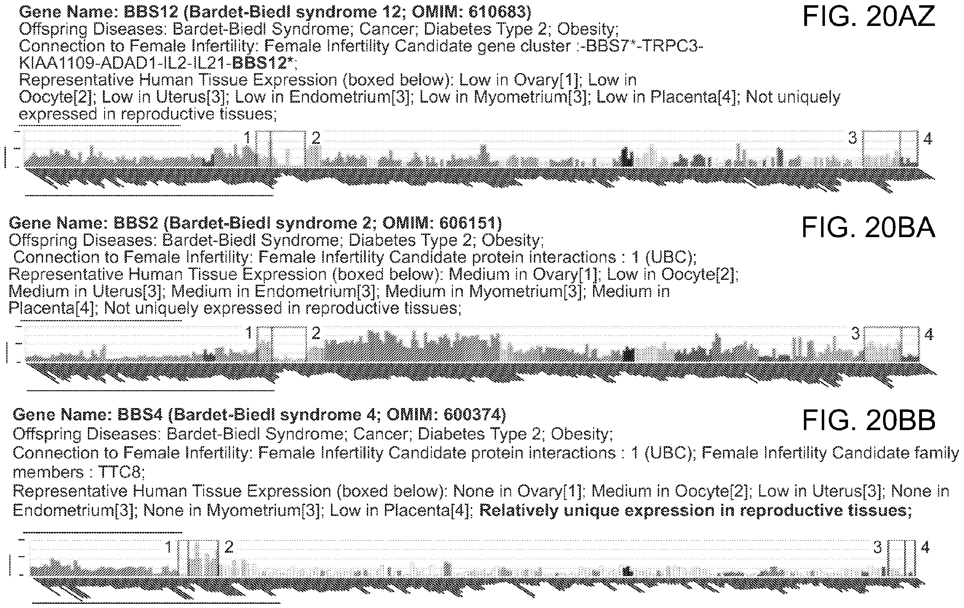

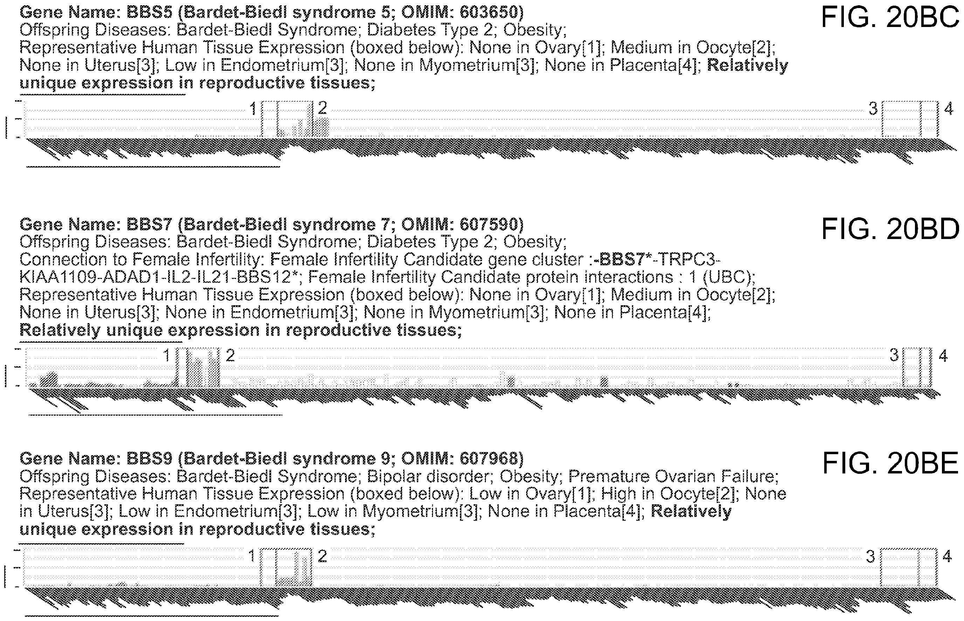

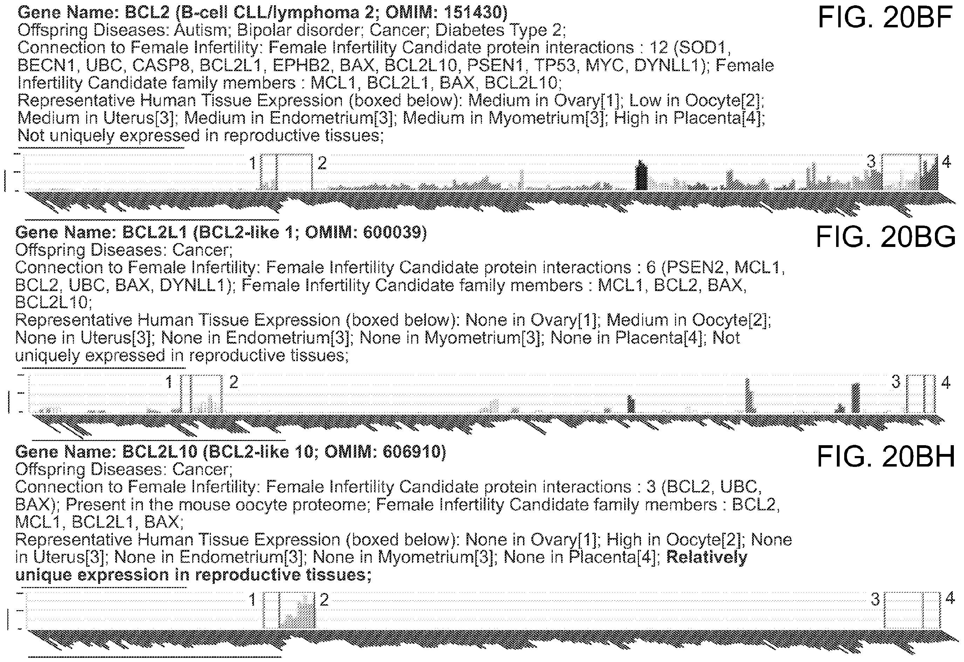

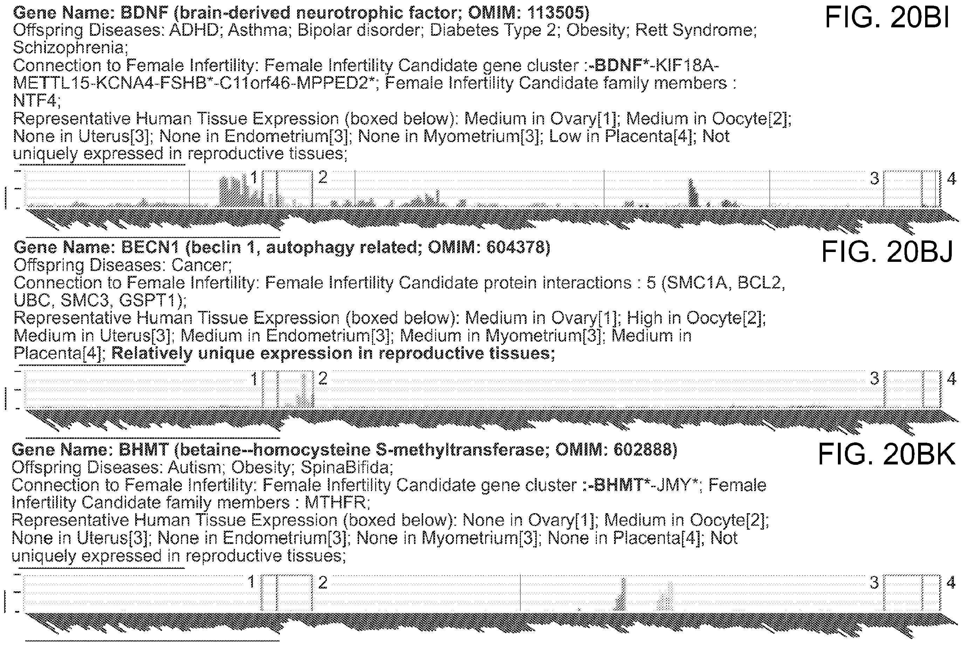

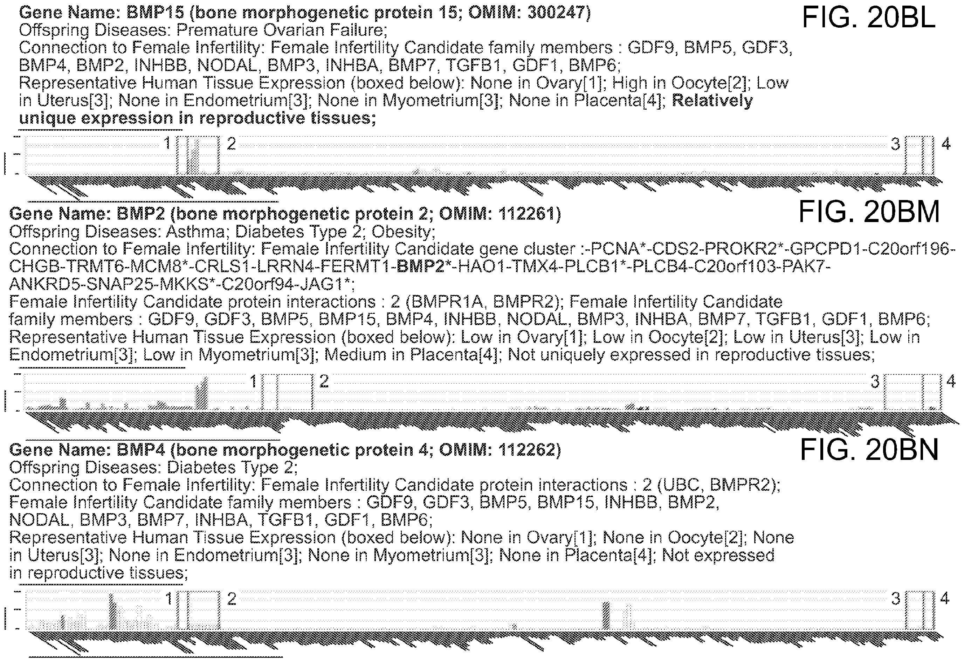

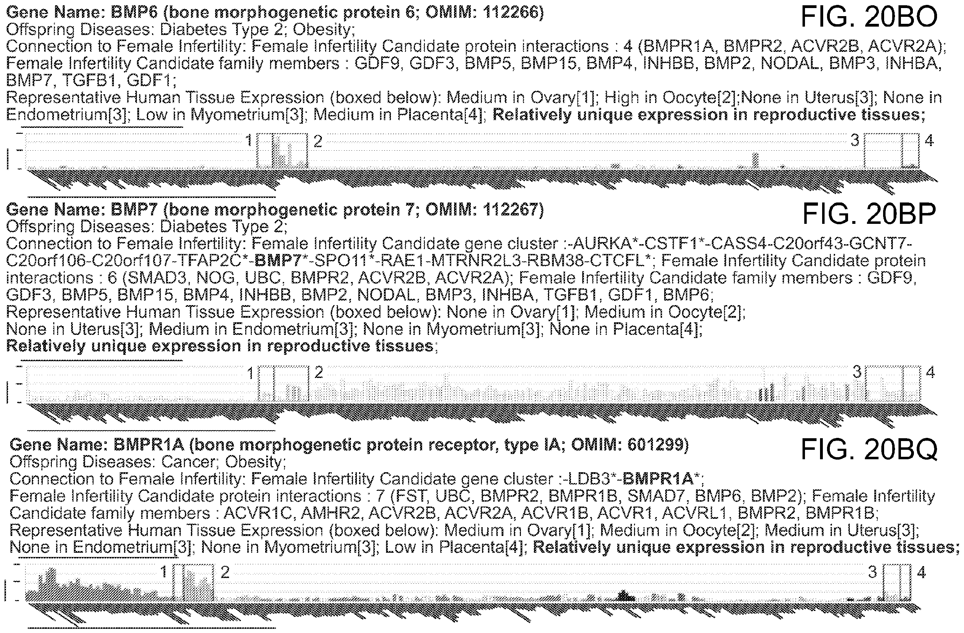

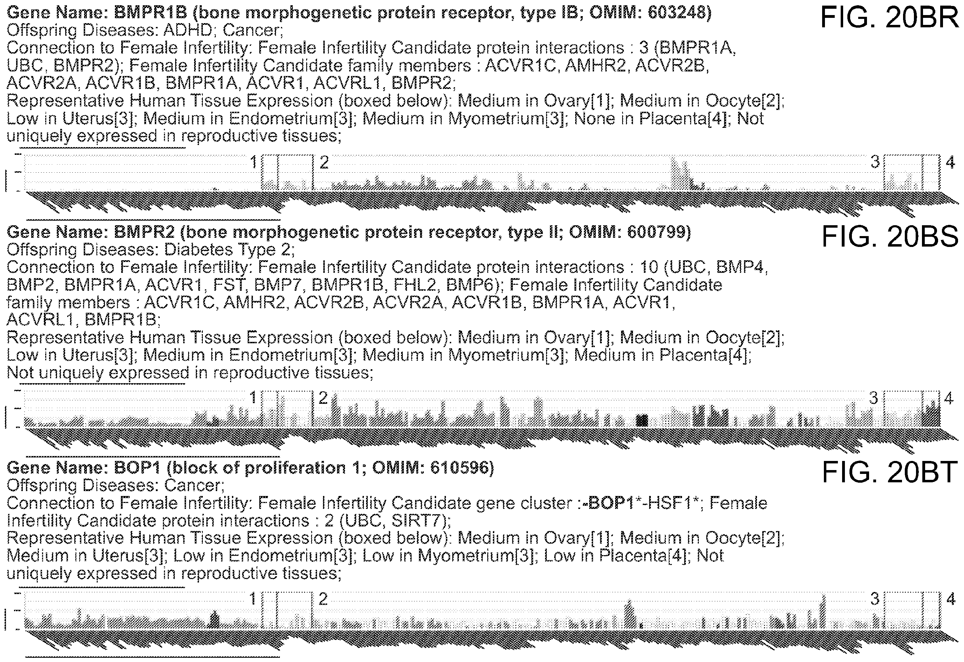

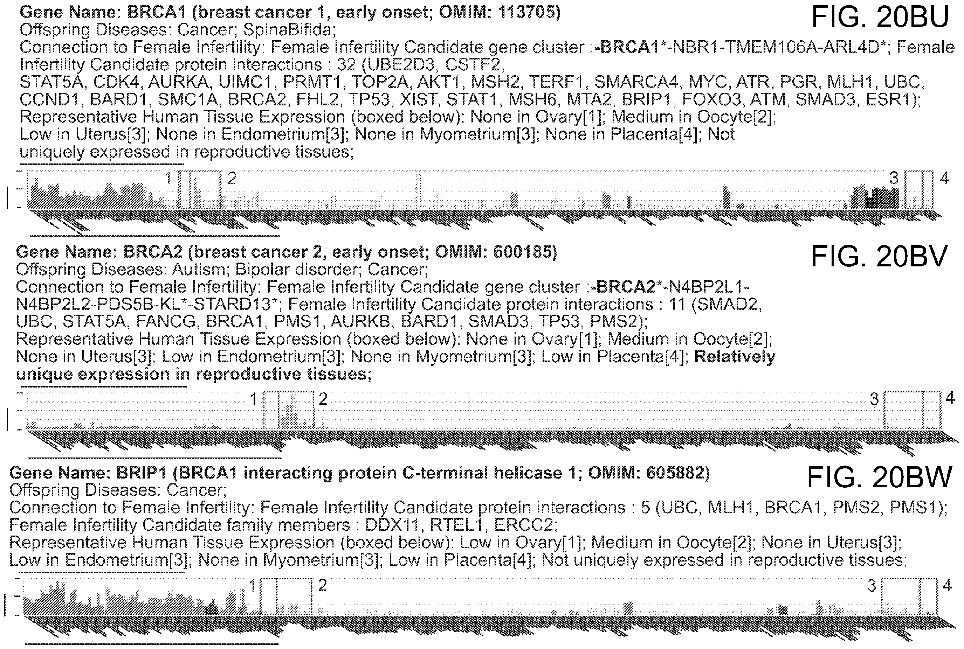

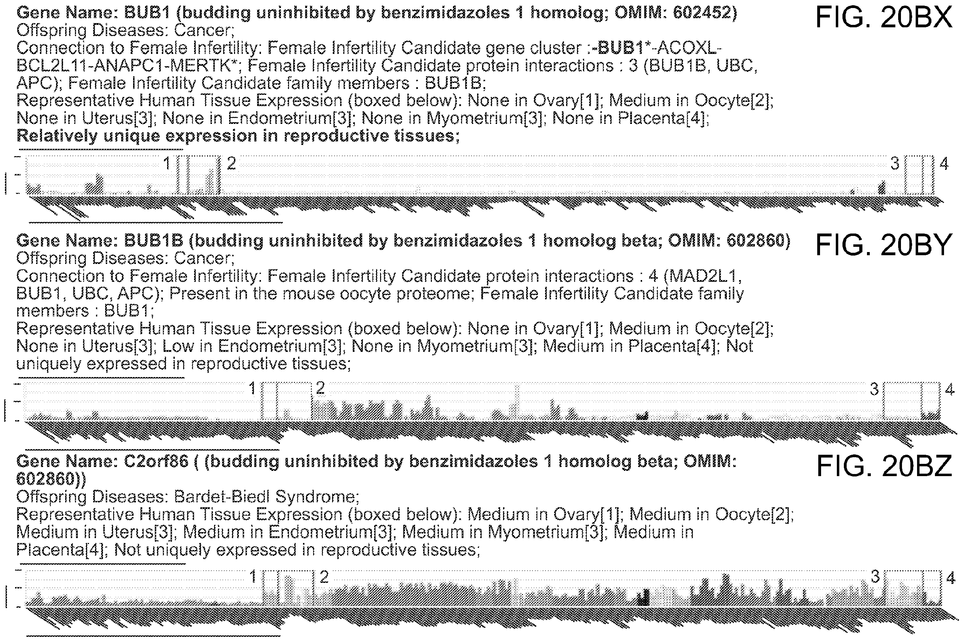

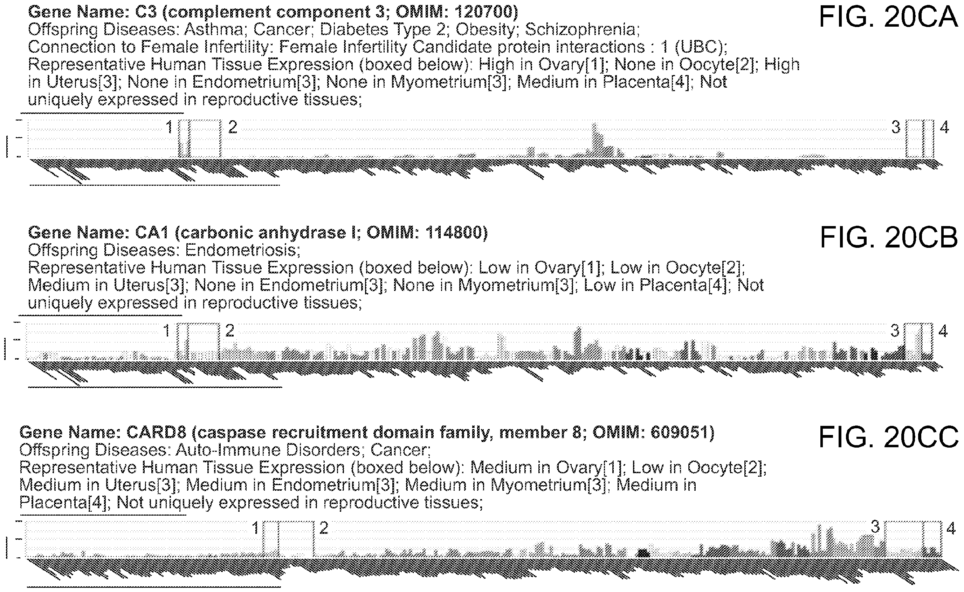

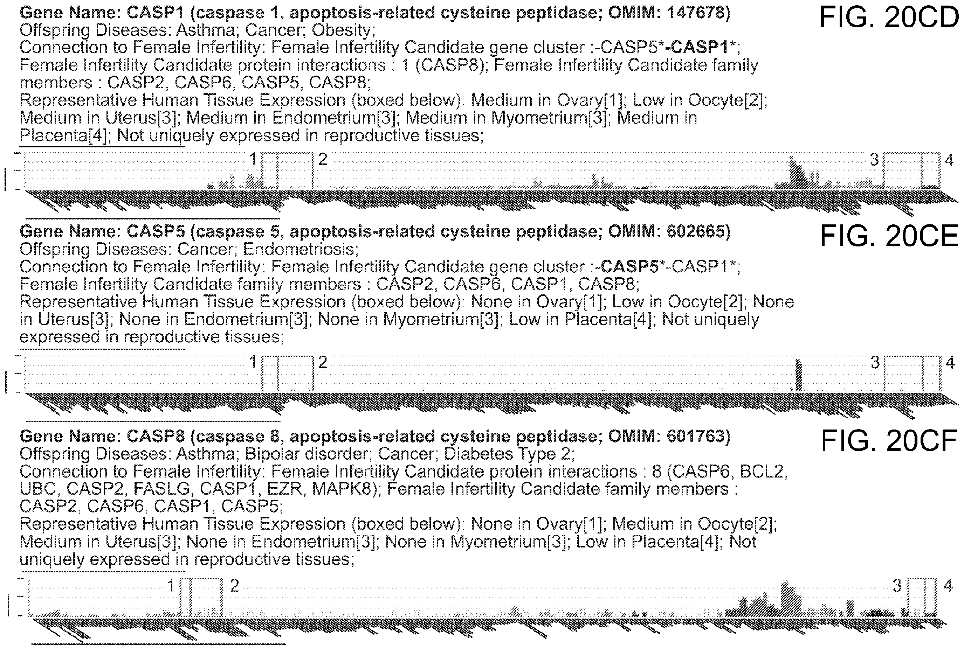

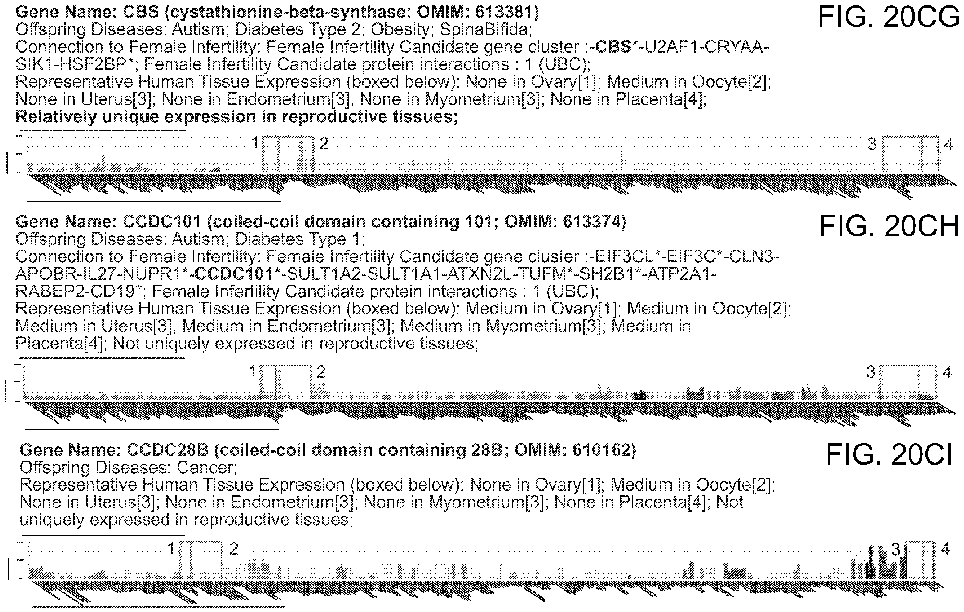

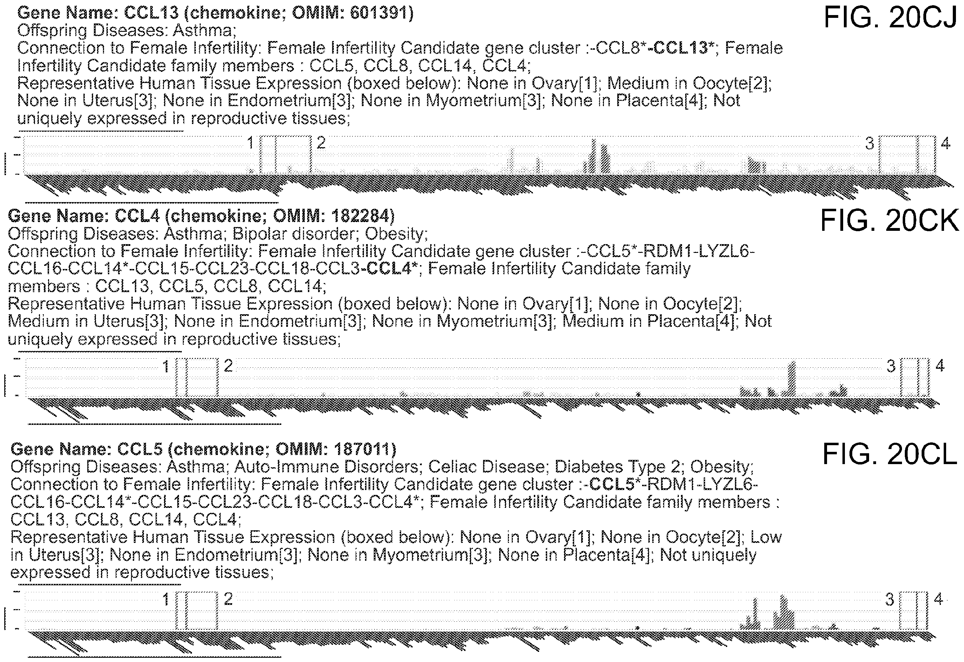

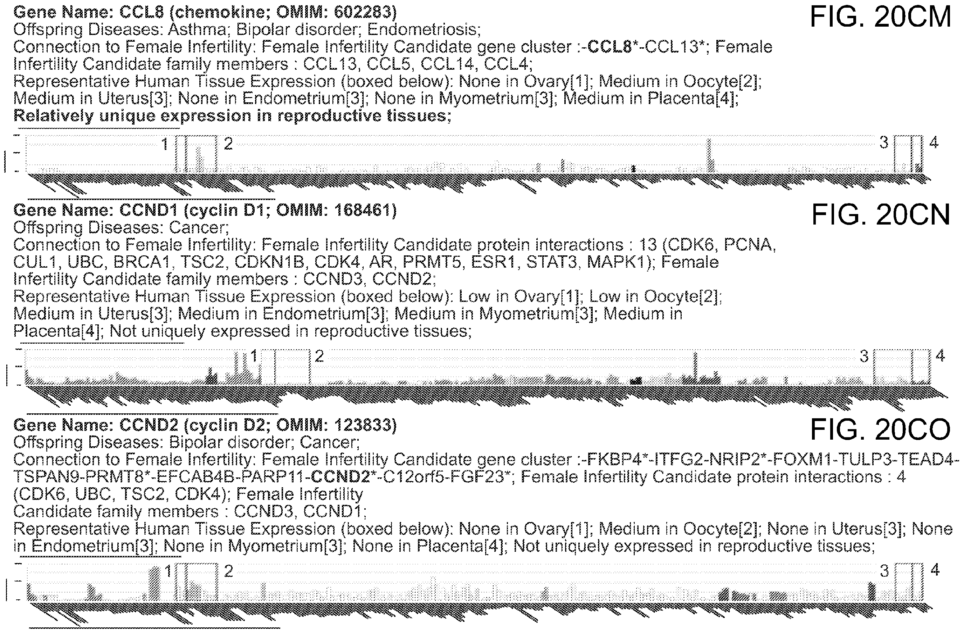

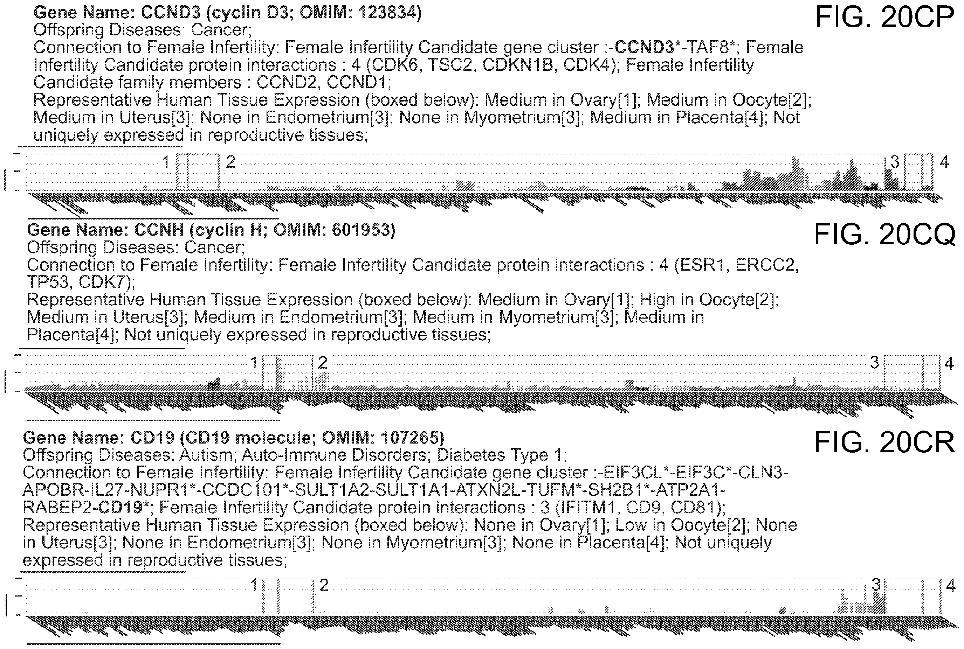

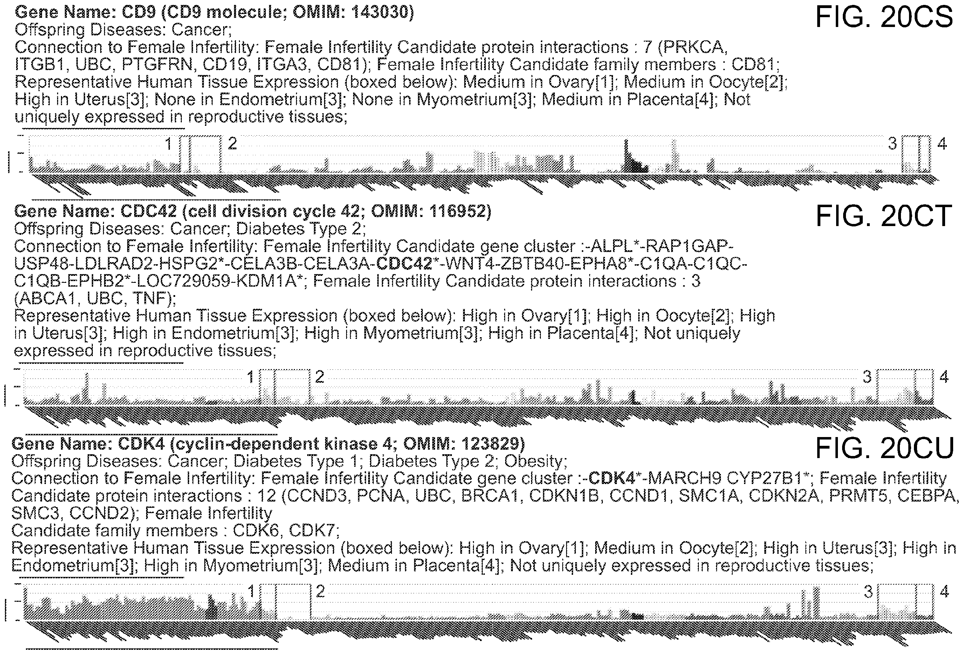

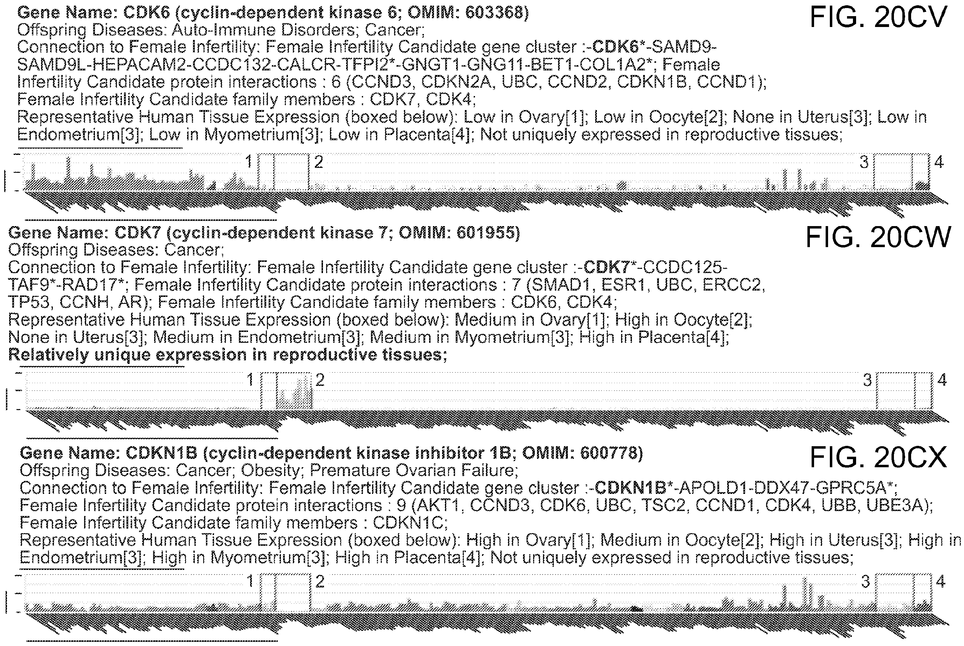

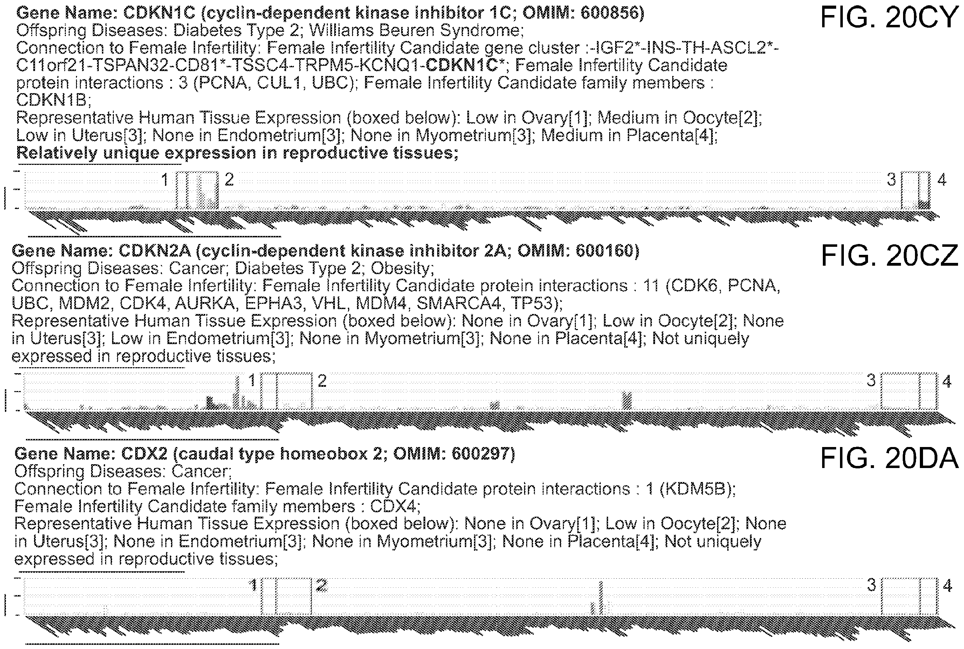

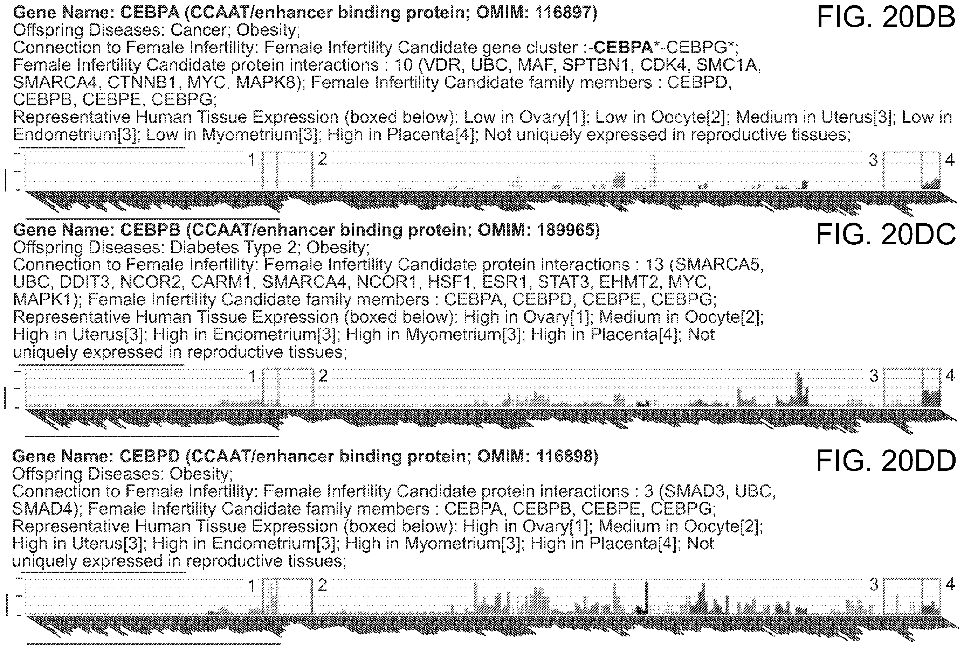

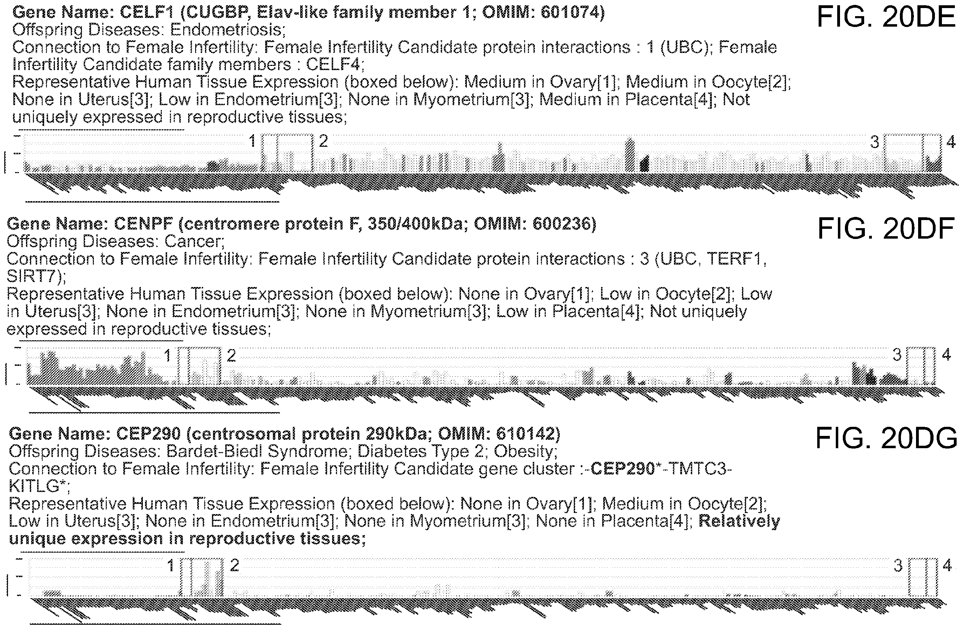

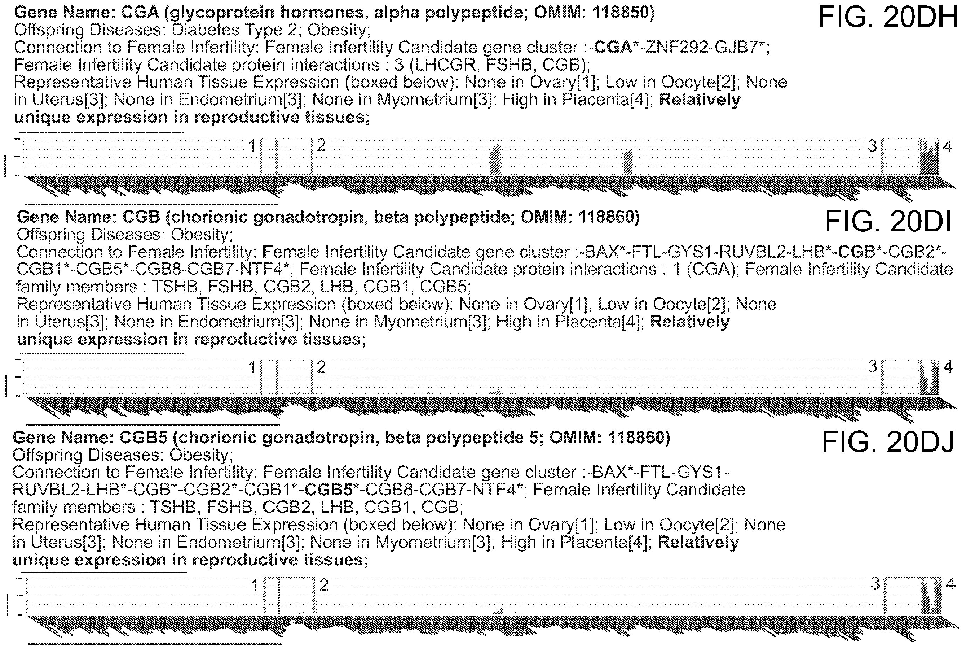

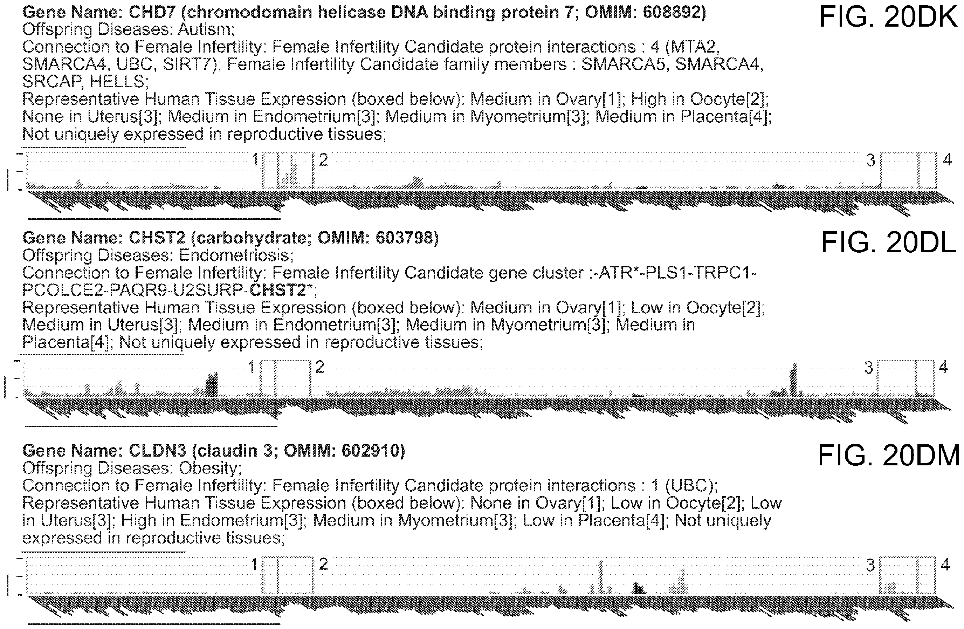

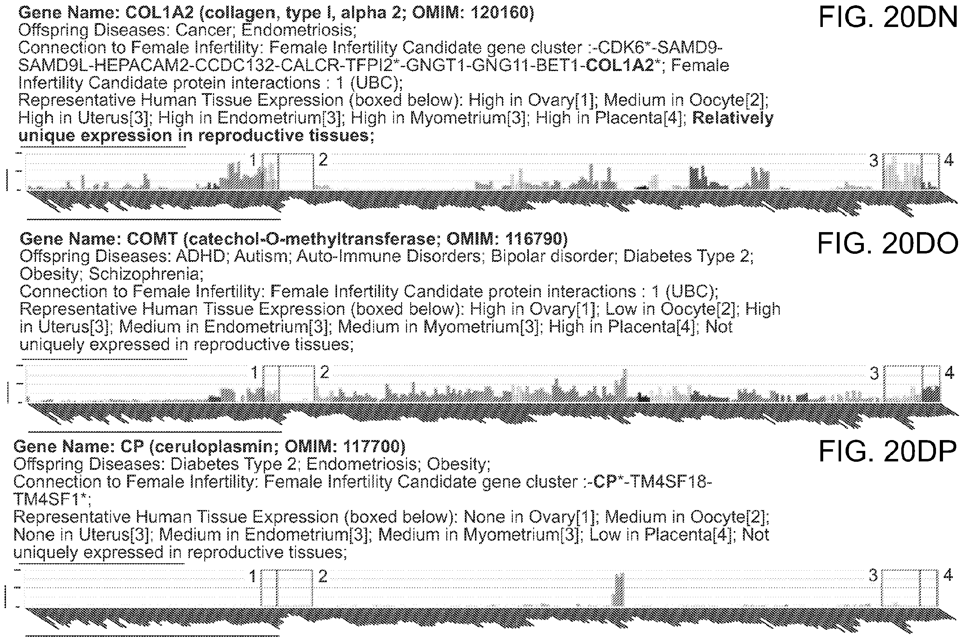

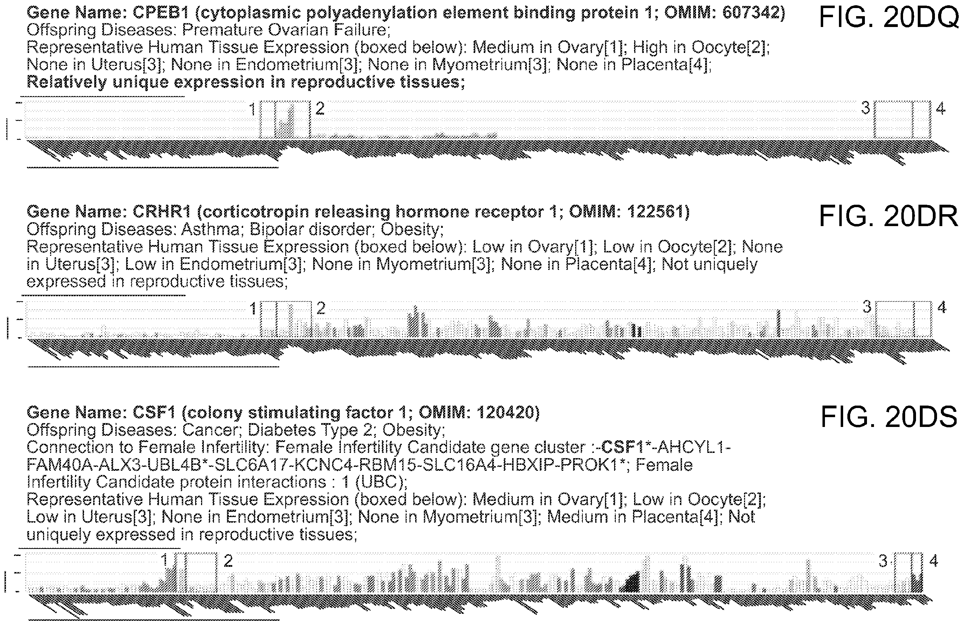

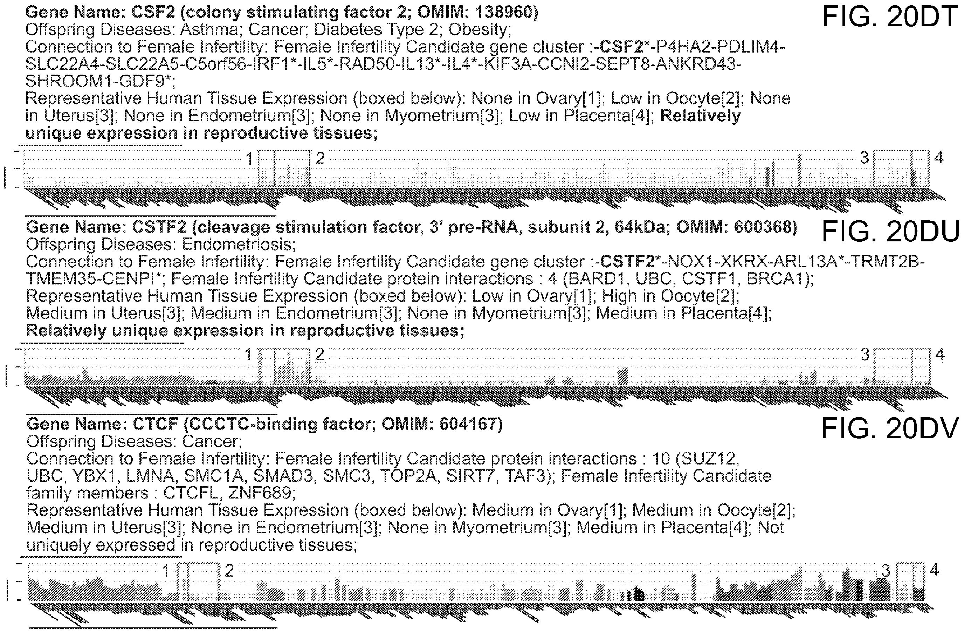

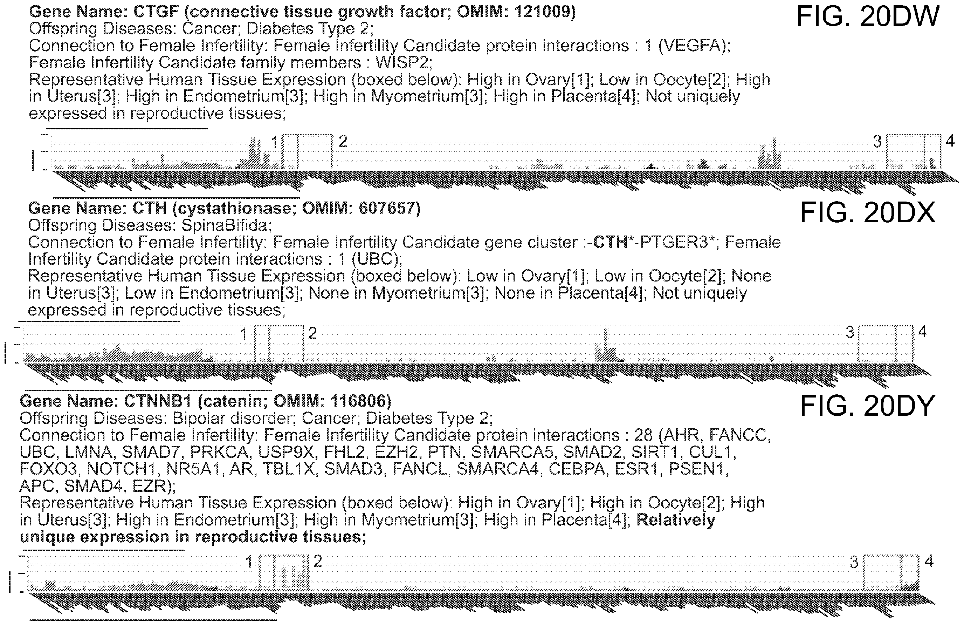

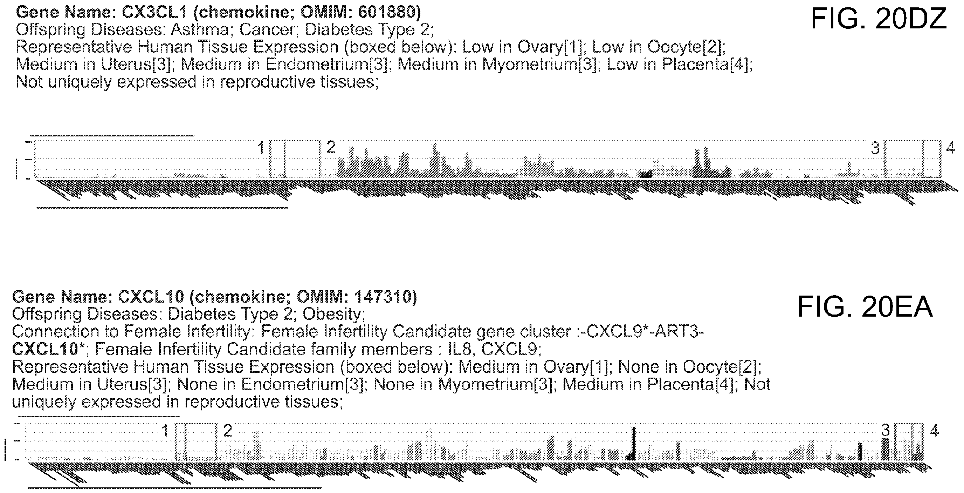

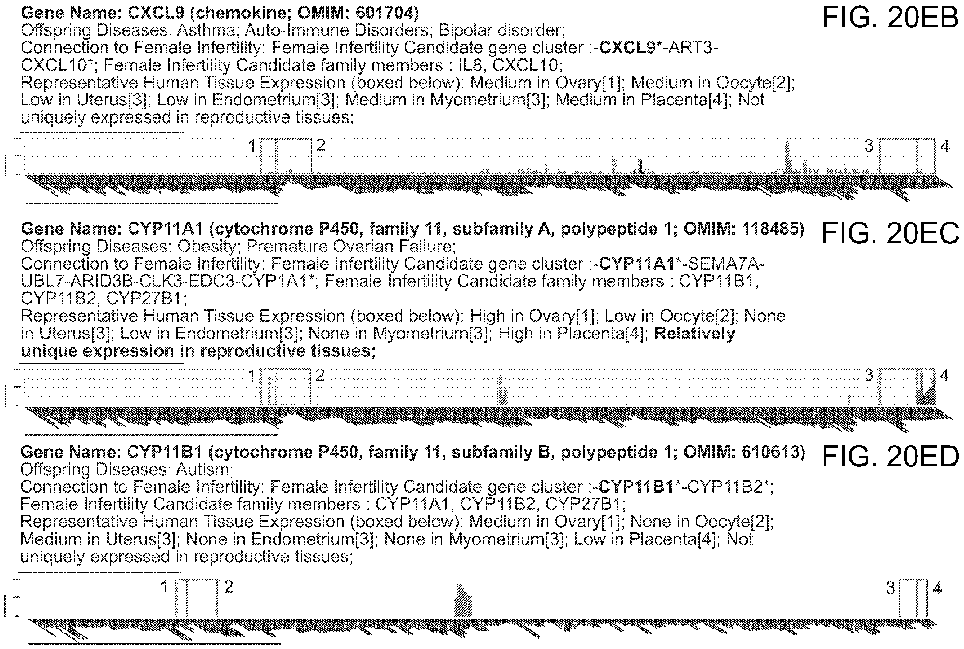

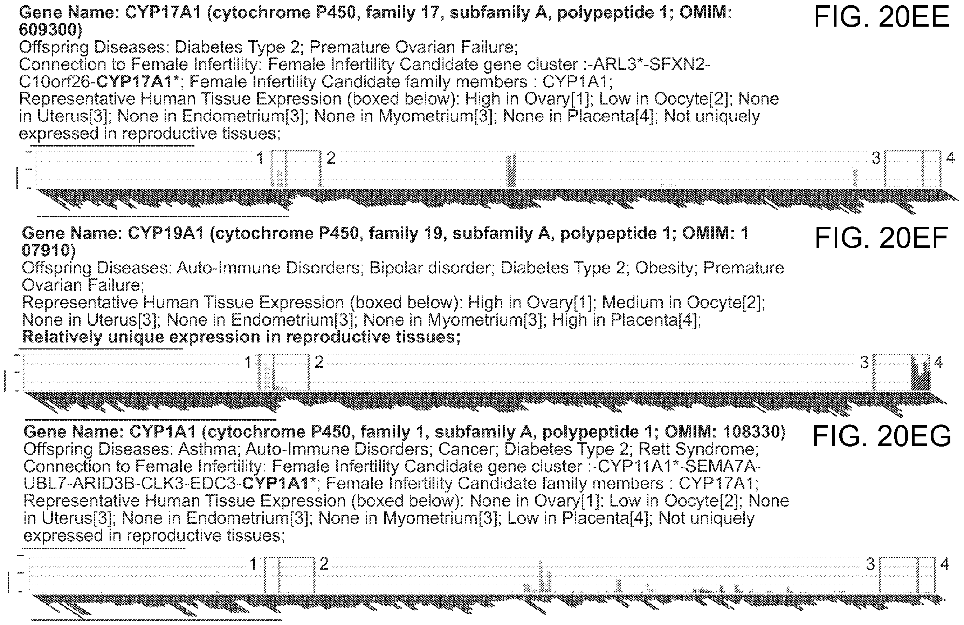

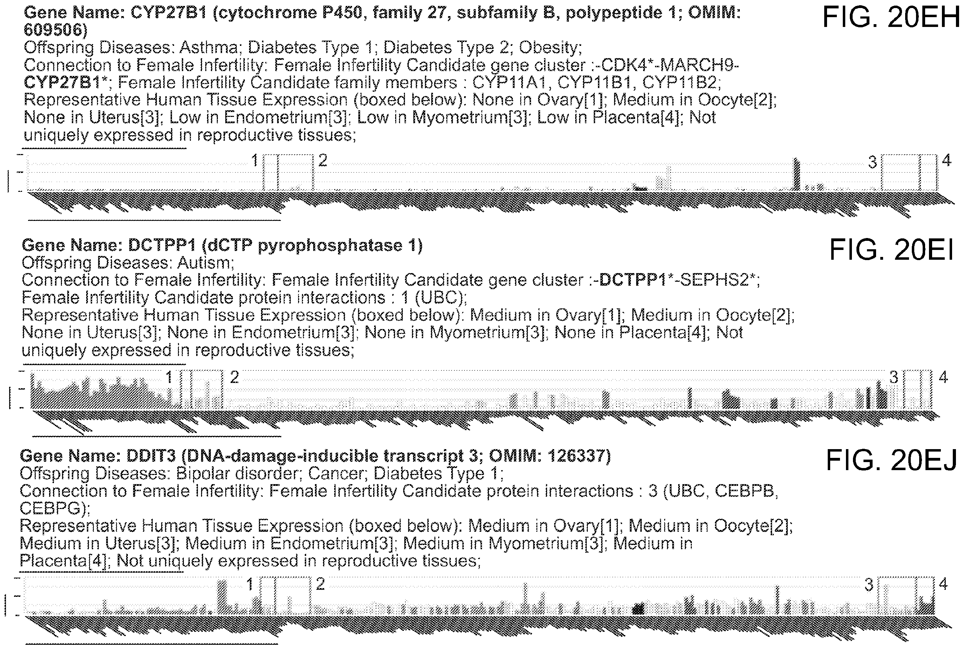

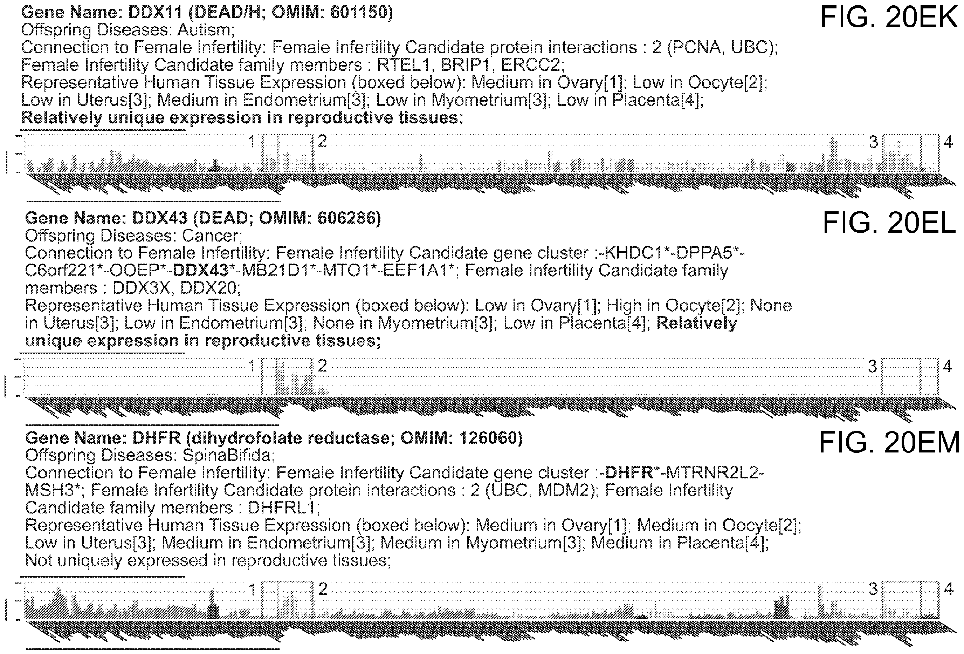

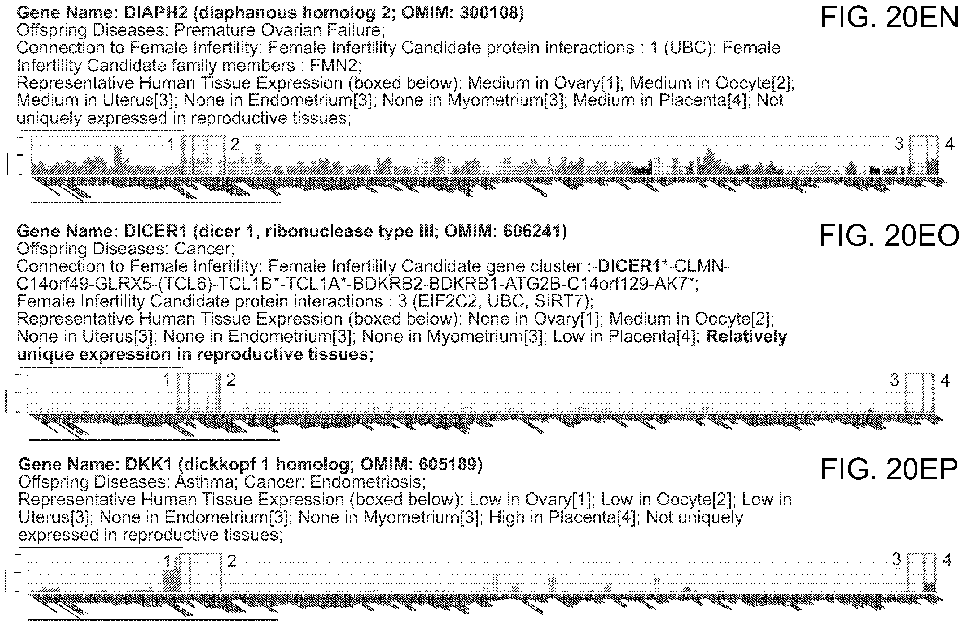

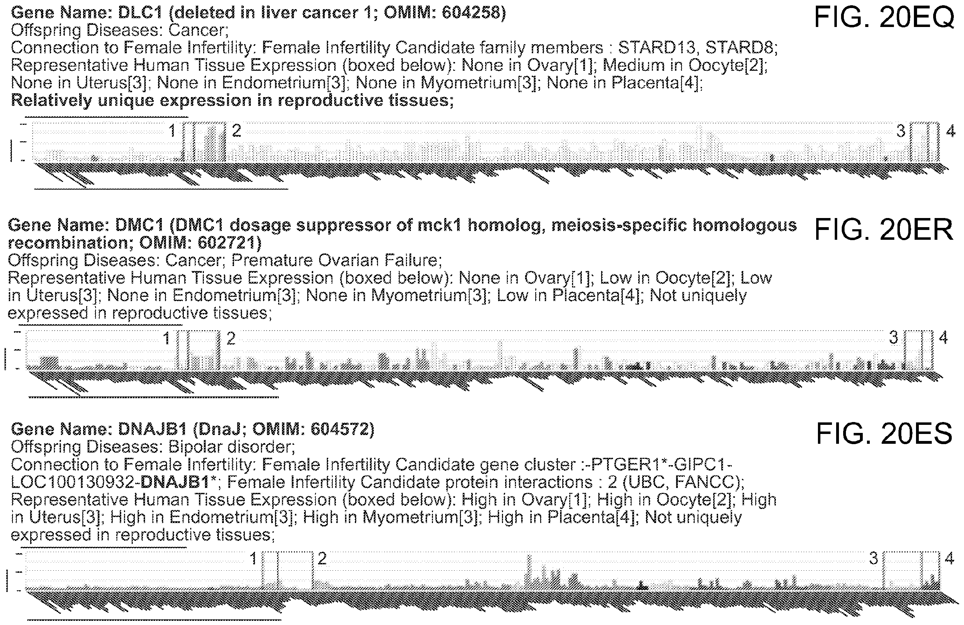

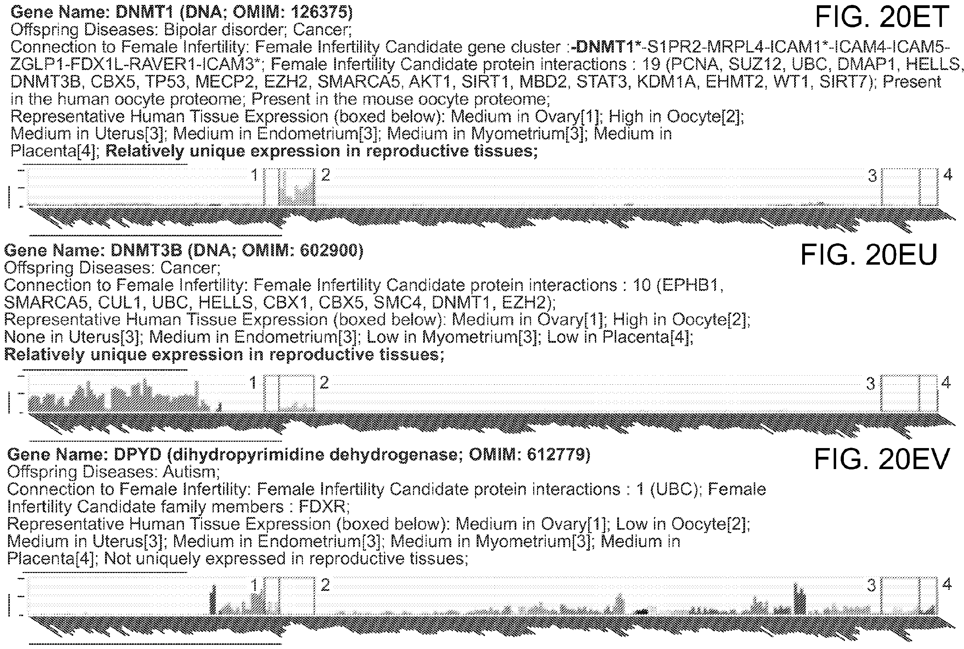

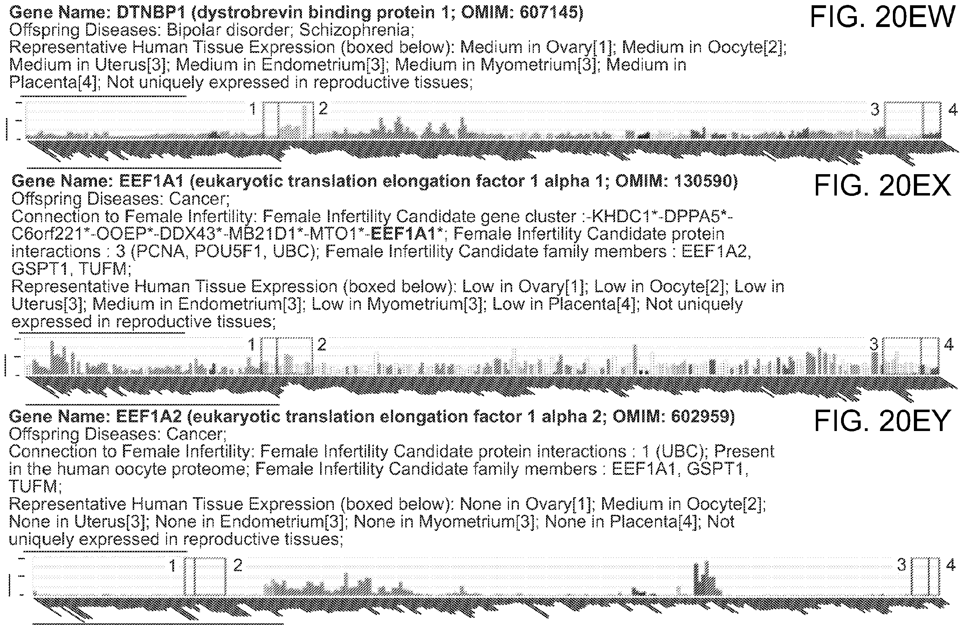

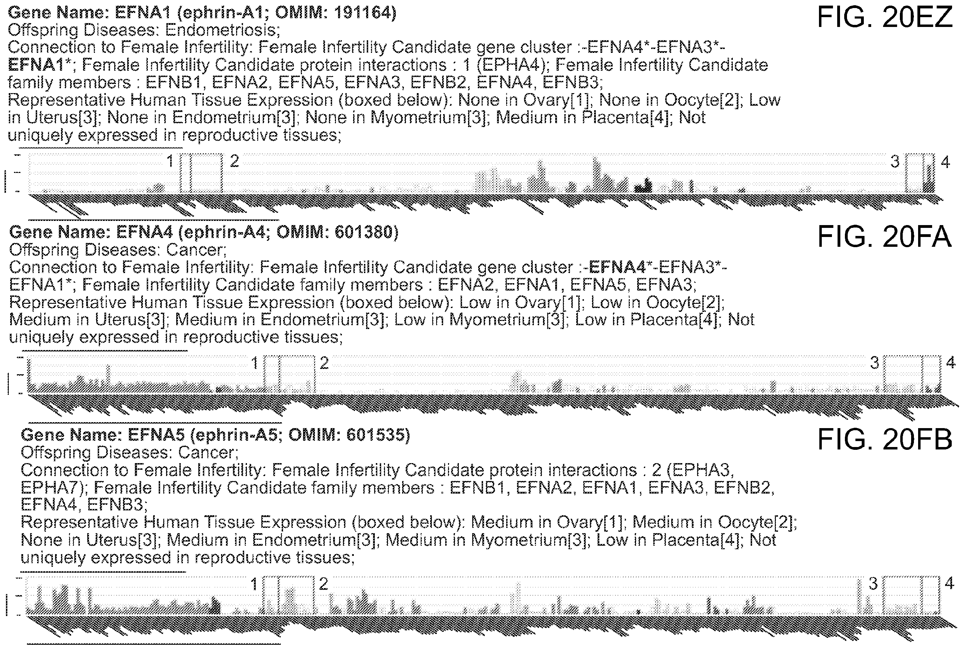

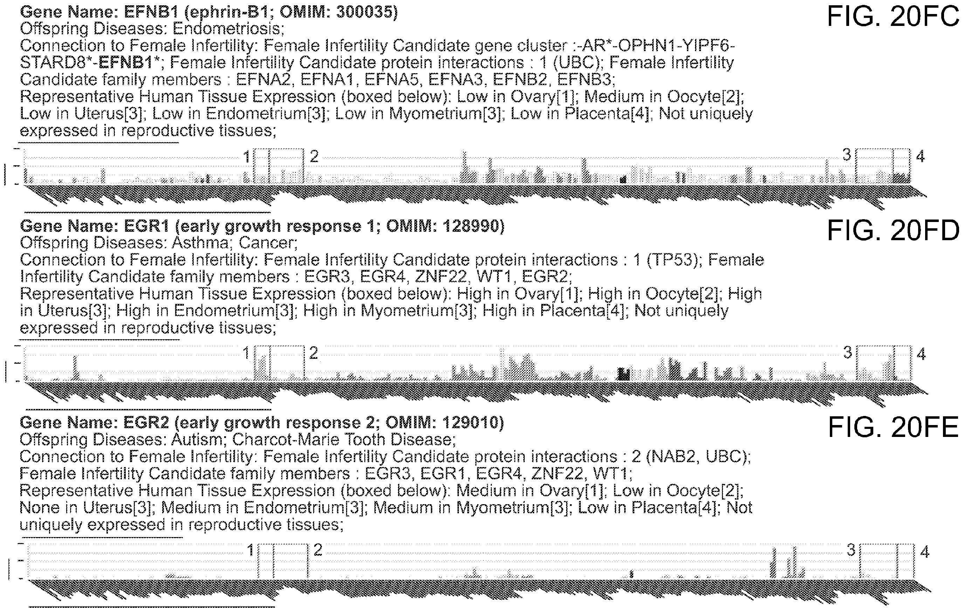

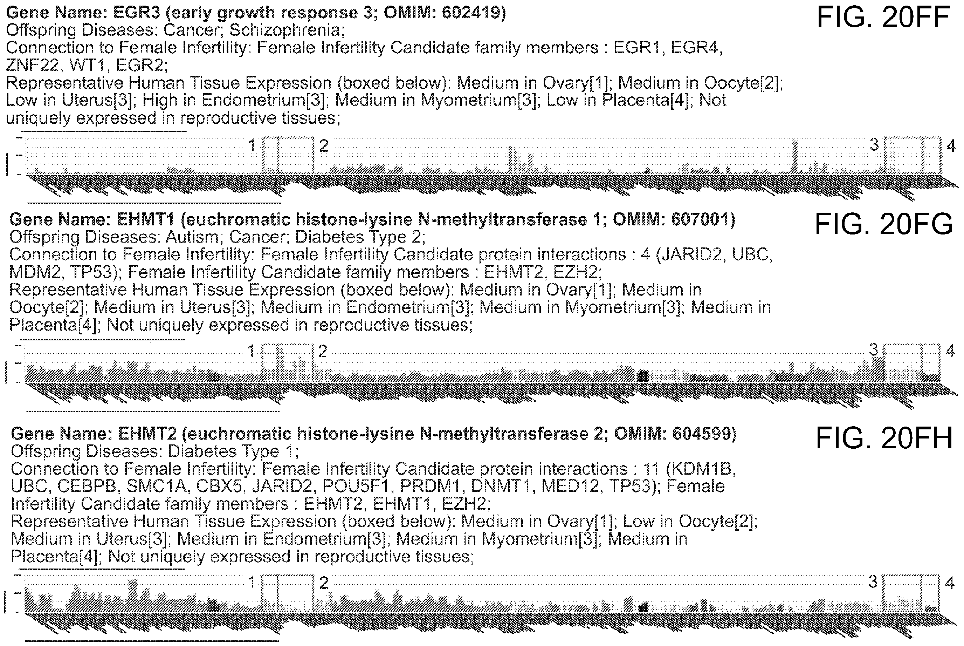

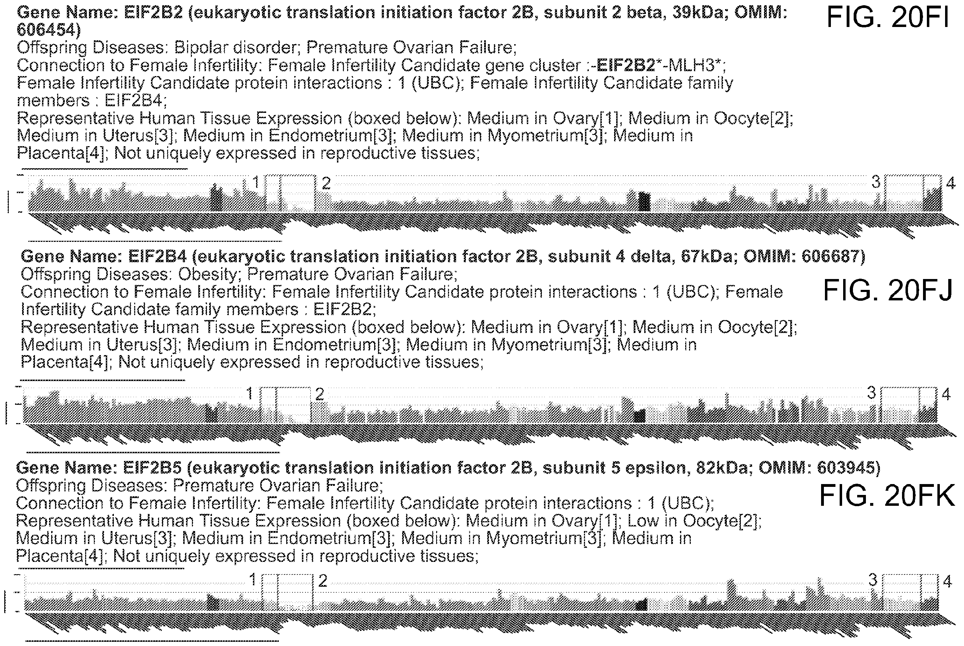

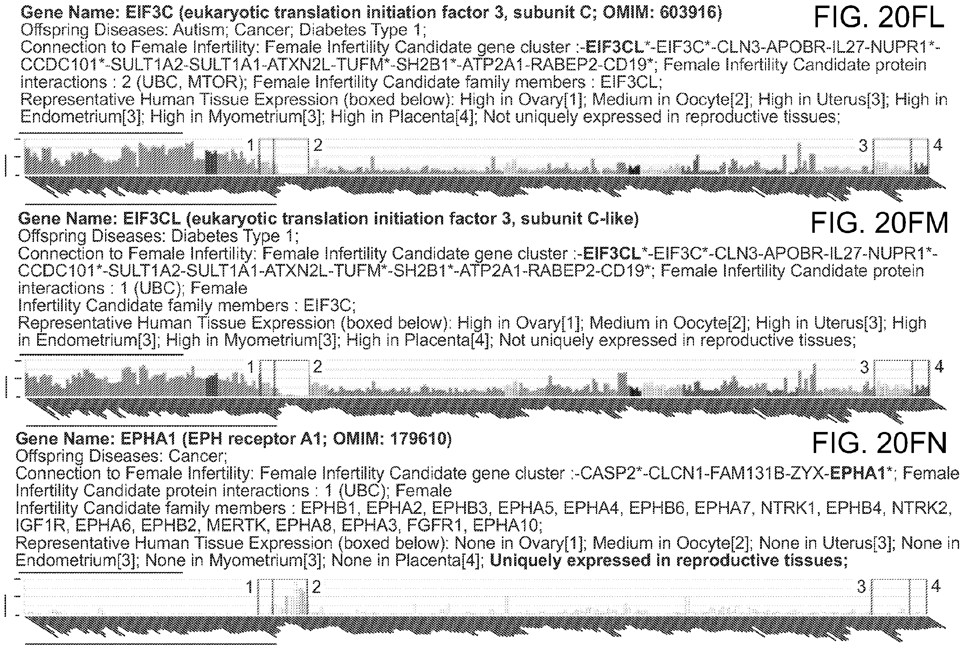

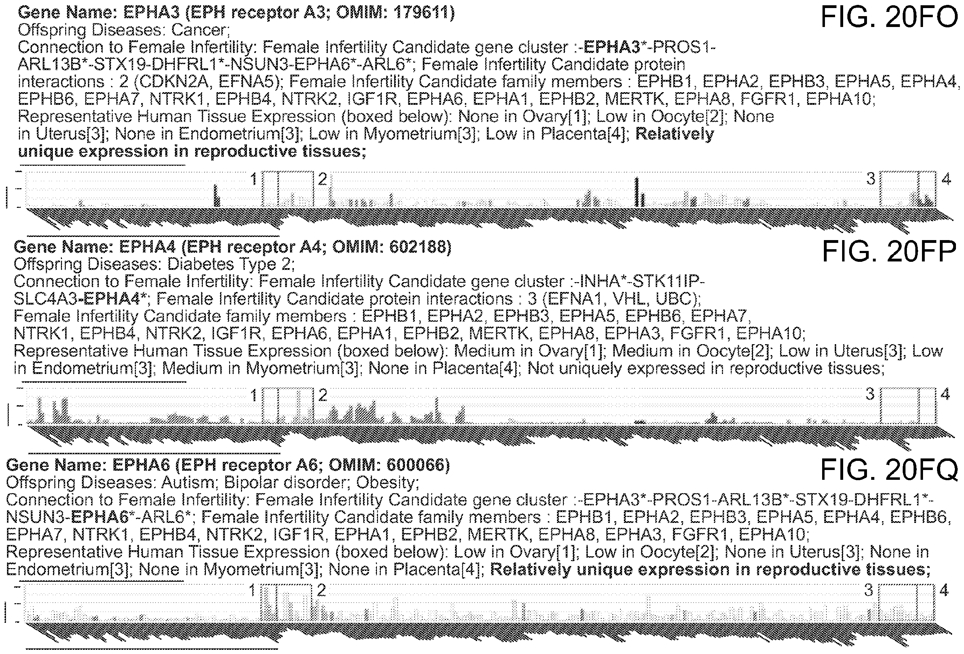

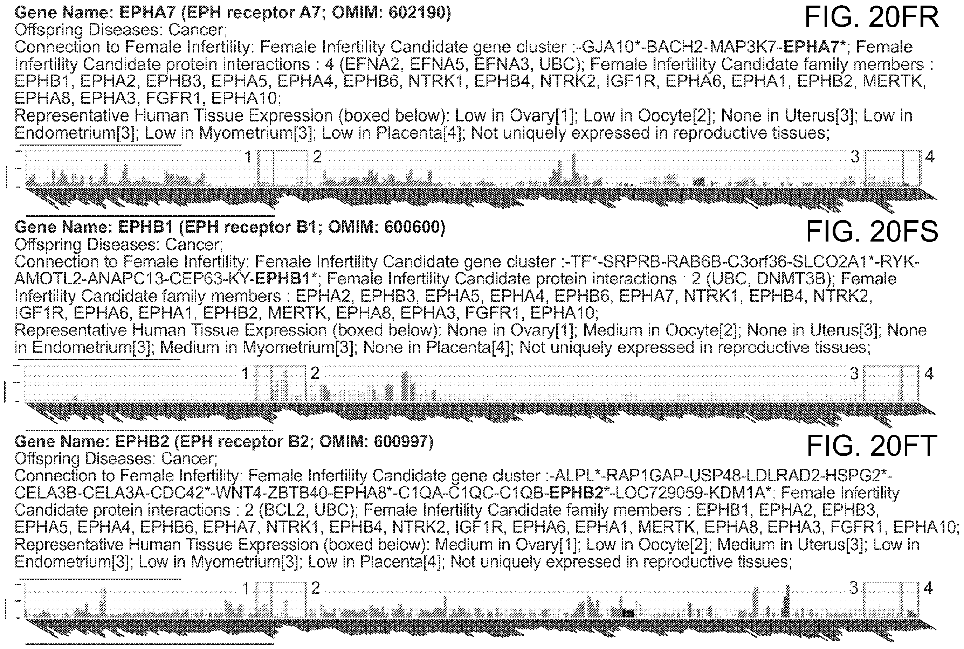

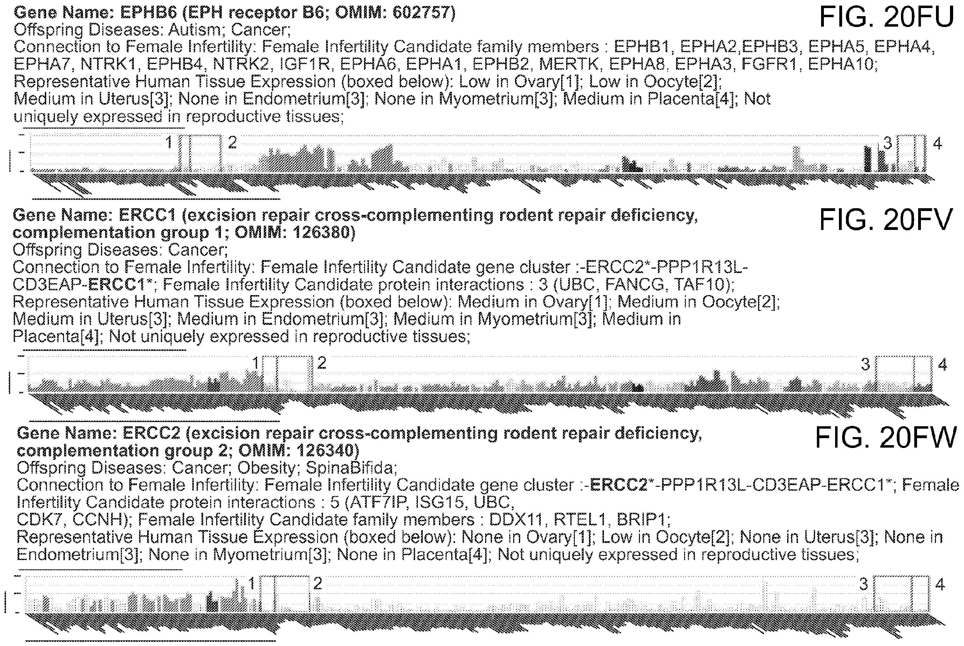

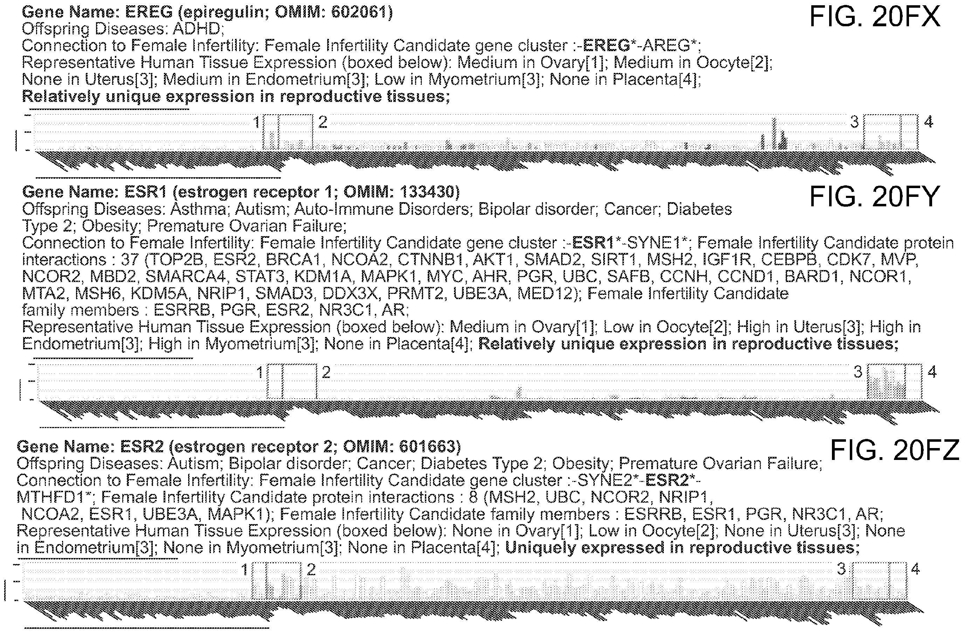

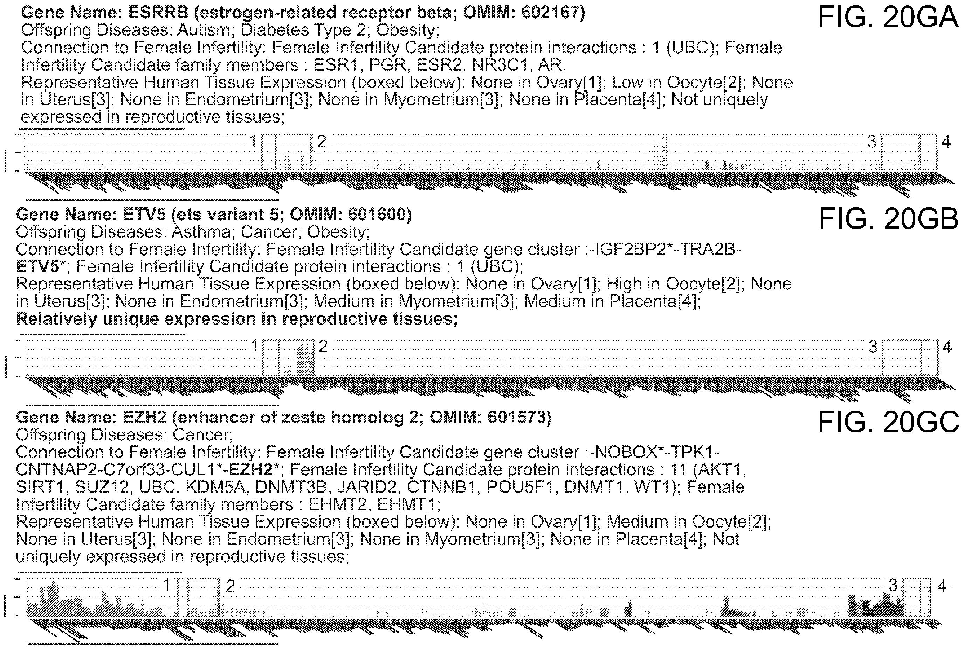

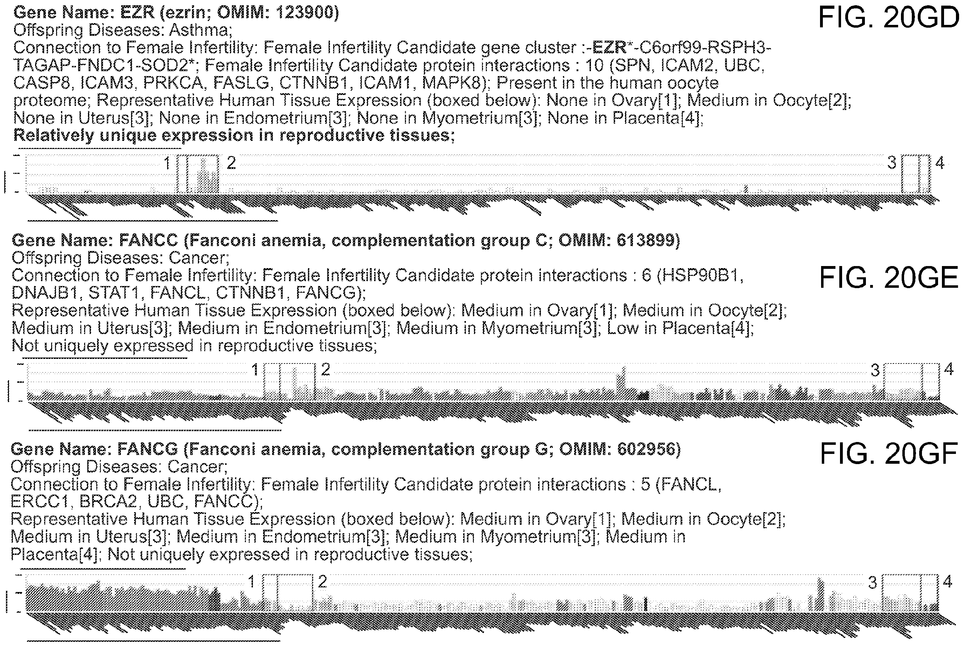

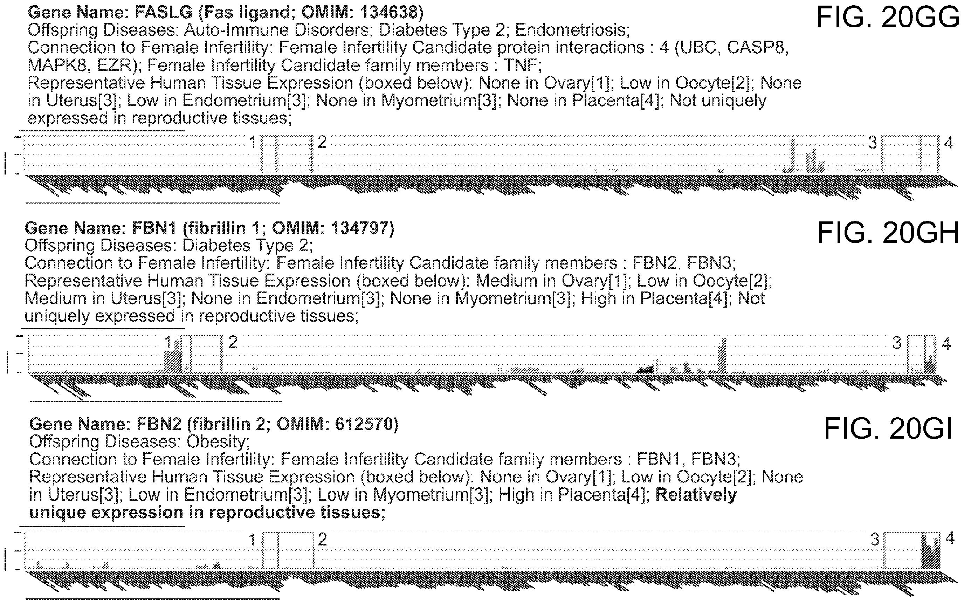

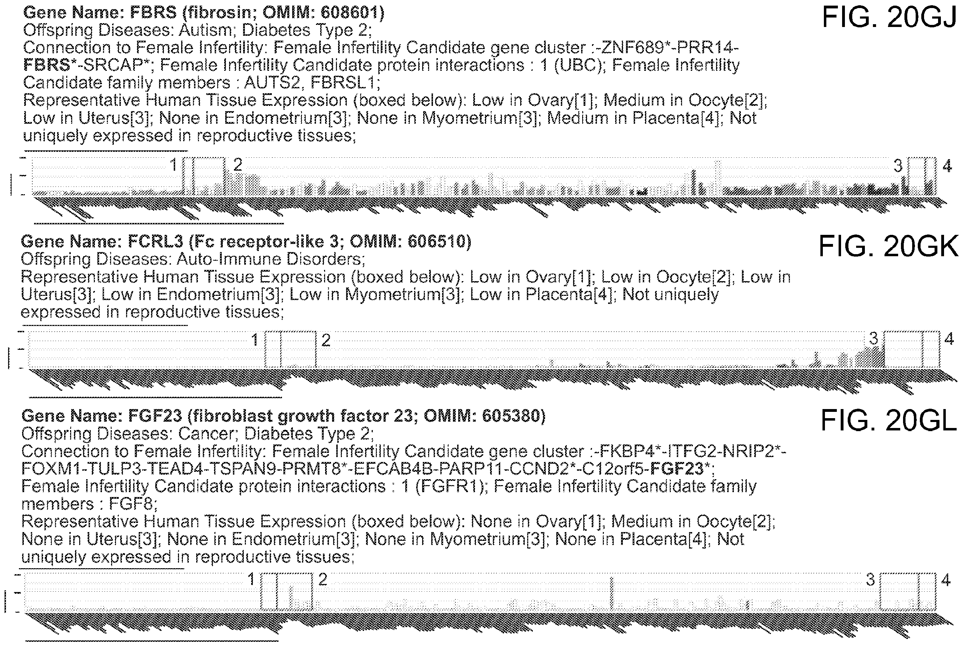

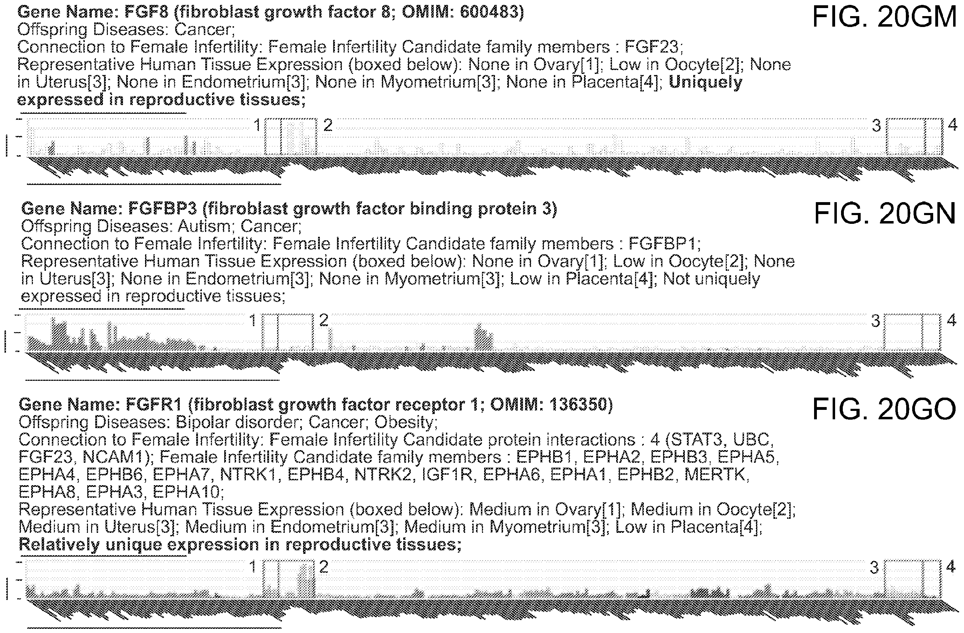

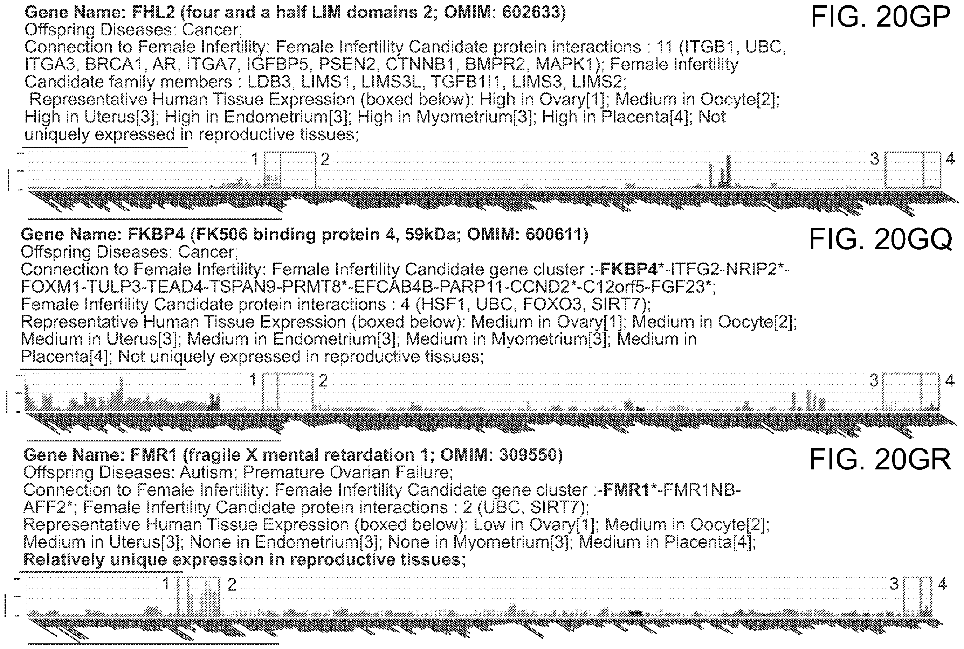

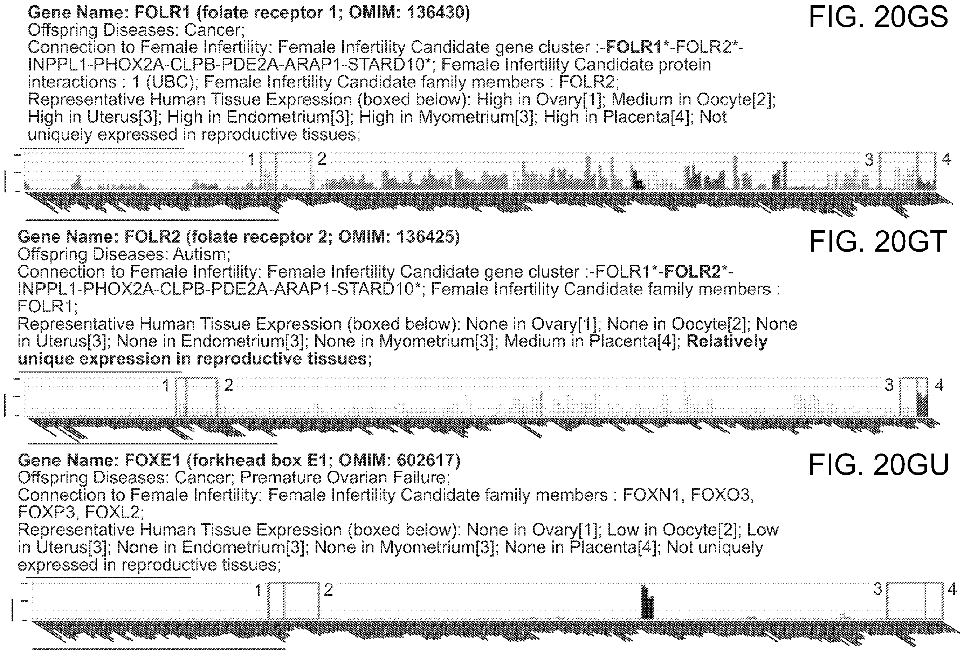

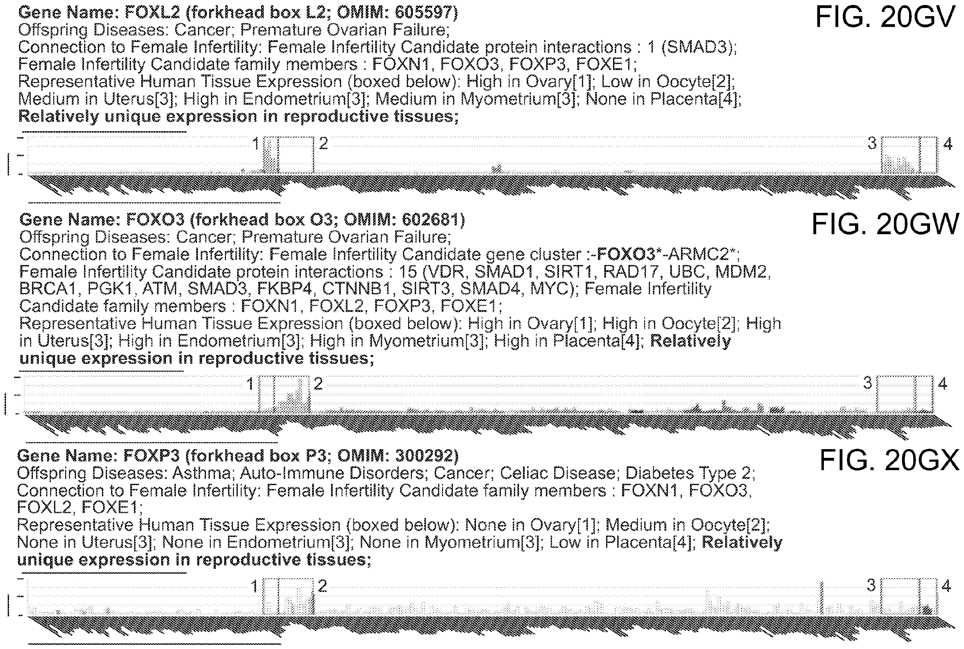

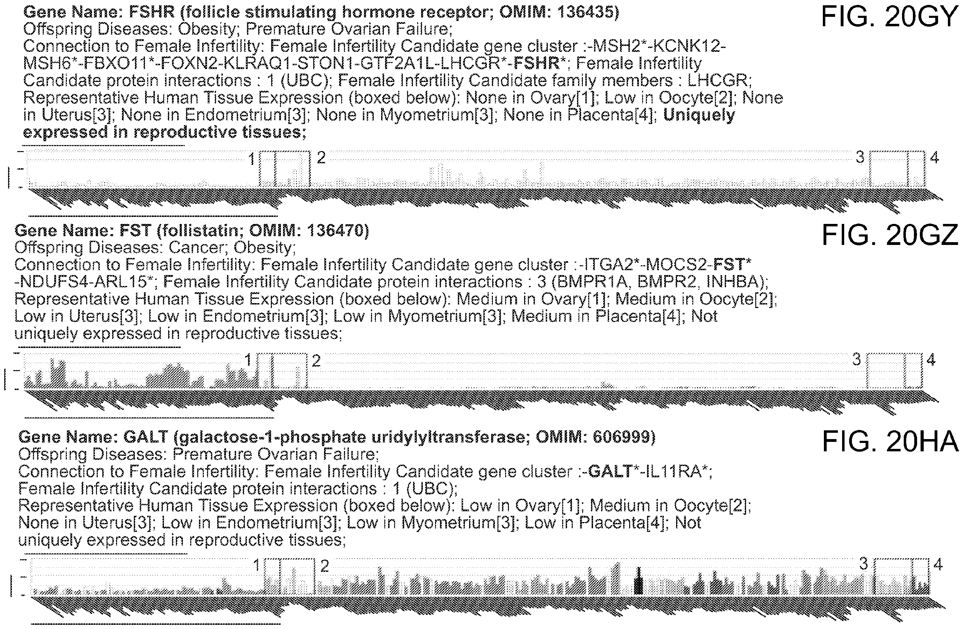

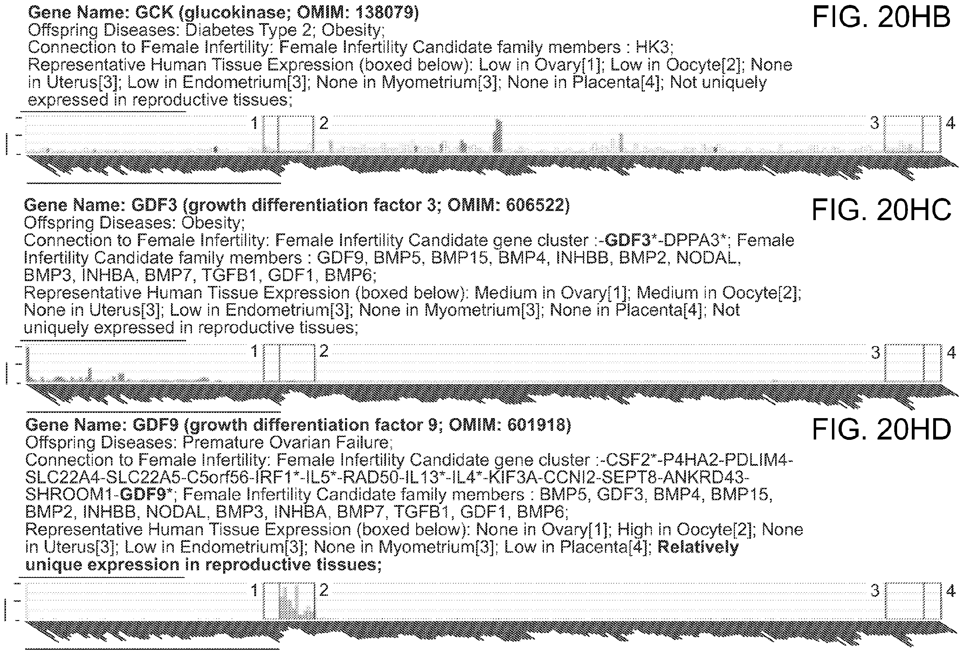

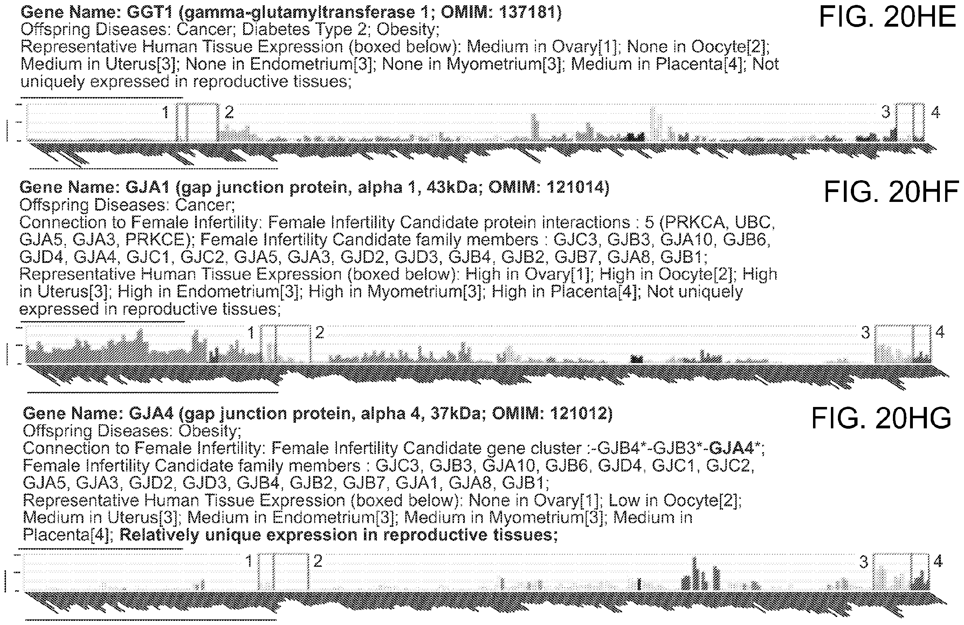

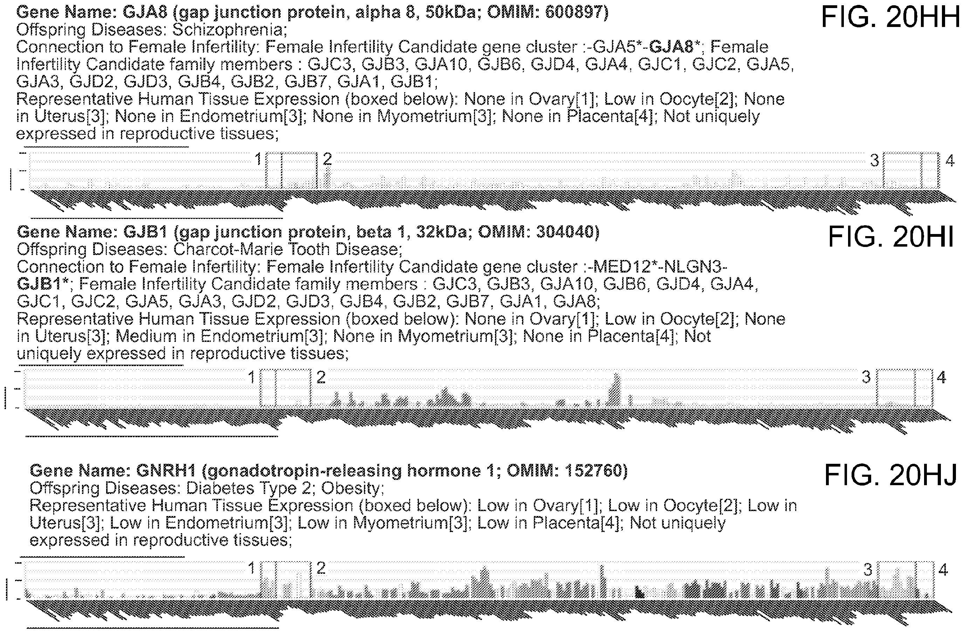

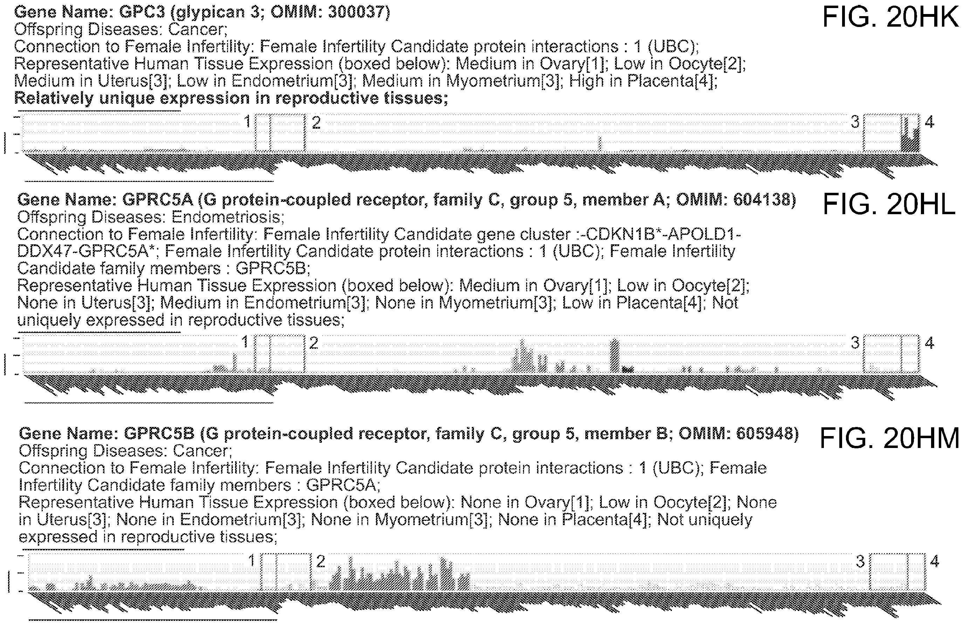

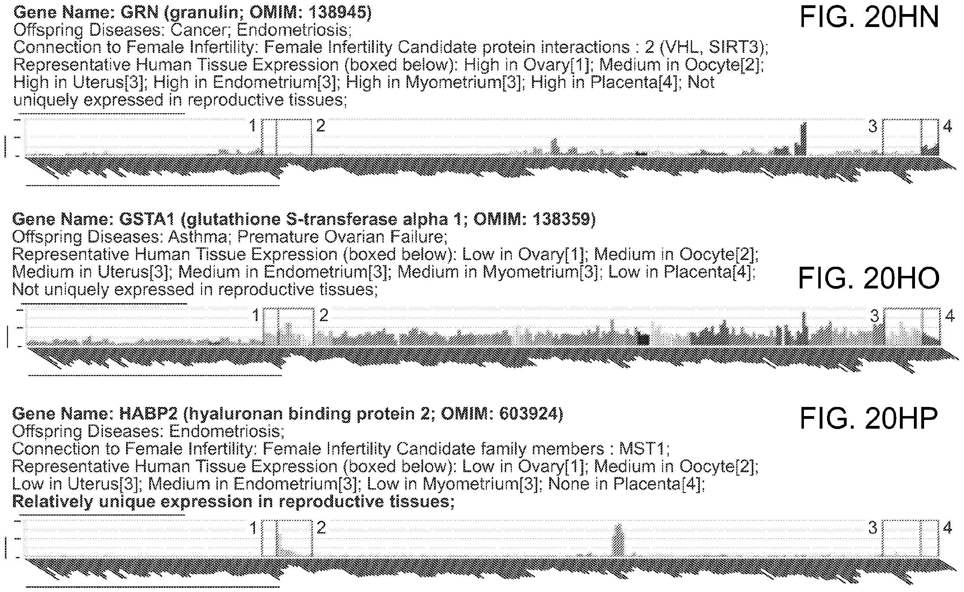

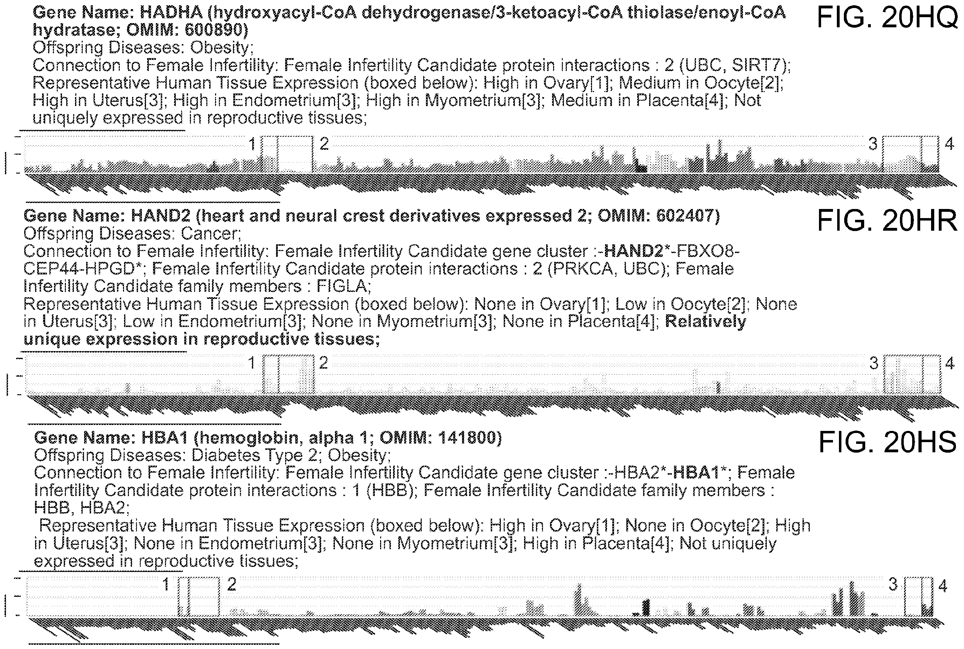

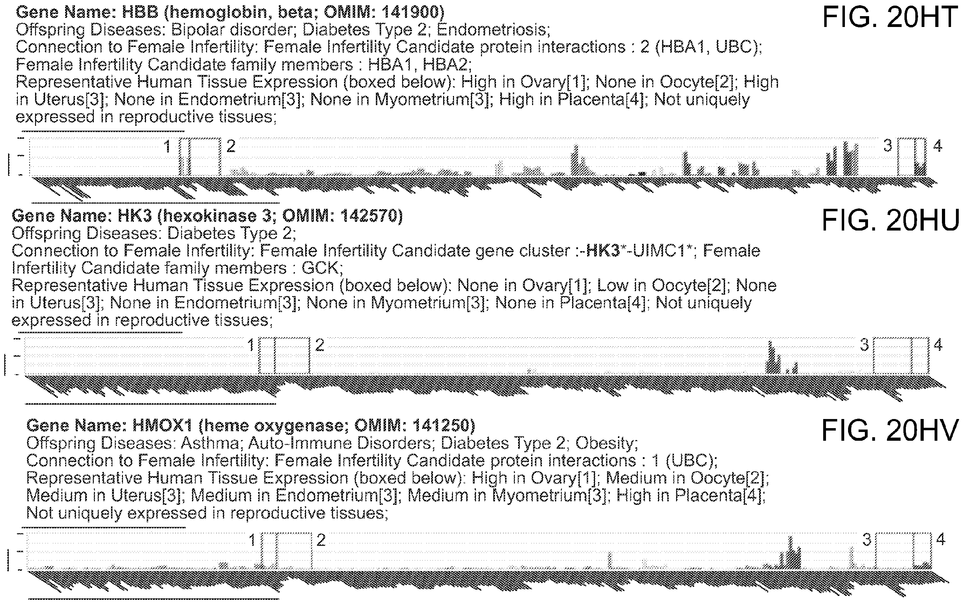

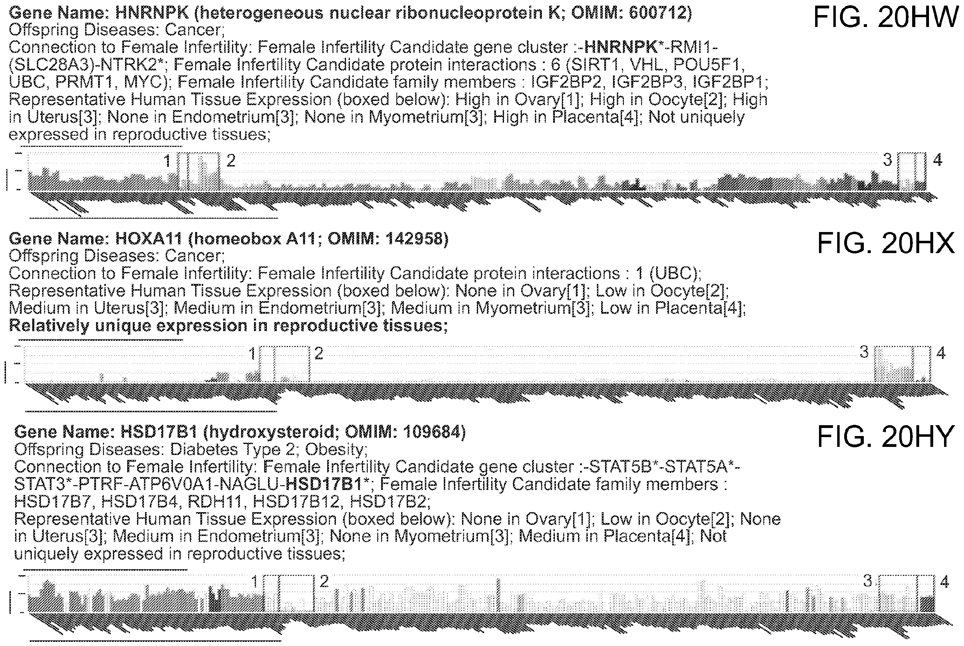

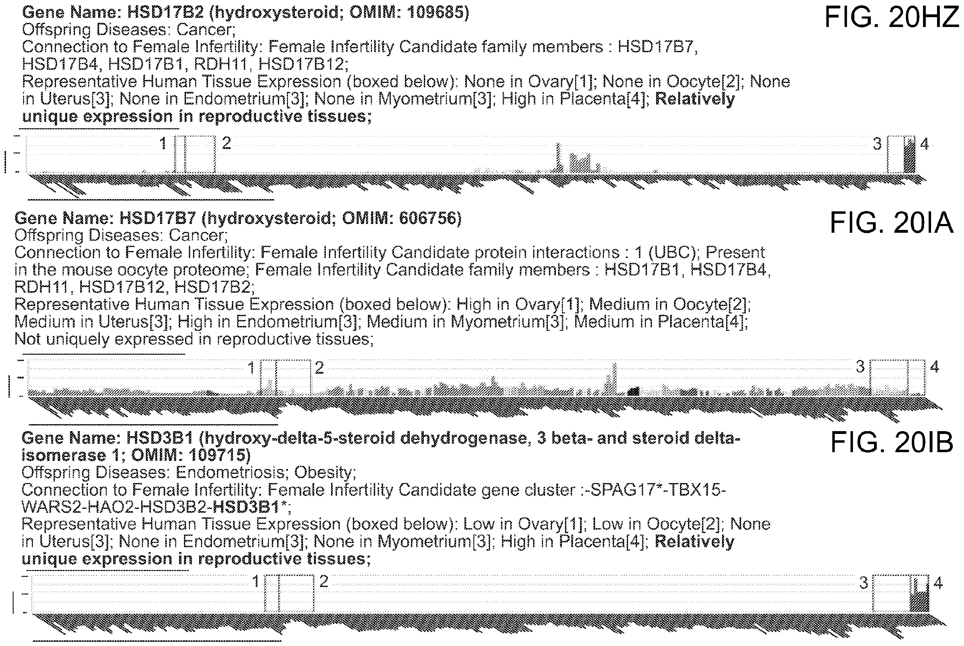

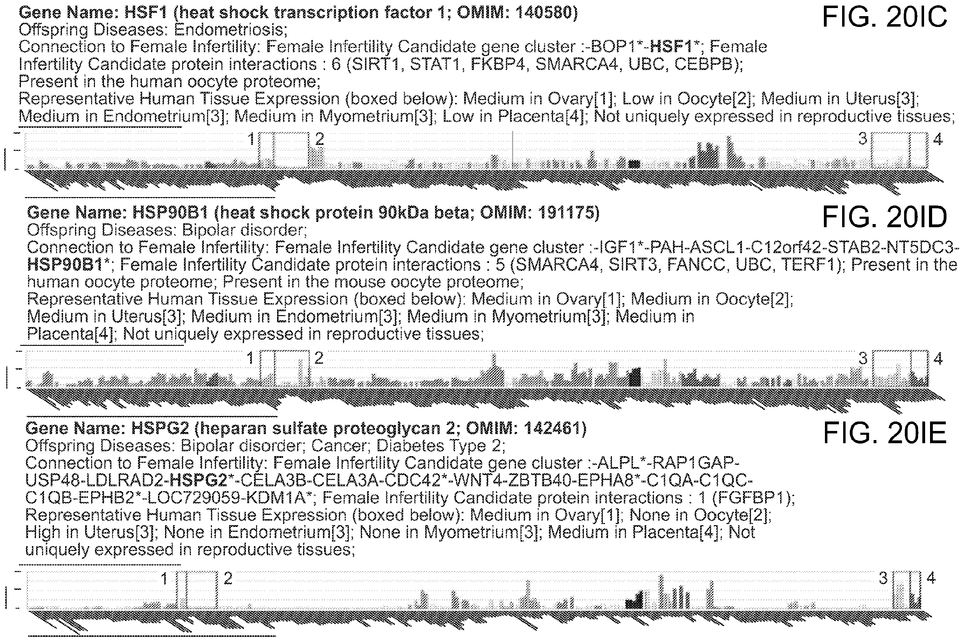

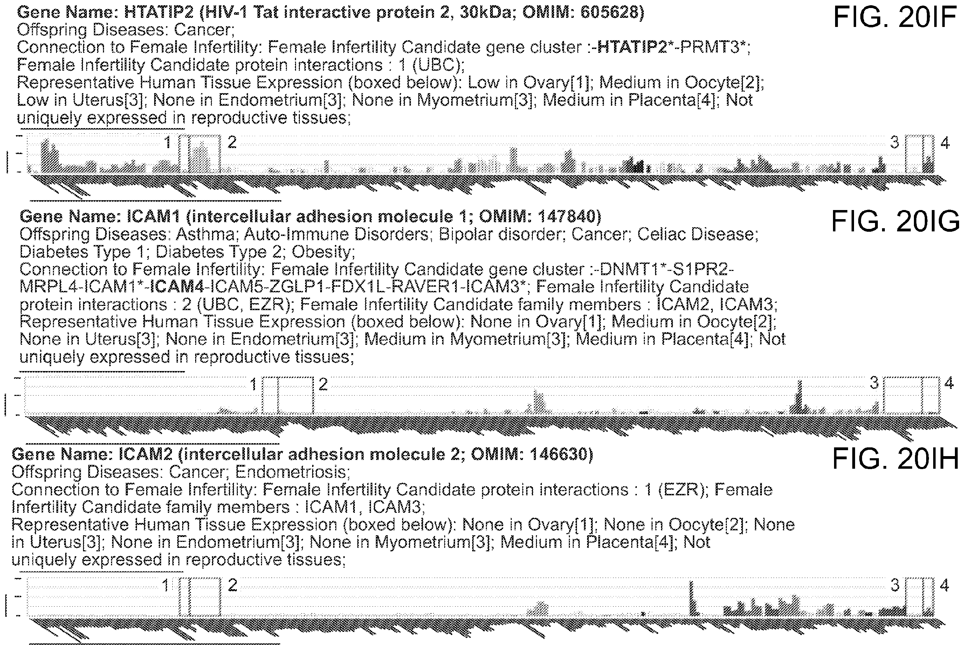

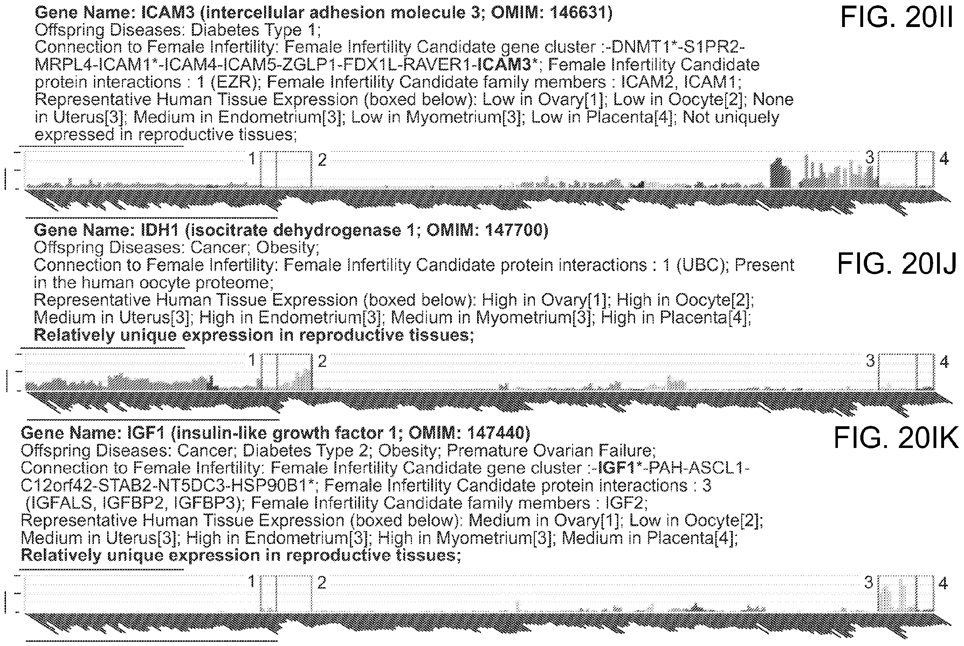

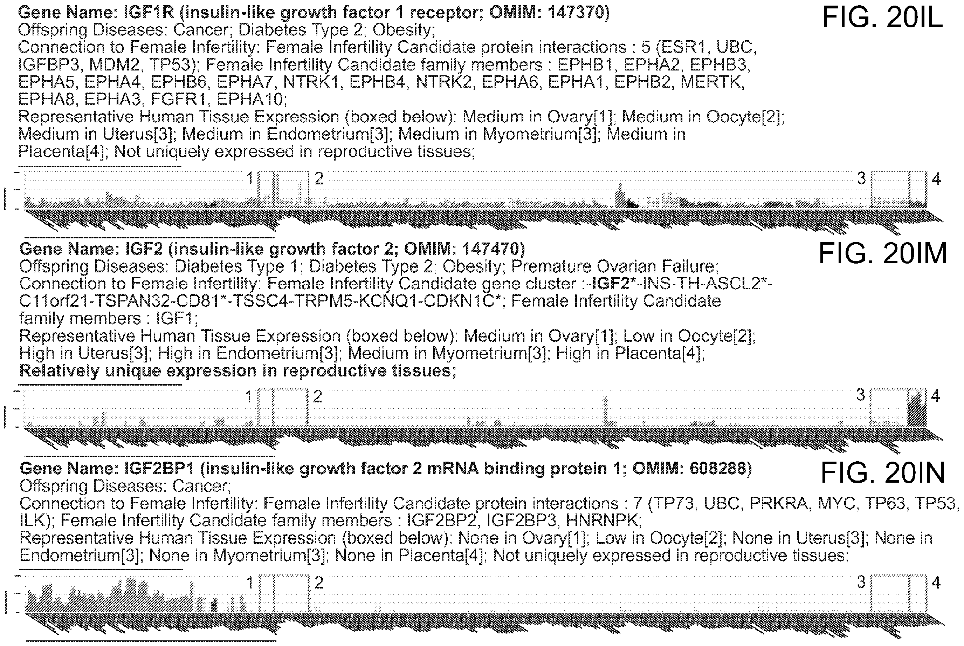

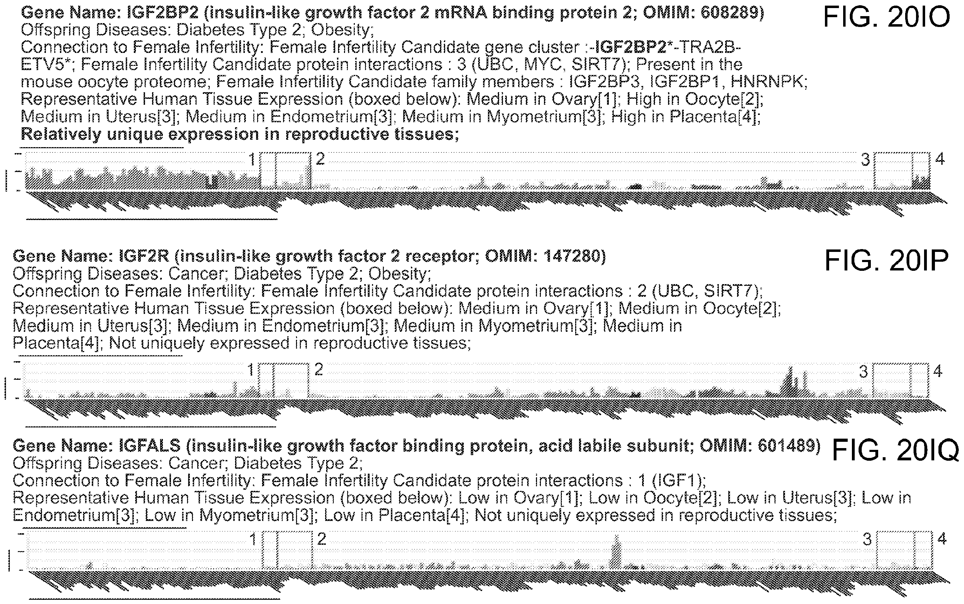

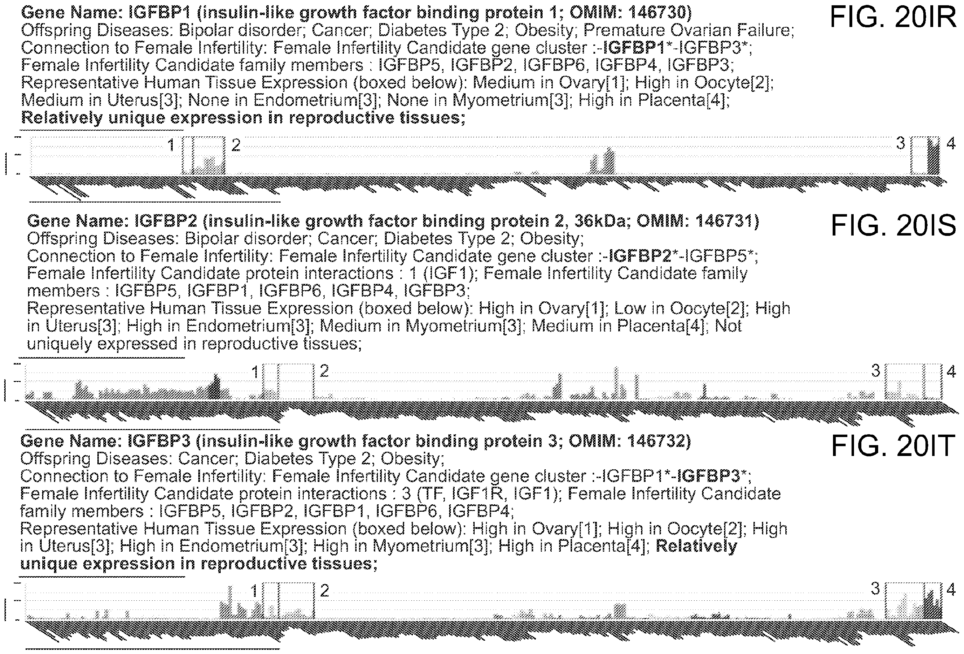

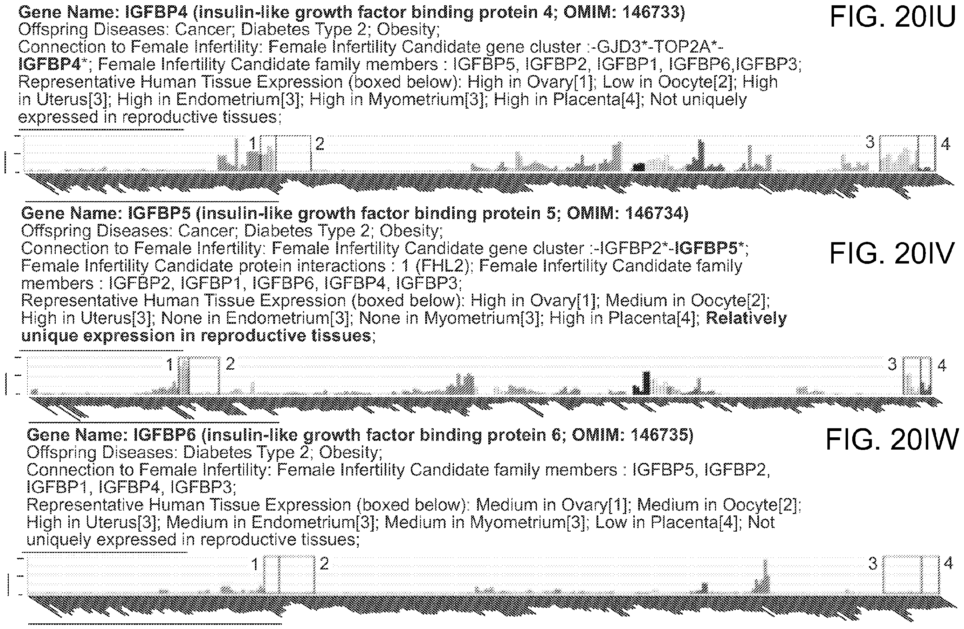

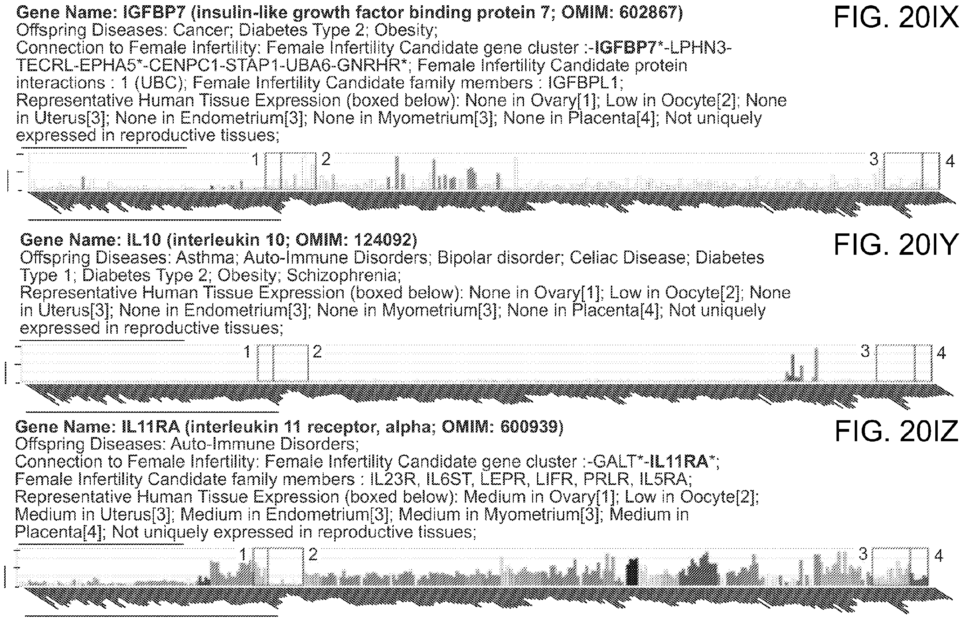

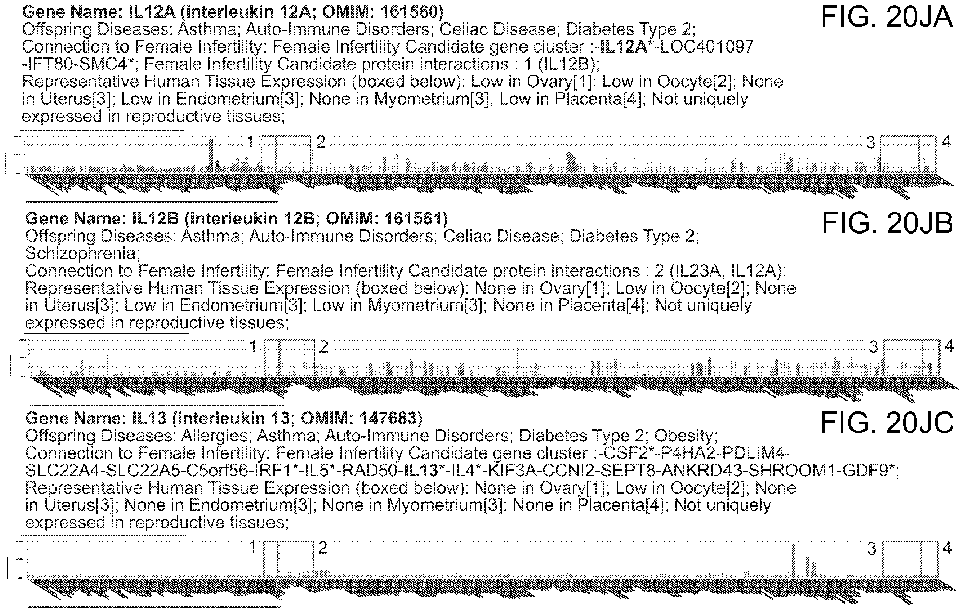

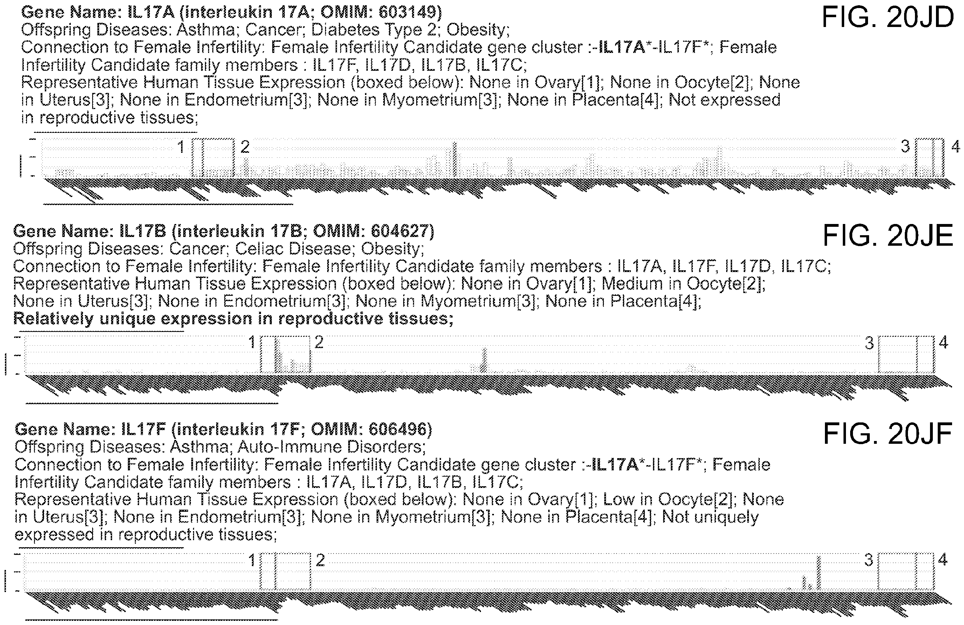

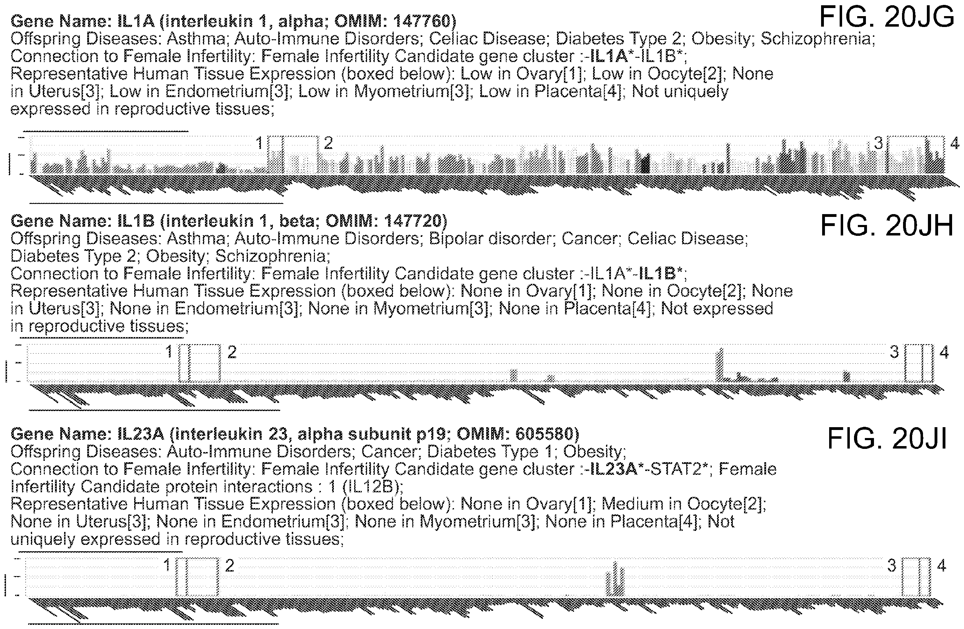

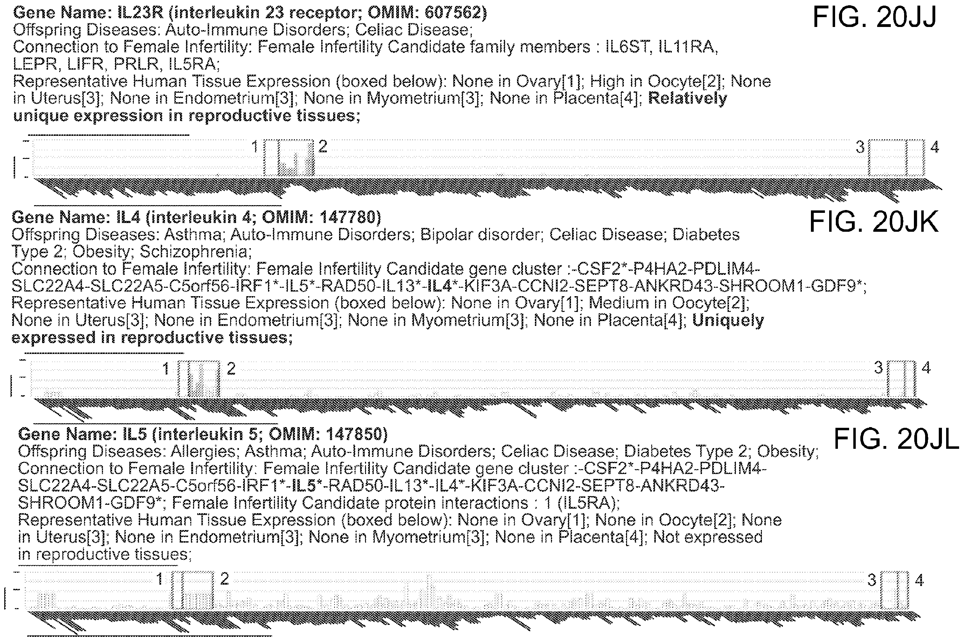

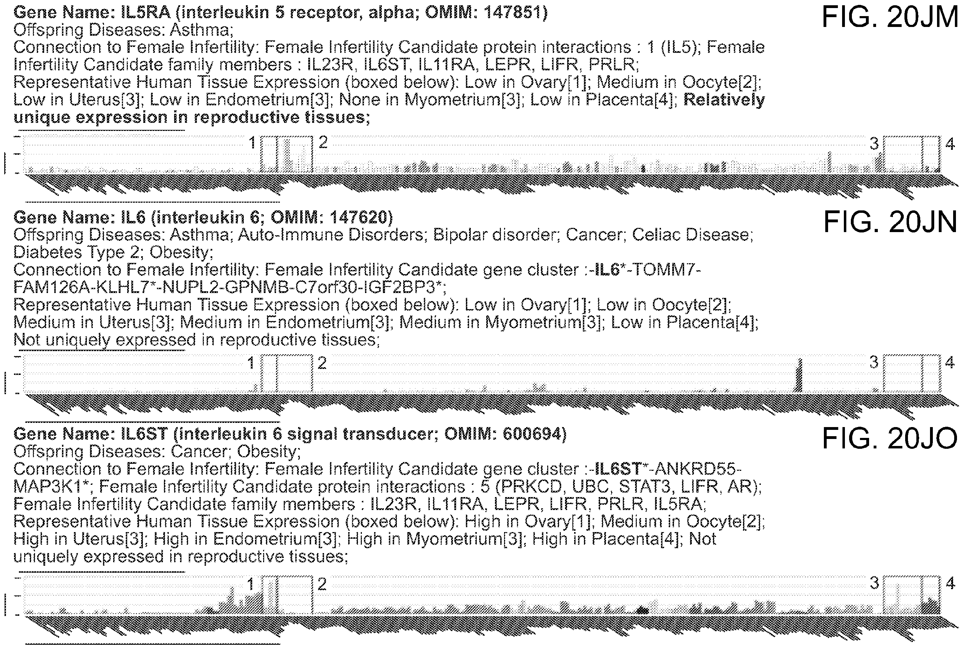

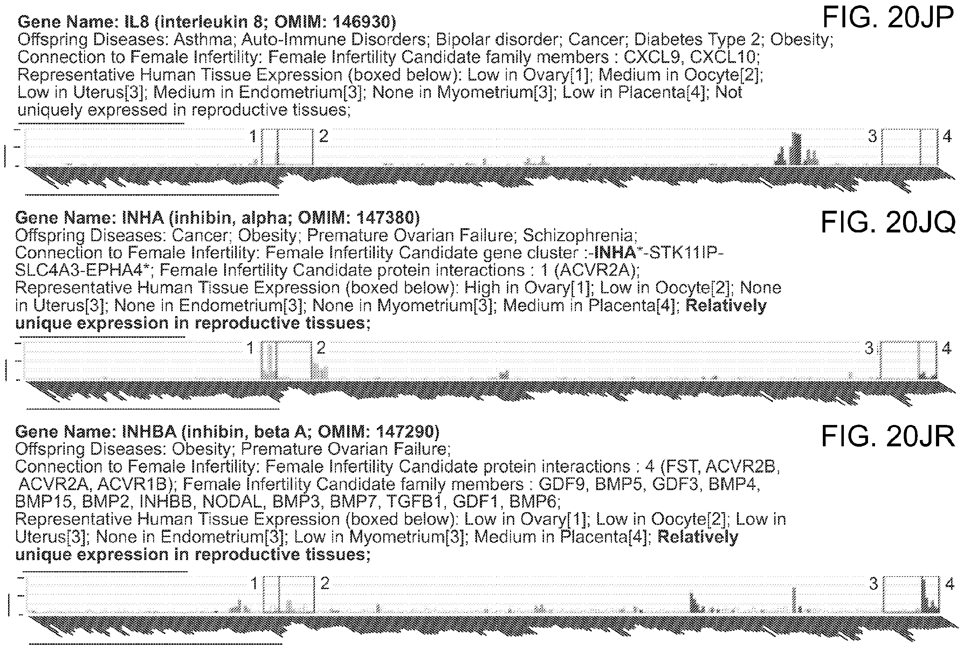

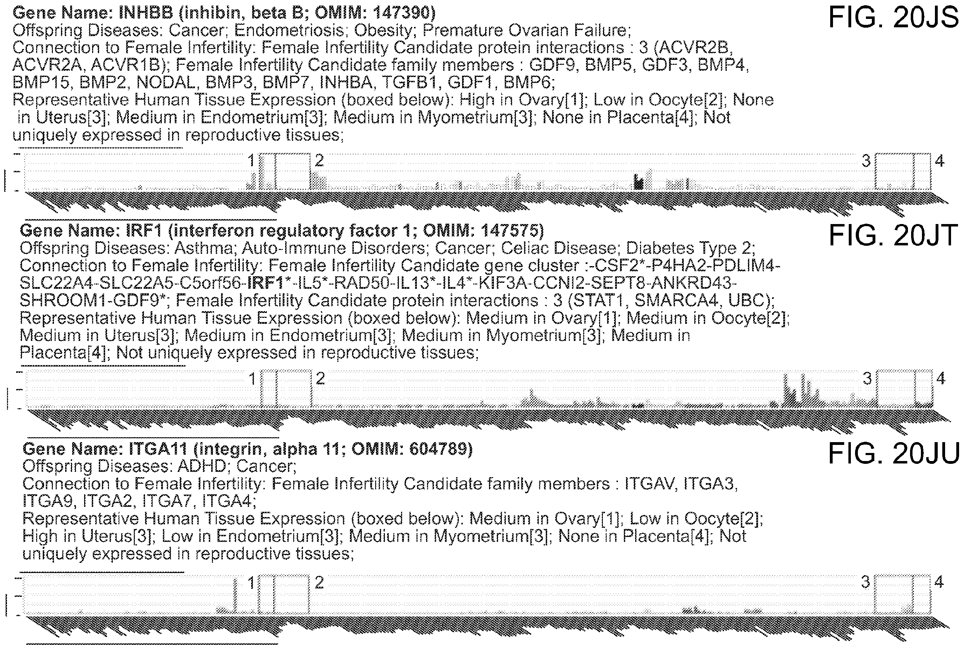

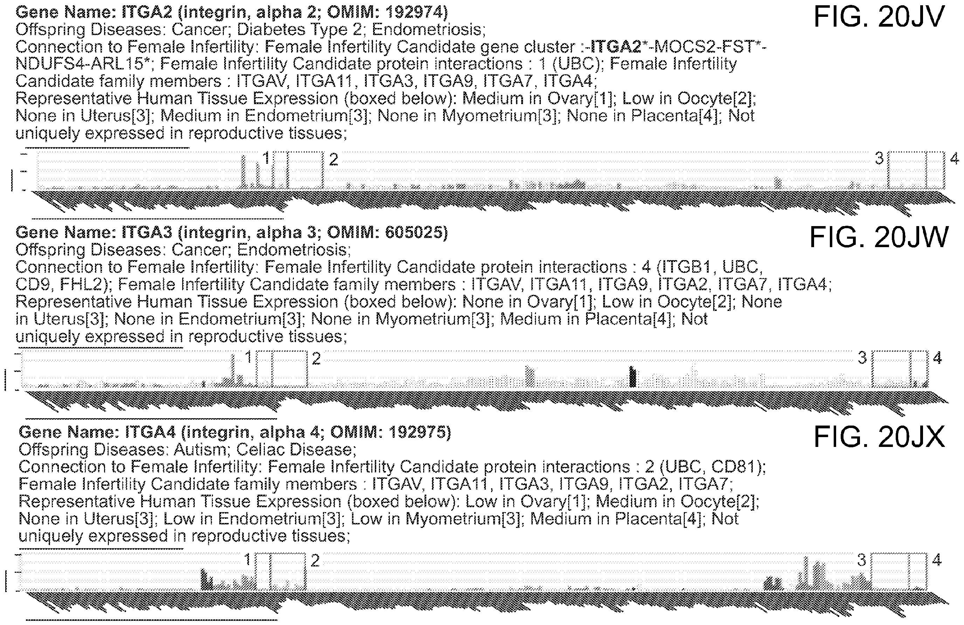

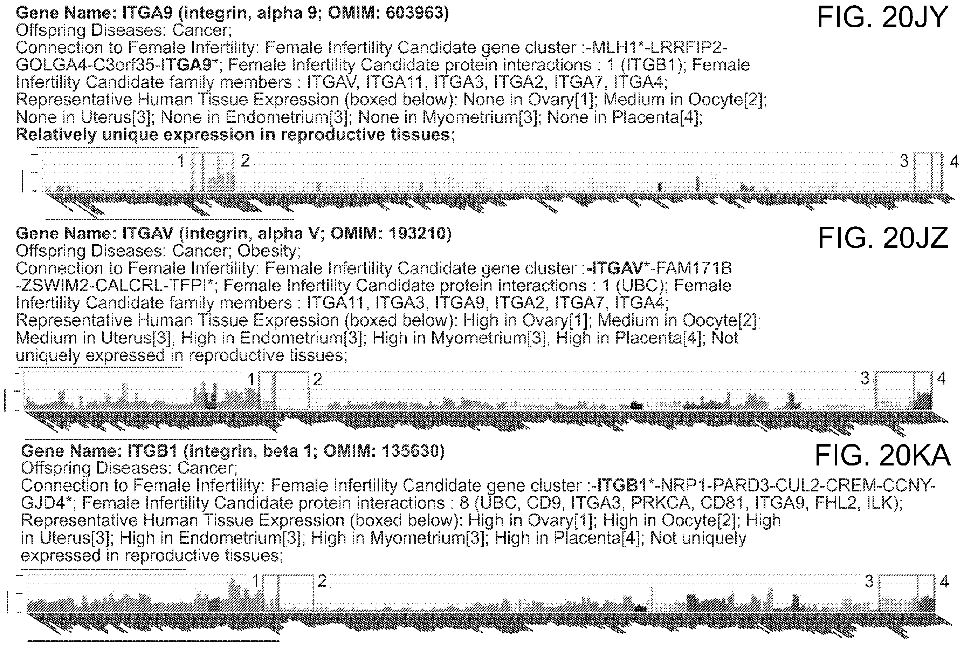

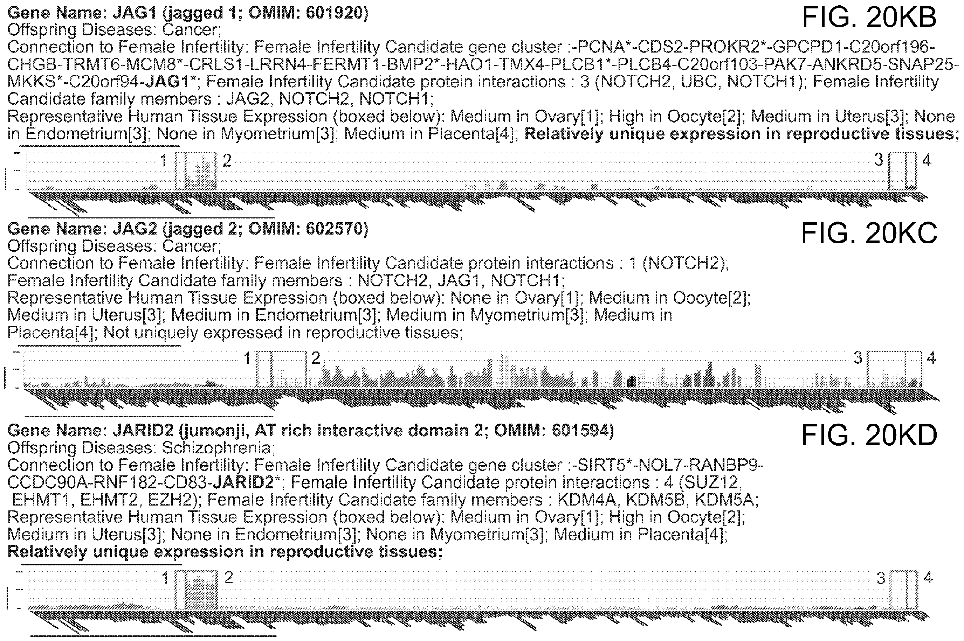

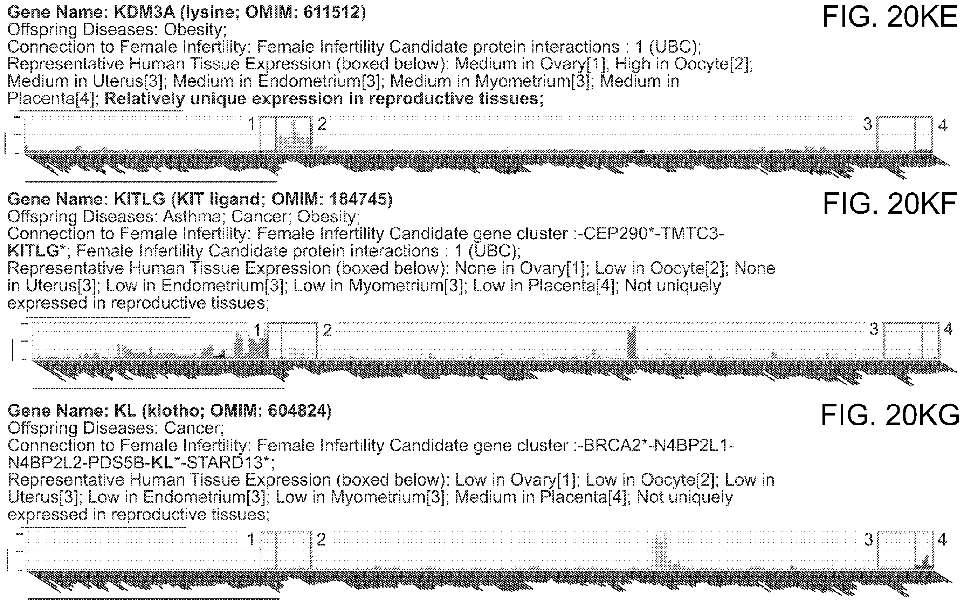

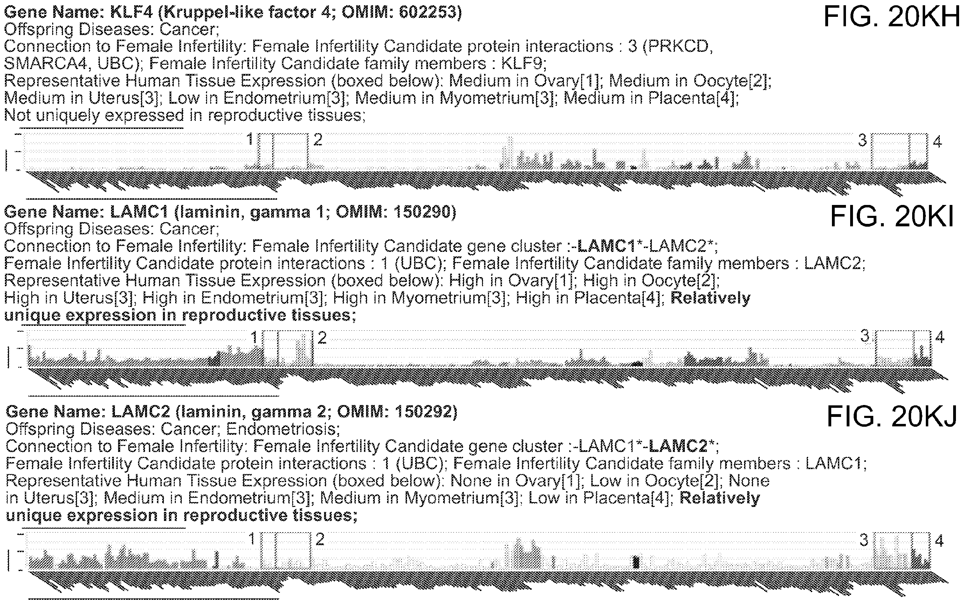

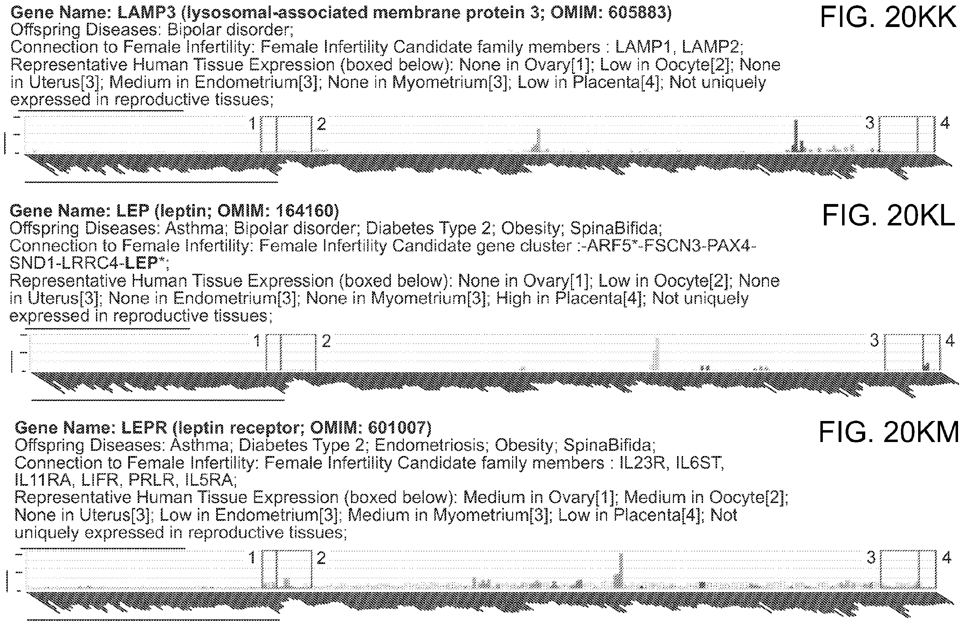

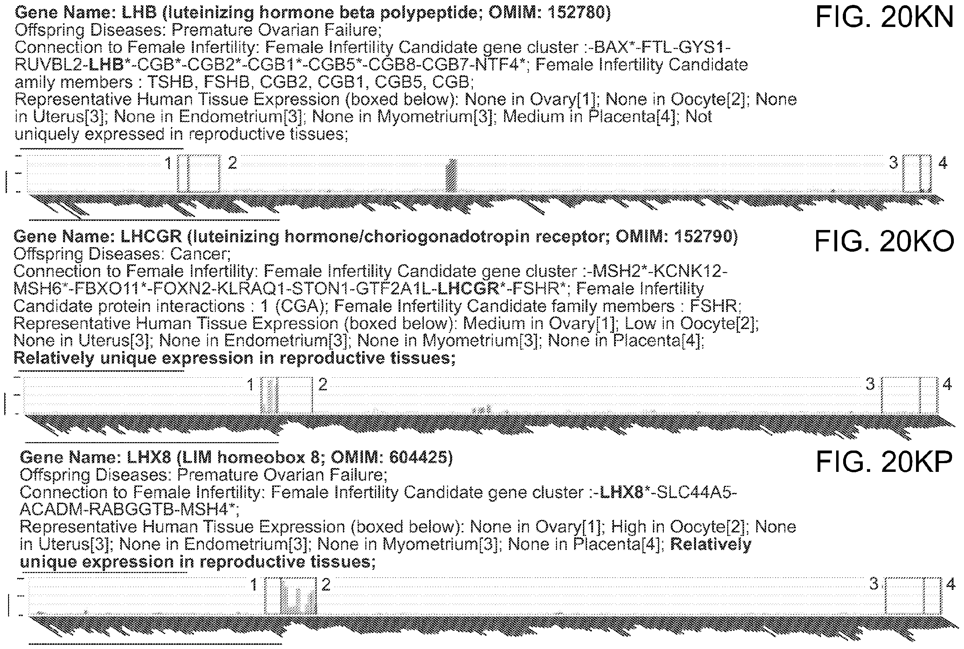

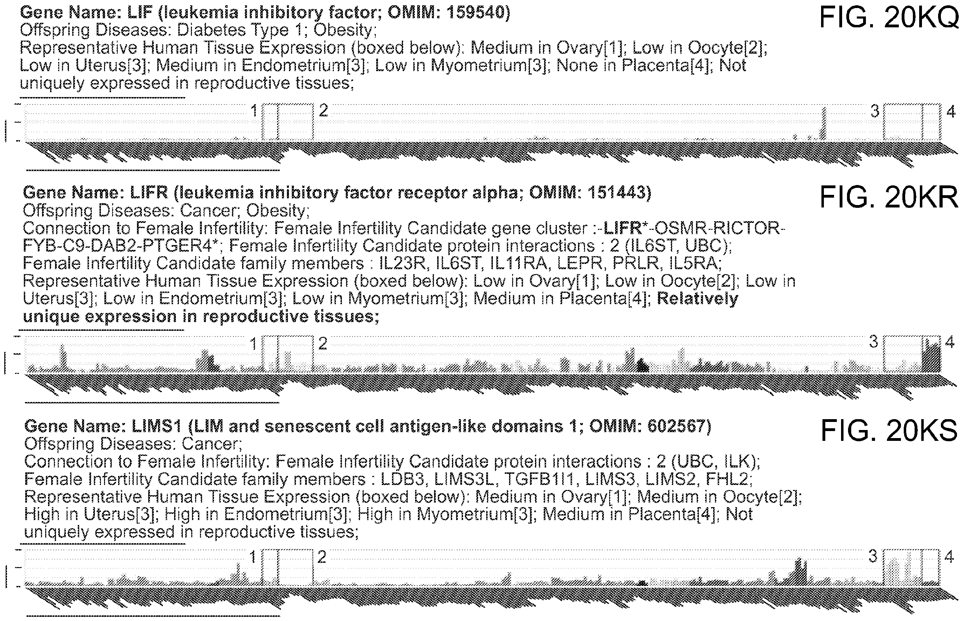

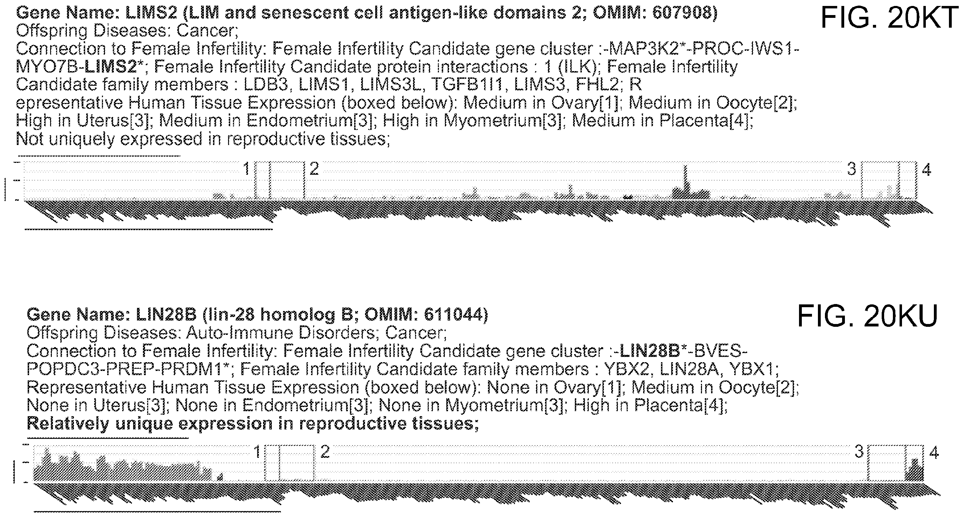

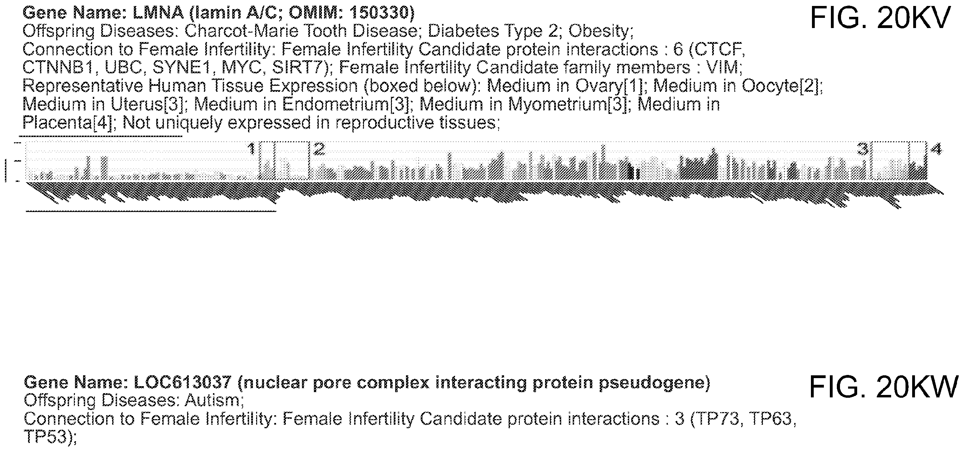

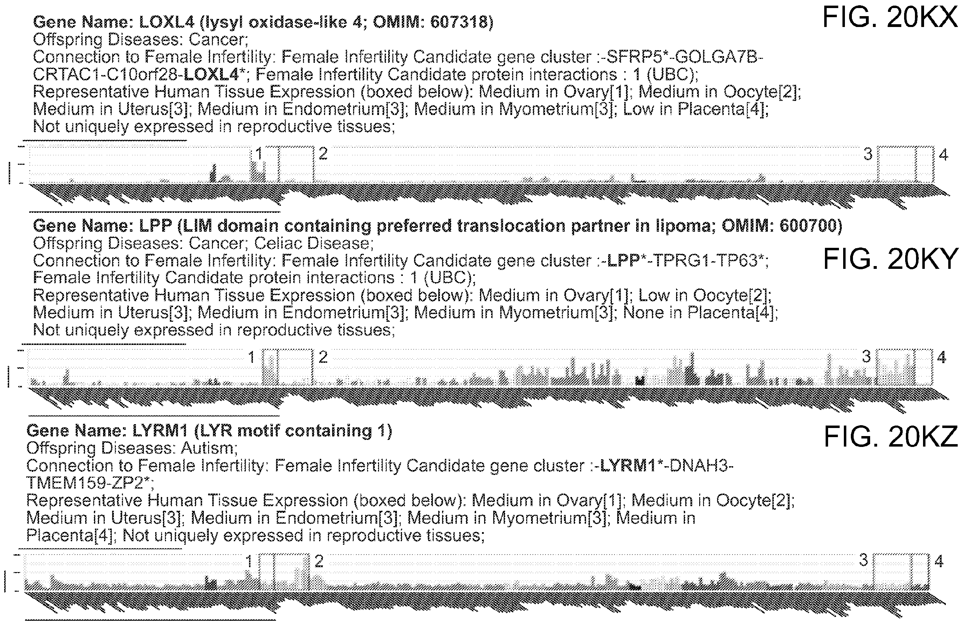

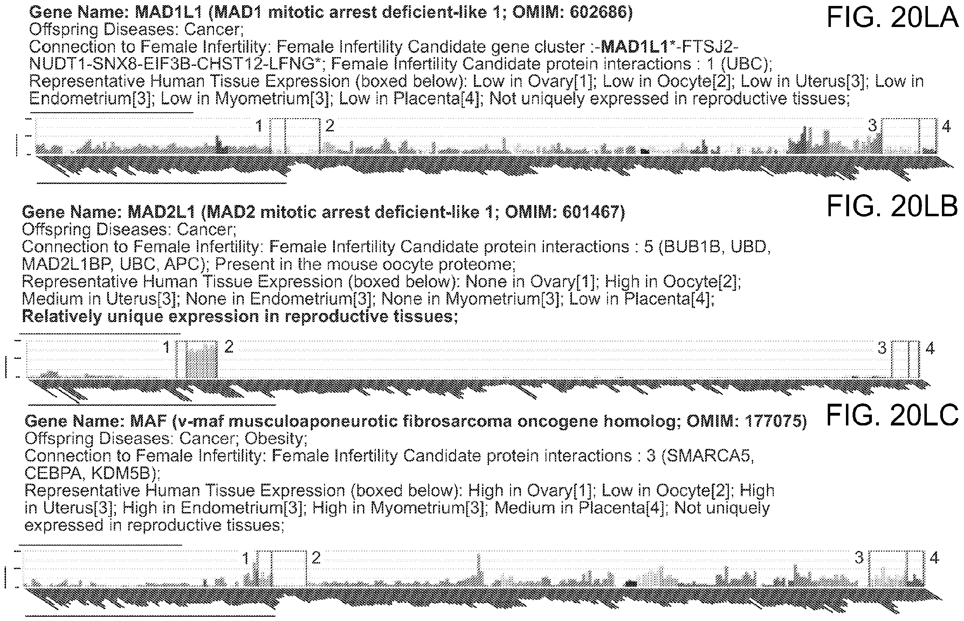

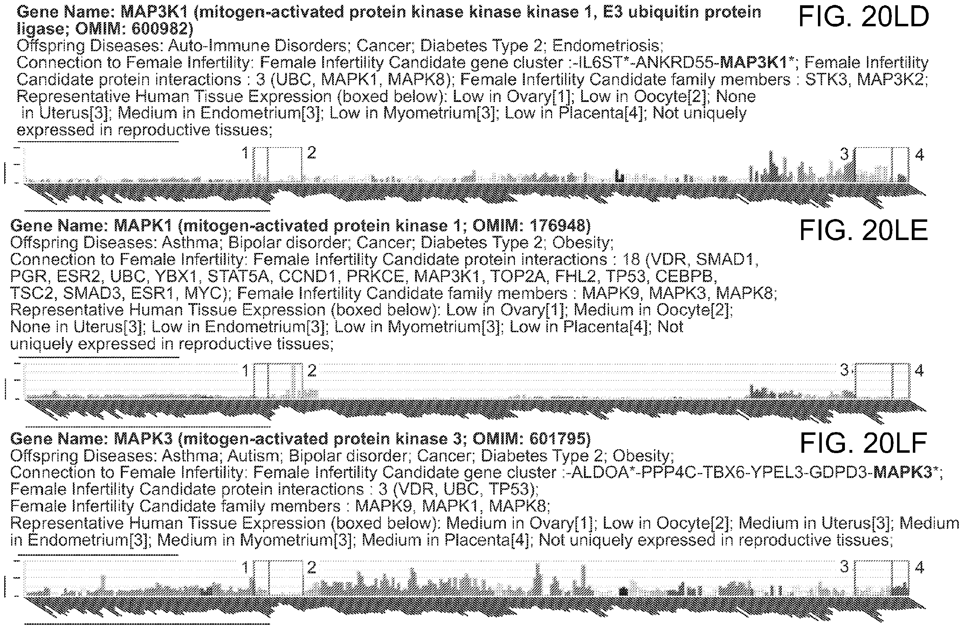

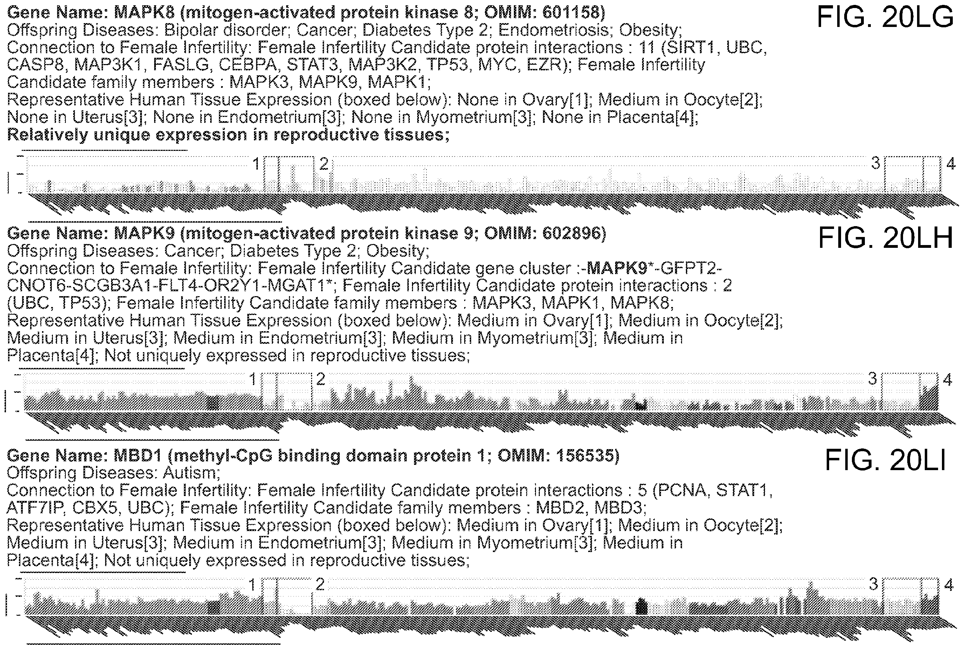

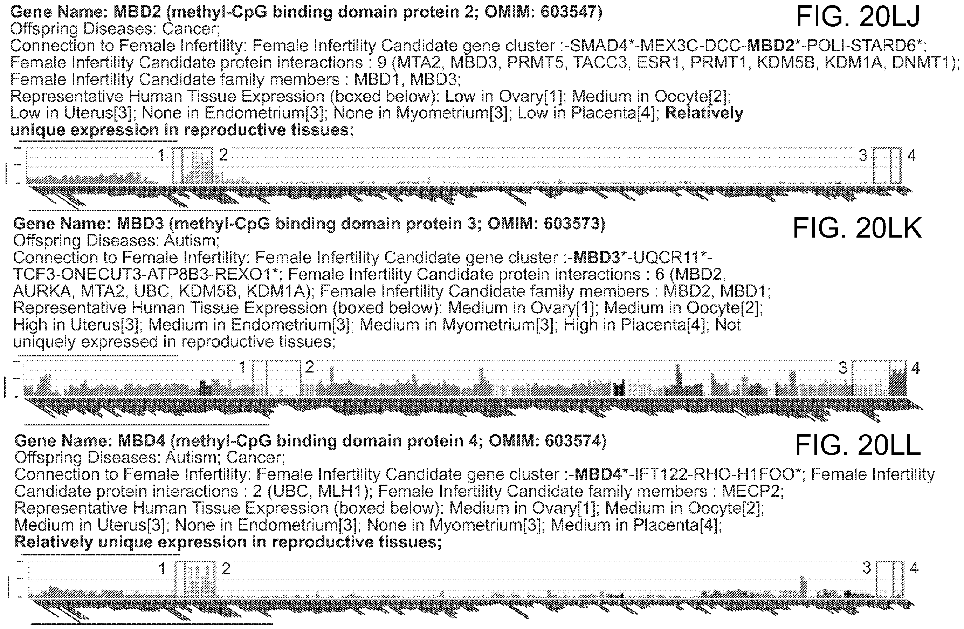

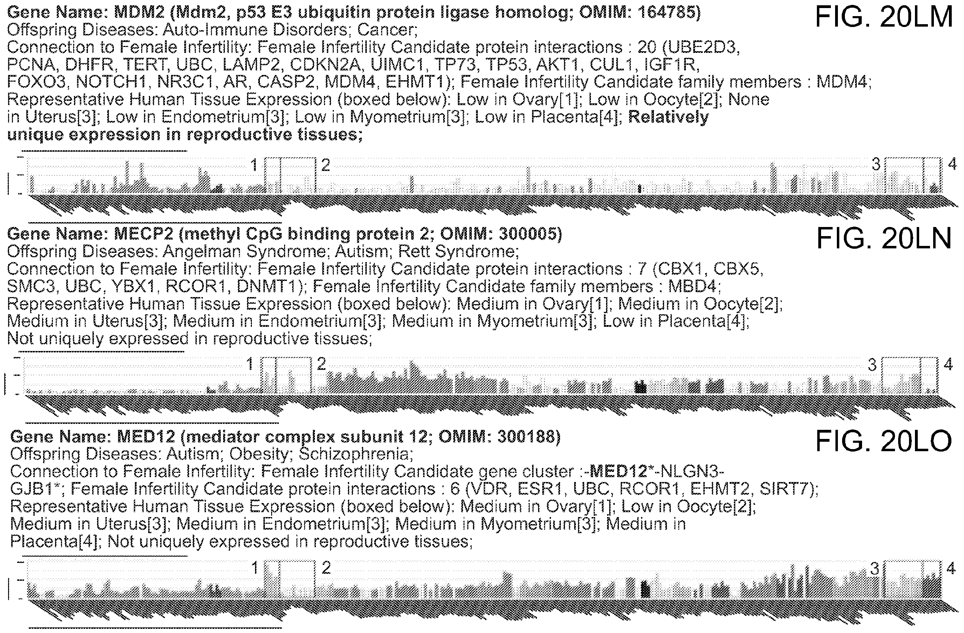

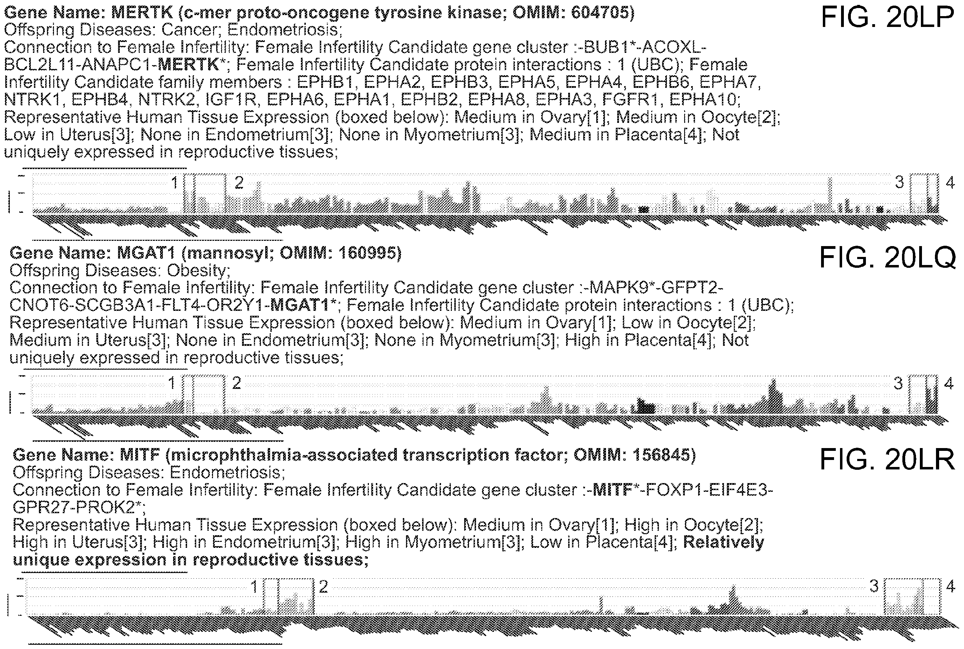

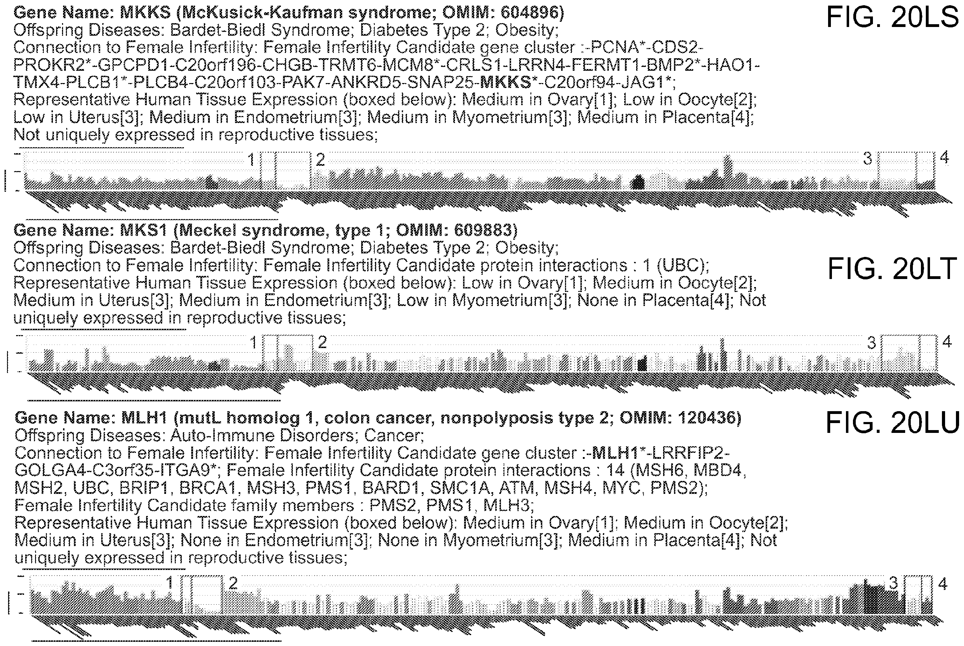

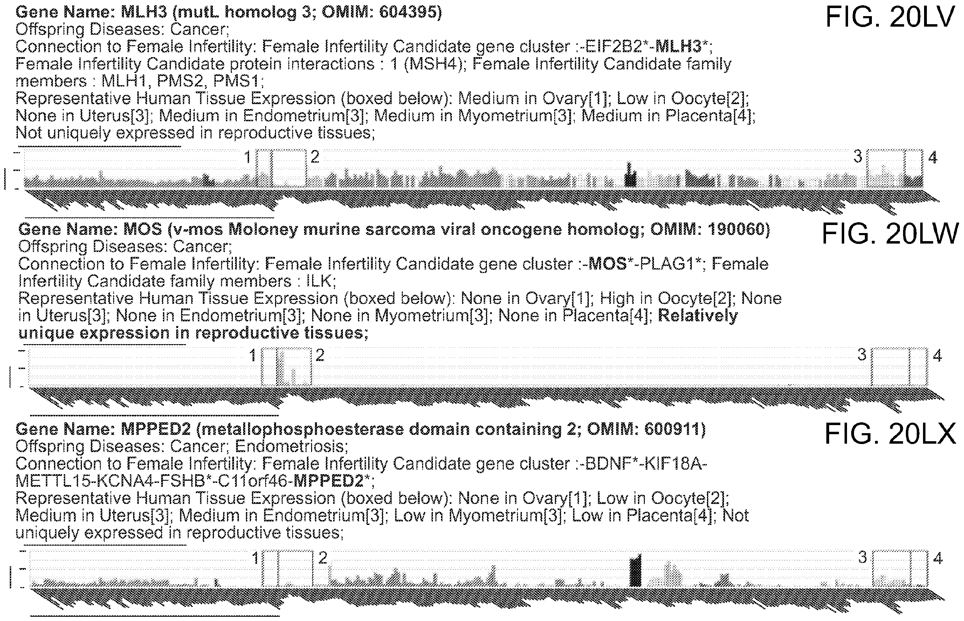

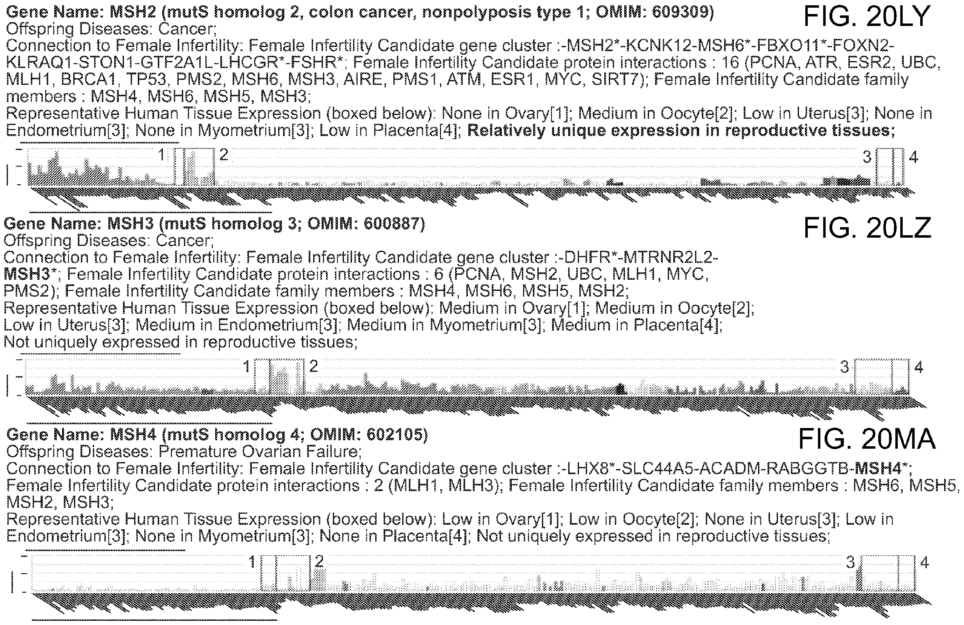

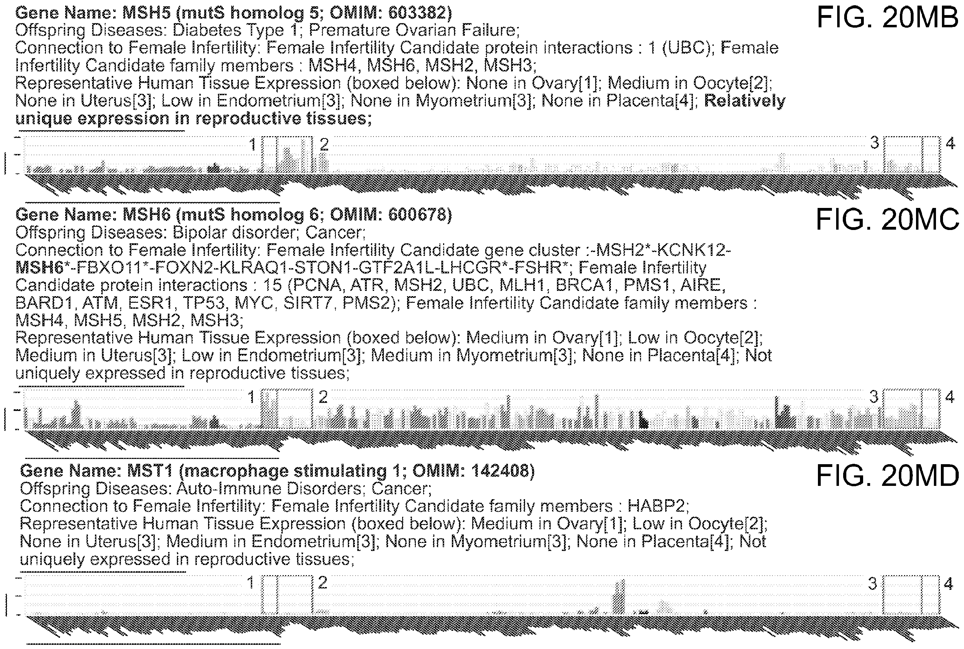

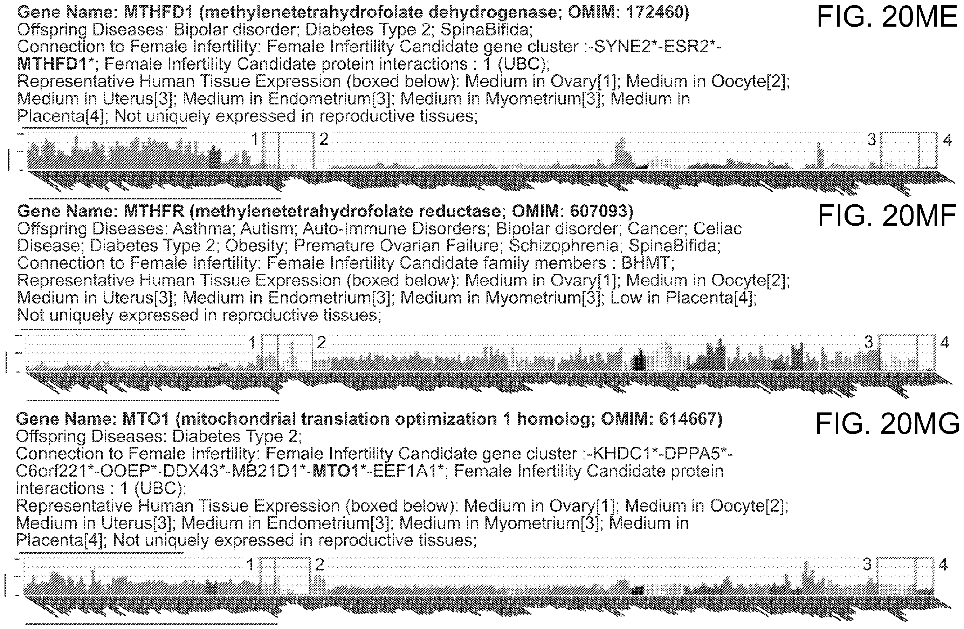

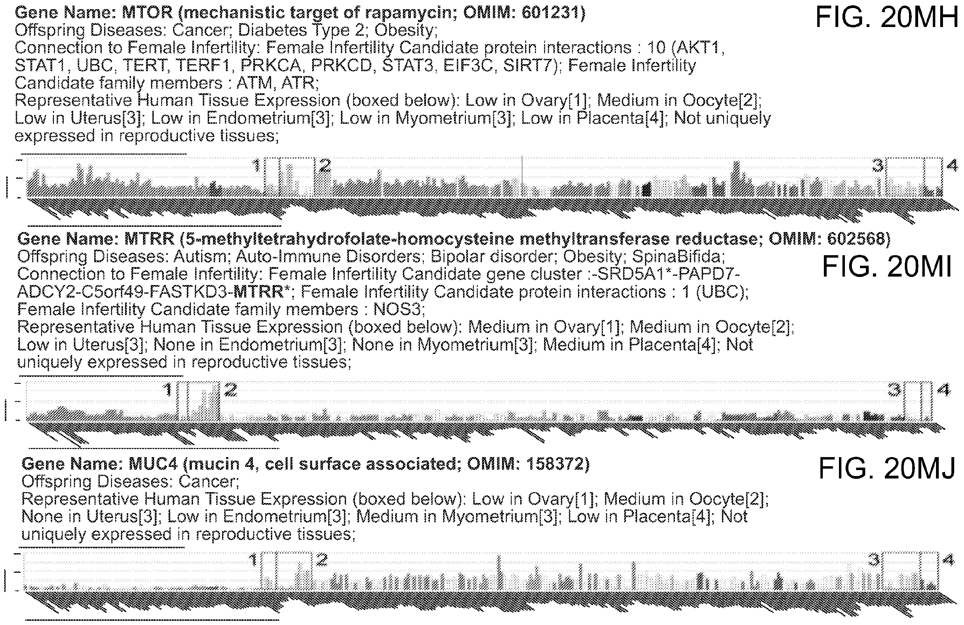

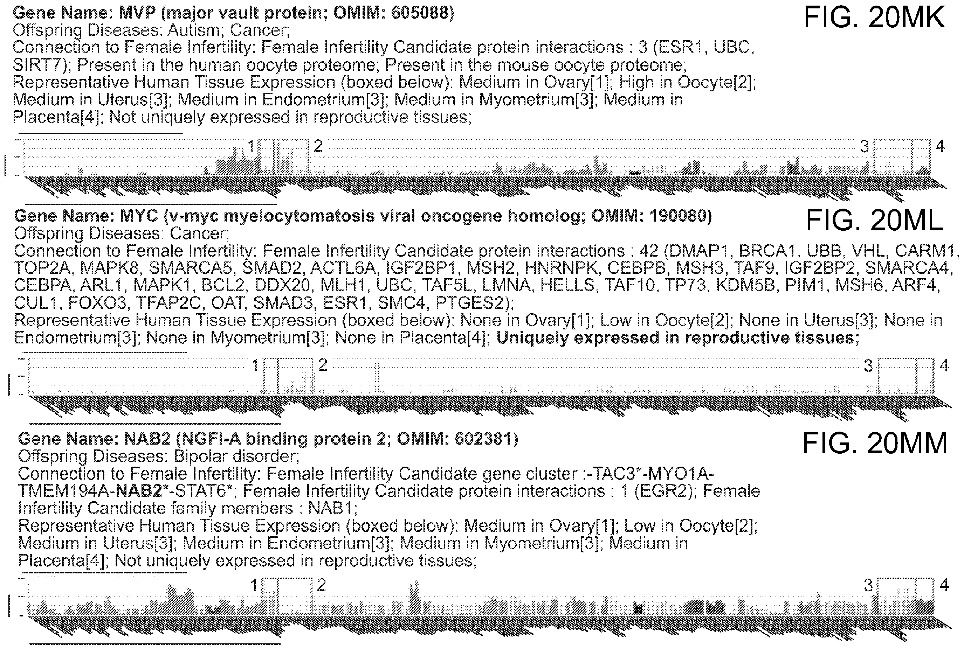

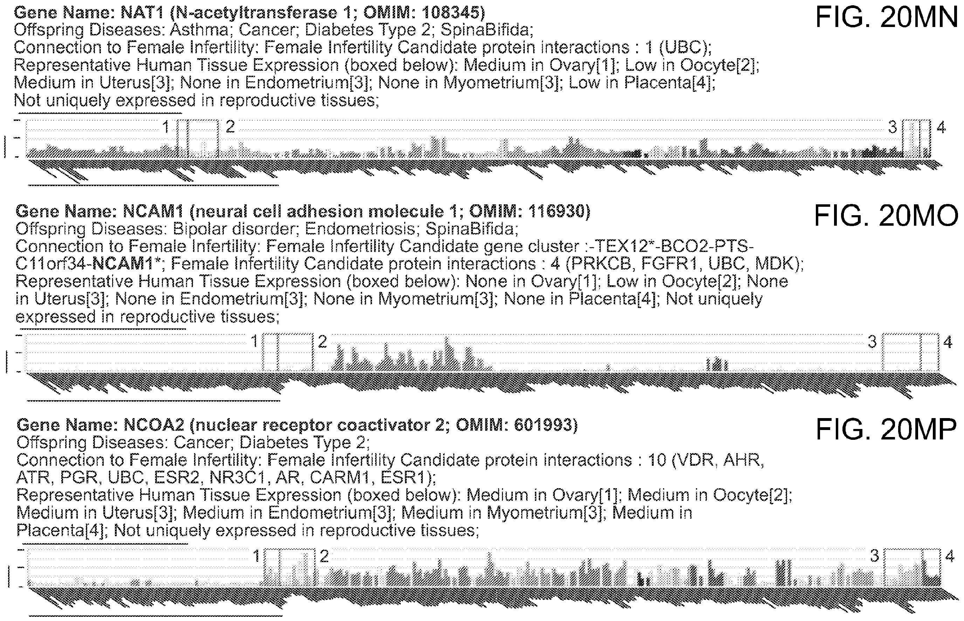

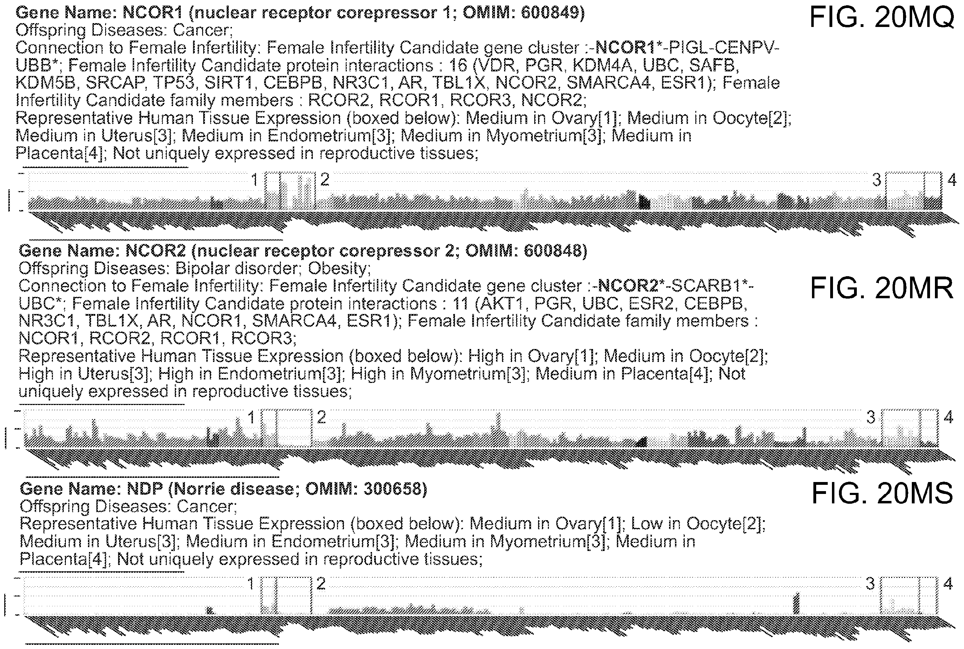

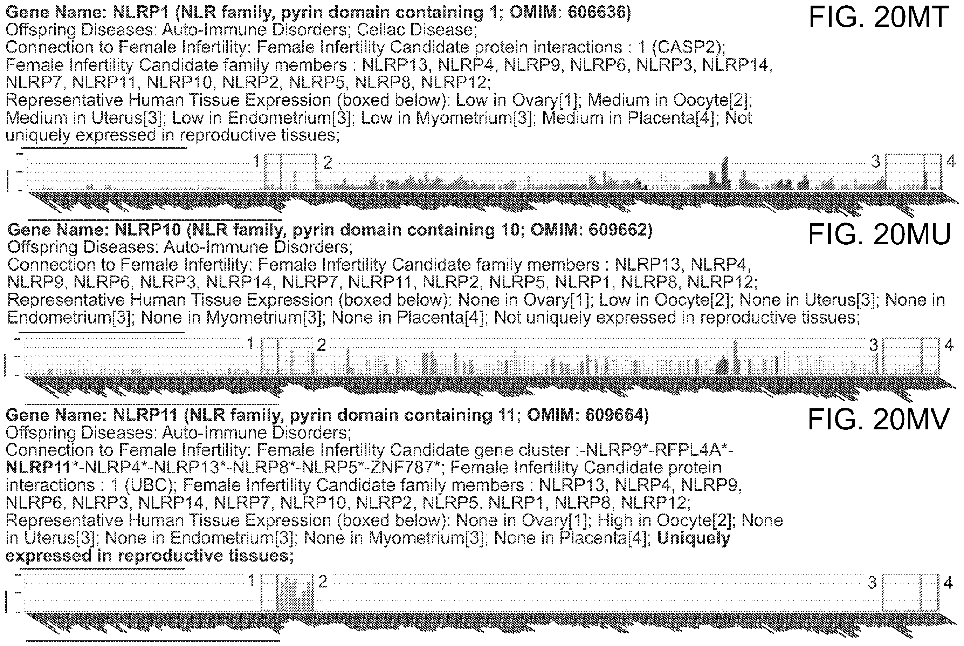

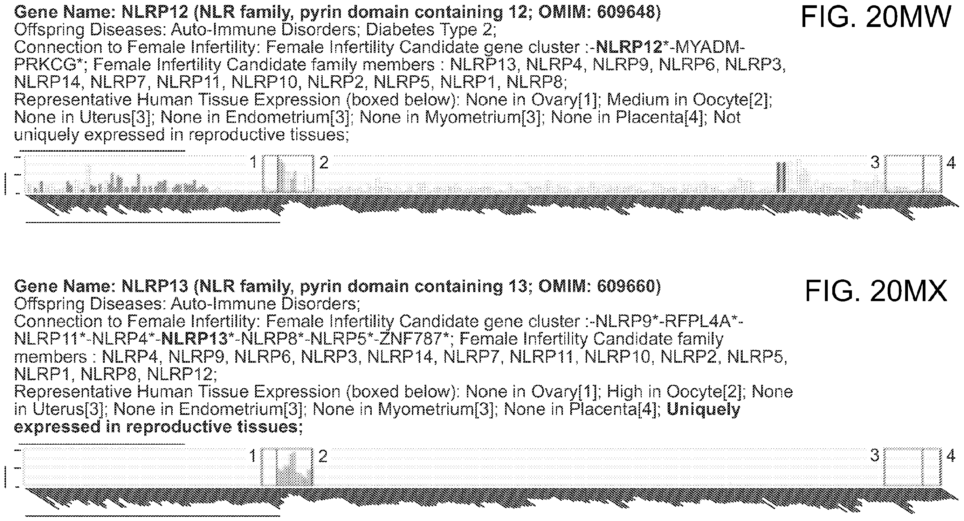

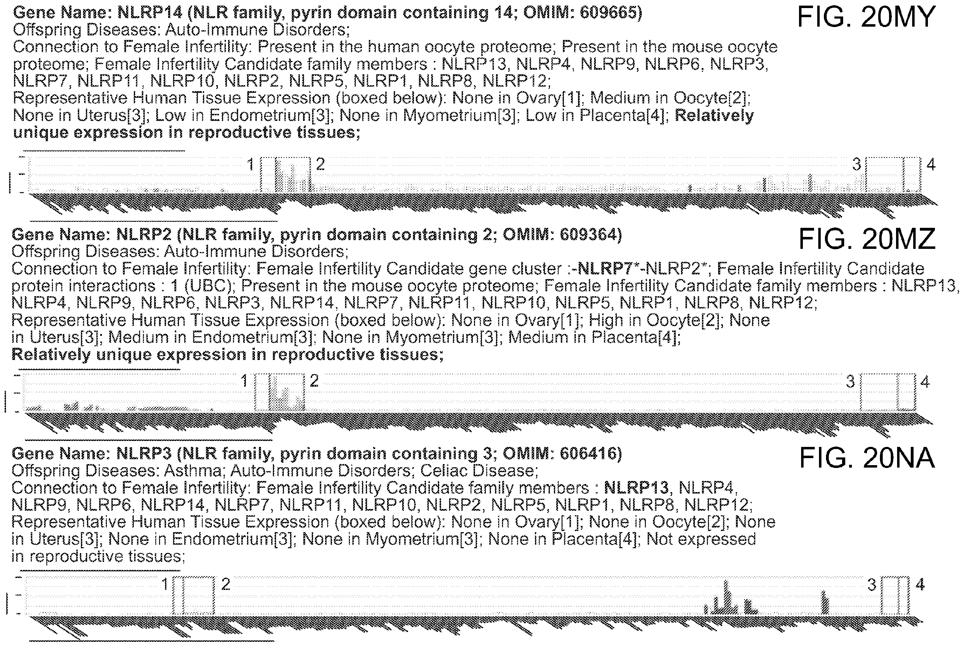

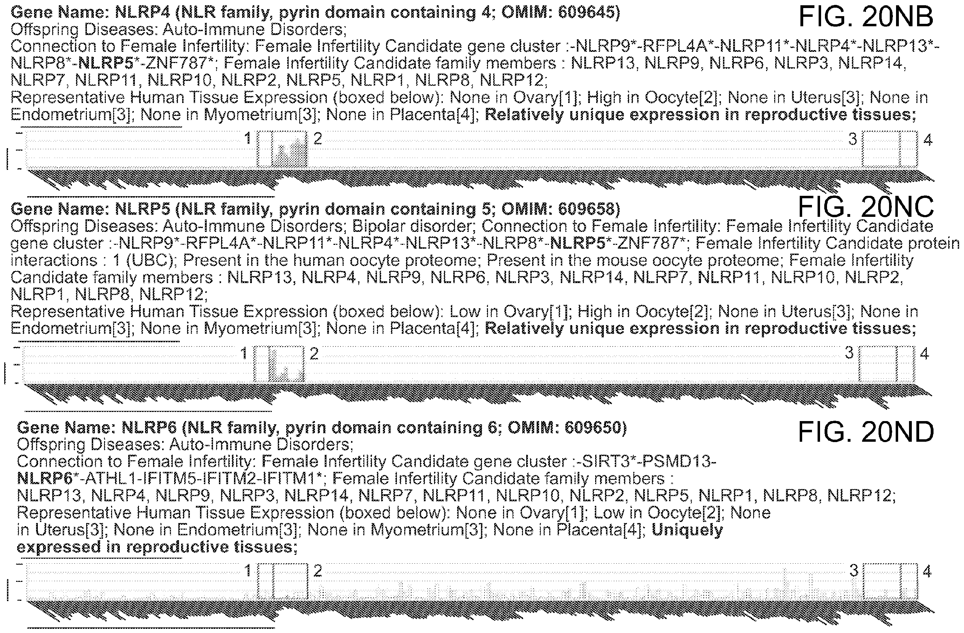

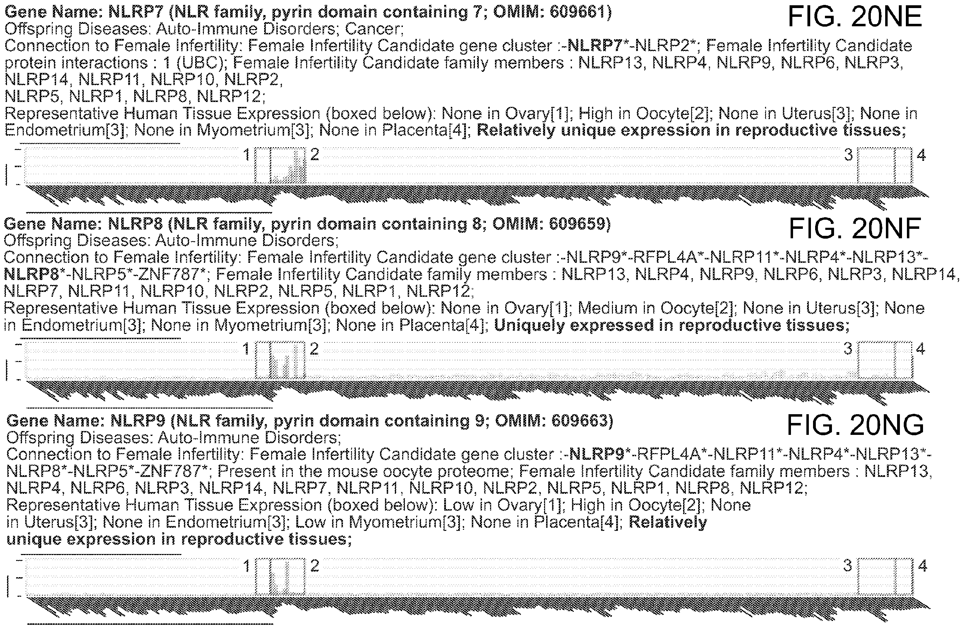

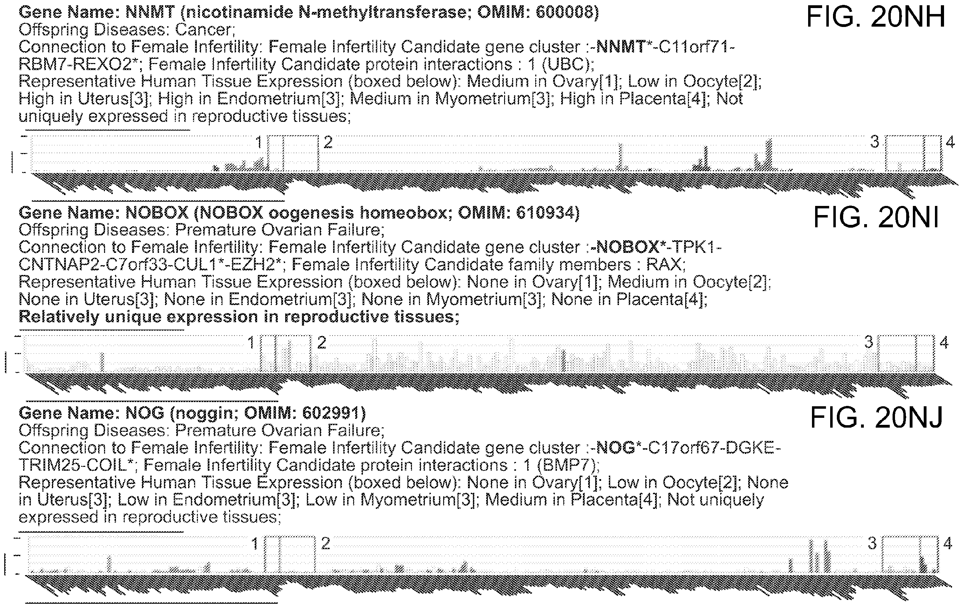

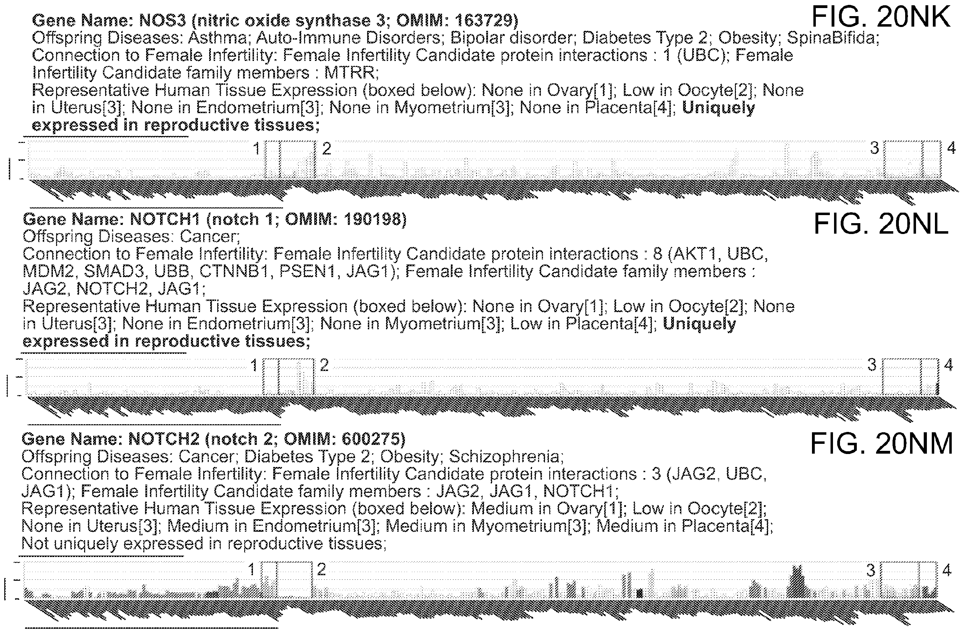

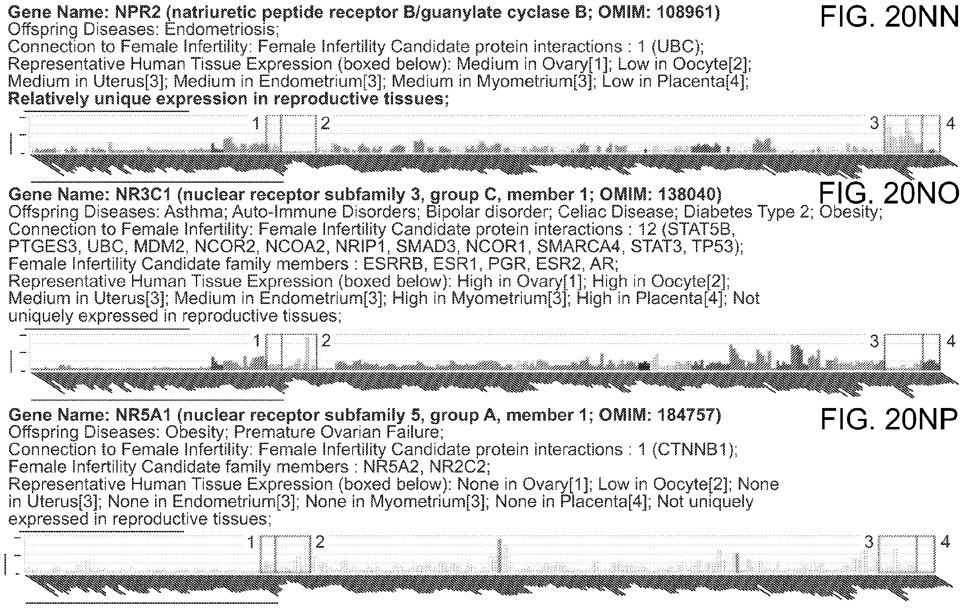

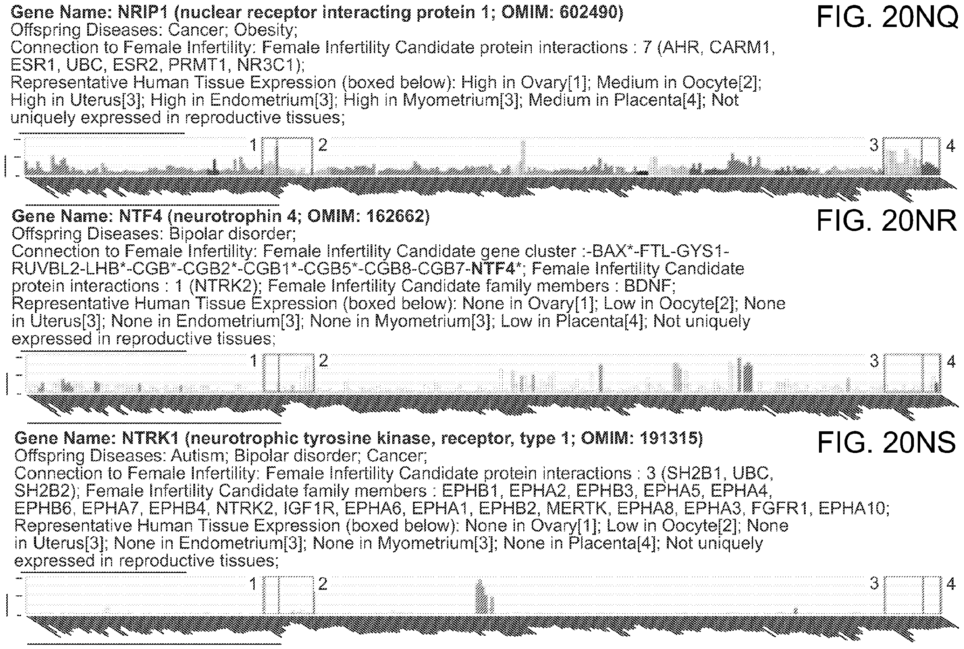

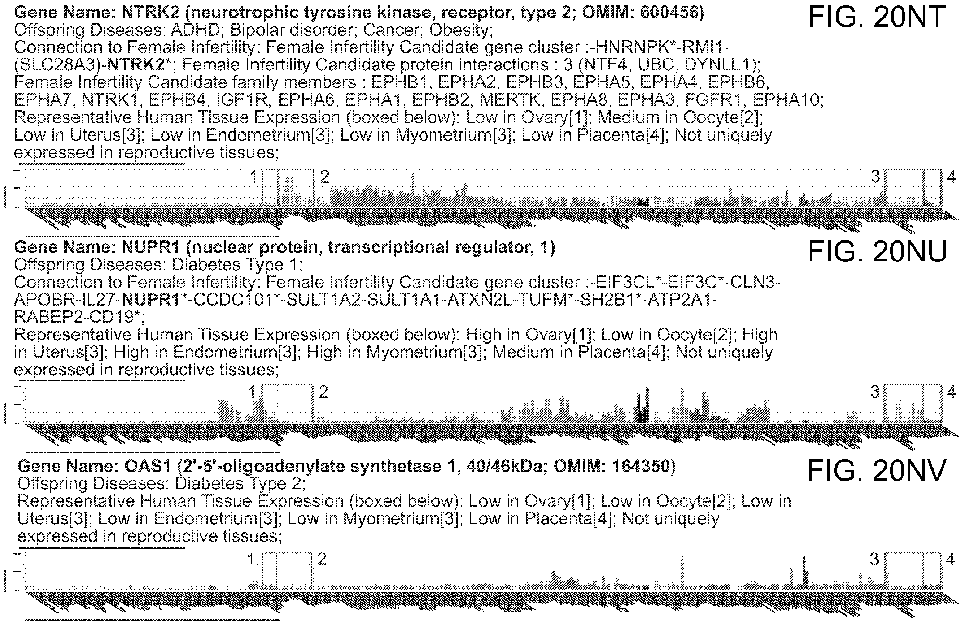

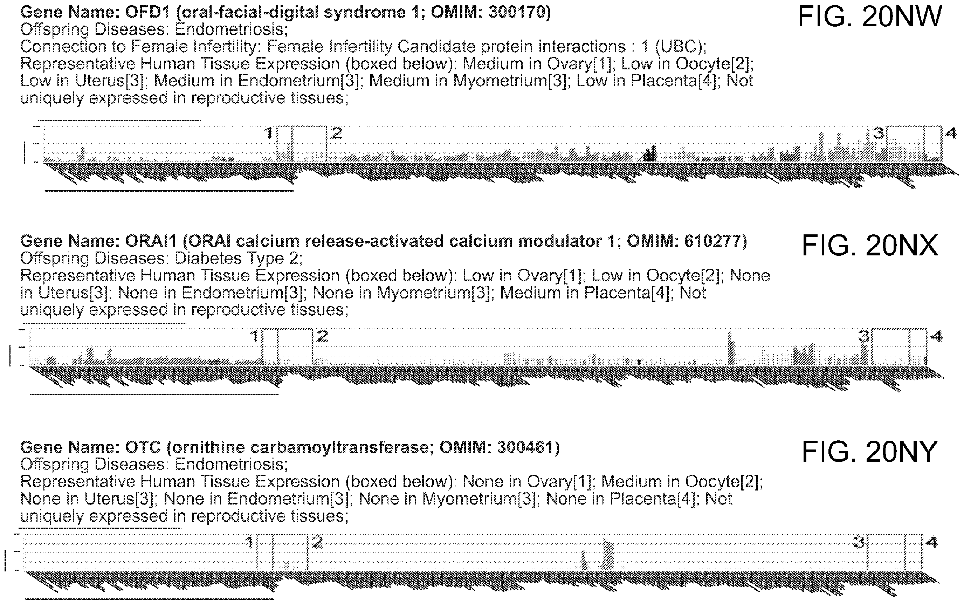

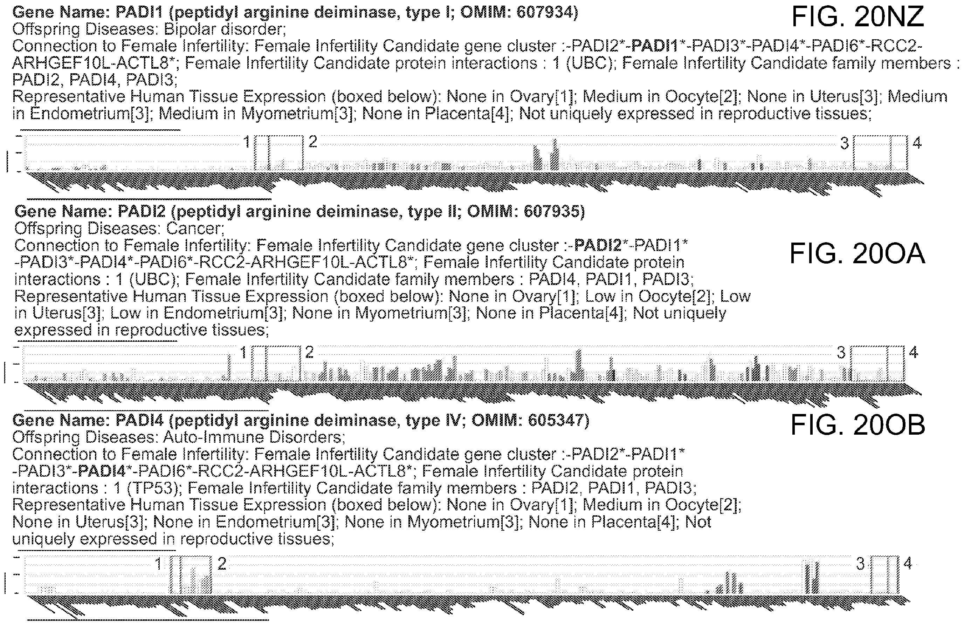

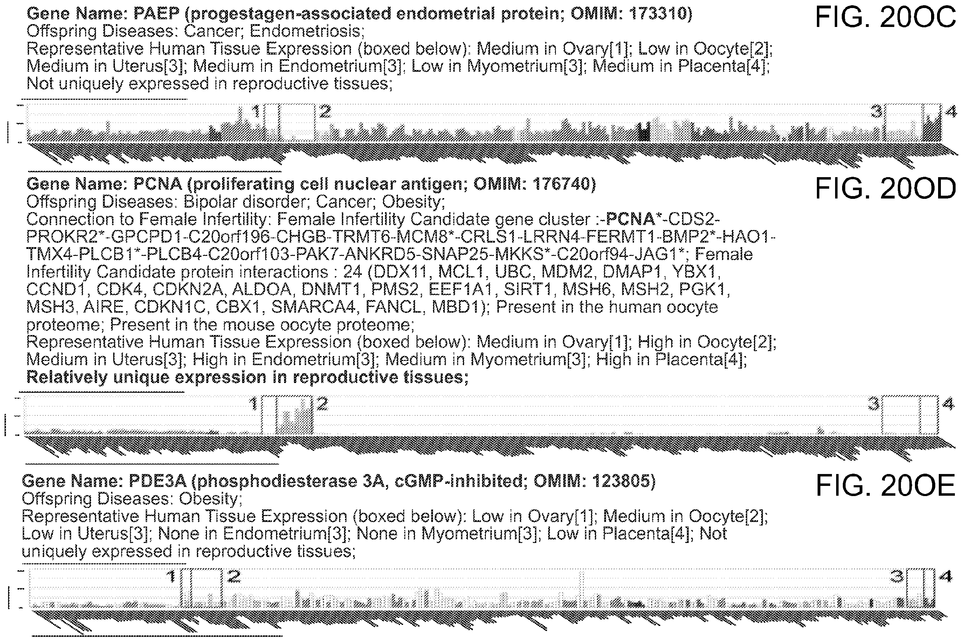

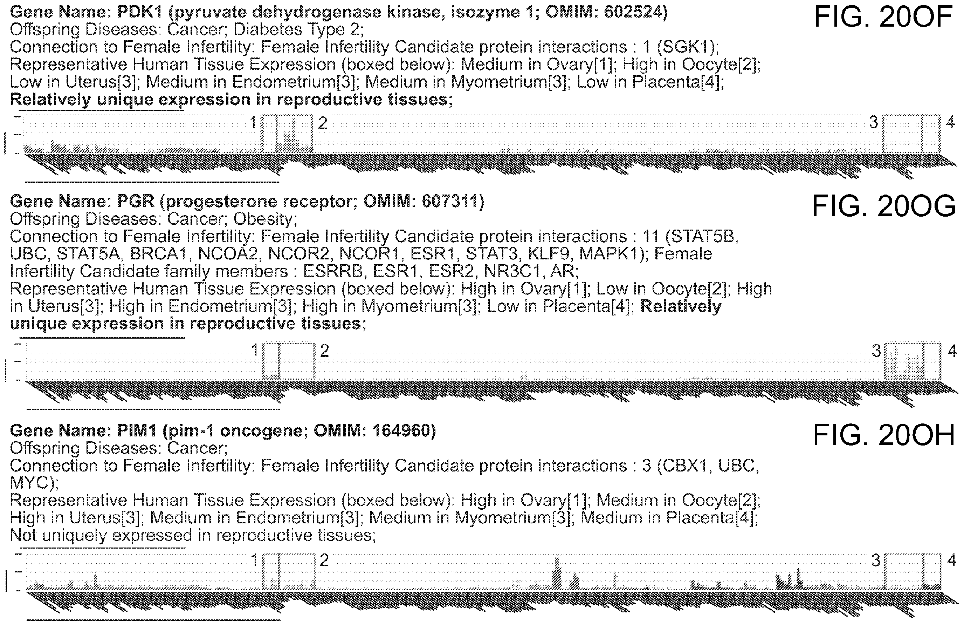

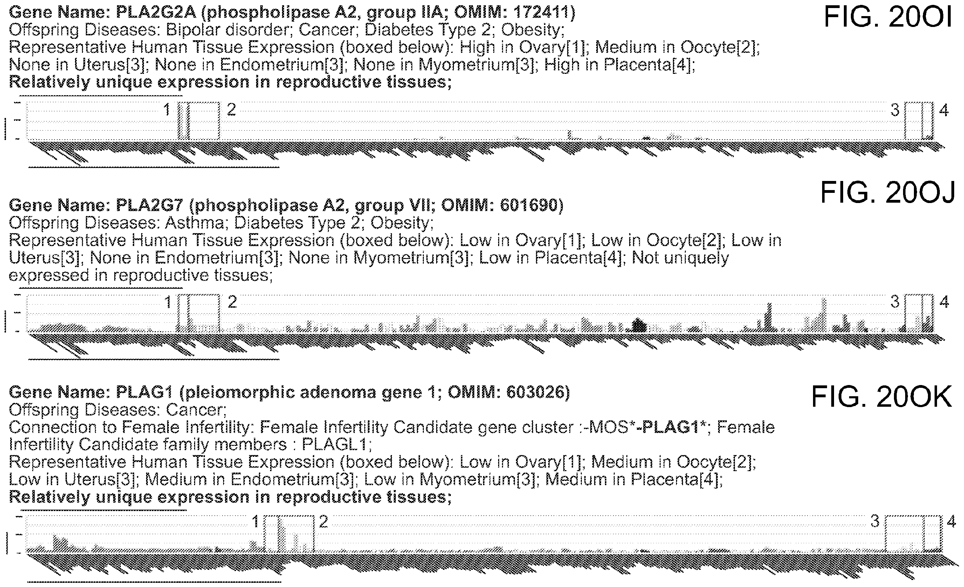

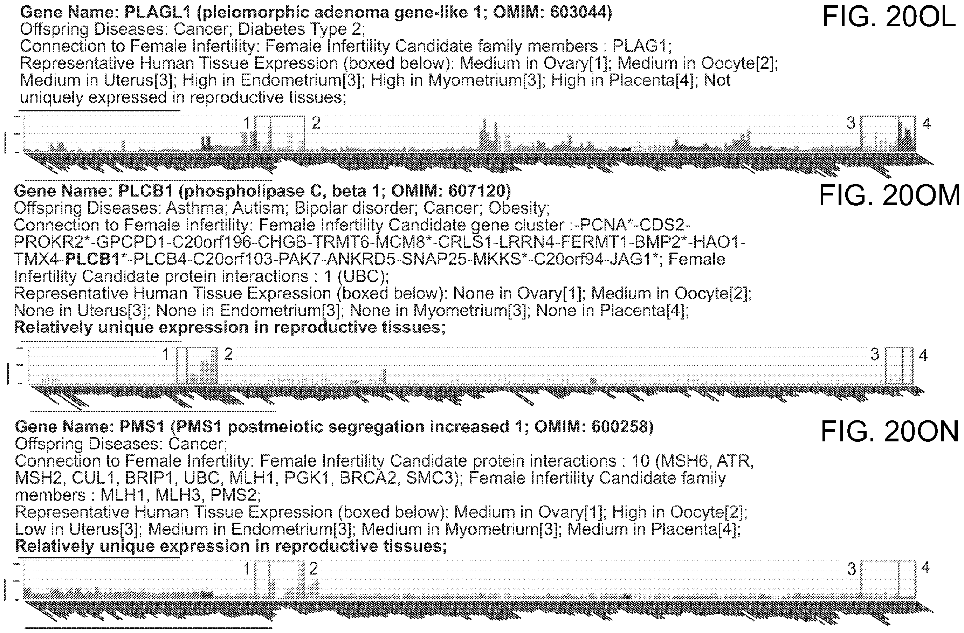

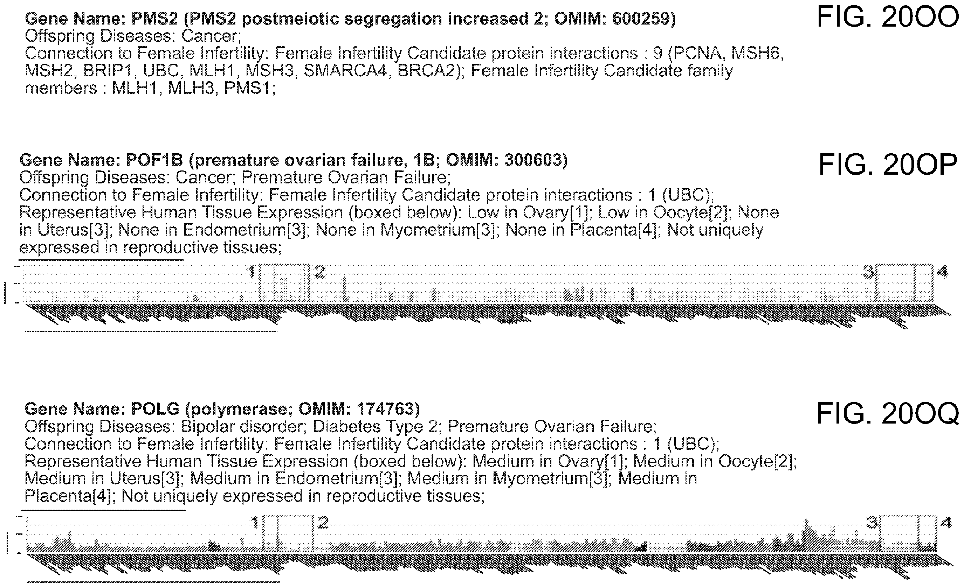

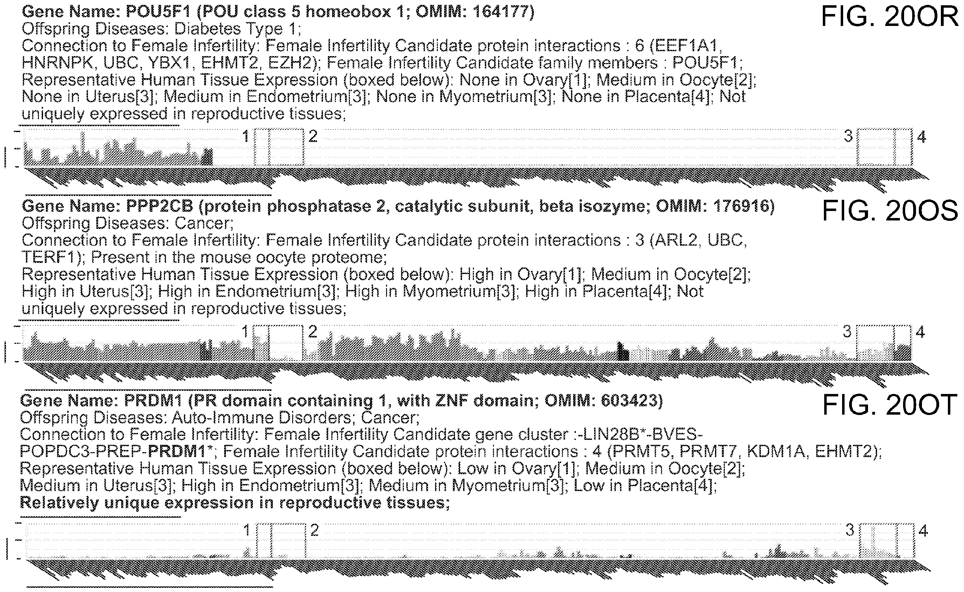

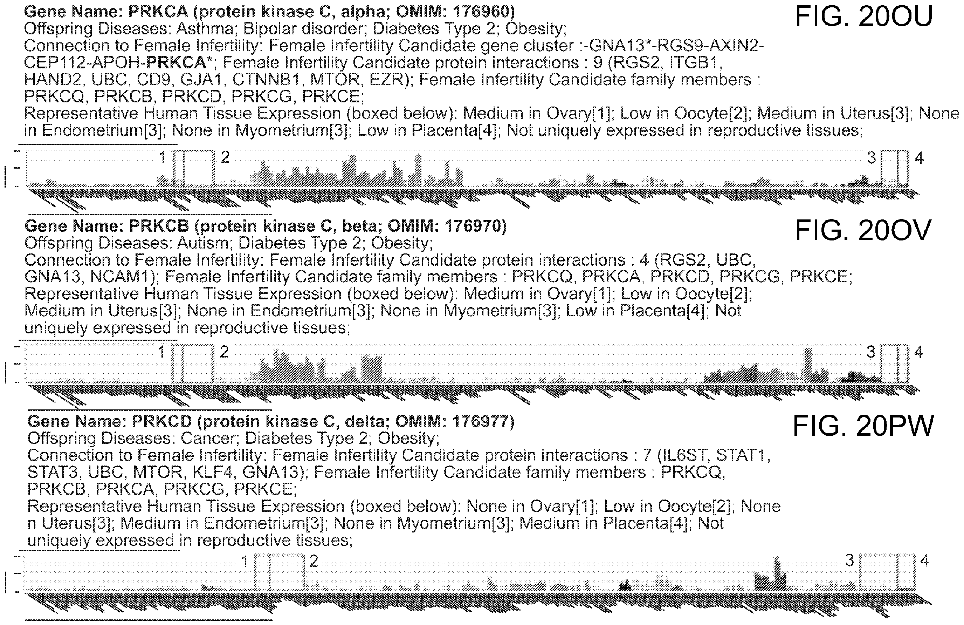

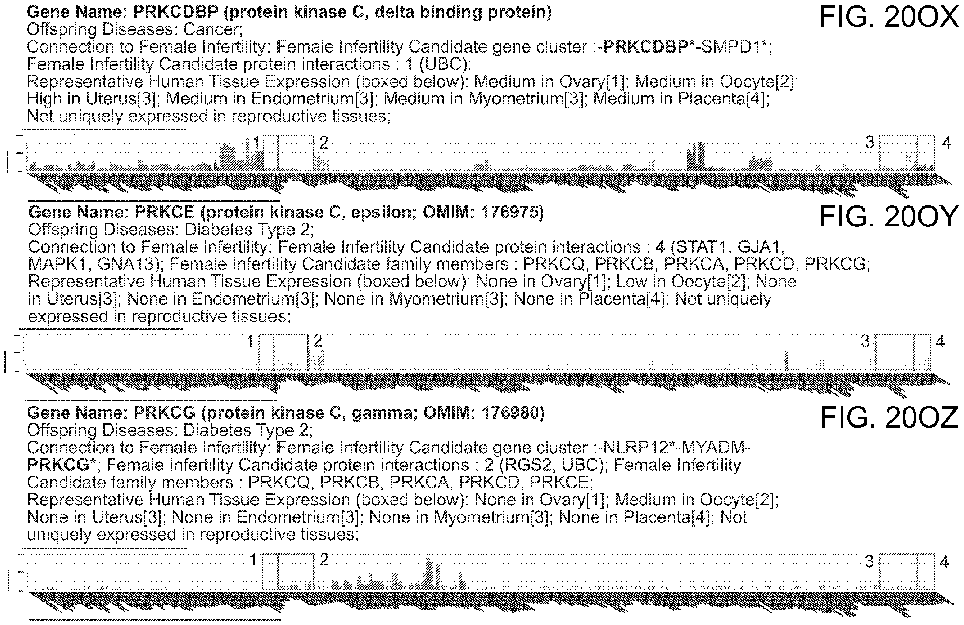

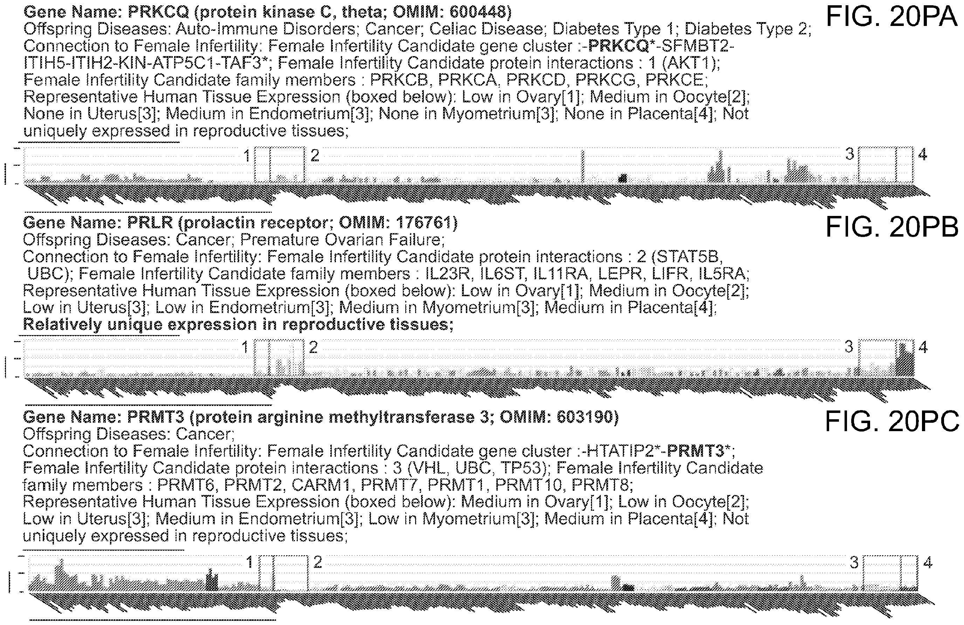

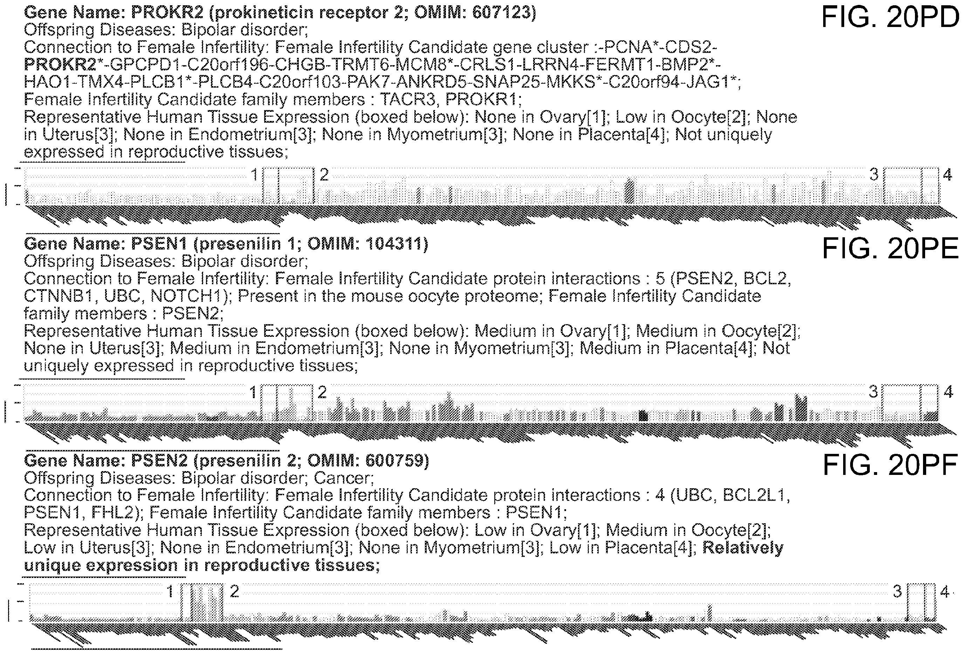

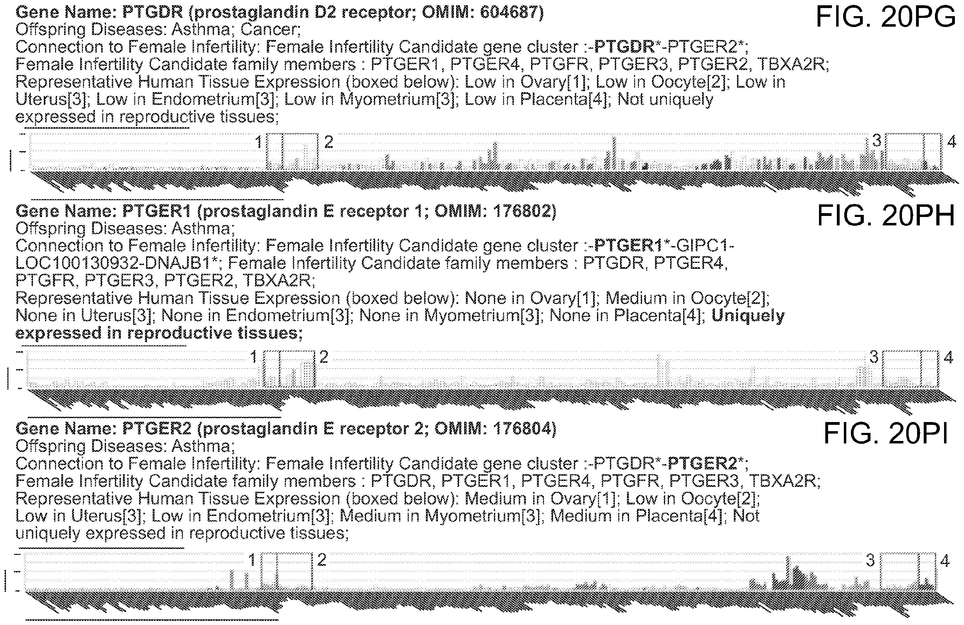

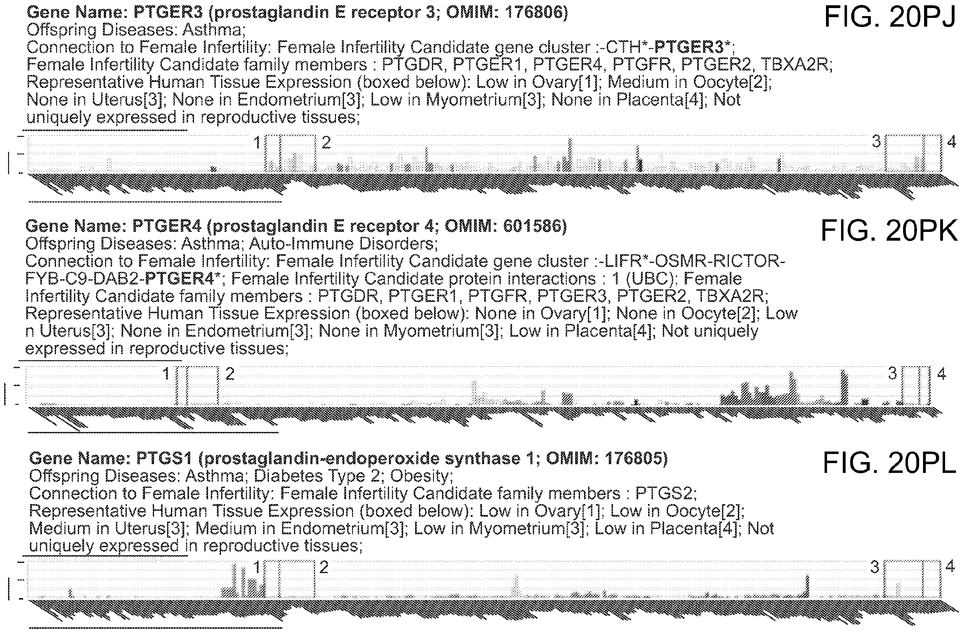

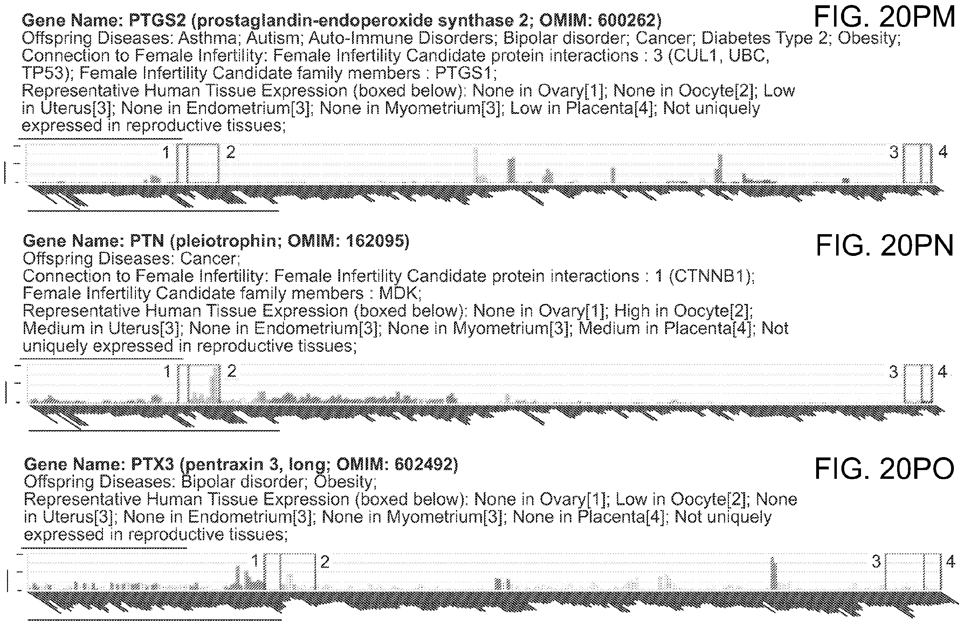

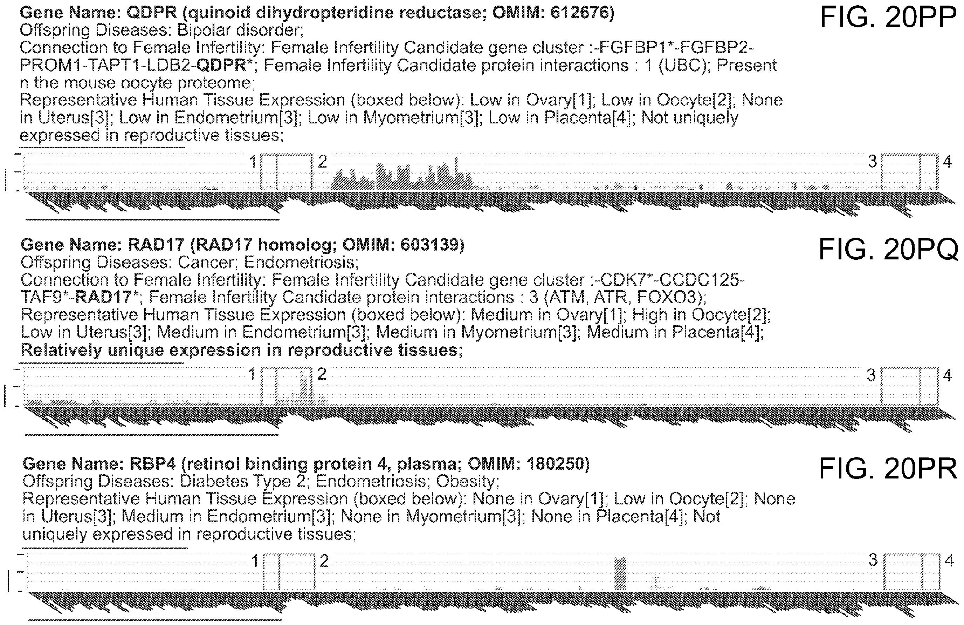

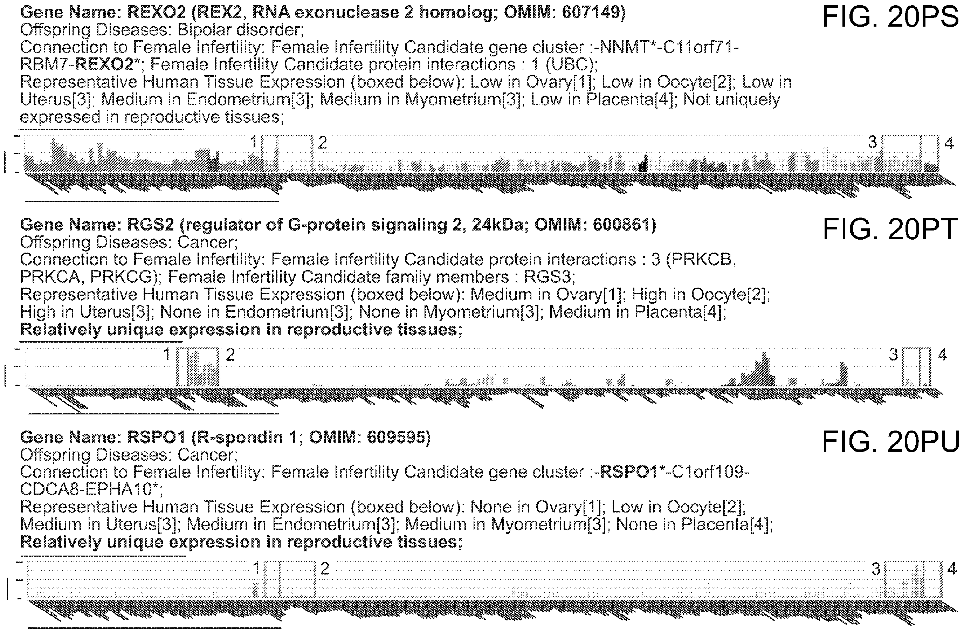

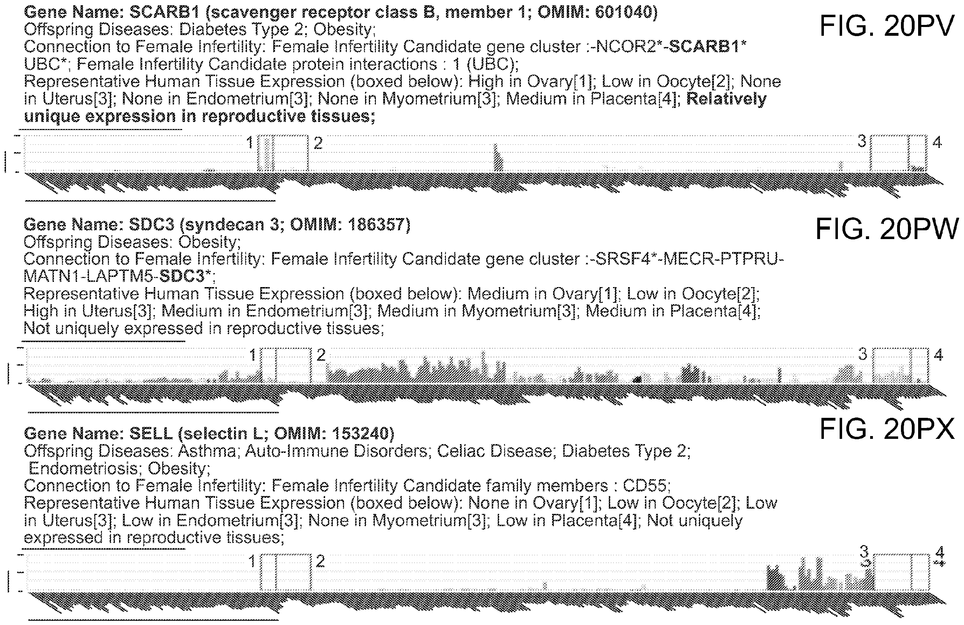

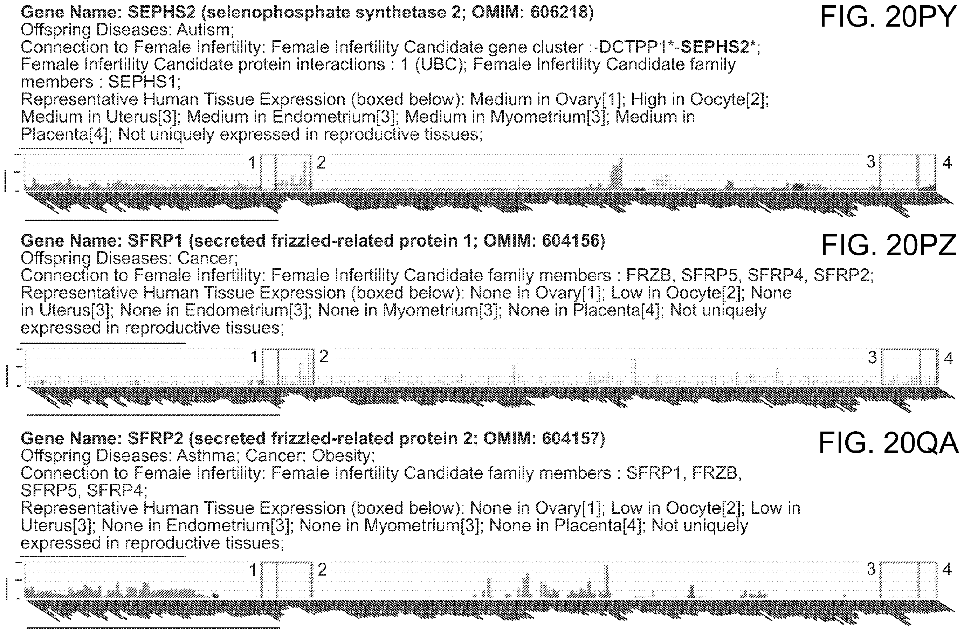

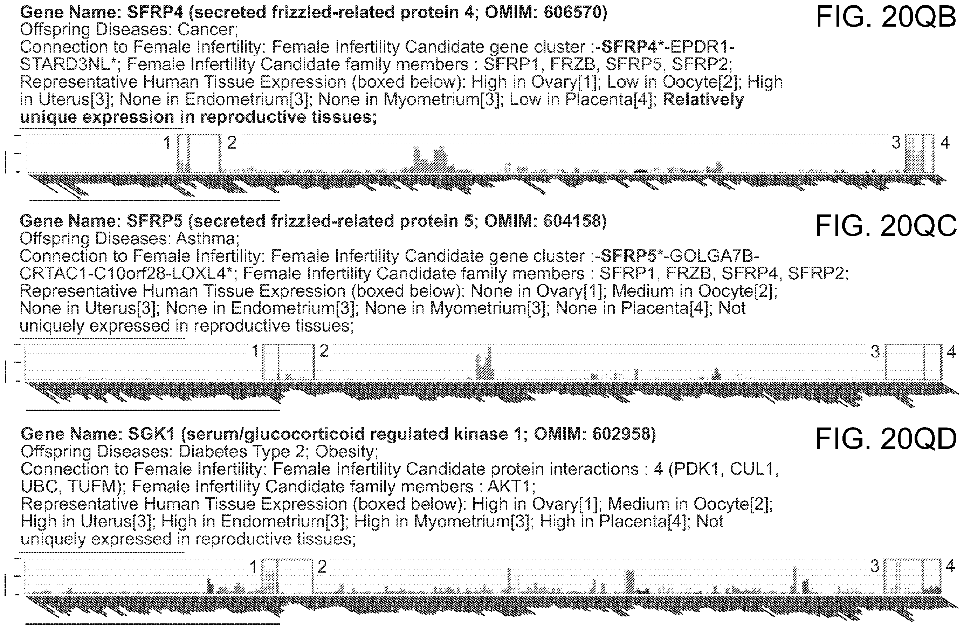

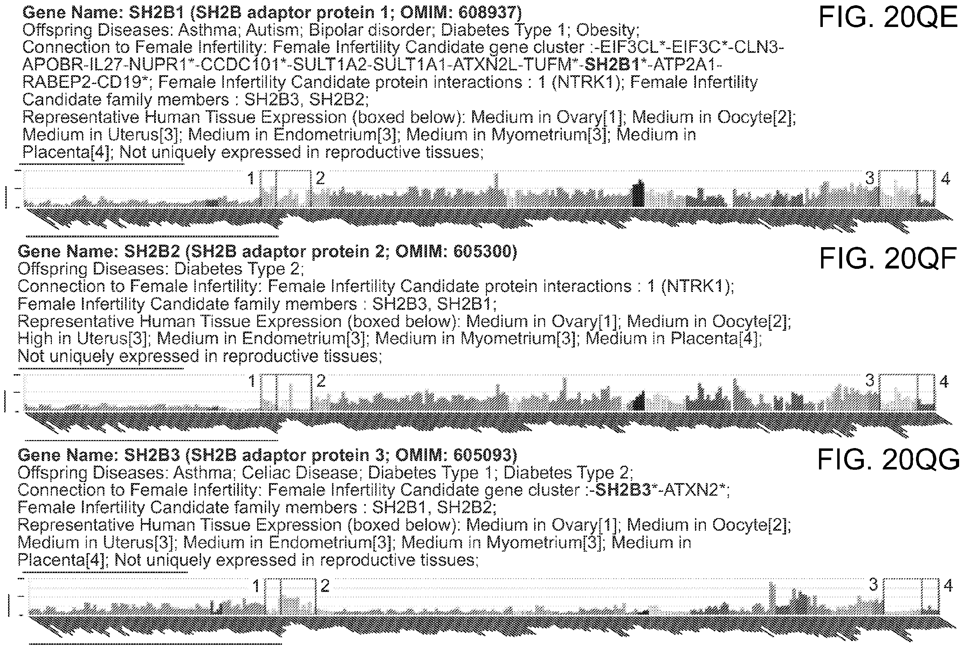

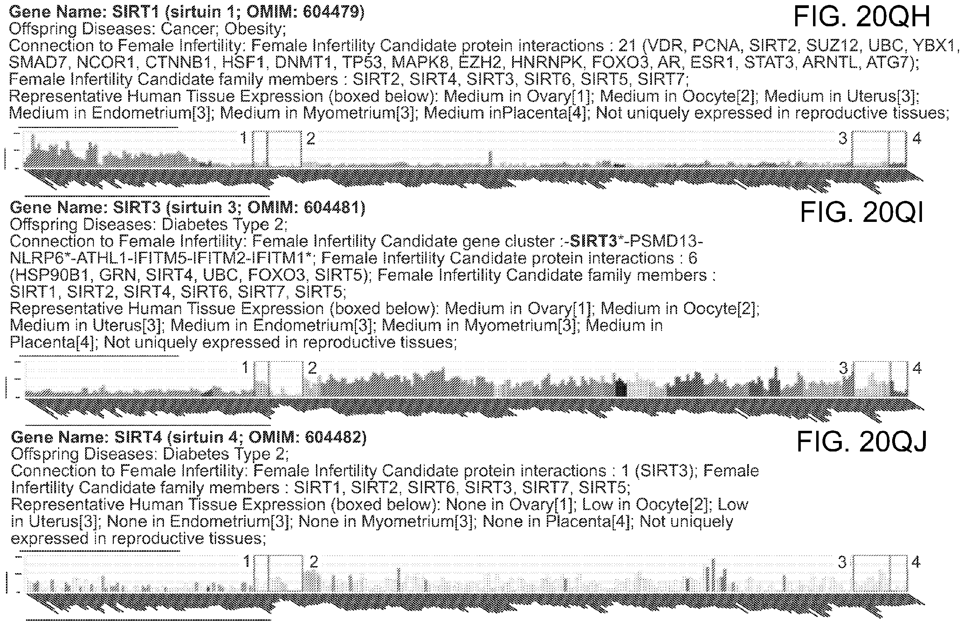

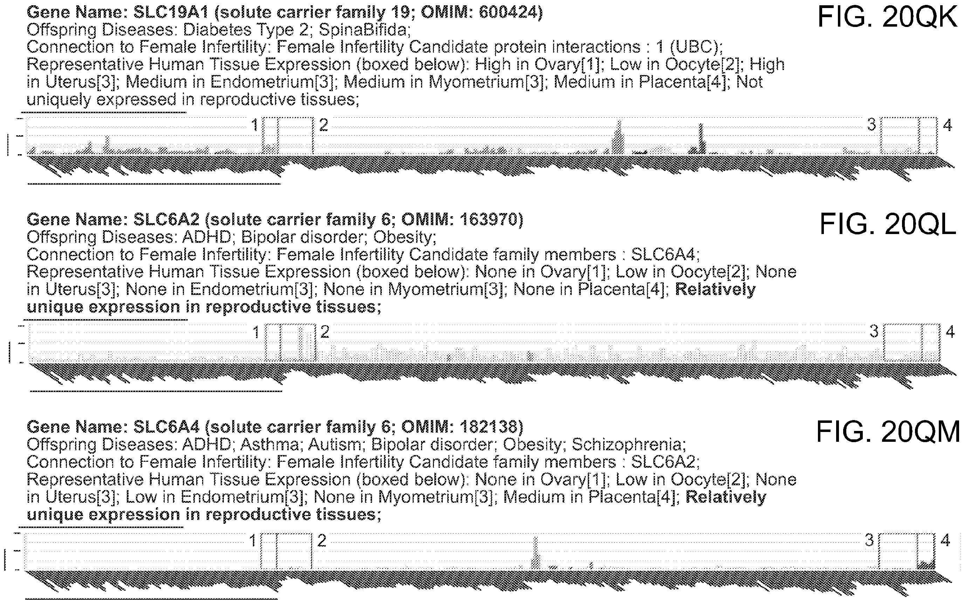

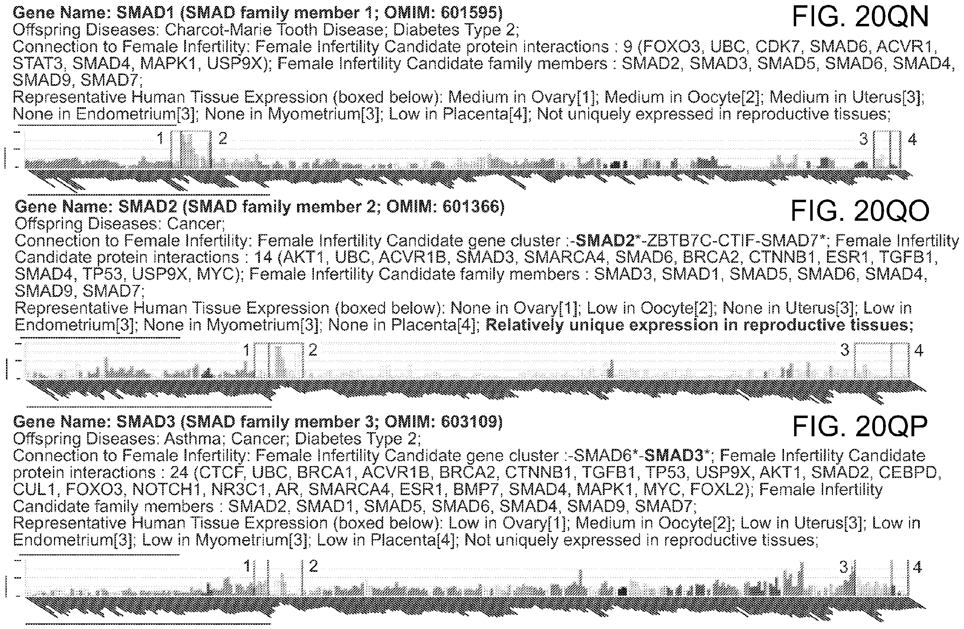

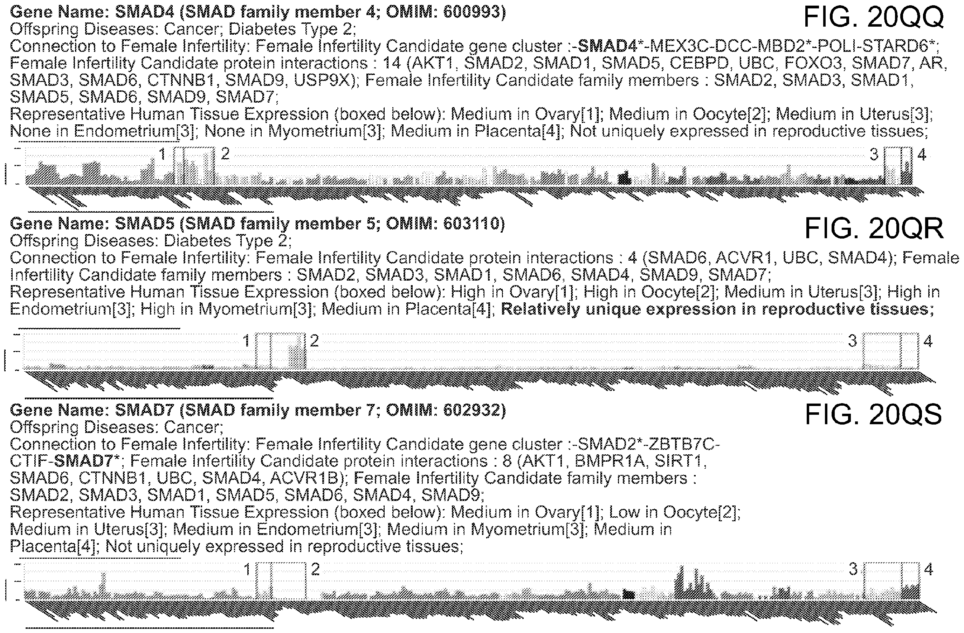

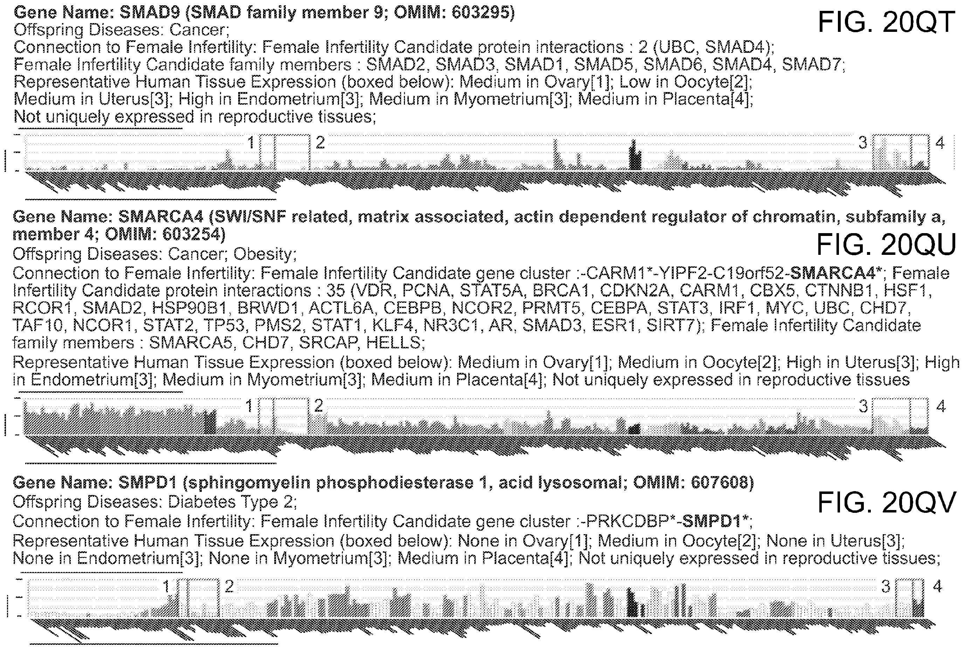

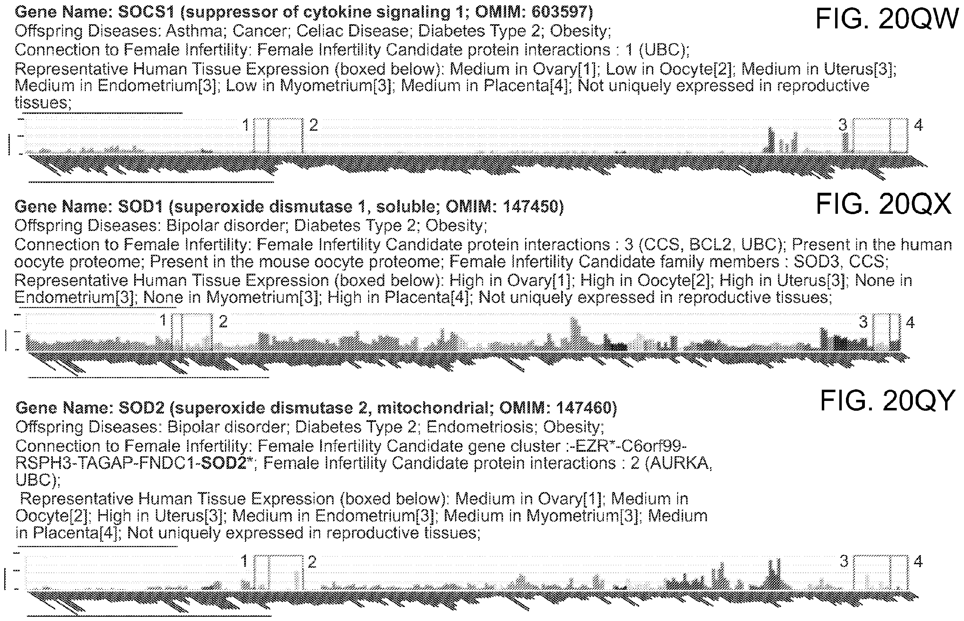

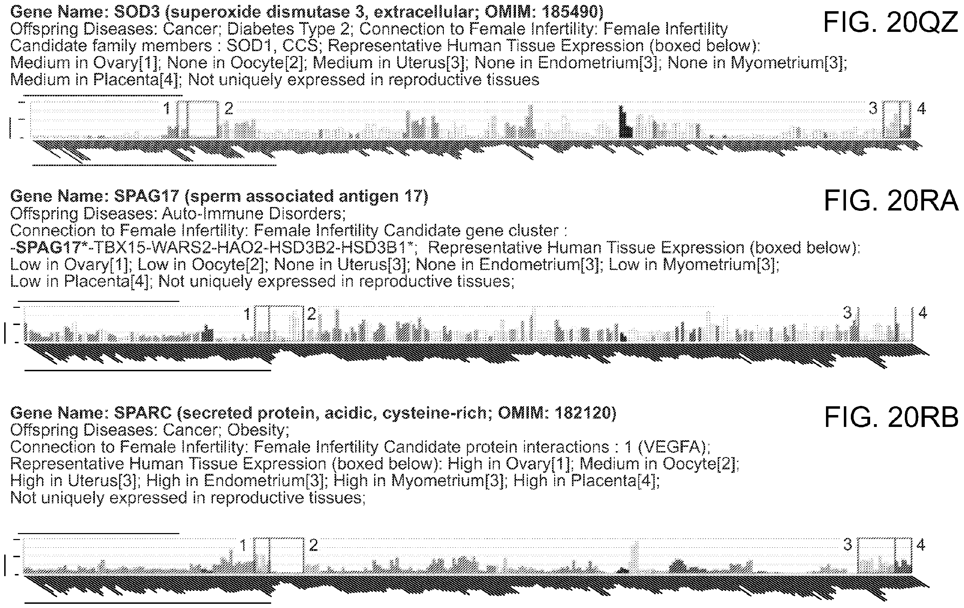

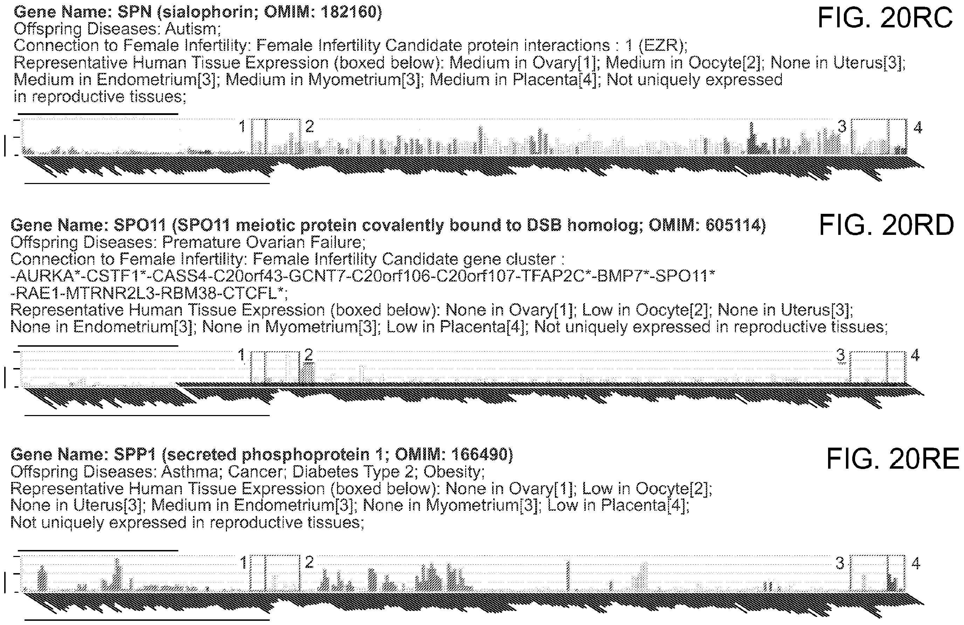

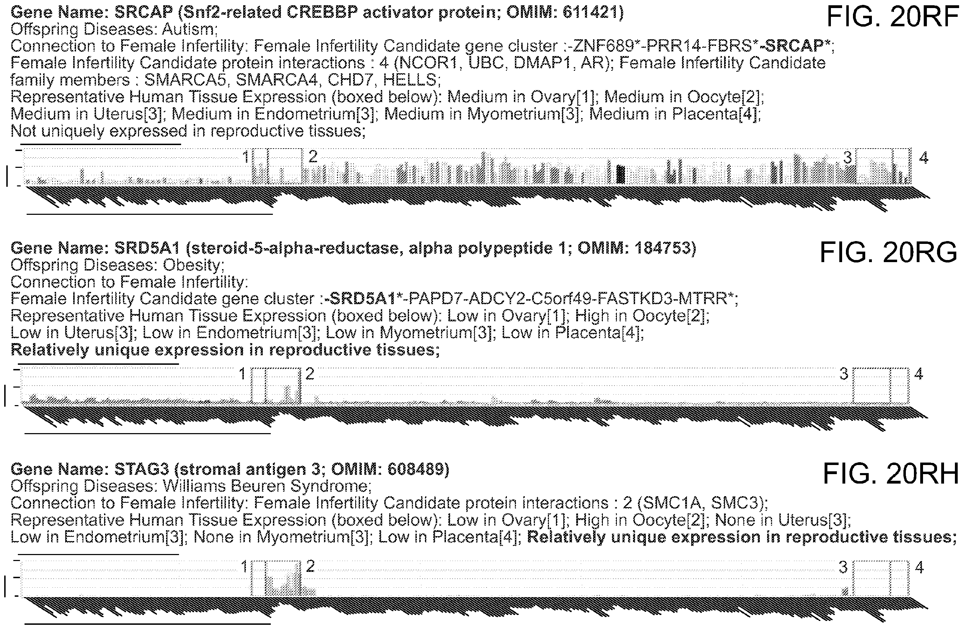

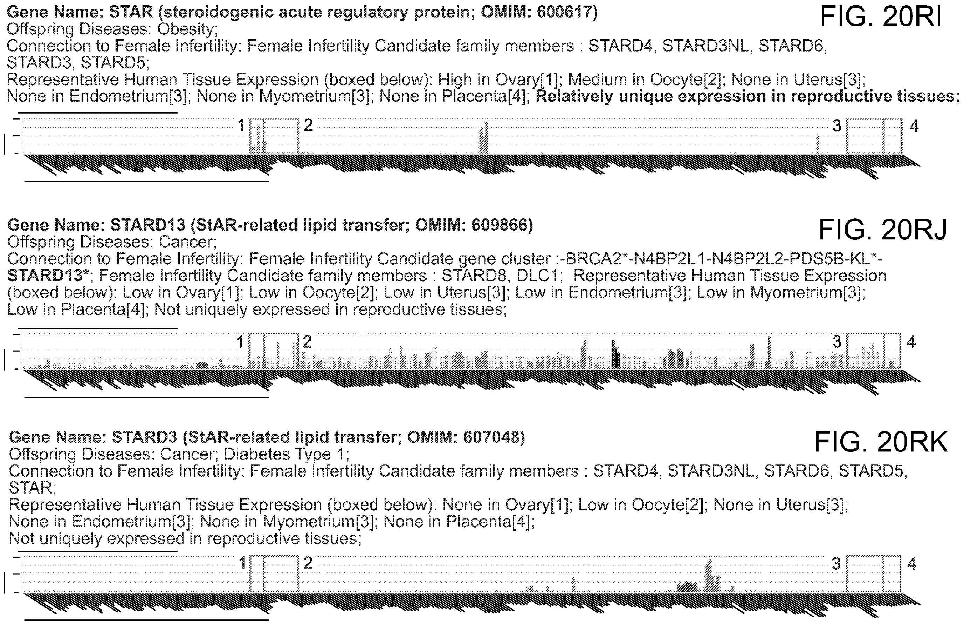

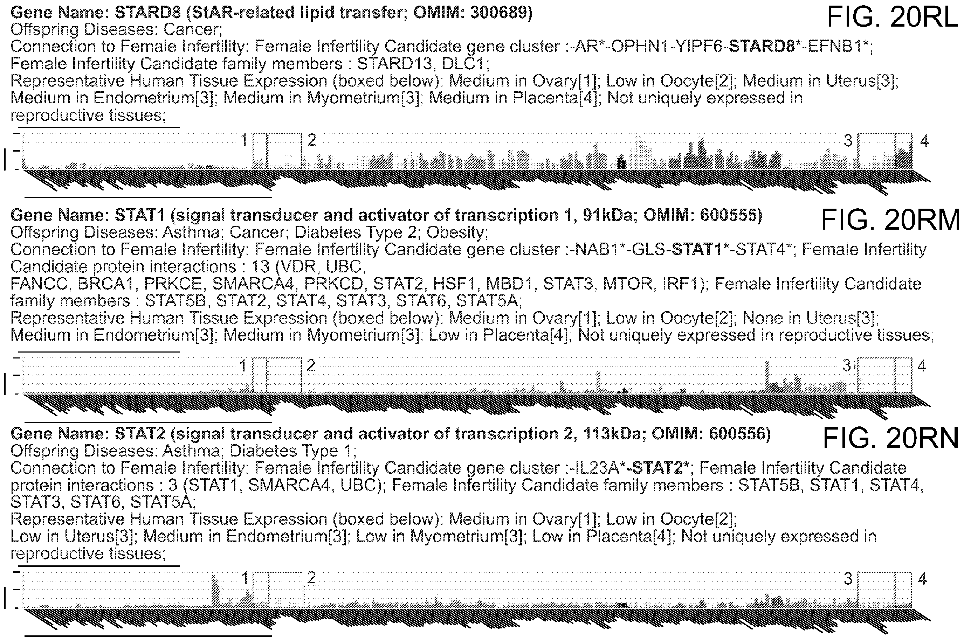

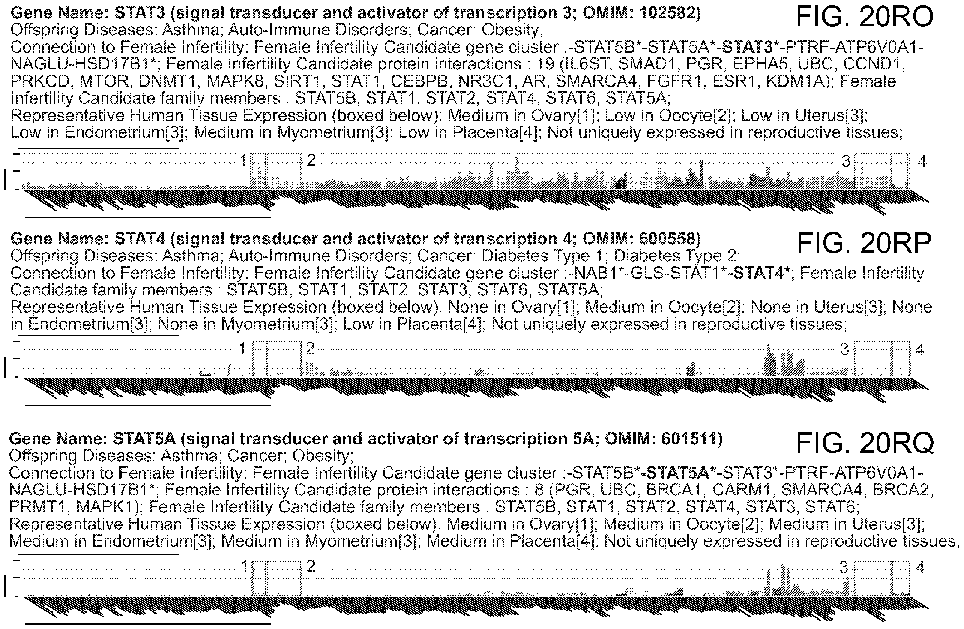

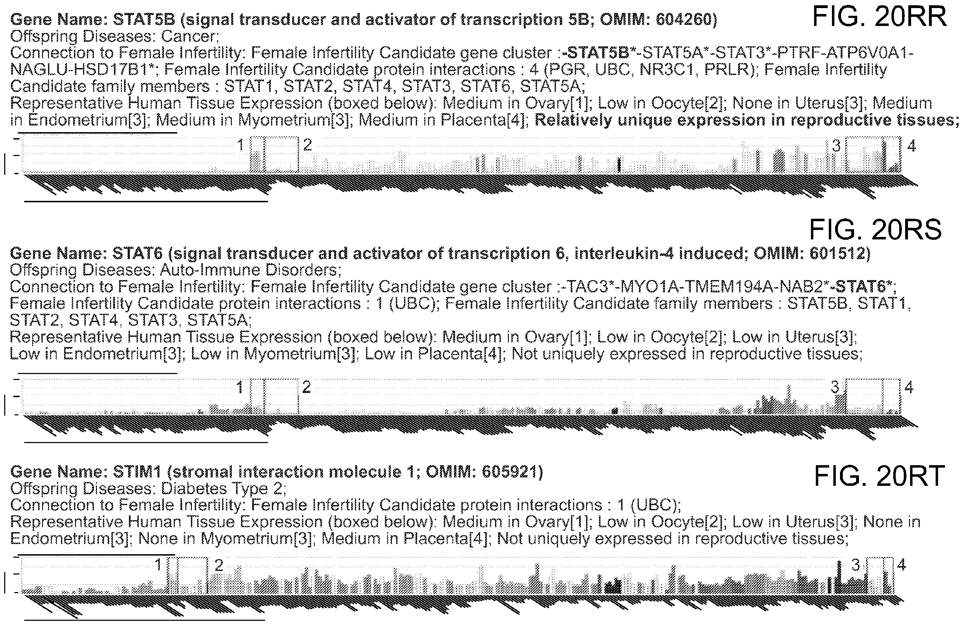

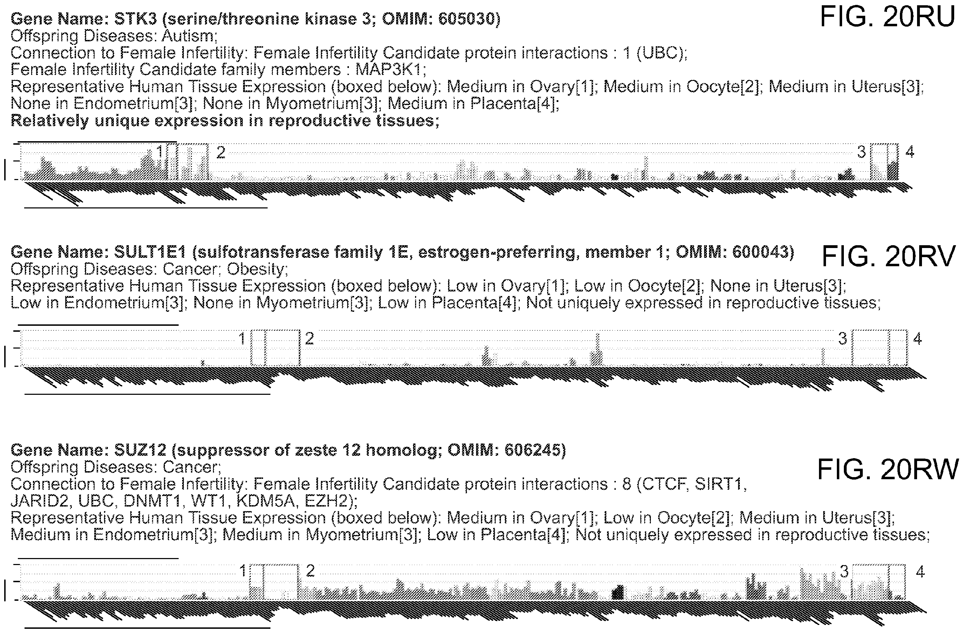

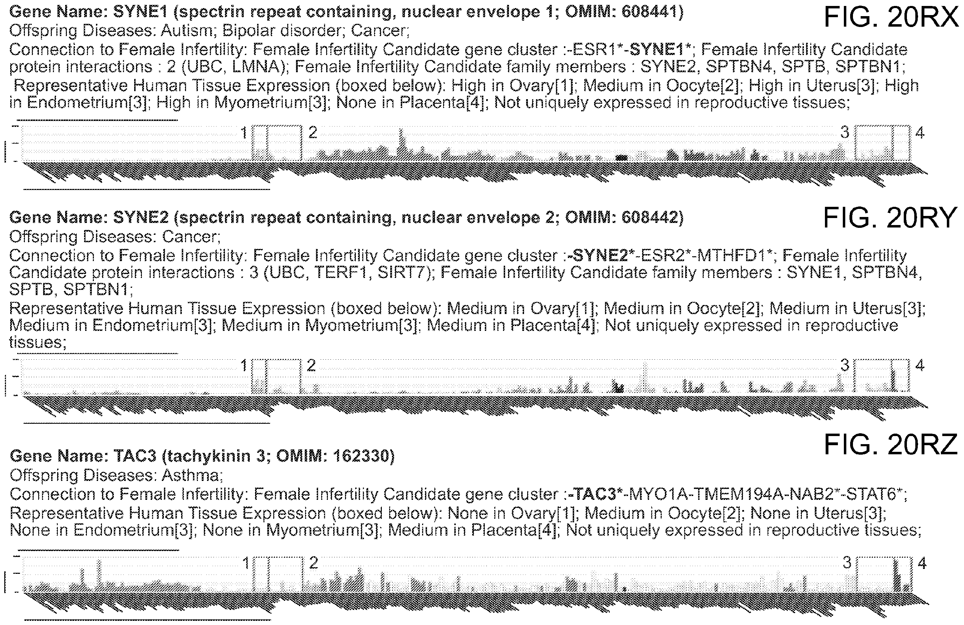

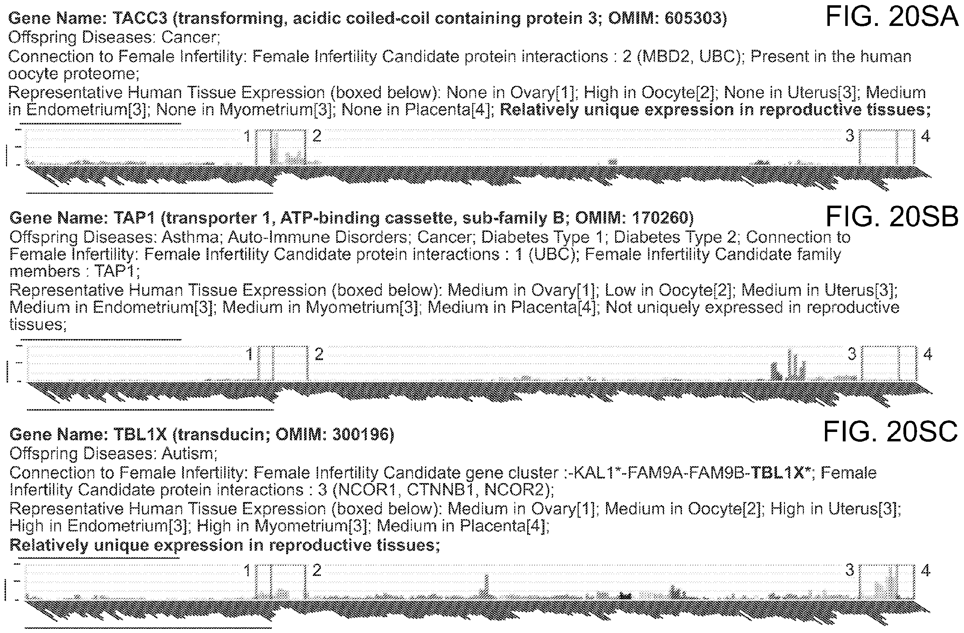

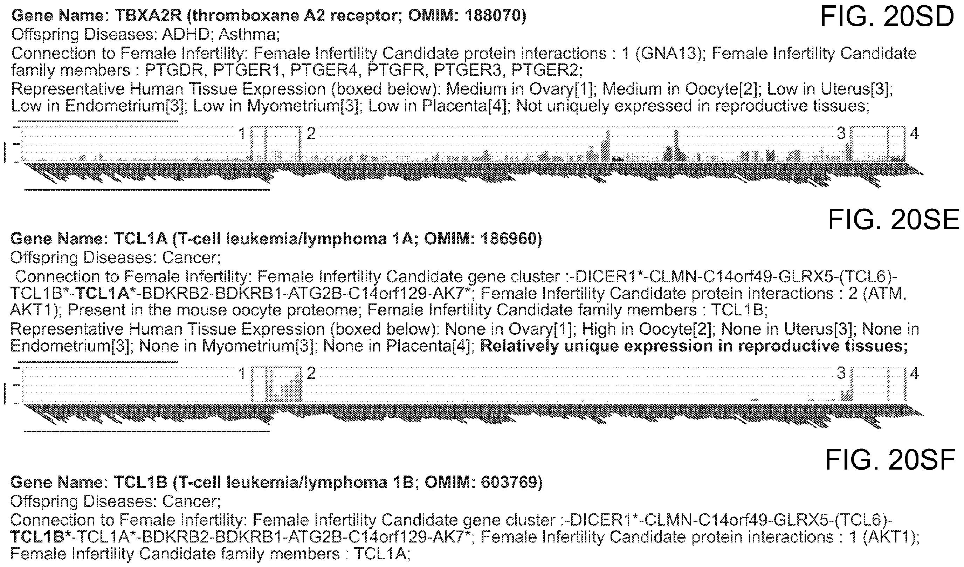

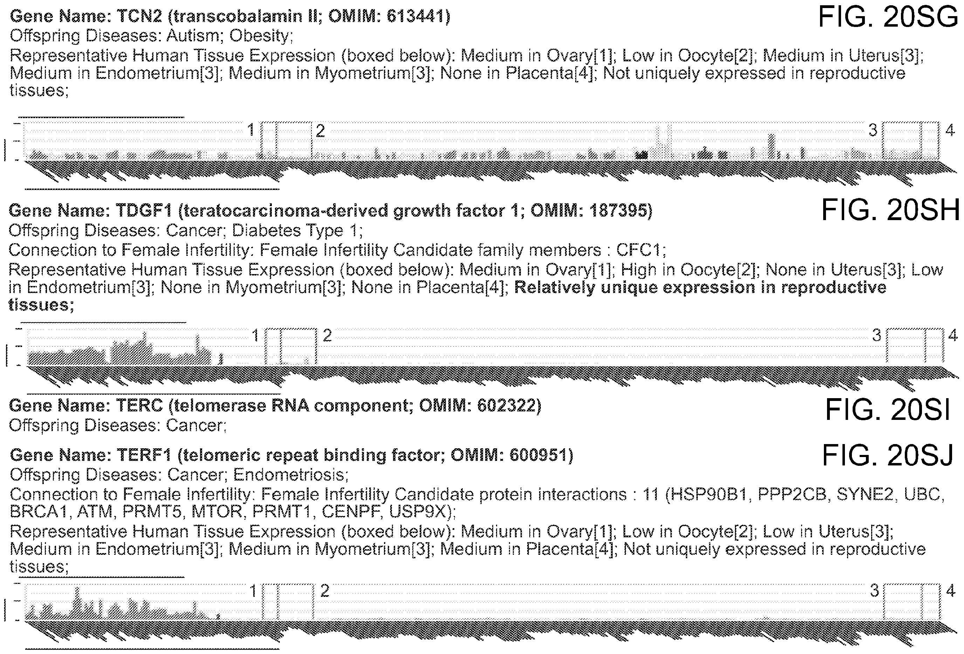

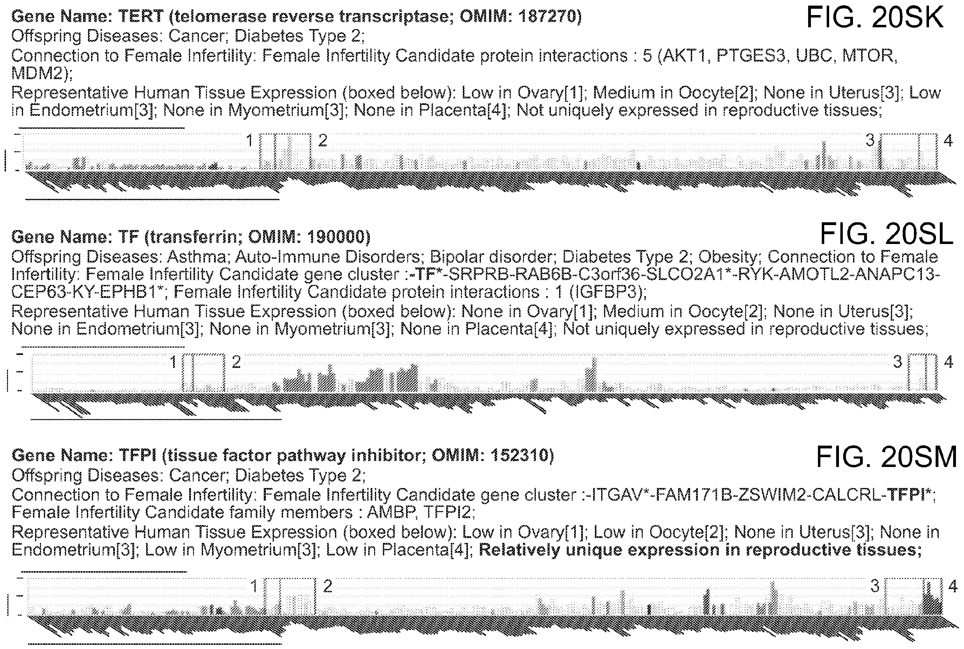

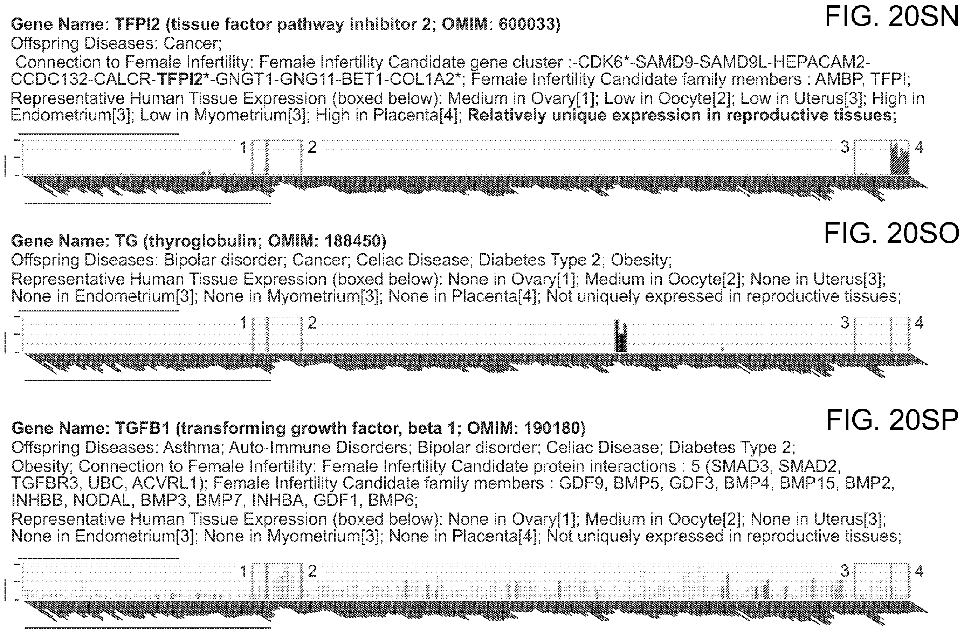

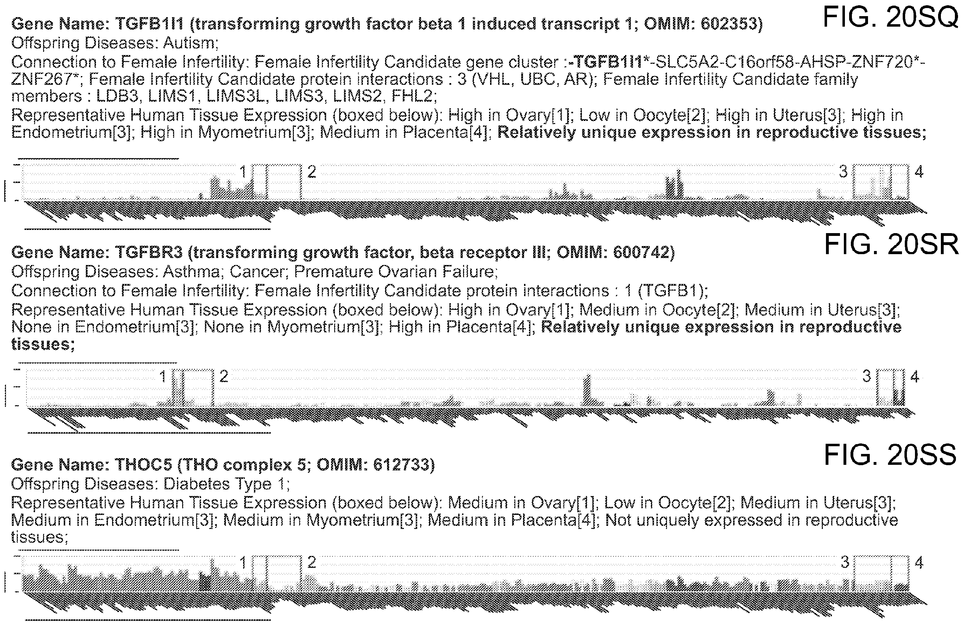

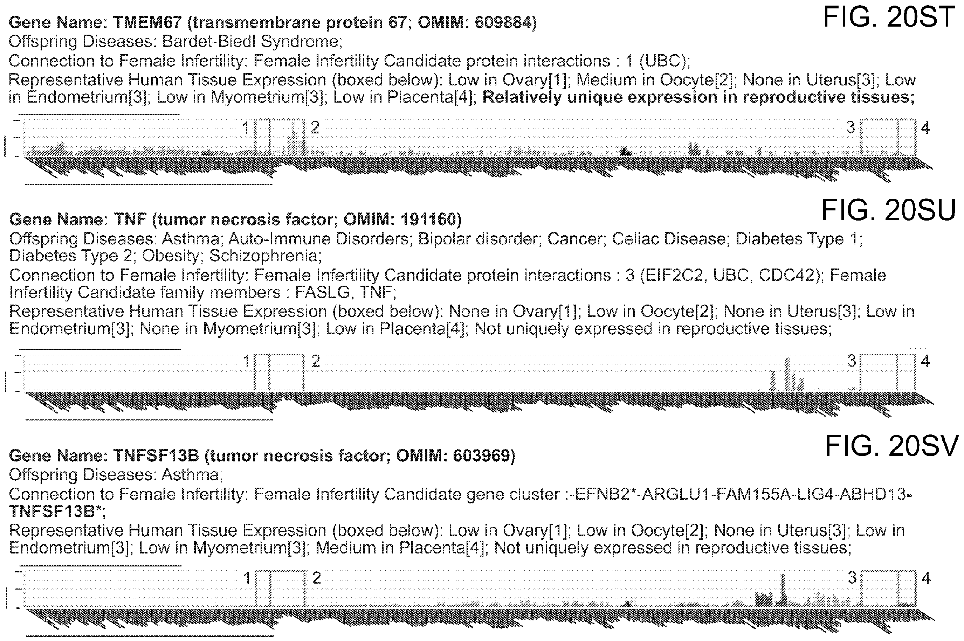

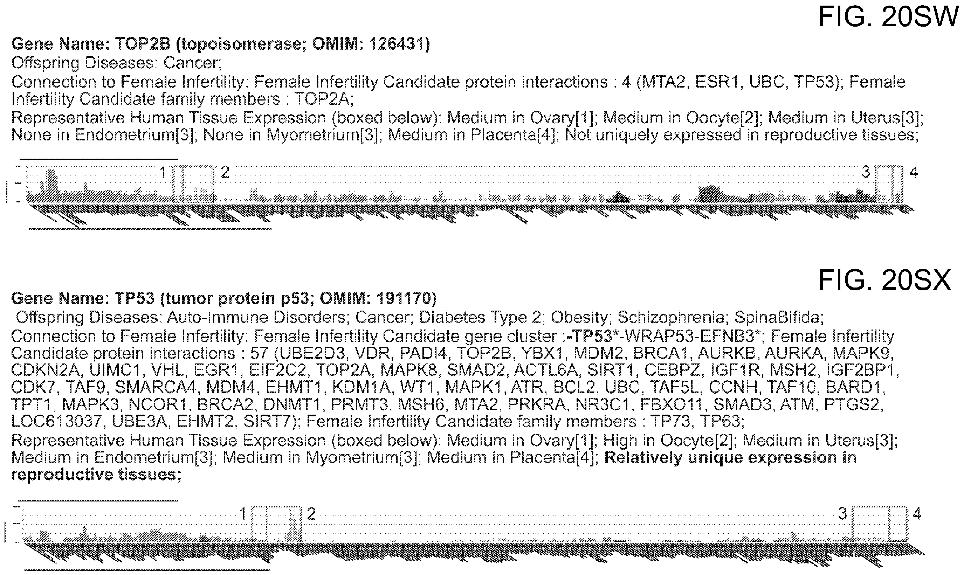

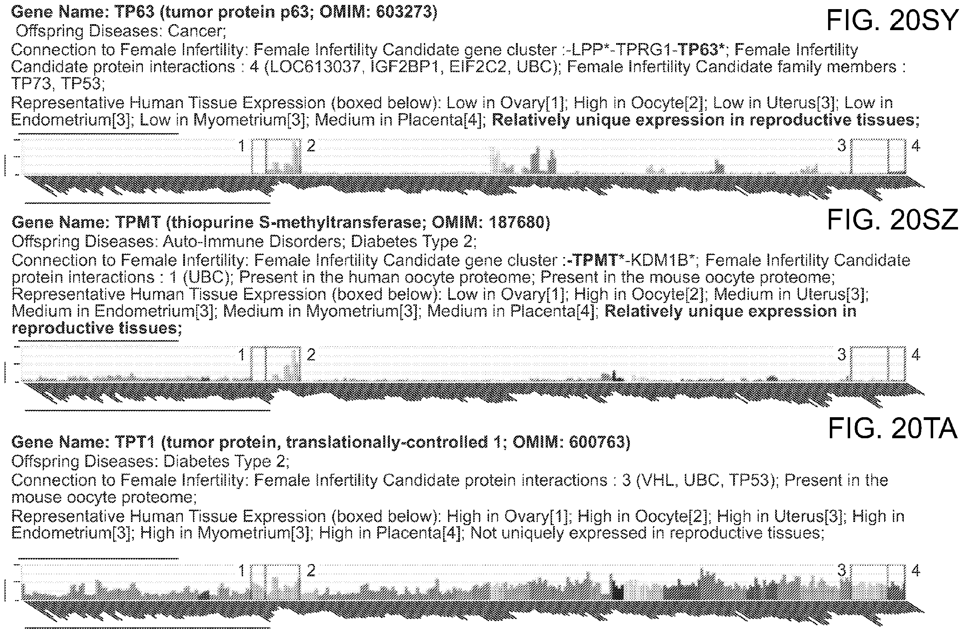

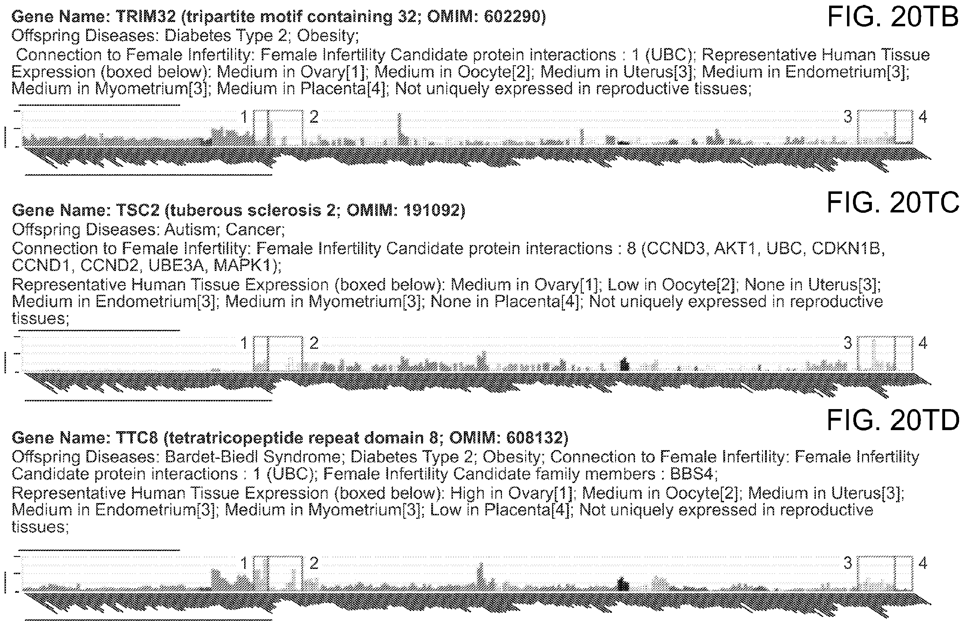

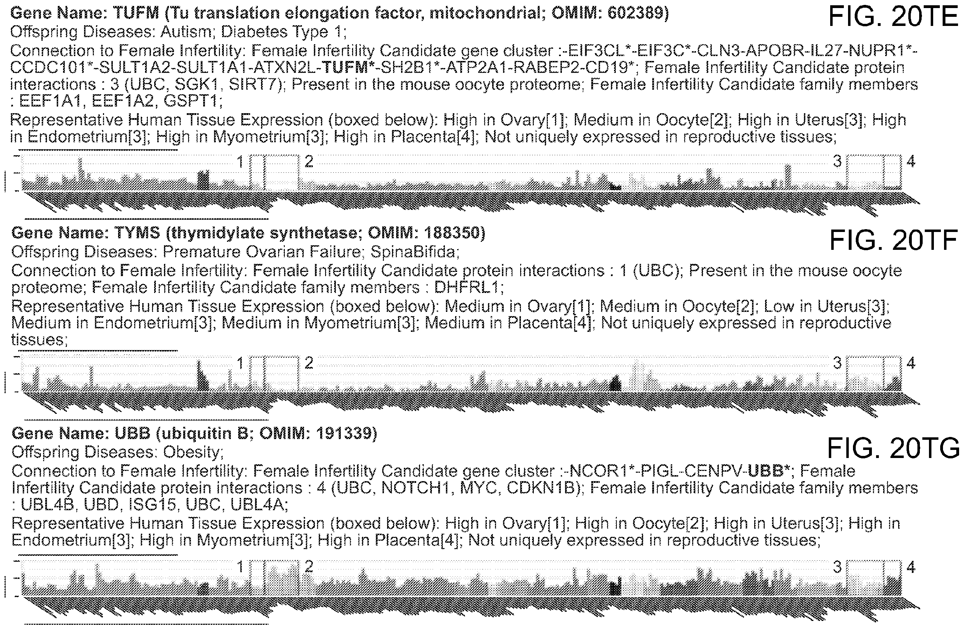

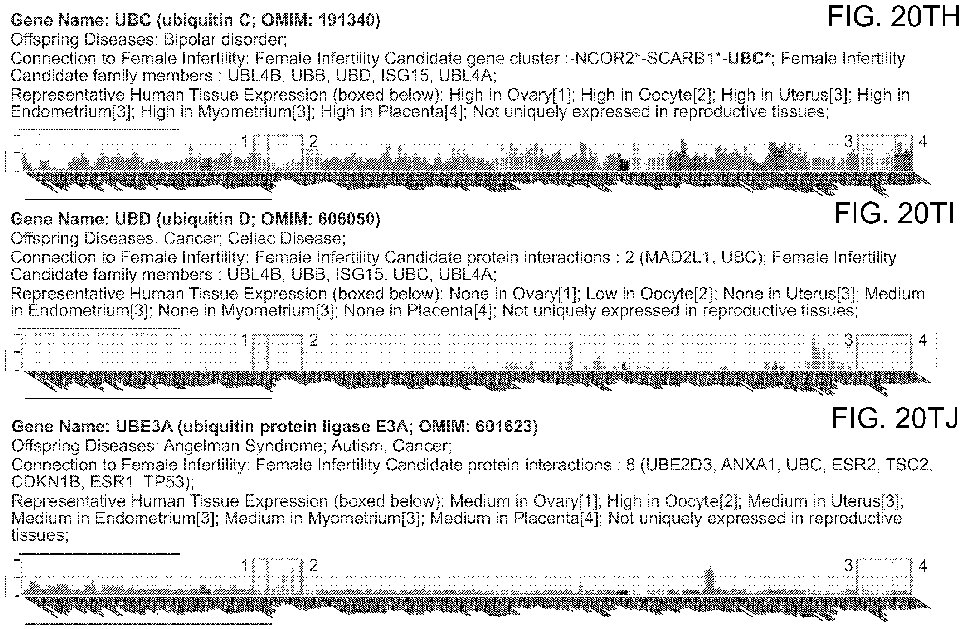

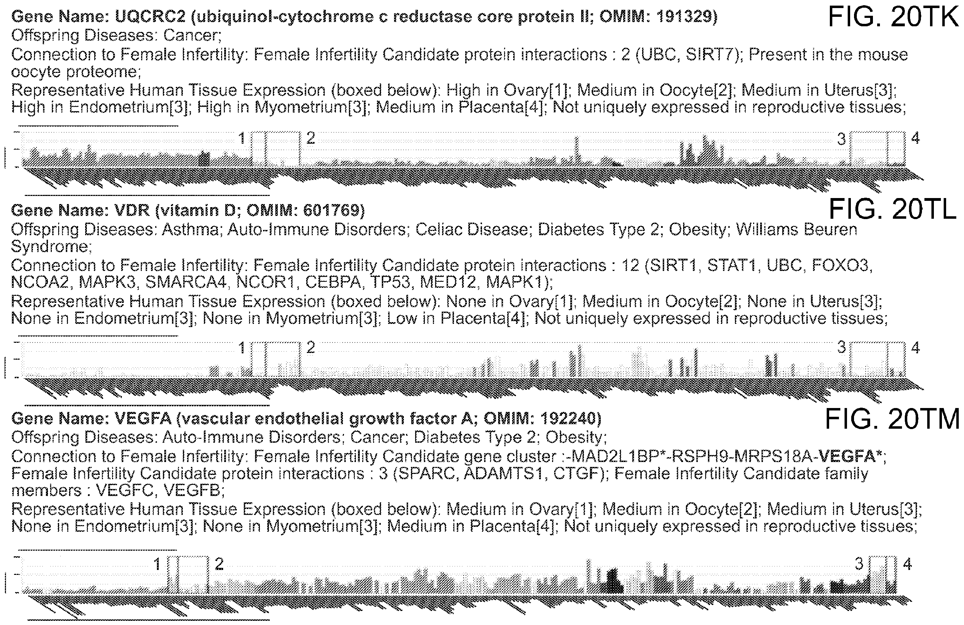

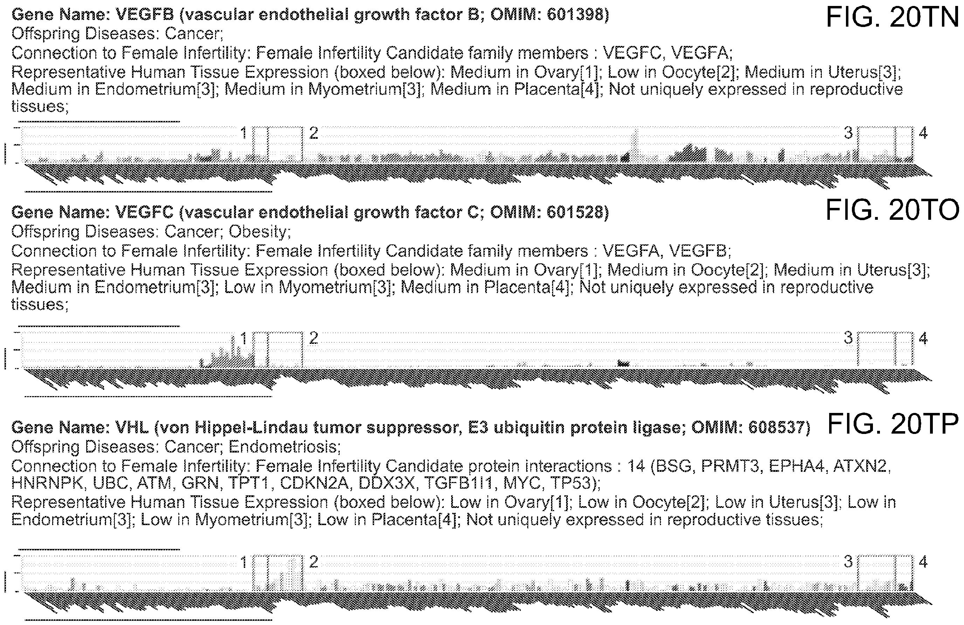

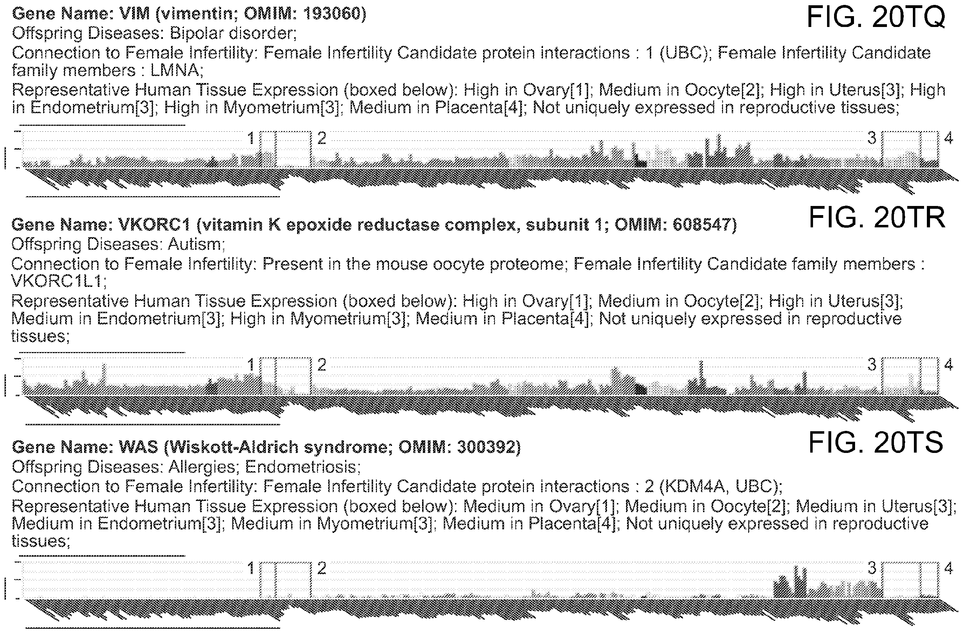

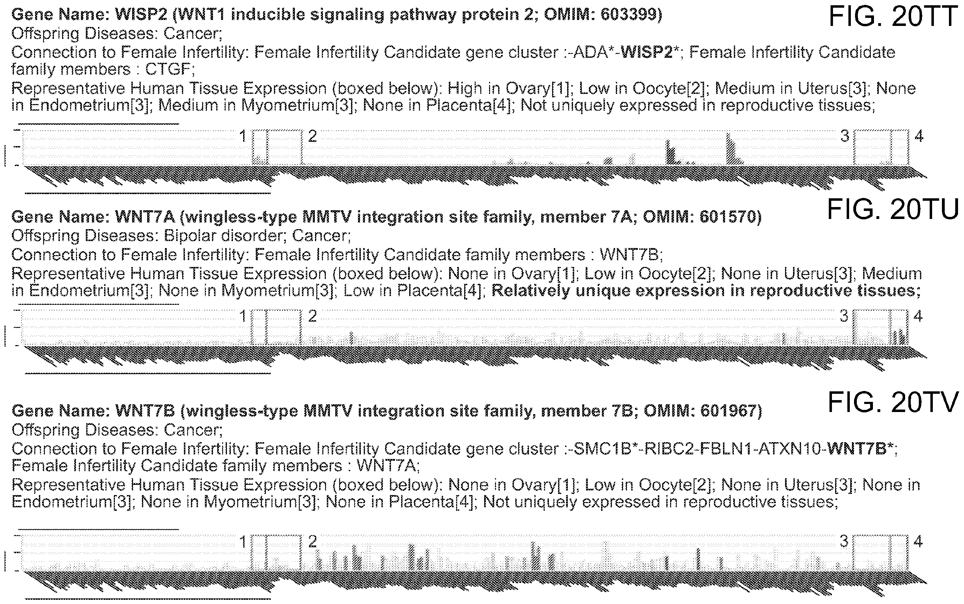

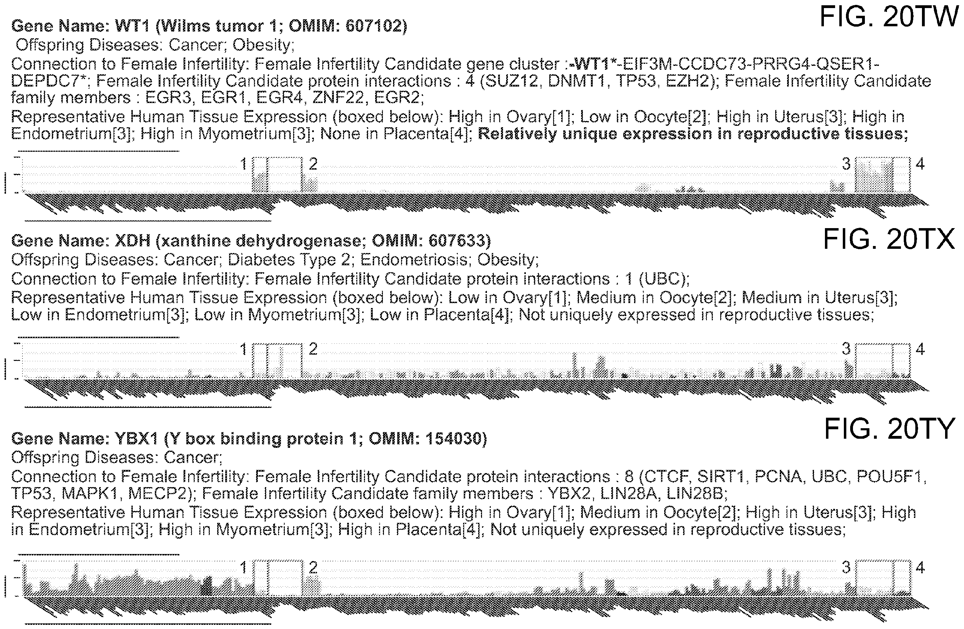

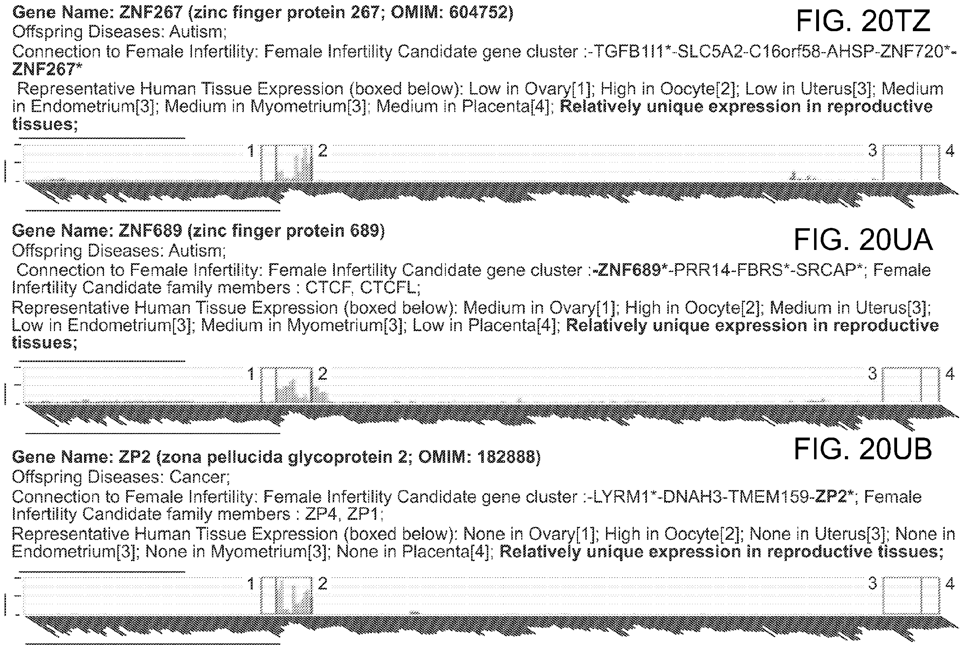

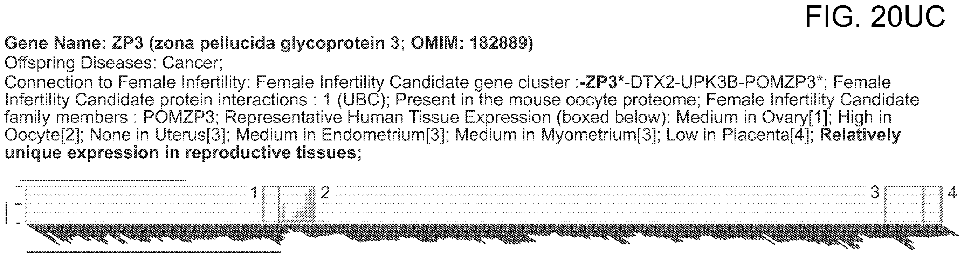

Fig. 20 depicts a series of human genes that, when carrying a certain variant, may give rise to infertility issues, and to offspring diseases if infertility issues are bypassed. Also shown is evidence for each gene's connection to female infertility, including protein interactions with other female infertility candidate genes, genomic clustering with other female infertility genes, and representative human tissue expression. Reproductive tissues are boxed with the following numbers: 1: ovary; 2: oocyte; 3: endometrium, myometrium, and/or uterus; and 4: placenta. Indications of extent of expression (relative to the expression of all genes) in these tissues is indicated by low (below 25^{th} percentile), medium (between 25^{th} -75^{th} percentile), high (above 75^{th} percentile). Histograms are shown with a variable y-axis, so should only be interpreted for relative gene expression across all tissues for a particular gene, not absolute gene expression.

### Detailed Description

The invention generally relates to methods and devices for assessing risk to a putative offspring of developing a condition. In certain embodiments, the invention provides methods for assessing risk to a putative offspring of developing a condition that involve obtaining a maternal sample, conducting an assay on at least one infertility-associated biomarker, and assessing risk to a putative offspring of developing a condition based upon results of the assay.

### Samples

Methods of the invention involve obtaining a sample, e.g., a tissue or body fluid, that is suspected to include an infertility-associated gene or gene product. In particular embodiments, the sample is a maternal sample. The sample may be collected in any clinically acceptable manner. A tissue is a mass of connected cells and/or extracellular matrix material, e.g. skin tissue, hair, nails, endometrial tissue, nasal passage tissue, CNS tissue, neural tissue, eye tissue, liver tissue, kidney tissue, placental tissue, mammary gland tissue, placental tissue, gastrointestinal tissue, musculoskeletal tissue, genitourinary tissue, bone marrow, and the like, derived from, for example, a human or other mammal and includes the connecting material and the liquid material in association with the cells and/or tissues. A body fluid is a liquid material derived from, for example, a human or other mammal. Such body fluids include, but are not limited to, mucous, blood, plasma, serum, serum derivatives, bile, blood, maternal blood, phlegm, saliva, sweat, amniotic fluid, menstrual fluid, endometrial aspirates, mammary fluid, follicular fluid of the ovary, fallopian tube fluid, peritoneal fluid, urine, and cerebrospinal fluid (CSF), such as lumbar or ventricular CSF. A sample may also be a fine needle aspirate or biopsied tissue. A sample also may be media containing cells or biological material. A sample may also be a blood clot, for example, a blood clot that has been obtained from whole blood after the serum has been removed. In certain embodiments, infertility-associated genes or gene products may be found in reproductive cells or tissues, such as gametic cells, gonadal tissue, fertilized embryos, and placenta. In certain embodiments, the sample is drawn maternal blood or saliva.

Nucleic acid is extracted from the sample according to methods known in the art. See for example, Maniatis, et al., Molecular Cloning: A Laboratory Manual, Cold Spring Harbor, N.Y., pp. 280-281, 1982, the contents of which are incorporated by reference herein in their entirety. In certain embodiments, a genomic sample is collected from a subject followed by enrichment for genetic regions or genetic fragments of interest, for example by hybridization to a nucleotide array comprising fertility-related genes or gene fragments of interest. The sample may be enriched for genes of interest (e.g., infertility-associated genes) using methods known in the art, such as hybrid capture. See for examples, Lapidus (U.S. patent number 7,666,593), the content of which is incorporated by reference herein in its entirety.

RNA may be isolated from eukaryotic cells by procedures that involve lysis of the cells and denaturation of the proteins contained therein. Tissue of interest includes gametic cells, gonadal tissue, endometrial tissue, fertilized embryos, and placenta. RNA may be isolated from fluids of interest by procedures that involve denaturation of the proteins contained therein. Fluids of interest include blood, menstrual fluid, mammary fluid, follicular fluid of the ovary, peritoneal fluid, or culture medium. Additional steps may be employed to remove DNA. Cell lysis may be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (Chirgwin et al., Biochemistry 18:5294-5299 (1979)). Poly(A)+ RNA is selected by selection with oligo-dT cellulose (see Sambrook et al., MOLECULAR CLONING--A LABORATORY MANUAL (2ND ED.), Vols. 1-3, Cold Spring Harbor Laboratory, Cold Spring Harbor, N.Y. (1989). Alternatively, separation of RNA from DNA can be accomplished by organic extraction, for example, with hot phenol or phenol/chloroform/isoamyl alcohol. If desired, RNase inhibitors may be added to the lysis buffer. Likewise, for certain cell types, it may be desirable to add a protein denaturation/digestion step to the protocol.

For many applications, it is desirable to preferentially enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or SEPHADEX (see Ausubel et al., CURRENT PROTOCOLS IN MOLECULAR BIOLOGY, vol. 2, Current Protocols Publishing, New York (1994). Once bound, poly(A)+ mRNA is eluted from the affinity column using 2 mM EDTA/0.1% SDS.

### Biomarkers

A biomarker generally refers to a molecule that may act as an indicator of a biological state. Biomarkers for use with methods of the invention may be any marker that is associated with infertility. Exemplary biomarkers include genes (e.g. any region of DNA encoding a functional product), genetic regions (e.g. regions including genes and intergenic regions with a particular focus on regions conserved throughout evolution in placental mammals), and gene products (e.g., RNA and protein). In certain embodiments, the biomarker is an infertility-associated genetic region. An infertility-associated genetic region is any DNA sequence in which variation is associated with a change in fertility. Examples of changes in fertility include, but are not limited to, the following: a homozygous mutation of an infertility-associated gene leads to a complete loss of fertility; a homozygous mutation of an infertility-associated gene is incompletely penetrant and leads to reduction in fertility that varies from individual to individual; a heterozygous mutation is completely recessive, having no effect on fertility; and the infertility-associated gene is X-linked, such that a potential defect in fertility depends on whether a non-functional allele of the gene is located on an inactive X chromosome (Barr body) or on an expressed X chromosome.

In particular embodiments, the infertility-associated genetic region is a maternal effect gene. Maternal effects genes are genes that have been found to encode key structures and functions in mammalian oocytes (Yurttas et al., Reproduction 139:809-823, 2010). Maternal effect genes are described, for example in, Christians et al. (Mol Cell Biol 17:778-88, 1997); Christians et al., Nature 407:693-694, 2000); Xiao et al. (EMBO J 18:5943-5952, 1999); Tong et al. (Endocrinology 145:1427-1434, 2004); Tong et al. (Nat Genet 26:267-268, 2000); Tong et al. (Endocrinology, 140:3720-3726, 1999); Tong et al. (Hum Reprod 17:903-911, 2002); Ohsugi et al. (Development 135:259-269, 2008); Borowczyk et al. (Proc Natl Acad Sci U S A., 2009); and Wu (Hum Reprod 24:415-424, 2009). The content of each of these is incorporated by reference herein in its entirety.

In particular embodiments, the infertility-associated genetic region is a gene (including exons, introns, and 10 kb of DNA flanking either side of said gene) selected from the genes shown in Table 1 below. In Table 1, OMIM reference numbers are provided when available.

| **Table 1 - Human Infertility-Related Genes (OMIM #)** | | | |
|---|---|---|---|
| ABCA1 (600046) | ACTL6A (604958) | ACTL8 | ACVR1 (102576) |
| ACVR1B (601300) | ACVR1C (608981) | ACVR2(102581) | ACVR2A (102581) |
| ACVR2B (602730) | ACVRL1 (601284) | ADA (608958) | ADAMTS1 (605174) |
| ADM (103275) | ADM2 (608682) | AFF2 (300806) | AGT (106150) |
| AHR (600253) | AIRE (607358) | AK2 (103020) | AK7 |
| AKR1C1 (600449) | AKR1C2 (600450) | AKR1C3 (603966) | AKR1C4 (600451) |
| AKT1 (164730) | ALDOA (103850) | ALDOB (612724) | ALDOC (103870) |
| ALPL (171760) | AMBP (176870) | AMD1 (180980) | AMH (600957) |
| AMHR2 (600956) | ANK3 (600465) | ANXA1 (151690) | APC (611731) |
| APOA1 (107680) | APOE (107741) | AQP4 (600308) | AR (313700) |
| AREG (104640) | ARF1 (103180) | ARF3 (103190) | ARF4 (601177) |
| ARF5 (103188) | ARFRP1 (604699) | ARL1 (603425) | ARL10 (612405) |
| ARL11 (609351) | ARL13A | ARL13B (608922) | ARL15 |
| ARL2 (601175) | ARL3 (604695) | ARL4A (604786) | ARL4C (604787) |
| ARL4D (600732) | ARL5A (608960) | ARL5B (608909) | ARL5C |
| ARL6 (608845) | ARL8A | ARL8B | ARMC2 |
| ARNTL (602550) | ASCL2 (601886) | ATF7IP (613644) | ATG7 (608760) |
| ATM (607585) | ATR (601215) | ATXN2 (601517) | AURKA (603072) |
| AURKB (604970) | AUTS2 (607270) | BARD1 (601593) | BAX (600040) |
| BBS1 (209901) | BBS10 (610148) | BBS12 (610683) | BBS2 (606151) |
| BBS4 (600374) | BBS5 (603650) | BBS7 (607590) | BBS9 (607968) |
| BCL2 (151430) | BCL2L1 (600039) | BCL2L10 (606910) | BDNF (113505) |
| BECN1 (604378) | BHMT (602888) | BLVRB (600941) | BMP15 (300247) |
| BMP2 (112261) | BMP3 (112263) | BMP4 (112262) | BMP5 (112265) |
| BMP6 (112266) | BMP7 (112267) | BMPR1A (601299) | BMPR1B (603248) |
| BMPR2 (600799) | BNC1 (601930) | BOP1 (610596) | BRCA1 (113705) |
| BRCA2 (600185) | BRIP1 (605882) | BRSK1 (609235) | BRWD1 |
| BSG (109480) | BTG4 (605673) | BUB1 (602452) | BUB1B (602860) |
| C2orf86 (613580) | C3 (120700) | C3orf56 | C6orf221 (611687) |
| CA1 (114800) | CARD8 (609051) | CARM1 (603934) | CASP1 (147678) |
| CASP2 (600639) | CASP5 (602665) | CASP6 (601532) | CASP8 (601763) |
| CBS (613381) | CBX1 (604511) | CBX2 (602770) | CBX5 (604478) |
| CCDC101 (613374) | CCDC28B (610162) | CCL13 (601391) | CCL14 (601392) |
| CCL4 (182284) | CCL5 (187011) | CCL8 (602283) | CCND1 (168461) |
| CCND2 (123833) | CCND3 (123834) | CCNH (601953) | CCS (603864) |
| CD19 (107265) | CD24 (600074) | CD55 (125240) | CD81 (186845) |
| CD9 (143030) | CDC42 (116952) | CDK4 (123829) | CDK6 (603368) |
| CDK7 (601955) | CDKN1B (600778) | CDKN1C (600856) | CDKN2A (600160) |
| CDX2 (600297) | CDX4 (300025) | CEACAM20 | CEBPA (116897) |
| CEBPB (189965) | CEBPD (116898) | CEBPE (600749) | CEBPG (138972) |
| CEBPZ (612828) | CELF1 (601074) | CELF4 (612679) | CENPB (117140) |
| CENPF (600236) | CENPI (300065) | CEP290 (610142) | CFC1 (605194) |
| CGA (118850) | CGB (118860) | CGB1 (608823) | CGB2 (608824) |
| CGB5 (608825) | CHD7 (608892) | CHST2 (603798) | CLDN3 (602910) |
| COIL (600272) | COL1A2 (120160) | COL4A3BP (604677) | COMT (116790) |
| COPE (606942) | COX2 (600262) | CP (117700) | CPEB1 (607342) |
| CRHR1 (122561) | CRYBB2 (123620) | CSF1 (120420) | CSF2 (138960) |
| CSTF1 (600369) | CSTF2 (600368) | CTCF (604167) | CTCFL (607022) |
| CTF2P | CTGF (121009) | CTH (607657) | CTNNB1 (116806) |
| CUL1 (603134) | CX3CL1 (601880) | CXCL10 (147310) | CXCL9 (601704) |
| CXorf67 | CYP11A1 (118485) | CYP11B1 (610613) | CYP11B2 (124080) |
| CYP17A1 (609300) | CYP19A1 (107910) | CYP1A1 (108330) | CYP27B1 (609506) |
| DAZ2 (400026) | DAZL (601486) | DCTPP1 | DDIT3 (126337) |
| DDX11 (601150) | DDX20 (606168) | DDX3X (300160) | DDX43 (606286) |
| DEPDC7 (612294) | DHFR (126060) | DHFRL1 | DIAPH2 (300108) |
| DICER1 (606241) | DKK1 (605189) | DLC1 (604258) | DLGAP5 |
| DMAP1 (605077) | DMC1 (602721) | DNAJB1 (604572) | DNMT1 (126375) |
| DNMT3B (602900) | DPPA3 (608408) | DPPA5 (611111) | DPYD (612779) |
| DTNBP1 (607145) | DYNLL1 (601562) | ECHS1 (602292) | EEF1A1 (130590) |
| EEF1A2 (602959) | EFNA1 (191164) | EFNA2 (602756) | EFNA3 (601381) |
| EFNA4 (601380) | EFNA5 (601535) | EFNB1 (300035) | EFNB2 (600527) |
| EFNB3 (602297) | EGR1 (128990) | EGR2 (129010) | EGR3 (602419) |
| EGR4 (128992) | EHMT1 (607001) | EHMT2 (604599) | EIF2B2 (606454) |
| EIF2B4 (606687) | EIF2B5 (603945) | EIF2C2 (606229) | EIF3C (603916) |
| EIF3CL (603916) | EPHA1 (179610) | EPHA10 (611123) | EPHA2 (176946) |
| EPHA3 (179611) | EPHA4 (602188) | EPHA5 (600004) | EPHA6 (600066) |
| EPHA7 (602190) | EPHA8 (176945) | EPHB1 (600600) | EPHB2 (600997) |
| EPHB3 (601839) | EPHB4 (600011) | EPHB6 (602757) | ERCC1 (126380) |
| ERCC2 (126340) | EREG (602061) | ESR1 (133430) | ESR2 (601663) |
| ESR2 (601663) | ESRRB (602167) | ETV5 (601600) | EZH2 (601573) |
| EZR (123900) | FANCC (613899) | FANCG (602956) | FANCL (608111) |
| FAR1 | FAR2 | FASLG (134638) | FBN1 (134797) |
| FBN2 (612570) | FBN3 (608529) | FBRS (608601) | FBRSL1 |
| FBXO10 (609092) | FBXO11 (607871) | FCRL3 (606510) | FDXR (103270) |
| FGF23 (605380) | FGF8 (600483) | FGFBP1 (607737) | FGFBP3 |
| FGFR1 (136350) | FHL2 (602633) | FIGLA (608697) | FILIP1L (612993) |
| FKBP4 (600611) | FMN2 (606373) | FMR1 (309550) | FOLR1 (136430) |
| FOLR2 (136425) | FOXE1 (602617) | FOXL2 (605597) | FOXN1 (600838) |
| FOXO3 (602681) | FOXP3 (300292) | FRZB (605083) | FSHB (136530) |
| FSHR (136435) | FST (136470) | GALT (606999) | GBP5 (611467) |
| GCK (138079) | GDF1 (602880) | GDF3 (606522) | GDF9 (601918) |
| GGT1 (612346) | GJA1 (121014) | GJA10 (611924) | GJA3 (121015) |
| GJA4 (121012) | GJA5 (121013) | GJA8 (600897) | GJB1 (304040) |
| GJB2 (121011) | GJB3 (603324) | GJB4 (605425) | GJB6 (604418) |
| GJB7 (611921) | GJC1 (608655) | GJC2 (608803) | GJC3 (611925) |
| GJD2 (607058) | GJD3 (607425) | GJD4 (611922) | GNA13 (604406) |
| GNB2 (139390) | GNRH1 (152760) | GNRH2 (602352) | GNRHR (138850) |
| GPC3 (300037) | GPRC5A (604138) | GPRC5B (605948) | GREM2 (608832) |
| GRN (138945) | GSPT1 (139259) | GSTA1 (138359) | H19 (103280) |
| H1FOO (142709) | HABP2 (603924) | HADHA (600890) | HAND2 (602407) |
| HBA1 (141800) | HBA2 (141850) | HBB (141900) | HELLS (603946) |
| HK3 (142570) | HMOX1 (141250) | HNRNPK (600712) | HOXA11 (142958) |
| HPGD (601688) | HS6ST1 (604846) | HSD17B1 (109684) | HSD17B12 (609574) |
| HSD17B2 (109685) | HSD17B4 (601860) | HSD17B7 (606756) | HSD3B1 (109715) |
| HSF1 (140580) | HSF2BP (604554) | HSP90B1 (191175) | HSPG2 (142461) |
| HTATIP2 (605628) | ICAM1 (147840) | ICAM2 (146630) | ICAM3 (146631) |
| IDH1 (147700) | IFI30 (604664) | IFITM1 (604456) | IGF1 (147440) |
| IGF1R (147370) | IGF2 (147470) | IGF2BP1 (608288) | IGF2BP2 (608289) |
| IGF2BP3 (608259) | IGF2BP3 (608259) | IGF2R (147280) | IGFALS (601489) |
| IGFBP1 (146730) | IGFBP2 (146731) | IGFBP3 (146732) | IGFBP4 (146733) |
| IGFBP5 (146734) | IGFBP6 (146735) | IGFBP7 (602867) | IGFBPL1 (610413) |
| IL10 (124092) | IL11RA (600939) | IL12A (161560) | IL12B (161561) |
| IL13 (147683) | IL17A (603149) | IL17B (604627) | IL17C (604628) |
| IL17D (607587) | IL17F (606496) | IL1A (147760) | IL1B (147720) |
| IL23A (605580) | IL23R (607562) | IL4 (147780) | IL5 (147850) |
| IL5RA (147851) | IL6 (147620) | IL6ST (600694) | IL8 (146930) |
| ILK (602366) | INHA (147380) | INHBA (147290) | INHBB (147390) |
| IRF1 (147575) | ISG15 (147571) | ITGA11 (604789) | ITGA2 (192974) |
| ITGA3 (605025) | ITGA4 (192975) | ITGA7 (600536) | ITGA9 (603963) |
| ITGAV (193210) | ITGB1 (135630) | JAG1 (601920) | JAG2 (602570) |
| JARID2 (601594) | JMY (604279) | KAL1 (300836) | KDM1A (609132) |
| KDM1B (613081) | KDM3A (611512) | KDM4A (609764) | KDM5A (180202) |
| KDM5B (605393) | KHDC1 (611688) | KIAA0430 (614593) | KIF2C (604538) |
| KISS1 (603286) | KISS1R (604161) | KITLG (184745) | KL (604824) |
| KLF4 (602253) | KLF9 (602902) | KLHL7 (611119) | LAMC1 (150290) |
| LAMC2 (150292) | LAMP1 (153330) | LAMP2 (309060) | LAMP3 (605883) |
| LDB3 (605906) | LEP (164160) | LEPR (601007) | LFNG (602576) |
| LHB (152780) | LHCGR (152790) | LHX8 (604425) | LIF (159540) |
| LIFR (151443) | LIMS1 (602567) | LIMS2 (607908) | LIMS3 |
| LIMS3L | LIN28 (611043) | LIN28B (611044) | LMNA (150330) |
| LOC613037 | LOXL4 (607318) | LPP (600700) | LYRM1 (614709) |
| MAD1L1 (602686) | MAD2L1 (601467) | MAD2L1BP | MAF (177075) |
| MAP3K1 (600982) | MAP3K2 (609487) | MAPK1 (176948) | MAPK3 (601795) |
| MAPK8 (601158) | MAPK9 (602896) | MB21D1 (613973) | MBD1 (156535) |
| MBD2 (603547) | MBD3 (603573) | MBD4 (603574) | MCL1 (159552) |
| MCM8 (608187) | MDK (162096) | MDM2 (164785) | MDM4 (602704) |
| MECP2 (300005) | MED12 (300188) | MERTK (604705) | METTL3 (612472) |
| MGAT1 (160995) | MITF (156845) | MKKS (604896) | MKS1 (609883) |
| MLH1 (120436) | MLH3 (604395) | MOS (190060) | MPPED2 (600911) |
| MRS2 | MSH2 (609309) | MSH3 (600887) | MSH4 (602105) |
| MSH5 (603382) | MSH6 (600678) | MST1 (142408) | MSX1 (142983) |
| MSX2 (123101) | MTA2 (603947) | MTHFD1 (172460) | MTHFR (607093) |
| MTO1 (614667) | MTOR (601231) | MTRR (602568) | MUC4 (158372) |
| MVP (605088) | MX1 (147150) | MYC (190080) | NAB1 (600800) |
| NAB2 (602381) | NAT1 (108345) | NCAM1 (116930) | NCOA2 (601993) |
| NCOR1 (600849) | NCOR2 (600848) | NDP (300658) | NFE2L3 (604135) |
| NLRP1 (606636) | NLRP10 (609662) | NLRP11 (609664) | NLRP12 (609648) |
| NLRP13 (609660) | NLRP14 (609665) | NLRP2 (609364) | NLRP3 (606416) |
| NLRP4 (609645) | NLRP5 (609658) | NLRP6 (609650) | NLRP7 (609661) |
| NLRP8 (609659) | NLRP9 (609663) | NNMT (600008) | NOBOX (610934) |
| NODAL (601265) | NOG (602991) | NOS3 (163729) | NOTCH1 (190198) |
| NOTCH2 (600275) | NPM2 (608073) | NPR2 (108961) | NR2C2 (601426) |
| NR3C1 (138040) | NR5A1 (184757) | NR5A2 (604453) | NRIP1 (602490) |
| NRIP2 | NRIP3 (613125) | NTF4 (162662) | NTRK1 (191315) |
| NTRK2 (600456) | NUPR1 (614812) | OAS1 (164350) | OAT (613349) |
| OFD1 (300170) | OOEP (611689) | ORAI1 (610277) | OTC (300461) |
| PADI1 (607934) | PADI2 (607935) | PADI3 (606755) | PADI4 (605347) |
| PADI6 (610363) | PAEP (173310) | PAIP1 (605184) | PARP12 (612481) |
| PCNA (176740) | PCP4L1 | PDE3A (123805) | PDK1 (602524) |
| PGK1 (311800) | PGR (607311) | PGRMC1 (300435) | PGRMC2 (607735) |
| PIGA (311770) | PIM1 (164960) | PLA2G2A (172411) | PLA2G4C (603602) |
| PLA2G7 (601690) | PLAC1L | PLAG1 (603026) | PLAGL1 (603044) |
| PLCB1 (607120) | PMS1 (600258) | PMS2 (600259) | POF1B (300603) |
| POLG (174763) | POLR3A (614258) | POMZP3 (600587) | POU5F1 (164177) |
| PPID (601753) | PPP2CB (176916) | PRDM1 (603423) | PRDM9 (609760) |
| PRKCA (176960) | PRKCB (176970) | PRKCD (176977) | PRKCDBP |
| PRKCE (176975) | PRKCG (176980) | PRKCQ (600448) | PRKRA (603424) |
| PRLR (176761) | PRMT1 (602950) | PRMT10 (307150) | PRMT2 (601961) |
| PRMT3 (603190) | PRMT5 (604045) | PRMT6 (608274) | PRMT7 (610087) |
| PRMT8 (610086) | PROK1 (606233) | PROK2 (607002) | PROKR1 (607122) |
| PROKR2 (607123) | PSEN1 (104311) | PSEN2 (600759) | PTGDR (604687) |
| PTGER1 (176802) | PTGER2 (176804) | PTGER3 (176806) | PTGER4 (601586) |
| PTGES (605172) | PTGES2 (608152) | PTGES3 (607061) | PTGFR (600563) |
| PTGFRN (601204) | PTGS1 (176805) | PTGS2 (600262) | PTN (162095) |
| PTX3 (602492) | QDPR (612676) | RAD17 (603139) | RAX (601881) |
| RBP4 (180250) | RCOR1 (607675) | RCOR2 | RCOR3 |
| RDH11 (607849) | REC8 (608193) | REXO1 (609614) | REXO2 (607149) |
| RFPL4A (612601) | RGS2 (600861) | RGS3 (602189) | RSPO1 (609595) |
| RTEL1 (608833) | SAFB (602895) | SAR1A (607691) | SAR1B (607690) |
| SCARB1 (601040) | SDC3 (186357) | SELL (153240) | SEPHS1 (600902) |
| SEPHS2 (606218) | SERPINA10 (605271) | SFRP1 (604156) | SFRP2 (604157) |
| SFRP4 (606570) | SFRP5 (604158) | SGK1 (602958) | SGOL2 (612425) |
| SH2B1 (608937) | SH2B2 (605300) | SH2B3 (605093) | SIRT1 (604479) |
| SIRT2 (604480) | SIRT3 (604481) | SIRT4 (604482) | SIRT5 (604483) |
| SIRT6 (606211) | SIRT7 (606212) | SLC19A1 (600424) | SLC28A1 (606207) |
| SLC28A2 (606208) | SLC28A3 (608269) | SLC2A8 (605245) | SLC6A2 (163970) |
| SLC6A4 (182138) | SLCO2A1 (601460) | SLITRK4 (300562) | SMAD1 (601595) |
| SMAD2 (601366) | SMAD3 (603109) | SMAD4 (600993) | SMAD5 (603110) |
| SMAD6 (602931) | SMAD7 (602932) | SMAD9 (603295) | SMARCA4 (603254) |
| SMARCA5 (603375) | SMC1A (300040) | SMC1B (608685) | SMC3 (606062) |
| SMC4 (605575) | SMPD1 (607608) | SOCS1 (603597) | SOD1 (147450) |
| SOD2 (147460) | SOD3 (185490) | SOX17 (610928) | SOX3 (313430) |
| SPAG17 | SPARC (182120) | SPIN1 (609936) | SPN (182160) |
| SPO11 (605114) | SPP1 (166490) | SPSB2 (611658) | SPTB (182870) |
| SPTBN1 (182790) | SPTBN4 (606214) | SRCAP (611421) | SRD5A1 (184753) |
| SRSF4 (601940) | SRSF7 (600572) | ST5 (140750) | STAG3 (608489) |
| STAR (600617) | STARD10 | STARD13 (609866) | STARD3 (607048) |
| STARD3NL (611759) | STARD4 (607049) | STARD5 (607050) | STARD6 (607051) |
| STARD7 | STARD8 (300689) | STARD9 (614642) | STAT1 (600555) |
| STAT2 (600556) | STAT3 (102582) | STAT4 (600558) | STAT5A (601511) |
| STAT5B (604260) | STAT6 (601512) | STC1 (601185) | STIM1 (605921) |
| STK3 (605030) | SULT1E1 (600043) | SUZ12 (606245) | SYCE1 (611486) |
| SYCE2 (611487) | SYCP1 (602162) | SYCP2 (604105) | SYCP3 (604759) |
| SYNE1 (608441) | SYNE2 (608442) | TAC3 (162330) | TACC3 (605303) |
| TACR3 (162332) | TAF10 (600475) | TAF3 (606576) | TAF4 (601796) |
| TAF4B (601689) | TAF5 (601787) | TAF5L | TAF8 (609514) |
| TAF9 (600822) | TAP1 (170260) | TBL1X (300196) | TBXA2R (188070) |
| TCL1A (186960) | TCL1B (603769) | TCL6 (604412) | TCN2 (613441) |
| TDGF1 (187395) | TERC (602322) | TERF1 (600951) | TERT (187270) |
| TEX12 (605791) | TEX9 | TF (190000) | TFAP2C (601602) |
| TFPI (152310) | TFPI2 (600033) | TG (188450) | TGFB1 (190180) |
| TGFB1I1 (602353) | TGFBR3 (600742) | THOC5 (612733) | THSD7B |
| TLE6 (612399) | TM4SF1 (191155) | TMEM67 (609884) | TNF (191160) |
| TNFAIP6 (600410) | TNFSF13B (603969) | TOP2A (126430) | TOP2B (126431) |
| TP53 (191170) | TP53I3 (605171) | TP63 (603273) | TP73 (601990) |
| TPMT (187680) | TPRXL (611167) | TPT1 (600763) | TRIM32 (602290) |
| TSC2 (191092) | TSHB (188540) | TSIX (300181) | TTC8 (608132) |
| TUBB4Q (158900) | TUFM (602389) | TYMS (188350) | UBB (191339) |
| UBC (191340) | UBD (606050) | UBE2D3 (602963) | UBE3A (601623) |
| UBL4A (312070) | UBL4B (611127) | UIMC1 (609433) | UQCR11 (609711) |
| UQCRC2 (191329) | USP9X (300072) | VDR (601769) | VEGFA (192240) |
| VEGFB (601398) | VEGFC (601528) | VHL (608537) | VIM (193060) |
| VKORC1 (608547) | VKORC1L1 (608838) | WAS (300392) | WISP2 (603399) |
| WNT7A (601570) | WNT7B (601967) | WT1 (607102) | XDH (607633) |
| XIST (314670) | YBX1 (154030) | YBX2 (611447) | ZAR1 (607520) |
| ZFX (314980) | ZNF22 (194529) | ZNF267 (604752) | ZNF689 |
| ZNF720 | ZNF787 | ZNF84 | ZP1 (195000) |
| ZP2 (182888) | ZP3 (182889) | ZP4 (613514) | |

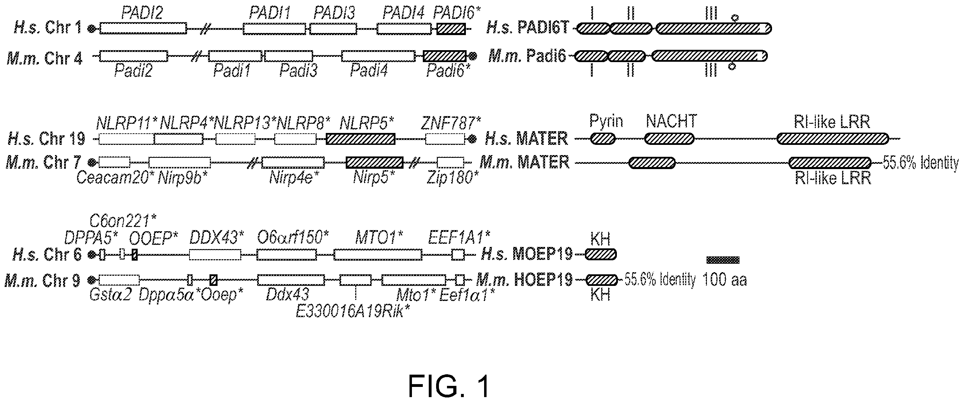

Additional genes to be used according to the invention are depicted in Fig. 1. Specifically, Fig. 1 depicts three maternal effect genes (*PADI6, NLRP5,* and *OOEP*; see Table 1) that are highly conserved, both at the protein level and genetic level in humans (H.s.) and mice (M.m.). Fig. 1 also depicts putative fertility-related genes that are clustered with each of the three maternal effect genes. These genes are located in genetic loci that are syntenic or highly conserved, and in most cases are observed to be expressed in murine and/or human eggs. Accordingly, fertility-related genes clustered with *PADI6, NLRP5, OOEP,* and other maternal effect genes including but not limited to those depicted in Fig. 1, can also be selected for use according to the invention. Additional gene clusters of relevance are shown in Figure 20.

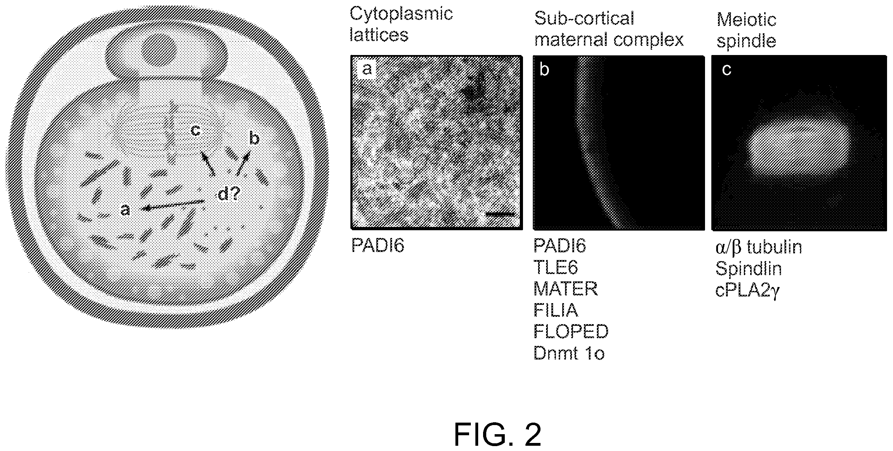

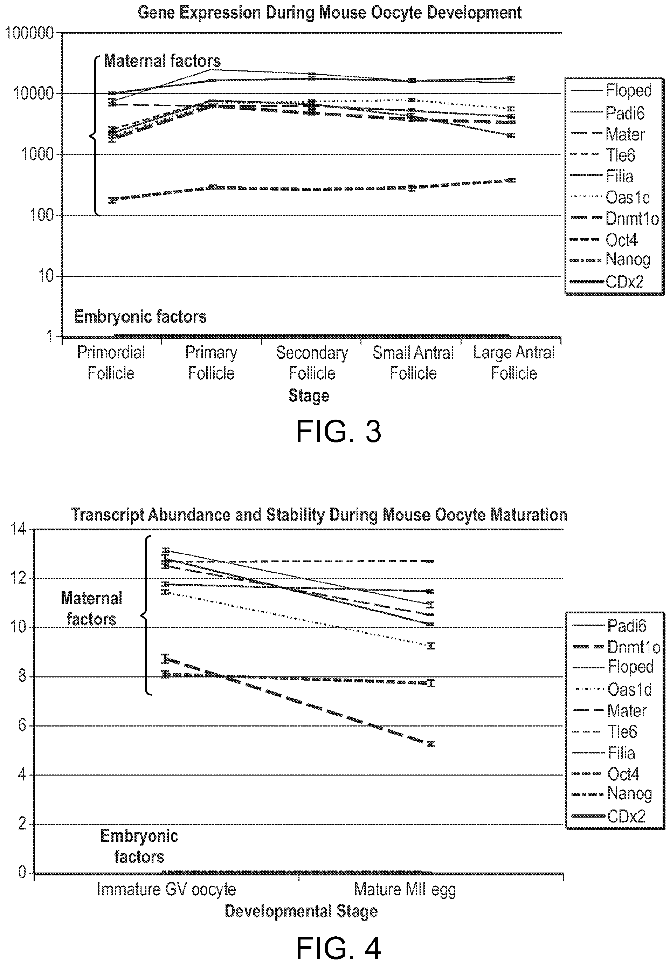

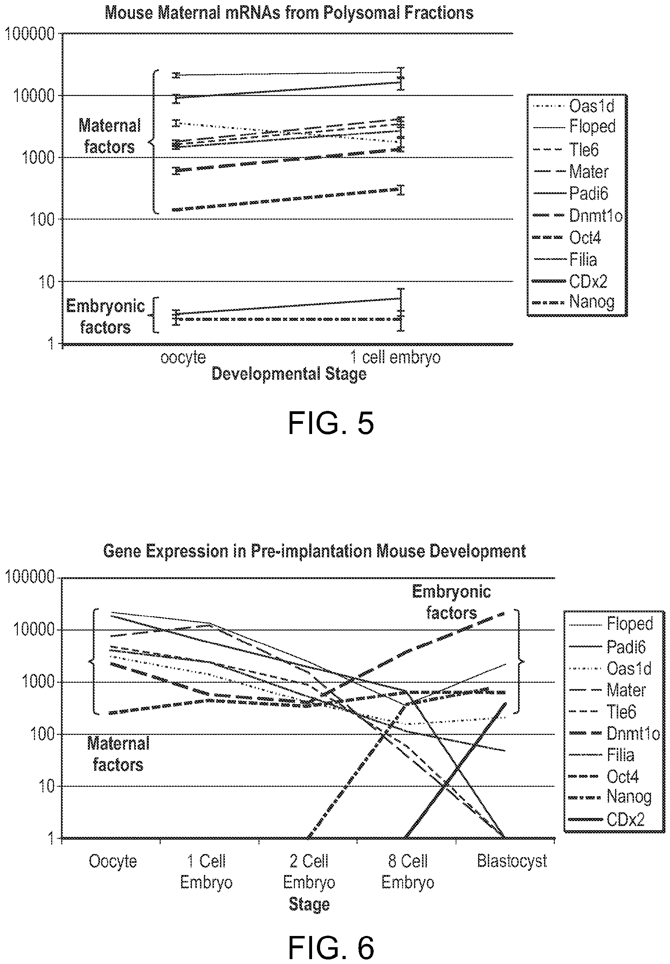

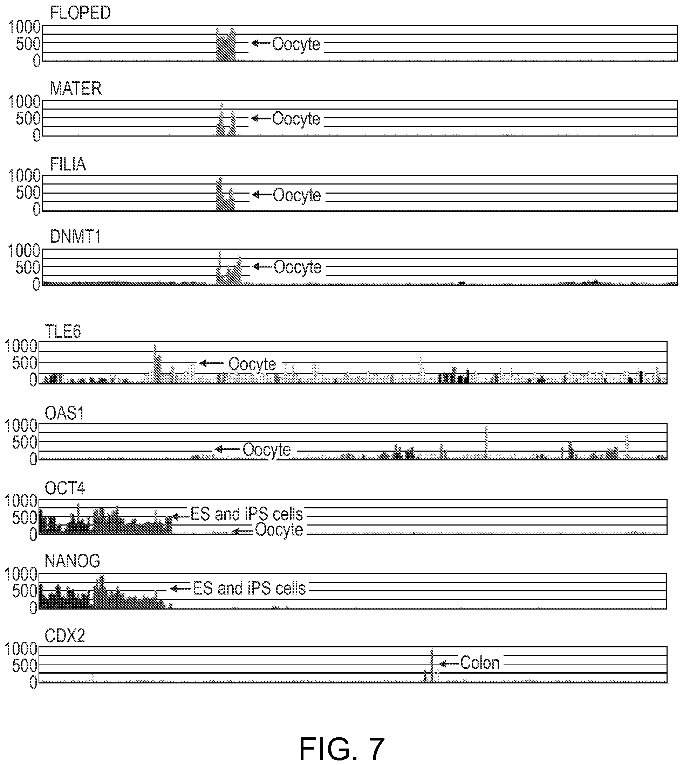

The molecular products of the genes in Table 1 are involved in different aspects of oocyte and embryo physiology from transcription and chromosome remodeling to RNA processing and binding. See Fig. 2. Fig. 2 depicts important mammalian egg structures: the Cytoplasmic Lattices, the Subcortical Maternal Complex (SCMC), and the Meiotic Spindle, that infertility-associated genes localize to and regulate. Fig. 3 depicts the abundant gene expression profile throughout egg development in mice of the maternal effect genes (MEG) associated with the SCMC. The relatively lower gene expression pattern of three genes (*Oct4, Cdx2, Nanog*) not associated with this complex are shown as a control. Fig. 4 depicts the relative decline in expression levels of the maternal effect genes associated with the SCMC during mouse oocyte maturation. Decline in MEG RNA and protein levels is commonly observed during oocyte maturation. Fig. 5 depicts the enrichment of MEGs associated with the SCMC in the polysomal fraction during the maternal to zygotic transition in mouse. Presence in this fraction is evidence that these mRNAs are being translated into protein. Fig. 6 depicts the dynamic RNA levels of MEGs associated with the SCMC (oocyte factors) relative to embryo factors (*Oct4, Cdx2, Nanog*) during the course of pre-implantation development in mice. In general, the levels of maternally inherited transcripts decline until the embryonic genome is fully activated. Fig. 7 depicts the tissue specific expression of human maternal effect genes.

**Peptidylarginine deiminase 6 (*PADI6*)** *Padi6* was originally cloned from a 2D murine egg proteome gel based on its relative abundance, and *Padi6* expression in mice appears to be almost entirely limited to the oocyte and pre-implantation embryo (Yurttas et al., 2010). *Padi6* is first expressed in primordial oocyte follicles and persists, at the protein level, throughout pre-implantation development to the blastocyst stage (Wright et al., Dev Biol, 256:73-88, 2003). Inactivation of *Padi6* leads to female infertility in mice, with the *Padi6*-null developmental arrest occurring at the two-cell stage (Yurttas et al., 2008).

**Nucleoplasmin 2 (*NPM2*)** Nucleoplasmin is another maternal effect gene, and is thought to be phosphorylated during mouse oocyte maturation. NPM2 exhibits a phosphate sensitive increase in mass during oocyte maturation. Increased phosphorylation is retained through the pronuclear stage of development. NPM2 then becomes dephosphorylated at the two- cell stage and remains in this form throughout the rest of pre-implantation development. Further, its expression pattern appears to be restricted to oocytes and early embryos. Immunofluorescence analysis of NPM2 localization shows that NPM2 primarily localizes to the nucleus in mouse oocytes and early embryos. In mice, maternally-derived NPM2 is required for female fertility (Burns et al., 2003).

**Maternal antigen the embryos require (MATER** / ***NLRP5*)** MATER, the protein encoded by the *Nlrp5* gene, is another highly abundant oocyte protein that is essential in mouse for embryonic development beyond the two-cell stage. MATER was originally identified as an oocyte-specific antigen in a mouse model of autoimmune premature ovarian failure (Tong et al., Endocrinology, 140:3720-3726, 1999). MATER demonstrates a similar expression and subcellular expression profile to PADI6. Like *Padi6*-null animals, *Nlrp5*-null females exhibit normal oogenesis, ovarian development, oocyte maturation, ovulation and fertilization. However, embryos derived from *Nlrp5*-null females undergo a developmental block at the two-cell stage and fail to exhibit normal embryonic genome activation (Tong et al., Nat Genet 26:267-268, 2000; and Tong et al. Mamm Genome 11:281-287, 2000b).

**Brahma-related gene 1 (*BRG1*)** Mammalian SWI/SNF-related chromatin remodeling complexes regulate transcription and are believed to be involved in zygotic genome activation (ZGA). Such complexes are composed of approximately nine subunits, which can be variable depending on cell type and tissue. The BRG1 catalytic subunit exhibits DNA-dependent APTase activity, and the energy derived from ATP hydrolysis alters the conformation and position of nucleosomes. *Brg1* is expressed in oocytes and has been shown to be essential in the mouse as null homozygotes do not progress beyond the blastocyst stage (Bultman et al., 2000).

**Factor located in oocytes permitting embryonic development (FLOPED /*OOEP*)** The subcortical maternal complex (SCMC) is a poorly characterized murine oocyte structure to which several maternal effect gene products localize (Li et al. Dev Cell 15:416-425, 2008). PADI6, MATER, FILIA, TLE6, and FLOPED have been shown to localize to this complex (Li et al. Dev Cell 15:416-425, 2008; Yurttas et al. Development 135:2627-2636, 2008). This complex is not present in the absence of *Floped* and *Nlrp5,* and similar to embryos resulting from *Nlrp5-*depleted oocytes, embryos resulting from *Floped*-null oocytes do not progress past the two cell stage of mouse development (Li et al., 2008). FLOPED is a small (19kD) RNA binding protein that has also been characterized under the name of MOEP19 (Herr et al., Dev Biol 314:300-316, 2008).

**KH domain containing 3-like, subcortical maternal complex member *(FILIA*/*KHDC3L)*** FILIA is another small RNA-binding domain containing maternally inherited murine protein. FILIA was identified and named for its interaction with MATER (Ohsugi et al. Development 135:259-269, 2008). Like other components of the SCMC, maternal inheritance of the *Khdc3* gene product is required for early embryonic development. In mice, loss of *Khdc3* results in a developmental arrest of varying severity with a high incidence of aneuploidy due, in part, to improper chromosome alignment during early cleavage divisions (Li et al., 2008). *Khdc3* depletion also results in aneuploidy, due to spindle checkpoint assembly (SAC) inactivation, abnormal spindle assembly, and chromosome misalignment (Zheng et al. Proc Natl Acad Sci USA 106:7473-7478, 2009).

**Basonuclin (*BNC1*)** Basonuclin is a zinc finger transcription factor that has been studied in mice. It is found expressed in keratinocytes and germ cells (male and female) and regulates rRNA (via polymerase I) and mRNA (via polymerase II) synthesis (Iuchi and Green, 1999; Wang et al., 2006). Depending on the amount by which expression is reduced in oocytes, embryos may not develop beyond the 8-cell stage. In *Bsn1* depleted mice, a normal number of oocytes are ovulated even though oocyte development is perturbed, but many of these oocytes cannot go on to yield viable offspring (Ma et al., 2006).

**Zygote Arrest 1 (ZAR1)** *Zar1* is an oocyte-specific maternal effect gene that is known to function at the oocyte to embryo transition in mice. High levels of *Zar1* expression are observed in the cytoplasm of murine oocytes, and homozygous-null females are infertile: growing oocytes from *Zar1*-null females do not progress past the two-cell stage.

**Cytosolic phospholipase A2y (PLA2G4C)** Under normal conditions, cPLA2y, the protein product of the murine *PLA2G4C* ortholog, expression is restricted to oocytes and early embryos in mice. At the subcellular level, cPLA2y mainly localizes to the cortical regions, nucleoplasm, and multivesicular aggregates of oocytes. It is also worth noting that while cPLA2y expression does appear to be mainly limited to oocytes and pre-implantation embryos in healthy mice, expression is considerably up-regulated within the intestinal epithelium of mice infected with *Trichinella spiralis.* This suggests that cPLA2y may also play a role in the inflammatory response. The human PLA2G4C differs in that rather than being abundantly expressed in the ovary, it is abundantly expressed in the heart and skeletal muscle. Also, the human protein contains a lipase consensus sequence but lacks a calcium-binding domain found in other PLA2 enzymes. Accordingly, another cytosolic phospholipase may be more relevant for human fertility.

**Transforming, Acidic Coiled-Coil Containing Protein 3 (*TACC3*)** In mice, TACC3 is abundantly expressed in the cytoplasm of growing oocytes, and is required for microtubule anchoring at the centrosome and for spindle assembly and cell survival (Fu et al., 2010).

In certain embodiments, the gene is a gene that is expressed in an oocyte. Exemplary genes include *CTCF, ZFP57, POU5F1, SEBOX,* and *HDAC1.*

In other embodiments, the gene is a gene that is involved in DNA repair pathways, including but not limited to, *MLH1, PMS1* and *PMS2.* In other embodiments, the gene is *BRCA1* or *BRCA2.*

In other embodiments, the biomarker is a gene product (e.g., RNA or protein) of an infertility-associated gene. In particular embodiments, the gene product is a gene product of a maternal effect gene. In other embodiments, the gene product is a product of a gene from Table 1. In certain embodiments, the gene product is a product of a gene that is expressed in an oocyte, such as a product of *CTCF, ZFP57, POU5F1, SEBOX,* and *HDAC1.* In other embodiments, the gene product is a product of a gene that is involved in DNA repair pathways, such as a product of *MLH1, PMS1,* or *PMS2.* In other embodiments, gene product is a product of *BRCA1* or *BRCA2.*

In other embodiments, the biomarker may be an epigenetic factor, such as methylation patterns (e.g., hypermethylation of CpG islands), genomic localization or post-translational modification of histone proteins, or general post-translational modification of proteins such as acetylation, ubiquitination, phosphorylation, or others.

In other embodiments, methods of the invention analyze infertility-associated biomarkers in order to assess the risk of an offspring developing a disorder. The invention recognizes that the exemplified genes may give rise to infertility issues while also being indicative of a putative offspring developing a disorder.

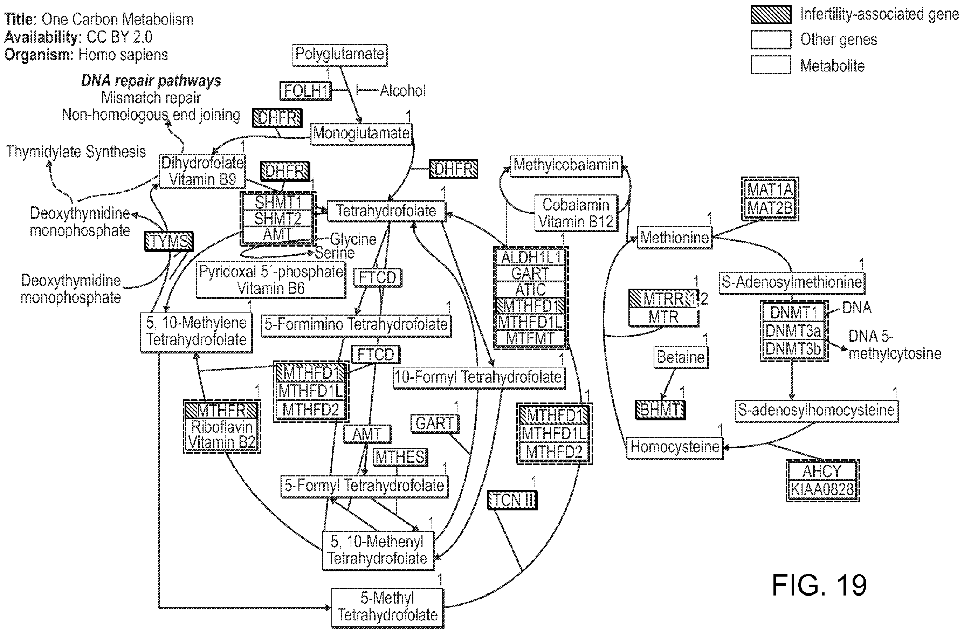

In certain embodiments, the biomarker is a genetic region, gene, or RNA/protein product of a gene associated with the one carbon metabolism pathway and other pathways that effect methylation of cellular macromolecules (Figure 19). Exemplary genes and products of those genes are described below and in Figure 19.

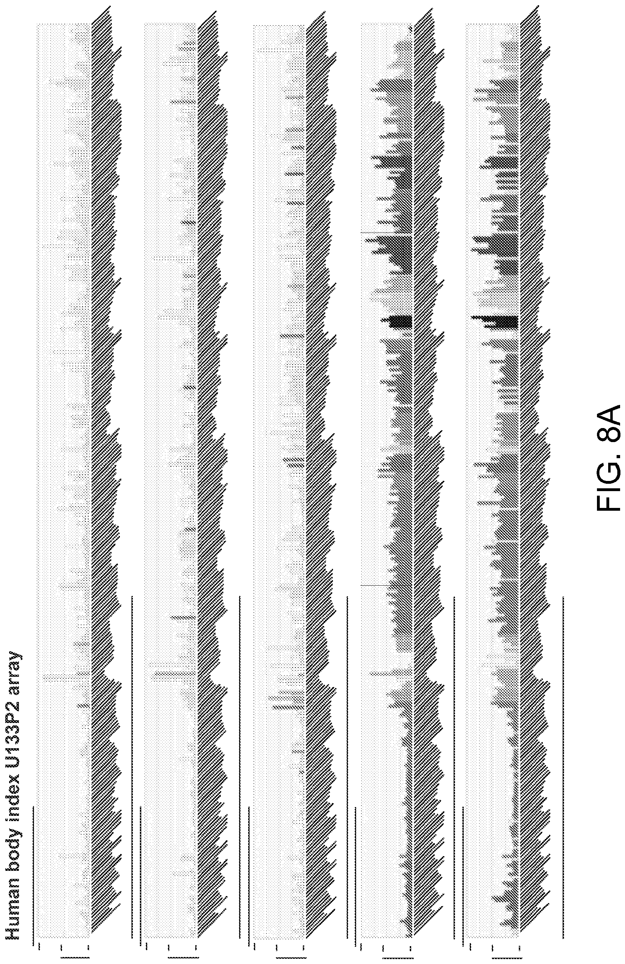

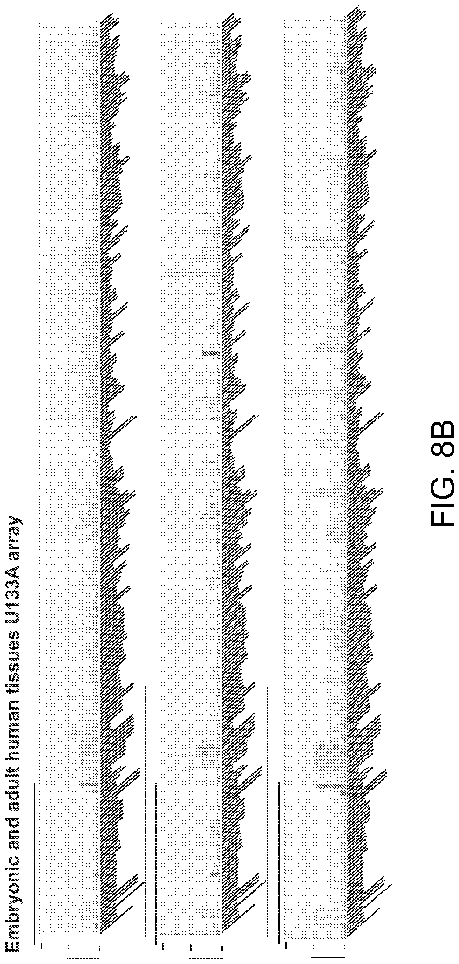

**Methylenetetrahydrofolate Reductase *(MTHFR)*** In particular embodiments a mutation (677C>T) in the *MTHFR* gene is associated with infertility. The enzyme 5,10-methylenetetrahydrofolate reductase regulates folate activity (Pavlik et al., Fertility and Sterility 95(7): 2257-2262, 2011). The 677TT genotype is known in the art to be associated with 60% reduced enzyme activity, inefficient folate metabolism, decreased blood folate, elevated plasma homocysteine levels, and reduced methylation capacity. Pavlik et al. (2011) investigated the effect of the *MTHFR* 677C>T on serum anti-Mullerian hormone (AMH) concentrations and on the numbers of oocytes retrieved (NOR) following controlled ovarian hyperstimulation (COH). Two hundred and seventy women undergoing COH for IVF were analyzed, and their AMH levels were determined from blood samples collected after 10 days of GnRH superagonist treatment and before COH. Average AMH levels of TT carriers were significantly higher than those of homozygous CC or heterozygous CT individuals. AMH serum concentrations correlated significantly with the NOR in all individuals studied. The study concluded that the *MTHFR* 677TT genotype is associated with higher serum AMH concentrations but paradoxically has a negative effect on NOR after COH. It was proposed that follicle maturation might be retarded in *MTHFR* 677TT individuals, which could subsequently lead to a higher proportion of initially recruited follicles that produce AMH, but fail to progress towards cyclic recruitment. The tissue gene expression patterns of *MTHFR* do not show any bias towards oocyte expression (Fig. 8). Analyzing a maternal sample for this mutation or other mutations (Table 2) in the *MTHFR* gene or abnormal gene expression of products of the *MTHFR* gene allows one to assess a risk of an offspring developing a disorder.

Jeddi-Tehrani et al. (American Journal of Reproductive Immunology 66(2):149-156, 2011) investigated the effect of the *MTHFR* 677TT genotype on Recurrant Pregnancy Loss (RPL). One hundred women below 35 years of age with two successive pregnancy losses and one hundred healthy women with at least two normal pregnancies were used to assess the frequency of five candidate genetic risk factors for RPL - *MTHFR* 677C>T, *MTHFR* 1298A>C, *PAI1* -675 4G/5G (Plasminogen Activator Inhibitor-1 promoter region), *BF* -455G/A (Beta Fibrinogen promoter region), and *ITGB3* 1565T/C (Integrin Beta 3). The frequencies of the polymorphisms were calculated and compared between case and control groups. Both the *MTHFR* polymorphisms (677C>T and 1298 A>C) and the *BF* -455G/A polymorphism were found to be positively and *ITGB3* 1565T/C polymorphism was found to be negatively associated with RPL. Homozygosity but not heterozygosity for the *PAI*-1 -6754G/5G polymorphism was significantly higher in patients with RPL than in the control group. The presence of both mutations of *MTHFR* genes highly increased the risk of RPL. Analyzing a maternal sample for these mutation and other mutations (Table 2) in the *MTHFR* gene or abnormal gene expression of products of the *MTHFR* gene allows one to assess a risk of an offspring developing a disorder.

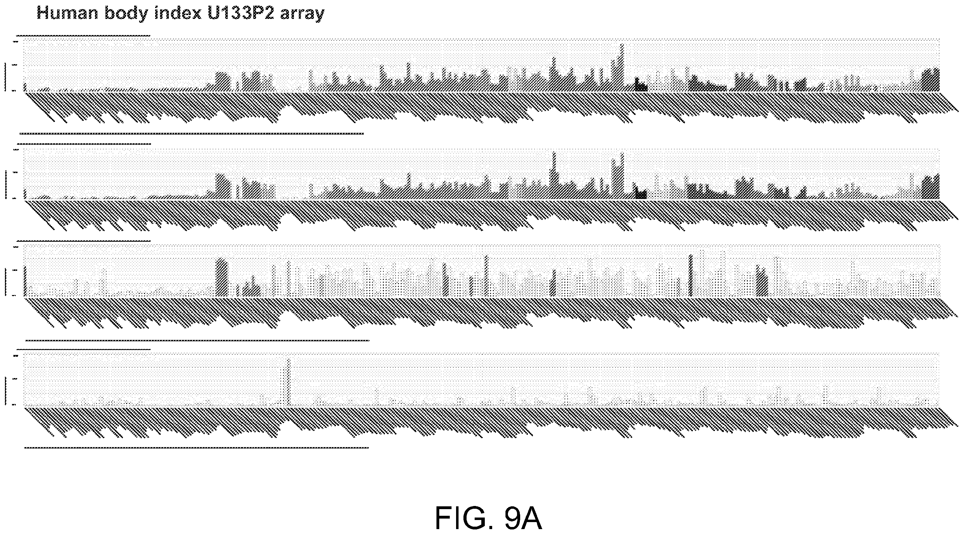

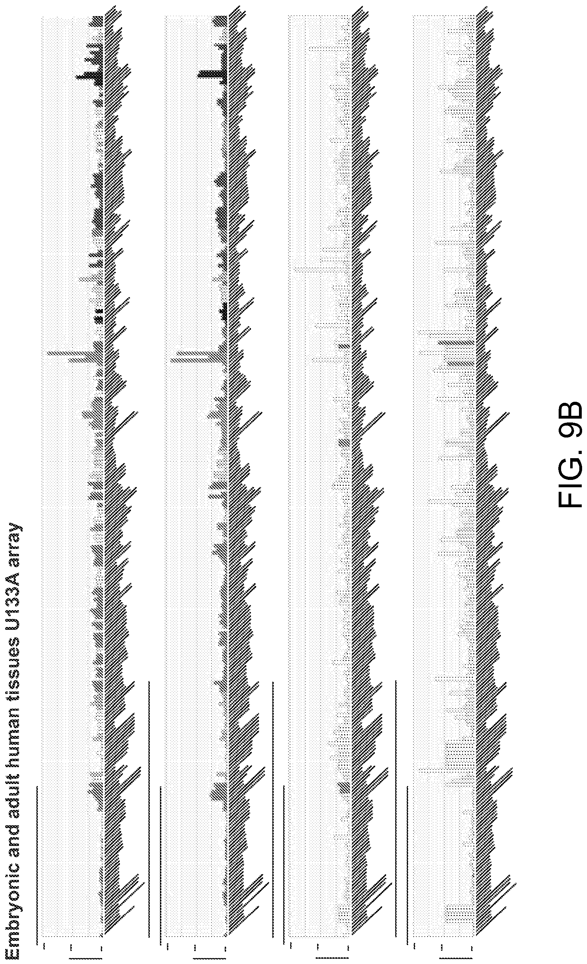

**Catechol-O-methyltransferase *(COMT)*** In particular embodiments a mutation (472G>A) in the *COMT* gene is associated with infertility. Catechol-O-methyltransferase is known in the art to be one of several enzymes that inactivates catecholamine neurotransmitters by transferring a methyl group from SAM (S-adenosyl methionine) to the catecholamine. The AA gene variant is known to alter the enzyme's thermostability and reduces its activity 3 to 4 fold (Schmidt et al., Epidemiology 22(4): 476-485, 2011). Salih et al. (Fertility and Sterility 89(5, Supplement 1): 1414-1421, 2008) investigated the regulation of COMT expression in granulosa cells and assessed the effects of 2-ME2 (COMT product) and COMT inhibitors on DNA proliferation and steroidogenesis in JC410 porcine and HGL5 human granulosa cell lines in *in vitro* experiments. They further assessed the regulation of COMT expression by DHT (Dihydrotestosterone), insulin, and ATRA (all-trans retinoic acid). They concluded that COMT expression in granulosa cells was up-regulated by insulin, DHT, and ATRA. Further, 2-ME2 decreased, and COMT inhibition increased granulosa cell proliferation and steroidogenesis. It was hypothesized that COMT overexpression with subsequent increased level of 2-ME2 may lead to ovulatory dysfunction. Fig. 9 illustrates the tissue expression patterns of *COMT* mRNA. Analyzing a maternal sample for this mutation in the *COMT* gene or abnormal gene expression of products of the COMT gene allows one to assess a risk of an offspring developing a disorder.

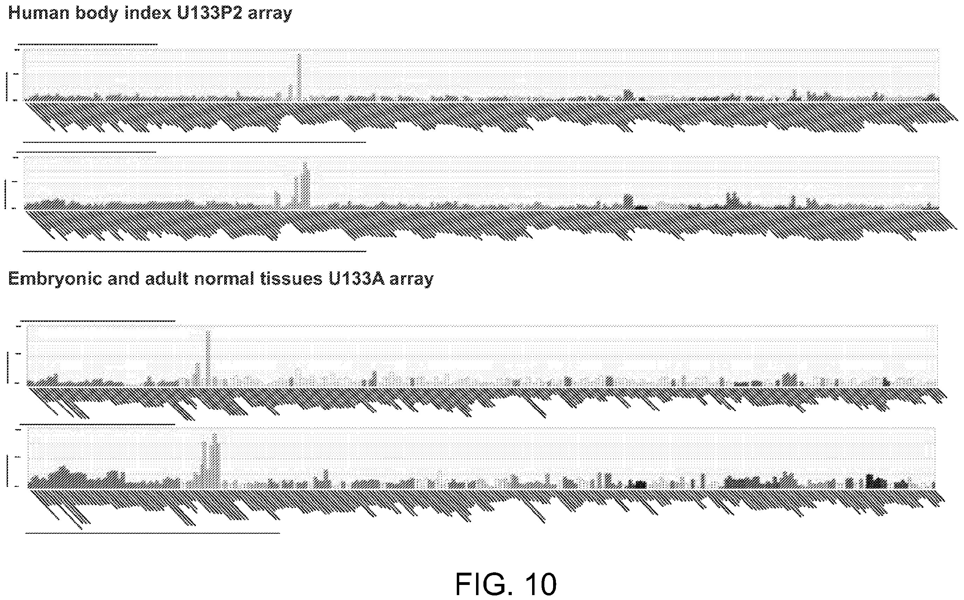

**Methionine Synthase Reductase (*MTRR*)** In particular embodiments a mutation (A66G) in the Methionine Synthase Reductase (*MTRR*) gene is associated with infertility. MTRR is required for the proper function of the enzyme Methionine Synthase (MTR). MTR converts homocysteine to methionine, and MTRR activates MTR, thereby regulating levels of homocysteine and methionine. The maternal variant A66G has been associated with early developmental disorders such as Down's syndrome (Pozzi et al., 2009) and Spina Bifida (Doolin et al., American journal of human genetics 71(5): 1222-1226, 2002). Fig. 10 illustrates the tissue expression patterns of *MTRR.* Analyzing a maternal sample for this mutation in the *MTRR* gene or abnormal gene expression of products of the *MTRR* gene allows one to assess a risk of an offspring developing a disorder.

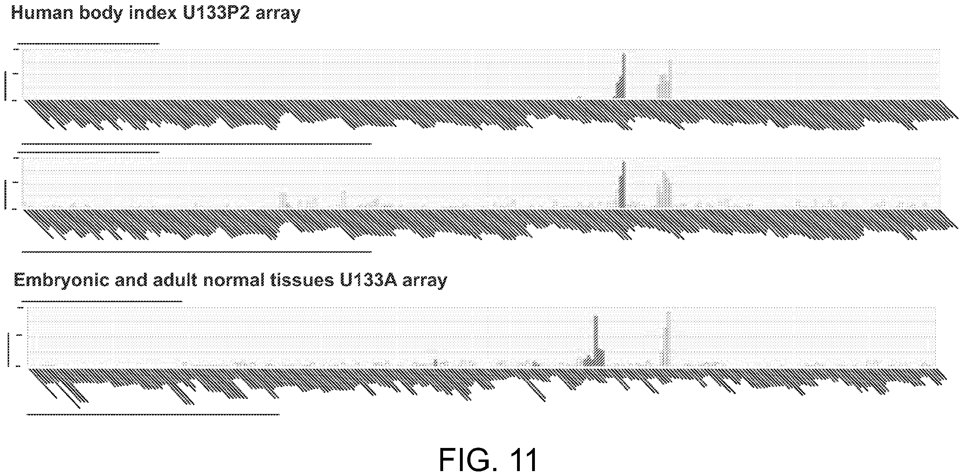

**Betaine-Homocysteine S-Methyltransferase (*BHMT*)** In particular embodiments a mutation (G716A) in the *BHMT* gene is associated with infertility. Betaine-Homocysteine S-Methyltransferase (BHMT), along with MTRR, assists in the Folate/B-12 dependent and choline/betaine-dependent conversions of homocysteine to methionine (See Figure 19). High homocysteine levels have been linked to female infertility (Berker et al., Human Reproduction 24(9): 2293-2302, 2009). Benkhalifa et al. (2010) discuss that controlled ovarian hyperstimulation (COH) affects homocysteine concentration in follicular fluid. Using germinal vescicle oocytes from patients involved in IVF procedures, the study concludes that the human oocyte is able to regulate its homocysteine level via remethylation using MTR and BHMT, but not CBS (Cystathione Beta Synthase). They further emphasize that this may regulate the risk of imprinting problems during IVF procedures. Fig. 11 illustrates the tissue expression patterns of *BHMT.* Analyzing a maternal sample for this mutation in the *BHMT* gene or abnormal gene expression of products of the *BHMT* gene allows one to assess a risk of an offspring developing a disorder.

Ikeda et al. (Journal of Experimental Zoology Part A: Ecological Genetics and Physiology 313A(3): 129-136, 2010) examined the expression patterns of all methylation pathway enzymes in bovine oocytes and preimplantation embryos. Bovine oocytes were demonstrated to have the mRNA of *MAT1A* (Methionine adenosyltransferase), *MAT2A, MAT2B, AHCY* (S-adenosylhomocysteine hydrolase), *MTR, BHMT, SHMT1* (Serine hydroxymethyltransferase), *SHMT2,* and *MTHFR.* All these transcripts were consistently expressed through all the developmental stages, except *MAT1A,* which was not detected from the 8-cell stage onward, and *BHMT,* which was not detected in the 8-cell stage. Furthermore, the effect of exogenous homocysteine on preimplantation development of bovine embryos was investigated *in vitro.* High concentrations of homocysteine induced hypermethylation of genomic DNA as well as developmental retardation in bovine embryos. Analyzing a maternal sample for these irregular methylation patterns allows one to assess a risk of an offspring developing a disorder.

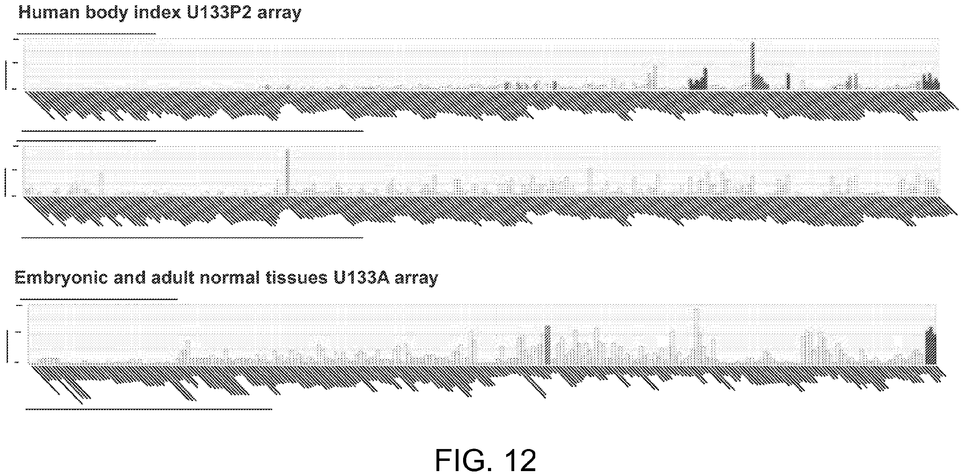

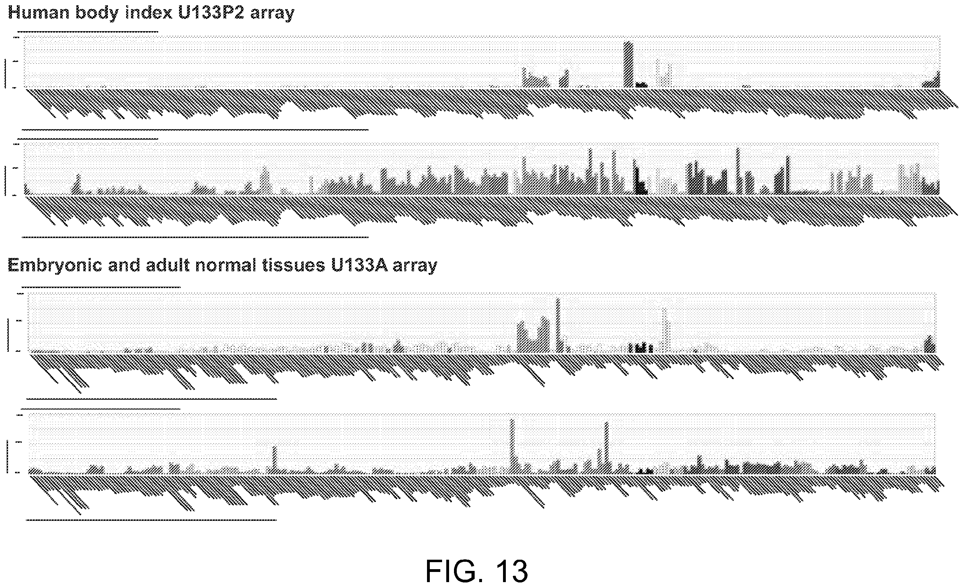

**Folate Receptor 2 (*FOLR2*)** In particular embodiments a mutation (rs2298444) in the *FOLR2* gene is associated with infertility. Folate Receptor 2 helps transport folate (and folate derivatives) into cells. Elnakat and Ratnam (Frontiers in bioscience: a journal and virtual library 11: 506-519, 2006) implicate FOLR2, along with FOLR1, in ovarian and endometrial cancers. Fig. 12 illustrates the tissue expression patterns of FOLR2. Fig. 13 illustrates the tissue expression patterns of FOLR1. Analyzing a maternal sample mutations in the *FOLR2* or *FOLR1* genes or abnormal gene expression of products of the *FOLR2* or *FOLR1* genes allows one to assess a risk of an offspring presenting with a developmental disorder.

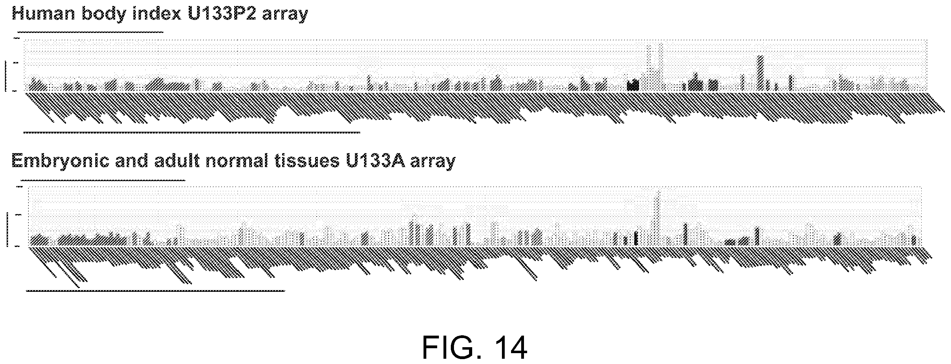

**Transcobalamin 2 (*TCN2*)** In particular embodiments a mutation (C776G) in the *TCN2* gene is associated with infertility. Transcobalamin 2 facilitates transport of cobalamin (Vitamin B12) into cells. Stanislawska-Sachadyn et al. (Eur J ClinNutr 64(11): 1338-1343, 2010) assessed the relationship between *TCN2* 776C>G polymorphism and both serum B12 and total homocysteine (tHcy) levels. Genotypes from 613 men from Northern Ireland were used to show that the *TCN2* 776CC genotype was associated with lower serum B12 concentrations when compared to the 776CG and 776GG genotypes. Furthermore, vitamin B12 status was shown to influence the relationship between *TCN2* 776C>G genotype and tHcy concentrations. The *TCN2* 776C>G polymorphism may contribute to the risk of pathologies associated with low B12 and high total homocysteine phenotype. Fig. 14 illustrates the tissue expression patterns of *TCN2.* Analyzing a maternal sample for this mutation in the *TCN2* gene or abnormal gene expression of products of the *TCN2* gene allows one to assess a risk of an offspring developing a disorder.

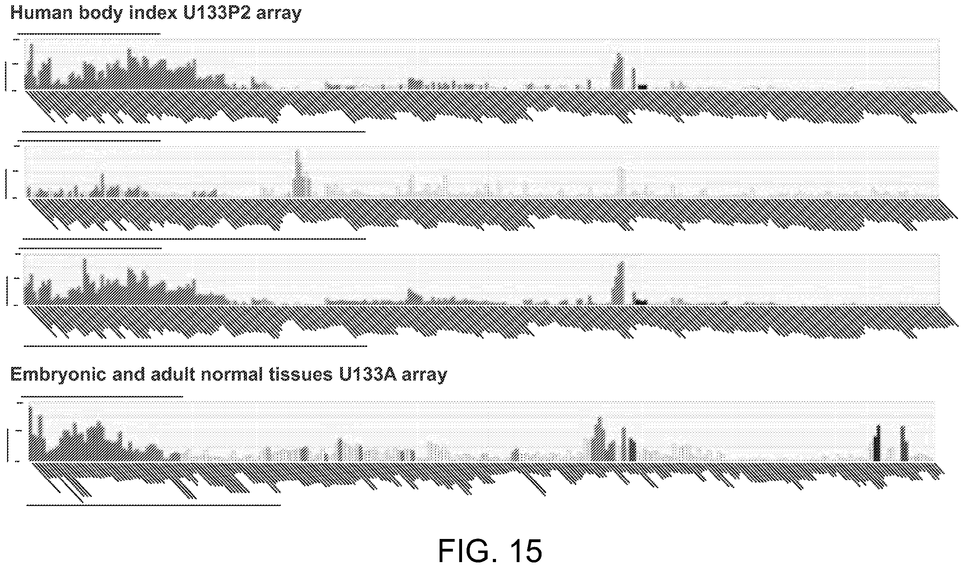

**Cystathionine-Beta-Synthase *(CBS)*** In particular embodiments a mutation (rs234715) in the *CBS* gene is associated with infertility. With vitamin B6 as a cofactor, the Cystathionine-Beta-Synthase (CBS) enzyme catalyzes a reaction that permanently removes homocysteine from the methionine pathway by diverting it to the transsulfuration pathway. *CBS* gene mutations associated with decreased CBS activity also lead to elevated plasma homocysteine levels. Guzman et al. (2006) demonstrate that *Cbs* knockout mice are infertile. They further explain that *Cbs*-null female infertility is a consequence of uterine failure, which is a consequence of hyperhomocysteinemia or other factor(s) in the uterine environment. Fig. 15 illustrates the tissue expression patterns of *CBS.* Analyzing a maternal sample for this mutation in the *CBS* gene or abnormal gene expression of products of the *CBS* gene allows one to assess a risk of an offspring presenting with a developmental disorder.

**DNA (cytosine-5)-methyltransferase 1 (*DNMT1*)** In particular embodiments a mutation (rs16999593) in the DNMT1 gene is associated with infertility. We identified the rs16999593 variant, which causes a histidine (H) to arginine (R) change at residue 97 in Dnmt1 protein, in a subset of infertile female patients. There are two isoforms of DNMT1 protein that are expressed in a sequential order during development (Carlson et al., 1992). The DNMTlo protein is known in mice to be a maternal effect protein that is synthesized in the oocyte and functions after fertilization to maintain methylation patterns on imprinted alleles. The DNMT1 protein, which has the same primary structure as DNMTlo with an additional 118-aa domain at its amino terminus, replaces DNMTlo after embryo implantation. Both DNMT1 isoforms maintain methylation at CpG dinucleotides by catalyzing the addition of methyl groups to cytosine bases in DNA, and are implicated in stabilizing repeat sequences, including CAG repeats.

Aberrant expression or function of both DNMT1 and/or DNMTlo could lead to female infertility and the presentation of disorders in putative offspring if infertility is bypassed. Depletion of *DNMT1* in mice leads to an increase in the expansion of CAG repeats during transmission from parents to offspring (Dion et al., 2008). Furthermore, a reduction of DNMT1 causes mismatch repair defects (Loughery et al., 2011) and destabilizes CAG triplet repeats (Dion et al., 2008) in human cells. Expanded CAG repeat tracks can cause an assortment of neurodegenerative diseases, including, but not limited to Huntington disease (HD), spino-cerebellar ataxia 1, 2, 3, 6, 7 and 17 (SCA1/2/3/6/7/17), and dentatorubral- pallidoluysian atrophy (DRPLA). Female mice with a homozygous *Dnmt1o* deletion appear phenotypically normal, but heterozygous fetuses of homozygous females generally die during the last 3^{rd} of gestation due to loss of allele-specific gene expression and methylation at certain imprinted loci{Howell:2001hf}. The rs16999593 variant in *DNMT1* could alter or attenuate DNMT1/DNMT1o function, which would subsequently lead to intergenerational CAG repeat track expansion and/or loss of imprinting, both of which could increase the risk of an offspring disorder. Analyzing a maternal sample for this or other mutations in the *DNMT1* gene or abnormal gene expression of products of the *DNMT1* gene allows one to assess a risk of an offspring developing a disorder.

In certain embodiments, the biomarker is a genetic region that has been previously associated with female infertility. A SNP association study by targeted re-sequencing was performed to search for new genetic variants associated with female infertility. Such methods have been successful in identifying significant variants associated in a wide range of diseases Rehman et al., 2010; Walsh et al., 2010). Briefly, a SNP association study is performed by collecting SNPs in genetic regions of interest in a number of samples and controls and then testing each of the SNPs that showed significant frequency differences between cases and controls. Significant frequency differences between cases and controls indicate that the SNP is associated with the condition of interest.

We performed a SNP association study by targeted re-sequencing and identified a total of 286 SNPs significantly associated with female infertility. Each variant was classified as novel or known.

For known variants, we retrieved the minor allele frequency from the 1000 genomes database (Durbin et al., 2010). We then performed an exact one-sided binomial test for each variant, testing whether the observed variant count was statistically greater than expected based on the minor allele frequency. P values less than .00043 were considered significant. For novel variants, the same method was employed, but using an imputed minor allele frequency of .001.

Table 2 identifies 286 SNPs associated with female infertility that fall within genetic regions that have also been associated with the risk of an offspring developing a disorder (see Table 3).

In particular embodiments, the infertility-associated genetic region is selected from the SNPs shown in Table 2 below.

**Table 2: Infertility-Associated SNPs**

| SNP ID | Locus | Position | Ref | Var | MAF | Score |
|---|---|---|---|---|---|---|
| novel | MUC4 | chr3:195512107 | T | A | - | 96.00 |
| rs62532306 | BOP1 | chr8:145505229 | C | A | - | 58.06 |
| rs4002465 | CGB | chr19:49527061 | G | C | - | 54.04 |
| rs62126025 | CGB | chr19:49527066 | T | C | - | 54.04 |
| rs76295320 | ZNF787 | chr19:56610009 | A | G | - | 53.81 |
| rs62126988 | RFPL4A | chr19:56272921 | A | T | - | 49.48 |
| rs3972768 | ZP3 | chr7:76063333 | T | C | - | 47.94 |
| rs9915824 | HSD17B1 | chr17:40702841 | A | G | - | 47.74 |
| rs74842155 | RFPL4A | chr19:56273736 | T | A | - | 46.46 |
| rs74182589 | ZNF787 | chr19:56610006 | T | C | - | 46.07 |
| rs79852613 | ZNF787 | chr19:56610038 | T | C | - | 46.07 |
| rs55956596 | MAD1L1 | chr7:1922620 | C | T | - | 45.89 |
| rs112808291 | MUC4 | chr3:195507242 | C | A | - | 44.66 |
| rs10835907 | WT1 | chr11:32434180 | G | A | - | 43.48 |
| rs2688498 | MUC4 | chr3:195476442 | G | C | - | 42.95 |
| rs28571120 | CGB | chr19:49526191 | G | A | - | 42.93 |
| rs116383373 | RFPL4A | chr19:56268106 | C | T | - | 41.07 |
| rs111519111 | MAD1L1 | chr7:1910705 | T | C | - | 41.07 |
| novel | ZNF787 | chr19:56610083 | T | C | - | 40.52 |
| rs13406100 | BARD1 | chr2:215627612 | C | A | - | 38.66 |
| rs10860866 | IGF1 | chr12:102835687 | T | G | - | 38.14 |
| rs2868052 | RFPL4A | chr19:56276194 | A | C | - | 38.14 |
| rs2450440 | ZNF787 | chr19:56628695 | A | G | - | 38.14 |
| rs17852109 | CGB | chr19:49526203 | G | C | - | 38.12 |
| rs1544809 | MUC4 | chr3:195472740 | C | T | - | 37.66 |
| rs59080007 | CARM1 | chr19:10996568 | T | C | - | 37.43 |
| rs7633103 | MUC4 | chr3:195471492 | C | G | - | 37.21 |
| rs62456177 | PMS2 | chr7:6025845 | C | T | - | 36.82 |
| rs1638149 | ZP3 | chr7:76064950 | A | C | - | 36.25 |
| rs1754208 | ZP3 | chr7:76066129 | A | G | - | 36.25 |
| rs4386875 | MAD1L1 | chr7:1928020 | G | A | - | 35.26 |
| rs12973299 | ZNF787 | chr19:56618129 | C | T | - | 34.40 |
| novel | MUC4 | chr3:195507846 | C | G | - | 33.33 |
| novel | MTO1 | chr6:74206880 | C | T | - | 33.33 |
| rs6969822 | MAD1L1 | chr7:2035814 | G | C | - | 33.33 |
| rs2045865 | RFPL4A | chr19:56273902 | C | A | - | 32.02 |
| novel | HS6ST1 | chr2:129006024 | C | A | - | 31.99 |
| rs12403346 | FMN2 | chr1:240620371 | G | A | - | 31.64 |
| rs113457754 | MUC4 | chr3:195512343 | G | A | - | 30.93 |
| rs1770345 | MTOR | chr1:11214580 | C | A | - | 30.47 |
| rs72626227 | CGB | chr19:49527339 | C | T | - | 30.47 |
| rs2948677 | MUC4 | chr3:195510786 | T | G | - | 30.47 |
| rs413807 | MUC4 | chr3:195510827 | C | T | - | 30.47 |
| rs71321841 | MUC4 | chr3:195513345 | A | C | - | 30.47 |
| rs67816107 | ESRRB | chr14:76944784 | T | C | - | 30.04 |
| rs2019946 | RFPL4A | chr19:56273293 | G | A | - | 30.04 |
| novel | EZR | chr6:159237011 | A | C | - | 29.57 |
| rs9283487 | PRKRA | chr2:179296271 | T | C | - | 29.28 |
| rs112638514 | MUC4 | chr3:195539373 | T | C | - | 29.02 |
| novel | UIMC1 | chr5:176396948 | A | C | - | 29.02 |
| rs3997878 | PRKRA | chr2:179296626 | G | A | - | 28.93 |
| rs71523271 | MAD1L1 | chr7:1910374 | G | A | - | 28.93 |
| rs9881716 | MUC4 | chr3:195472148 | C | T | - | 28.52 |
| novel | MUC4 | chr3:195506267 | C | T | - | 28.52 |
| rs5022939 | MAD1L1 | chr7:2035966 | G | C | - | 28.50 |
| novel | ZP3 | chr7:76072644 | A | G | - | 28.50 |
| novel | ZNF787 | chr19:56610075 | C | A | - | 28.07 |
| rs78772566 | ZNF787 | chr19:56610076 | A | G | - | 28.07 |
| rs62475576 | ZP3 | chr7:76073794 | A | T | - | 28.07 |
| novel | COMT | chr22:19947360 | C | T | - | 27.78 |
| novel | COMT | chr22:19947361 | A | T | - | 27.78 |
| rs10408967 | CGB | chr19:49528275 | C | T | - | 27.65 |
| novel | MUC4 | chr3:195507827 | G | A | - | 27.65 |
| novel | MTO1 | chr6:74192512 | C | T | - | 27.16 |
| novel | GPC3 | chrX:132910905 | C | T | - | 27.16 |
| novel | ESR1 | chr6:152306694 | G | A | - | 26.61 |
| novel | STK3 | chr8:99891810 | A | C | - | 26.61 |
| rs3997877 | PRKRA | chr2:179296666 | T | C | - | 26.58 |
| rs3997879 | PRKRA | chr2:179296511 | C | T | - | 26.26 |
| rs58599527 | MUC4 | chr3:195476305 | G | A | - | 26.12 |
| rs79313055 | ZP3 | chr7:76073796 | G | A | - | 26.12 |
| novel | ZNF787 | chr19:56610073 | A | G | - | 25.67 |
| rs78006633 | MUC4 | chr3:195481809 | A | G | - | 24.90 |
| novel | MUC4 | chr3:195481867 | A | G | - | 24.90 |
| novel | NLRP11 | chr19:56312625 | G | T | - | 24.74 |
| novel | ESR1 | chr6:152201316 | G | T | - | 24.74 |
| novel | MAD1L1 | chr7:2264910 | A | G | - | 24.55 |
| novel | MUC4 | chr3:195508709 | A | G | - | 24.19 |
| rs79018306 | HSD17B1 | chr17:40708178 | C | T | - | 23.93 |
| novel | FMR1 | chrX:147021719 | G | A | - | 23.71 |
| novel | DIAPH2 | chrX:96726717 | G | A | - | 23.71 |
| novel | CGB5 | chr19:49546958 | T | C | - | 23.28 |
| novel | CGB5 | chr19:49546960 | T | A | - | 23.28 |
| novel | MUC4 | chr3:195499065 | G | A | - | 23.28 |
| novel | MUC4 | chr3:195508500 | G | C | - | 23.28 |
| rs12278181 | HSD17B12 | chr11:43704110 | A | C | - | 22.89 |
| novel | CTCF | chr16:67640652 | G | T | - | 22.89 |
| novel | NLRP5 | chr19:56542077 | C | T | - | 22.89 |
| rs2906996 | ZP3 | chr7:76069937 | C | T | - | 22.89 |
| rs437805 | MUC4 | chr3:195512103 | G | A | - | 22.53 |
| novel | MTO1 | chr6:74192508 | C | T | - | 22.32 |
| novel | MAD1L1 | chr7:2042637 | C | T | - | 22.32 |
| novel | MAD1L1 | chr7:2042638 | T | C | - | 22.32 |
| rs34718891 | CBS | chr21:44477834 | C | G | - | 22.20 |
| novel | ZNF787 | chr19:56618184 | G | T | - | 21.89 |
| novel | MAD1L1 | chr7:1898732 | C | G | - | 21.89 |
| novel | EIF2B2 | chr14:75465532 | T | A | - | 21.78 |
| rs12489838 | MUC4 | chr3:195514109 | C | A | - | 21.78 |
| rs76816122 | MUC4 | chr3:195514144 | T | C | - | 21.78 |
| novel | NLRP11 | chr19:56332403 | G | T | - | 21.31 |
| rs7621695 | MUC4 | chr3:195475989 | G | A | - | 21.08 |
| rs112829487 | MUC4 | chr3:195539376 | G | A | - | 20.89 |
| novel | EZR | chr6:159232393 | A | T | - | 20.89 |
| rs61762234 | TP73 | chr1:3610404 | A | C | - | 20.52 |
| novel | HS6ST1 | chr2:129024942 | C | G | - | 20.51 |
| novel | MUC4 | chr3:195514728 | T | A | - | 20.51 |
| novel | MUC4 | chr3:195481823 | G | A | - | 20.17 |
| novel | TP73 | chr1:3610392 | G | A | - | 19.89 |
| novel | STK3 | chr8:99693203 | A | C | - | 19.89 |
| novel | DIAPH2 | chrX:96444382 | C | A | - | 19.89 |
| rs62126040 | CGB2 | chr19:49539560 | T | C | - | 19.85 |
| rs13046562 | CBS | chr21:44477803 | C | G | - | 19.85 |
| novel | MUC4 | chr3:195507262 | T | G | - | 19.56 |
| rs71522278 | MAD1L1 | chr7:1943503 | C | T | - | 19.56 |
| rs10950559 | MAD1L1 | chr7:2172705 | C | T | - | 19.56 |
| novel | ESRRB | chr14:76929788 | G | T | - | 19.36 |
| novel | TLE6 | chr19:2993943 | T | G | - | 19.36 |
| novel | GALT | chr9:34637819 | G | C | - | 19.36 |
| rs3176202 | POLG | chr15:89866286 | G | A | - | 18.80 |
| rs798767 | TACC3 | chr4:1734281 | A | G | - | 18.58 |
| novel | MDM4 | chr1:204521995 | C | T | - | 18.50 |
| novel | GDF1 | chr19:18985313 | T | G | - | 18.14 |
| rs113643133 | ZNF787 | chr19:56609885 | A | G | - | 17.81 |
| novel | ZNF787 | chr19:56625688 | T | C | - | 17.81 |
| novel | TP73 | chr1:3585751 | G | C | - | 17.47 |
| novel | ESRRB | chr14:76936269 | G | T | - | 17.47 |
| novel | ATR | chr3:142282826 | A | C | - | 17.47 |
| novel | BRCA1 | chr17:41264749 | C | G | - | 17.24 |
| rs12539440 | MAD1L1 | chr7:1980610 | C | G | - | 16.95 |
| rs7936739 | NLRP14 | chr11:7068543 | C | T | - | 16.75 |
| novel | MAD2L1BP | chr6:43605162 | C | T | - | 16.50 |
| novel | DIAPH2 | chrX:96149969 | C | T | - | 16.50 |
| rs55976062 | TP73 | chr1:3622892 | T | C | - | 14.93 |
| rs62282465 | MUC4 | chr3:195506315 | T | C | - | 14.93 |
| rs55782426 | PMS2 | chr7:6023033 | T | C | - | 14.70 |
| rs6802709 | MUC4 | chr3:195473776 | T | C | - | 14.48 |
| rs6768267 | MUC4 | chr3:195473783 | G | A | - | 14.48 |
| rs10271093 | MAD1L1 | chr7:1885221 | C | T | - | 14.48 |
| rs1558180 | ZP3 | chr7:76066189 | A | G | - | 14.48 |
| rs9835456 | MUC4 | chr3:195471522 | G | A | - | 13.74 |
| rs13239578 | MAD1L1 | chr7:1980636 | G | C | - | 13.42 |
| rs80209248 | ZP3 | chr7:76069486 | G | A | - | 12.65 |
| rs61124766 | NLRP5 | chr19:56511465 | T | C | - | 12.43 |
| rs7251474 | NLRP7 | chr19:55443228 | A | T | - | 12.24 |
| rs12155464 | MAD1L1 | chr7:1899547 | G | C | - | 12.24 |
| rs12155227 | MAD1L1 | chr7:1899553 | T | C | - | 12.24 |
| rs80123784 | MUC4 | chr3:195513383 | T | A | - | 12.05 |
| rs75239380 | RFPL4A | chr19:56274453 | G | A | - | 11.88 |
| rs578069 | ZNF787 | chr19:56631356 | A | G | - | 11.88 |
| rs75200017 | MUC4 | chr3:195473784 | C | T | - | 11.43 |
| rs2880893 | MUC4 | chr3:195491180 | C | T | - | 11.07 |
| rs10408095 | CGB | chr19:49527991 | A | G | - | 10.82 |
| rs10408850 | CGB | chr19:49528047 | G | A | - | 10.82 |
| rs35280967 | MAD1L1 | chr7:1922667 | C | G | - | 10.16 |
| rs55740147 | MAD1L1 | chr7:2089773 | G | A | - | 9.71 |
| rs62075836 | HSD17B1 | chr17:40702252 | C | T | - | 9.43 |
| rs4662595 | HS6ST1 | chr2:129024915 | C | T | - | 9.00 |
| rs71635066 | MUC4 | chr3:195477586 | T | C | - | 9.00 |
| rs80197731 | PRKRA | chr2:179315890 | G | C | - | 8.74 |
| rs34406439 | TP73 | chr1:3610322 | A | G | - | 8.52 |
| rs11757217 | FOXO3 | chr6:108882570 | C | T | - | 8.32 |
| rs78190054 | TP73 | chr1:3610357 | G | A | - | 8.14 |
| rs13123646 | TACC3 | chr4:1740539 | T | C | - | 8.14 |
| rs60234611 | MAD1L1 | chr7:1885242 | T | C | - | 7.98 |
| rs7257437 | NLRP5 | chr19:56532128 | T | C | - | 7.70 |
| rs4719408 | MAD1L1 | chr7:2089757 | T | C | - | 7.70 |
| rs62284989 | MUC4 | chr3:195479484 | G | A | - | 7.57 |
| rs113971556 | BOP1 | chr8:145505306 | A | C | - | 7.45 |
| rs13078480 | MUC4 | chr3:195499099 | C | T | - | 7.14 |
| rs77907507 | MUC4 | chr3:195514104 | T | A | - | 6.66 |
| rs112413534 | ZNF787 | chr19:56603294 | A | C | - | 6.44 |
| rs112012272 | ZNF787 | chr19:56625720 | T | C | - | 5.95 |
| rs71355791 | PLA2G4C | chr19:48564911 | T | C | 0.01 | 5.86 |
| rs4801657 | NLRP5 | chr19:56515593 | C | T | - | 5.82 |
| rs55846169 | MAD1L1 | chr7:2089802 | C | T | - | 5.82 |
| rs113631808 | PMS2 | chr7:6019274 | C | T | - | 5.70 |
| rs115280443 | HADHA | chr2:26451922 | A | G | 0.02 | 5.57 |
| rs10407868 | CGB | chr19:49527907 | A | C | - | 5.22 |
| rs34349826 | LHB | chr19:49519883 | A | G | - | 5.09 |
| rs79014552 | TP63 | chr3:189499326 | G | T | 0.01 | 5.04 |
| rs117469552 | FMN2 | chr1:240525487 | C | T | 0.00 | 4.83 |
| rs77453381 | TP73 | chr1:3610436 | C | G | - | 4.83 |
| rs11667729 | ZNF787 | chr19:56628807 | T | C | - | 4.83 |
| rs11806926 | FMN2 | chr1:240569344 | C | G | 0.05 | 4.78 |
| rs79723785 | BRSK1 | chr19:55818225 | T | C | 0.00 | 4.73 |
| rs75739598 | HADHA | chr2:26412802 | A | G | 0.02 | 4.73 |
| rs78522745 | NLRP8 | chr19:56461003 | G | T | 0.00 | 4.67 |
| rs79288206 | RFPL4A | chr19:56276174 | G | A | - | 4.55 |
| rs2637100 | ZNF787 | chr19:56628814 | C | G | - | 4.55 |
| rs2016745 | AFF2 | chrX:147628728 | A | T | - | 4.55 |
| rs56034002 | AFF2 | chrX:147628775 | A | T | - | 4.55 |
| rs73102793 | ACVR1B | chr12:52390574 | C | T | 0.02 | 4.52 |
| rs11798175 | DIAPH2 | chrX:96741405 | G | A | 0.06 | 4.46 |
| rs12145827 | FMN2 | chr1:240596675 | C | T | 0.36 | 4.35 |
| rs67341807 | PADI6 | chr1:17710800 | A | G | 0.04 | 4.34 |
| rs12025170 | PADI6 | chr1:17704934 | G | C | 0.14 | 4.29 |
| rs3800869 | MAD1L1 | chr7:2109933 | C | T | 0.24 | 4.22 |
| rs55765849 | ZNF787 | chr19:56603305 | G | A | 0.24 | 4.22 |
| rs1204891 | PADI6,PADI4 | chr1:17694615 | G | A | 0.18 | 4.18 |
| rs1464541 | MAD1L1 | chr7:1960887 | A | G | 0.34 | 4.14 |
| rs34647879 | MAD1L1 | chr7:1960646 | G | A | 0.34 | 4.14 |
| rs74392263 | BMP7 | chr20:55775929 | A | G | 0.05 | 4.13 |
| rs75100250 | TAF4B | chr18:23814766 | C | T | 0.05 | 4.09 |
| rs34008721 | MAD1L1 | chr7:1959975 | T | C | 0.35 | 4.08 |
| rs1107238 | HADHA | chr2:26458244 | T | C | 0.02 | 4.07 |
| rs74406635 | ZNF787 | chr19:56603338 | G | A | - | 4.04 |
| rs17627983 | EZH2 | chr7:148528058 | A | G | 0.08 | 4.00 |
| rs116832355 | FMN2 | chr1:240299858 | T | A | 0.01 | 3.99 |
| rs10265212 | MAD1L1 | chr7:1860733 | G | C | - | 3.92 |
| rs78501539 | TP63 | chr3:189431642 | A | G | 0.07 | 3.91 |
| rs55815720 | LIFR | chr5:38588247 | C | A | 0.33 | 3.90 |
| rs72912353 | SMAD2 | chr18:45369316 | T | C | 0.06 | 3.89 |
| rs12563231 | PADI6 | chr1:17703861 | C | T | 0.14 | 3.85 |
| rs117748025 | MYC | chr8:128747011 | C | A | 0.05 | 3.85 |
| rs76633823 | ZNF787 | chr19:56610026 | A | G | 0.33 | 3.85 |
| rs75288315 | EZH2 | chr7:148530212 | T | C | 0.08 | 3.84 |
| rs4618595 | MAD1L1 | chr7:1946106 | A | G | 0.31 | 3.82 |
| rs2631635 | ZNF787 | chr19:56628705 | G | A | 0.20 | 3.79 |
| rs10497192 | ACVR1 | chr2:158671700 | C | T | 0.49 | 3.78 |
| rs9812619 | TP63 | chr3:189391823 | C | A | 0.31 | 3.77 |
| rs4721162 | MAD1L1 | chr7:1930350 | A | G | 0.29 | 3.76 |
| rs113242110 | ZNF787 | chr19:56609882 | T | C | 0.06 | 3.75 |
| rs1671187 | NLRP2,NLRP7 | chr19:55476685 | A | T | - | 3.73 |
| rs11571700 | BRCA2 | chr13:32927017 | G | A | 0.05 | 3.71 |
| rs77497094 | KHDC1 | chr6:73958003 | T | C | 0.00 | 3.69 |
| rs6978209 | MAD1L1 | chr7:1931544 | G | A | 0.32 | 3.68 |
| rs73173010 | BRCA2 | chr13:32975041 | G | A | 0.05 | 3.68 |
| rs2280549 | MAD1L1 | chr7:1976635 | G | A | 0.26 | 3.65 |
| rs511297 | ZNF787 | chr19:56609944 | C | T | - | 3.64 |
| rs57766906 | ZP3 | chr7:76064351 | G | A | - | 3.64 |
| rs3812043 | LIFR | chr5:38597478 | A | G | 0.32 | 3.63 |
| rs66617818 | MAD1L1 | chr7:1872132 | C | A | 0.44 | 3.62 |
| rs564533 | C6orf221 | chr6:74072937 | G | C | 0.07 | 3.60 |
| rs76955461 | FMN2 | chr1:240565203 | C | T | 0.01 | 3.57 |
| rs117986556 | TP63 | chr3:189505273 | G | T | 0.00 | 3.57 |
| rs7616592 | MUC4 | chr3:195476380 | A | G | - | 3.57 |
| rs10802871 | FMN2 | chr1:240613707 | G | A | 0.24 | 3.56 |
| rs28572042 | NLRP7 | chr19:55447341 | T | C | 0.06 | 3.55 |
| rs28617363 | NLRP7 | chr19:55447342 | C | T | 0.06 | 3.55 |
| rs111468036 | ESR1 | chr6:152418902 | C | T | 0.00 | 3.53 |
| rs12733042 | GREM2 | chr1:240719255 | T | C | 0.15 | 3.53 |
| rs11739017 | LIFR | chr5:38519462 | T | C | 0.29 | 3.52 |
| rs75584969 | TFPI | chr2:188388402 | G | C | 0.04 | 3.51 |
| rs12859009 | DIAPH2 | chrX:96707326 | C | T | 0.40 | 3.50 |
| rs73173011 | BRCA2 | chr13:32975487 | T | C | 0.05 | 3.50 |
| rs60506574 | CGB2 | chr19:49534393 | T | C | - | 3.50 |
| rs6641437 | AFF2 | chrX:147660644 | C | T | 0.17 | 3.49 |
| rs2228948 | ACVR1 | chr2:158593905 | C | T | 0.13 | 3.49 |
| rs7810386 | MAD1L1 | chr7:1952031 | T | A | 0.32 | 3.48 |
| rs7535085 | FMN2 | chr1:240183908 | C | T | 0.18 | 3.48 |
| rs80009068 | FMN2 | chr1:240554884 | T | G | 0.02 | 3.48 |
| rs12760504 | PADI6 | chr1:17696247 | T | C | 0.20 | 3.47 |
| rs12880959 | ESRRB | chr14:76787228 | C | T | 0.40 | 3.46 |
| rs75301713 | ESR1 | chr6:152295764 | C | G | 0.01 | 3.46 |
| rs74501504 | ESR1 | chr6:152306334 | C | T | 0.01 | 3.46 |
| rs72914334 | SMAD2 | chr18:45452084 | C | T | 0.02 | 3.46 |
| rs6700319 | PADI6 | chr1:17698891 | C | T | 0.20 | 3.45 |
| rs7785626 | MAD1L1 | chr7:2103005 | A | G | 0.28 | 3.45 |
| rs10274701 | EZH2 | chr7:148552456 | C | T | 0.24 | 3.44 |
| rs511291 | ZNF787 | chr19:56609952 | A | G | - | 3.43 |
| rs12859787 | DIAPH2 | chrX:96658300 | A | G | 0.40 | 3.43 |
| rs77404600 | GREM2 | chr1:240754703 | G | A | 0.00 | 3.42 |
| rs12886462 | ESRRB | chr14:76793400 | T | G | 0.40 | 3.39 |
| rs73945411 | CGB5 | chr19:49542070 | G | C | - | 3.37 |
| rs314287 | LIN28B | chr6:105434661 | A | G | 0.43 | 3.36 |
| rs6429209 | GREM2 | chr1:240707877 | C | G | 0.46 | 3.35 |
| rs3748697 | CENPF | chr1:214820099 | A | G | 0.27 | 3.33 |
| rs17318323 | AFF2 | chrX:148079412 | A | G | 0.03 | 3.33 |
| rs2067948 | MAD1L1 | chr7:1976749 | G | A | 0.28 | 3.33 |
| rs2072408 | EZH2 | chr7:148508197 | A | G | 0.25 | 3.32 |
| rs12132059 | FMN2 | chr1:240588861 | A | T | 0.00 | 3.31 |
| rs6944660 | MAD1L1 | chr7:2083782 | A | G | 0.27 | 3.31 |
| rs17646552 | NLRP5 | chr19:56565263 | T | C | 0.00 | 3.28 |
| rs10512687 | LIFR | chr5:38581262 | C | T | 0.33 | 3.28 |
| rs269955 | NLRP7 | chr19:55443424 | A | G | 0.28 | 3.28 |
| rs12699596 | MAD1L1 | chr7:2065828 | A | G | 0.25 | 3.27 |
| rs5921851 | DIAPH2 | chrX:96811308 | T | A | 0.41 | 3.27 |
| rs385564 | KL | chr13:33592409 | C | G | 0.31 | 3.27 |
| rsl0853881 | NLRP4 | chr19:56373608 | A | G | 0.28 | 3.25 |
| rs2561821 | LIFR | chr5:38488174 | C | A | 0.43 | 3.24 |
| rs12760851 | FMN2 | chr1:240569361 | T | G | 0.02 | 3.22 |
| rs8105736 | TLE6 | chr19:2973274 | C | T | 0.01 | 3.22 |
| rs17445954 | TP63 | chr3:189411830 | G | A | 0.10 | 3.21 |
| rs12735781 | FMN2 | chr1:240569335 | G | C | 0.10 | 3.21 |
| rs1204889 | PADI6,PADI4 | chr1:17694717 | T | A | 0.22 | 3.21 |
| rs112915167 | ESRRB | chr14:76899709 | G | A | 0.00 | 3.21 |

| | | | | | | |
|---|---|---|---|---|---|---|
| The SNPs listed in the Table independently and significantly associated with Female Infertility. Position refers to NCBI Build 37. Alleles are reported on the forward strand. Ref = Reference allele, Var = Variant (risk) allele, MAF = minor allele frequency, Score = P-value of one-sided exact binomial test -Log₁₀p-value | | | | | | |

In certain embodiments, the biomarker is a genetic region, gene or gene product of a gene associated with human fertility and another disorder. Examples of offspring disorders include, but are not limited to, neurodevelopmental, neuropsychological and neuro-genetic disorder, e.g. neural tube defects, an autism spectrum disorder (including, but not limited to classical autism, asperger syndrome, rett syndrome, childhood disintegrative disorder, and pervasive developmental disorder not otherwise specified (PDD-NOS)), Bardet-Beidl syndrome, Attention Deficit Hyperactivity Disorder (ADHD), Angelman Syndrome, Prader-Willi Syndrome, Bipolar Disorder, Charcot Marie Tooth Syndrome, or Schizophrenia; metabolic disorder, e.g. obesity and Diabetes Mellitus (Type I or II); gynecological and/or infertility disorder, e.g. Endometriosis and Premature ovarian failure (POF); autoimmune disorder, e.g. asthma, juvenile idiopathic arthritis, allergies, Addison's disease, Crohn's disease, and Celiac disease; muscular dystrophy; cancer; and cardiovascular disease, e.g. early onset coronary heart disease.

In particular embodiments, an infertility/offspring disease-associated genetic region is a gene selected from the genes shown in Table 3 below. The OMIM reference number is provided where available.

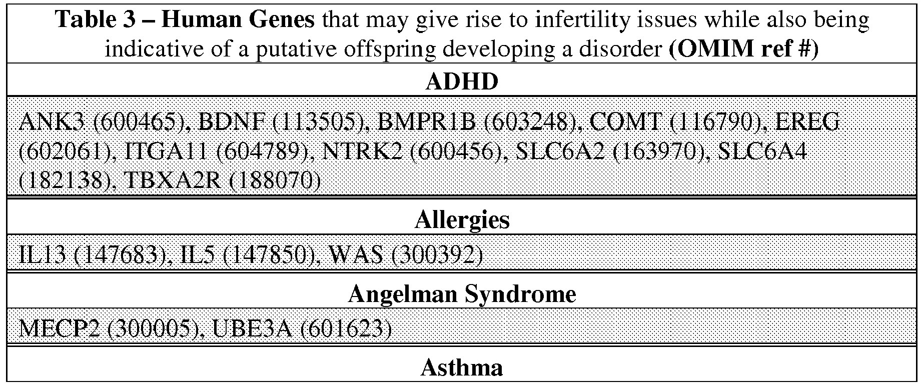

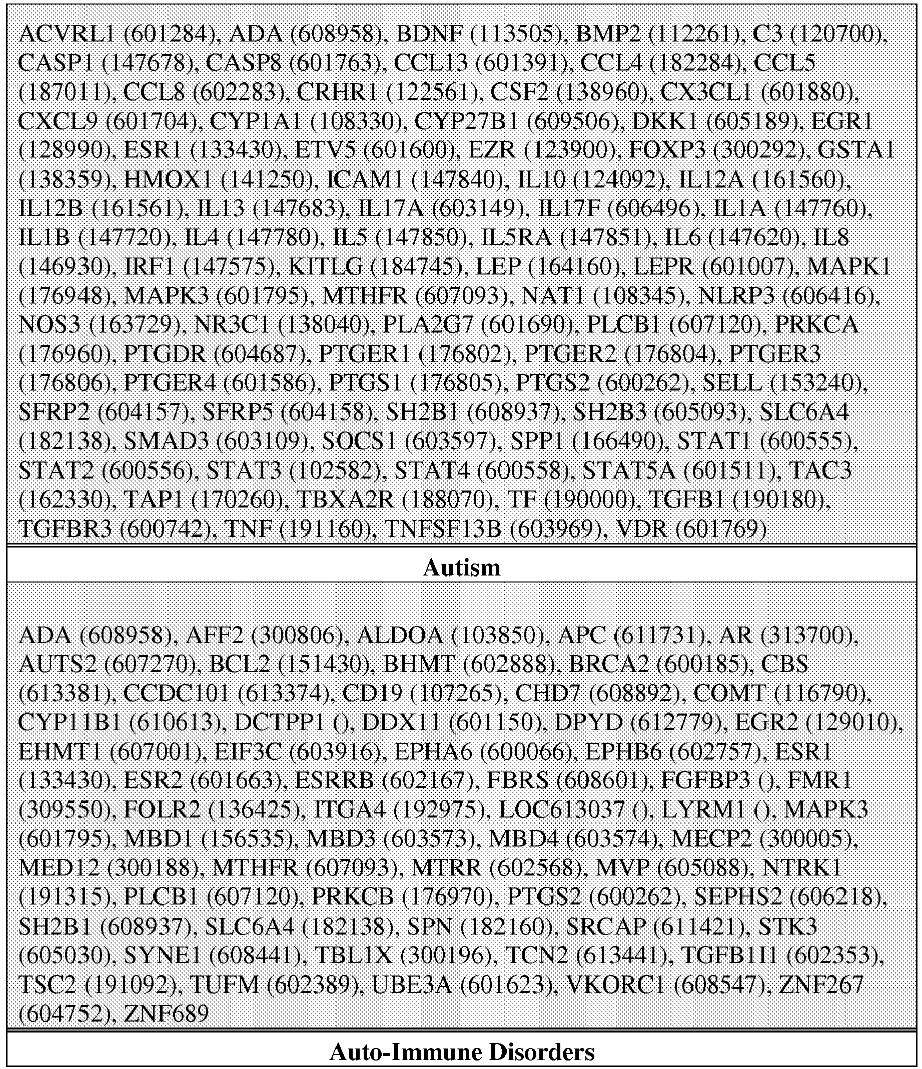

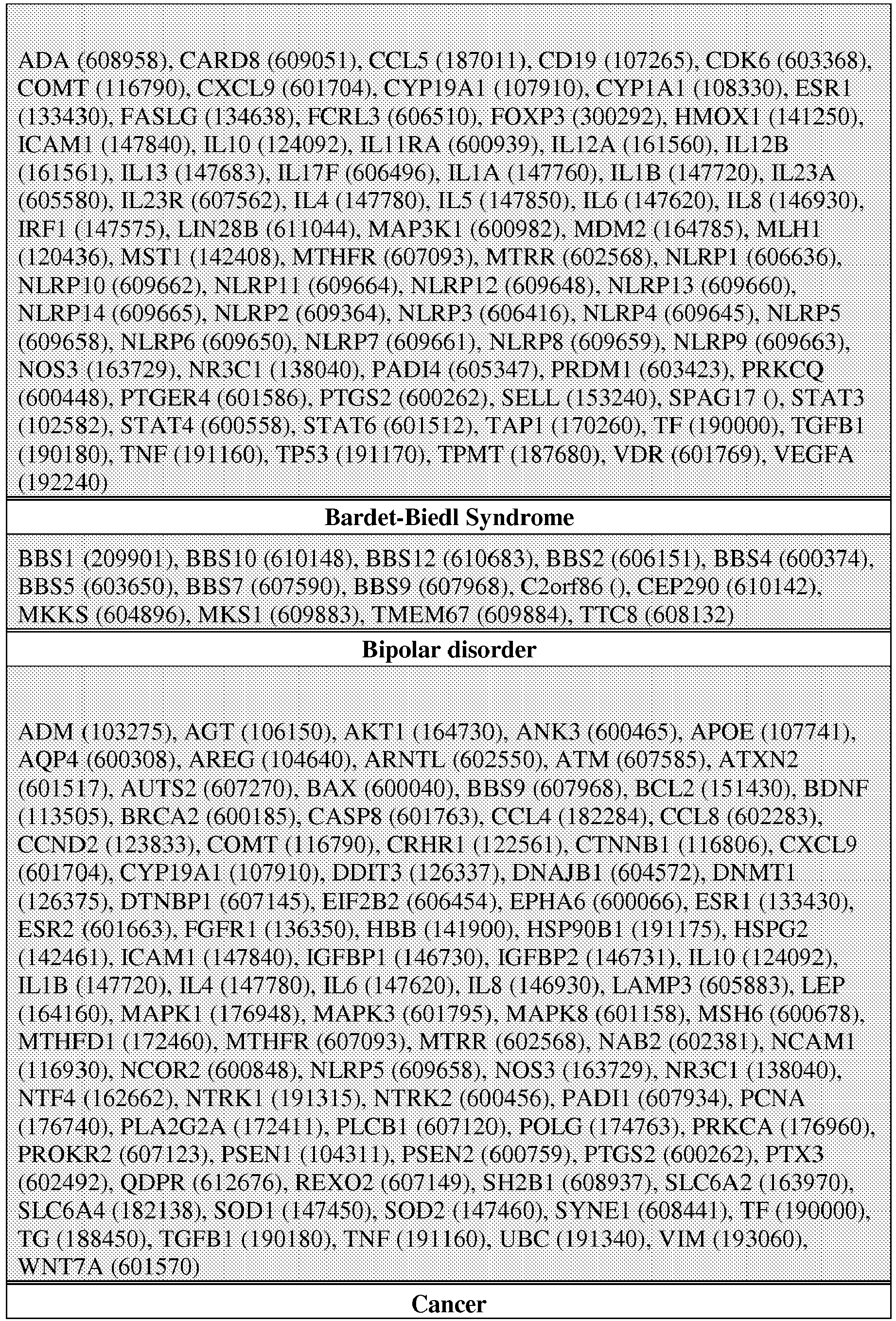

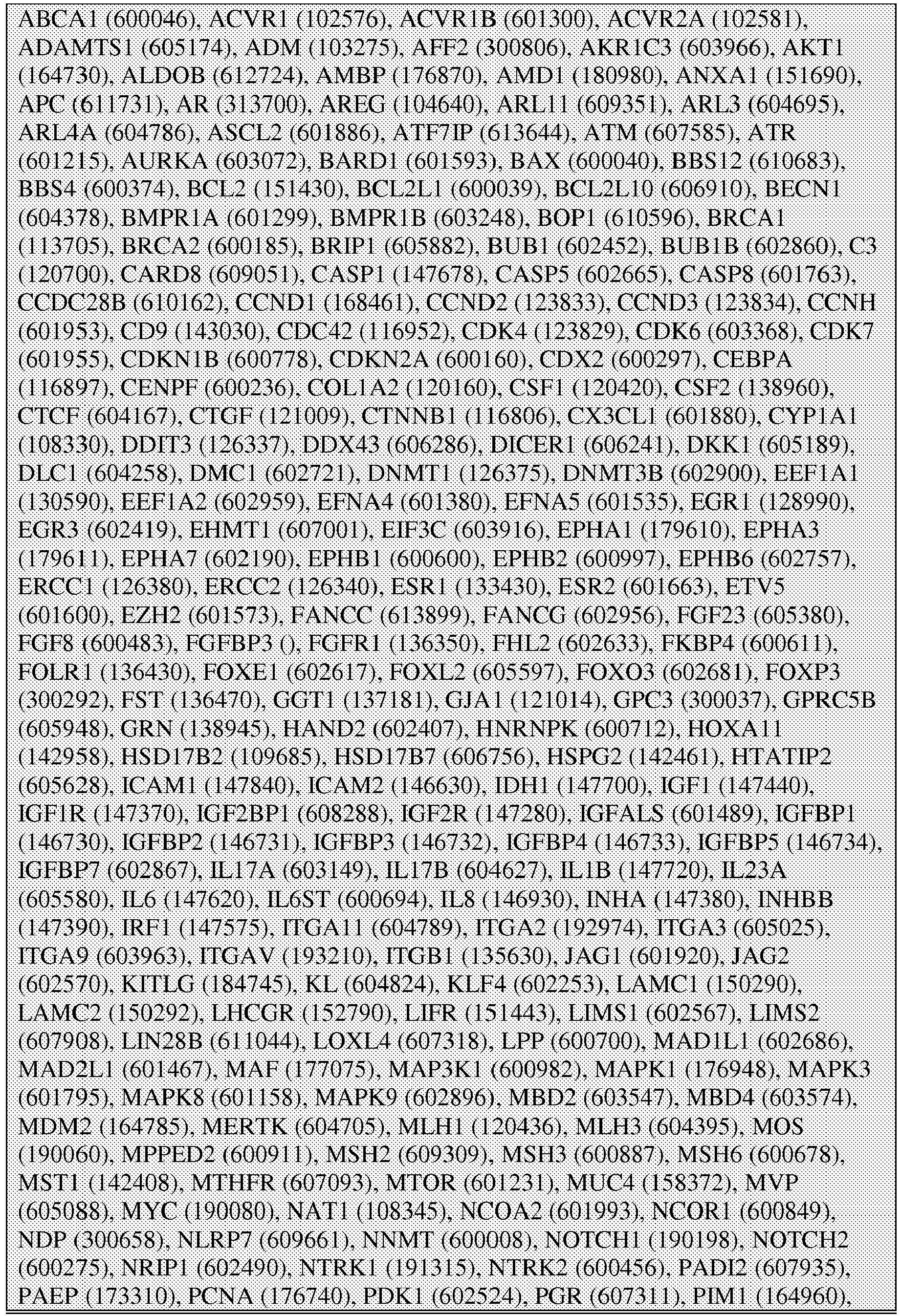

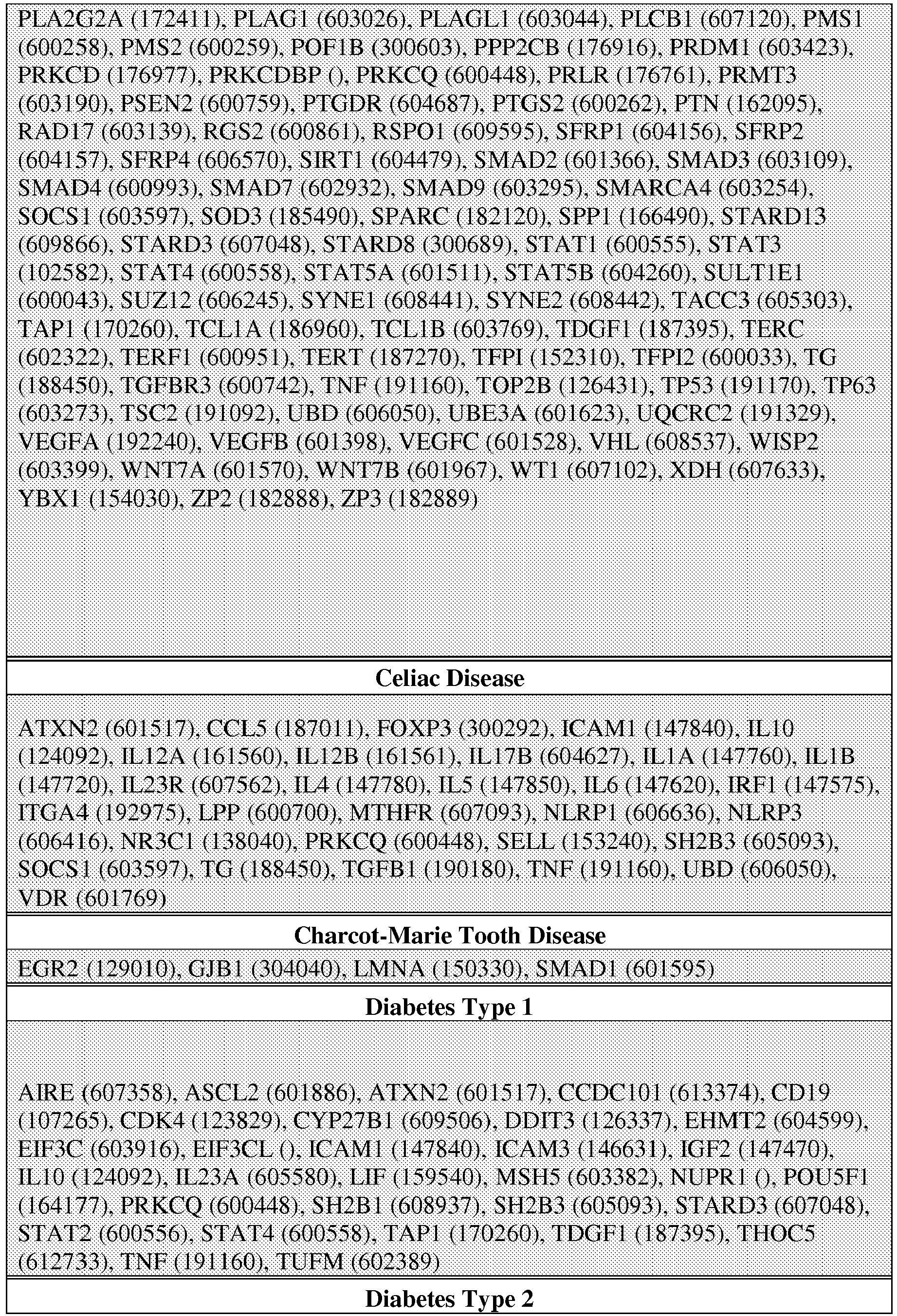

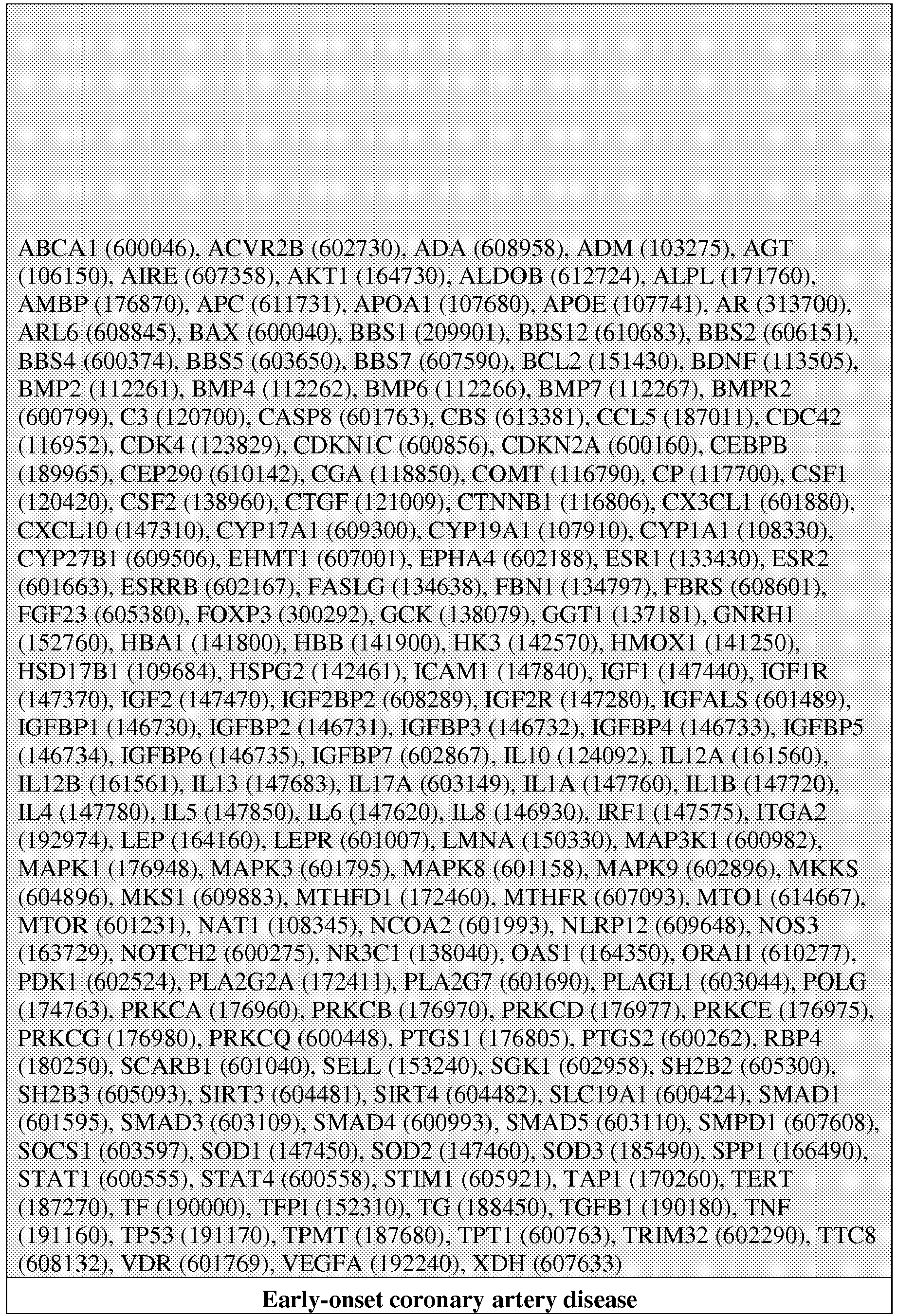

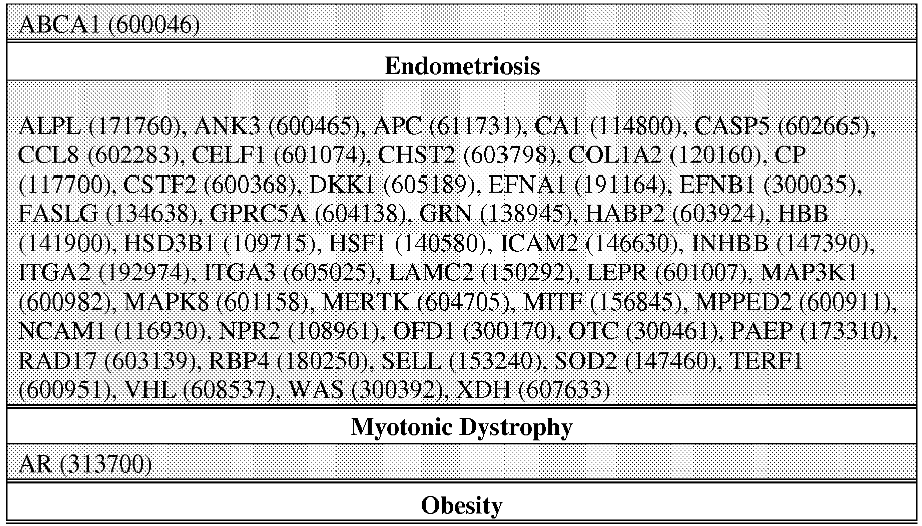

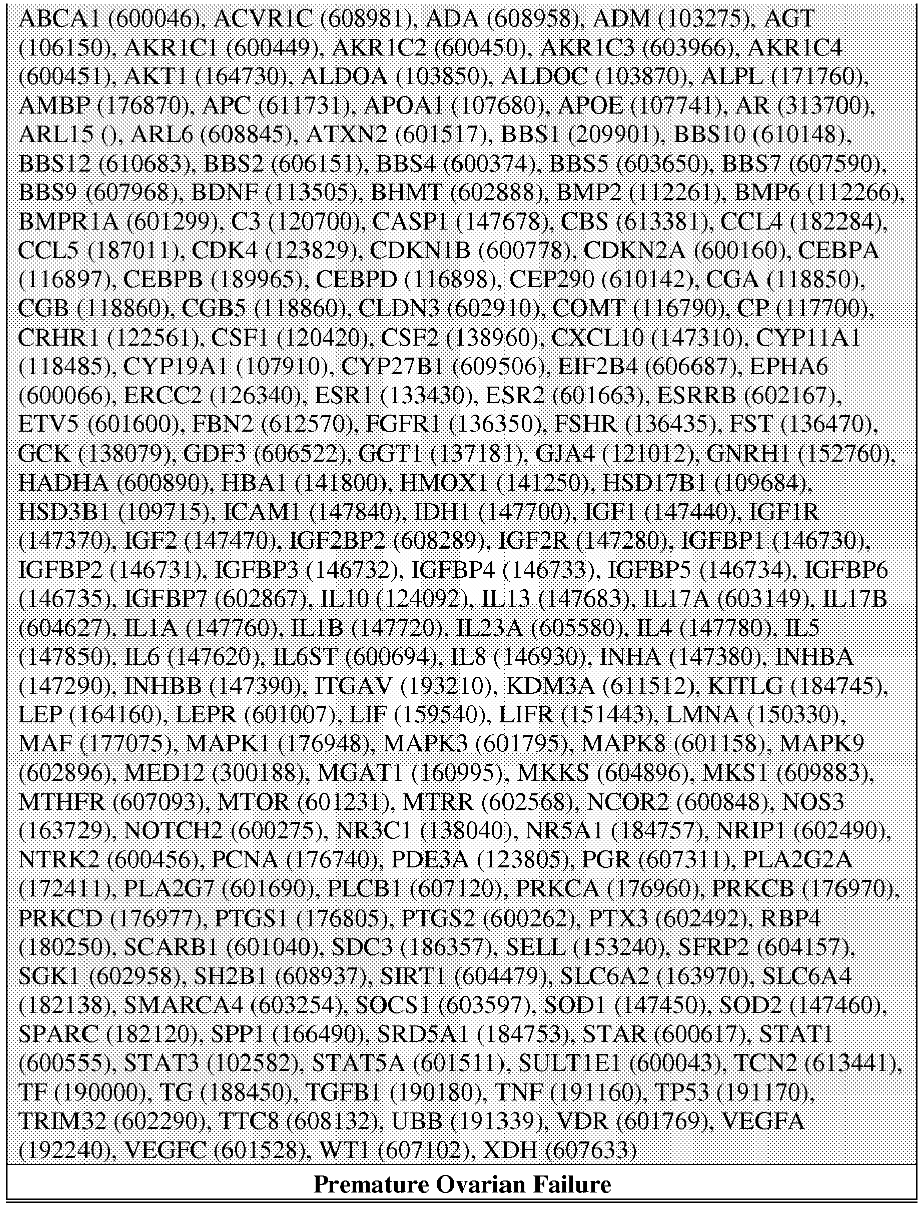

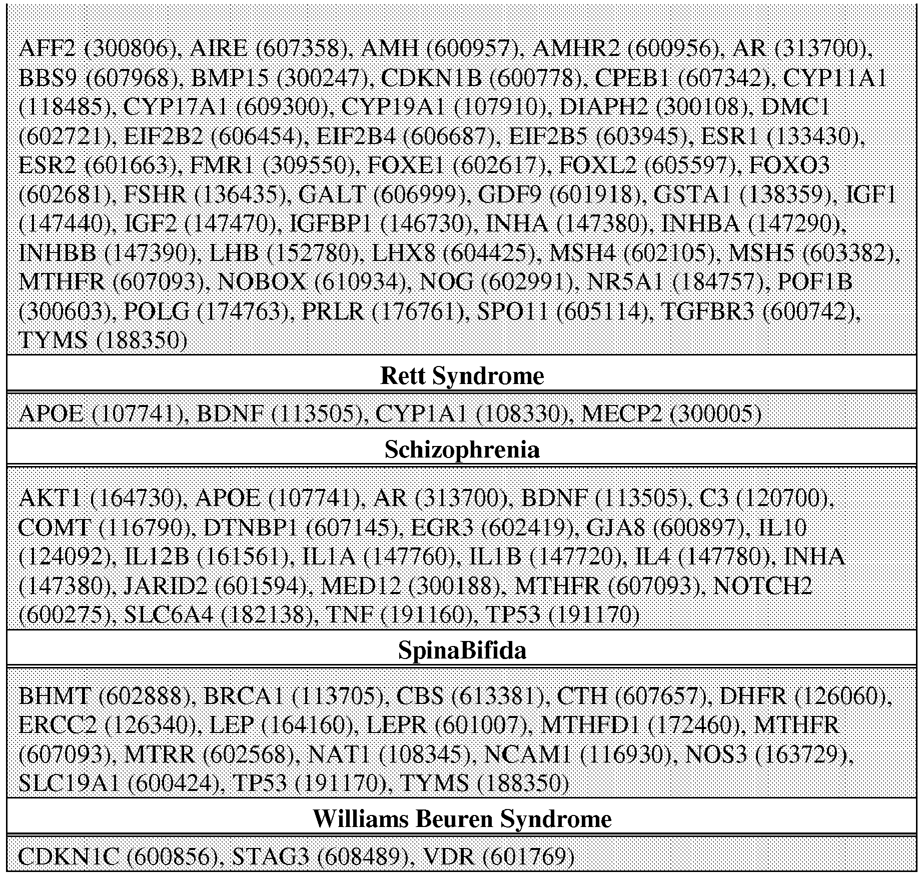

**ATP-Binding cassette subfamily A, member 1 (ABCA1)** encodes a protein that functions in cholesterol efflux, which is critical to female fertility and several other biological processes. ABCA1 is expressed in human female reproductive tissues and contributes to human female fertility (Fujimoto et al., 2010). In addition, ABCA1 knockout mice exhibit severe placental malformation which subsequently compromises mouse fertility (Trudy A Christiansen-Weber, 2000). Some genetic variants in ABCA1 have also been associated with human early-onset coronary heart disease (CHD). A study of the presence of the arg219-to-lys (R219K) variant in the ABCA1 gene revealed that the R allele was significantly more frequent in patients with early onset CHD (Cenarro, 2003).

**Adenosine Deaminase (ADA)** is an enzyme that catalyzes the deamination of adenosine in the purine catabolic pathway. ADA RNA transcript is highly expressed in human oocytes and the protein is also detected in the mouse oocyte proteome. In addition to being associated with female infertility (Nicotra et al., 1998), a search of public databases revealed that ADA is also associated with several other diseases, including Autism Spectrum Disorder (ASD), Asthma, Type II Diabetes, and Obesity. ADA-deficient mice die at approximately week 3 due to severe respiratory distress (Blackburn et al., 2000) and several variants in ADA have been associated with asthma (Bottini et al., 2002). Furthermore, autistic children show a reduced ADA activity in sera compared to controls and the ADA Asp8Asn polymorphism is overrepresented in autistic children compared to controls (Bottini et al., 2001).

**AF4/FMR2 family, member 2 (AFF2)** is an RNA binding protein and putative transcriptional activator that co-localizes with the splicing factor SC35 in nuclear speckles, dense regions of the nucleus that are thought to modulate pre-mRNA splicing. The AFF2 RNA transcript is significantly upregulated in human oocytes compared to other tissues, according to public databases. In a small study that we conducted on infertile patients, we identified a subset of patients with a rare copy number variation in the AFF2 locus (chrX: 147737851-147739165), suggesting that variants in this gene could contribute to female infertility. Similarly, another study revealed that micro-deletions in AFF2 were associated with an increased risk of premature ovarian failure (POF) (Murray et al., 1999). In addition to its association with female infertility, mutations in AFF2 are also associated with mental retardation. Microdeletions within the AFF2 locus can result in the silencing of this gene, causing Fragile X E syndrome, a form of X-linked mental retardation (Sahoo et al., 2011).

**Autism susceptibility candidate 2 (AUTS2)** According to public databases, AUTS2 RNA is uniquely expressed in the adult human female oocyte, implying that the protein product functions exclusively in this tissue. Indeed, genetic variants that appear to alter or abrogate the function of AUTS2 in pigs are associated with a reduced number of corpora lutea, an endocrine structure in porcine females produces high levels of progesterone is critical for successful reproduction (Sato et al., 2011). In addition to functioning in the adult oocyte, AUTS2 is also expressed in the developing frontal cerebral cortex and could contribute to proper brain formation (Francesco Bedogni, 2010). Several genetic variants in AUTS2 have been associated with abnormal neural development disorders, including Autism Spectrum Disorder (ASD), Attention Deficit Hyperactivity Disorder (ADHD), Epilepsy, and Addiction (Abrams et al., 1997; Gunter Schumann, 2011; Talkowski et al., 2012).

**Bardet-Biedl Syndrome Genes (BBS1, BBS2, BBS4, BBS5, BBS6, BBS7, BBS8, BBS9, BBS10, and BBS12)** encode proteins that compose or mediate the function of the BBSome, a basal body involved in the formation of the primary cilium of multiple cell types. Deleterious mutations in any of the BBS genes can cause Bardet-Biedl Syndrome, a ciliopathic human genetic disorder characterized by obesity, retinopathy, polydactyly, hypogonadism, renal dysfunction, and mental retardation. As illustrated in Figure 20, BBS1, BBS4, BBS5, BBS7, BBS9, and BBS10 are significantly overexpressed in the adult human oocyte, indicating that these genes play an important role in female fertility.

### Assays

Methods of the invention involve conducting an assay that detects either a mutation in an infertility-associated gene or abnormal expression (over or under) of an infertility-associated gene product. In particular embodiments, the assay is conducted on infertility-associated genetic regions or products of these region. Detailed descriptions of conventional methods, such as those employed to make and use nucleic acid arrays, amplification primers, hybridization probes, and the like can be found in standard laboratory manuals such as: Genome Analysis: A Laboratory Manual Series (Vols. I-IV), Cold Spring Harbor Laboratory Press; PCR Primer: A Laboratory Manual, Cold Spring Harbor Laboratory Press; and Sambrook, J et al., (2001) Molecular Cloning: A Laboratory Manual, 2nd ed. (Vols. 1-3), Cold Spring Harbor Laboratory Press. Custom nucleic acid arrays are commercially available from, e.g., Affymetrix (Santa Clara, CA), Applied Biosystems (Foster City, CA), and Agilent Technologies (Santa Clara, CA).

Methods of detecting mutations in genetic regions are known in the art. In certain embodiments, a mutation in a single infertility-associated genetic region indicates infertility. In other embodiments, the assay is conducted on more than one genetic region, and a mutation in at least two of the genetic regions indicates infertility. In other embodiments, a mutation in at least three of the genetic regions indicates infertility; a mutation in at least four of the genetic regions indicates infertility; a mutation in at least five of the genetic regions indicates infertility; a mutation in at least six of the genetic regions indicates infertility; a mutation in at least seven of the genetic regions indicates infertility; a mutation in at least eight of the genetic regions indicates infertility; a mutation in at least nine of the genetic regions indicates infertility; a mutation in at least 10 of the genetic regions indicates infertility; a mutation in at least 15 of the genetic regions indicates infertility; or a mutation in all of the genetic regions from Table 1 indicates infertility.

In certain embodiments, a known single nucleotide polymorphism at a particular position can be detected by single base extension for a primer that binds to the sample DNA adjacent to that position. See for example Shuber et al. (U.S. patent number 6,566,101), the content of which is incorporated by reference herein in its entirety. In other embodiments, a hybridization probe might be employed that overlaps the SNP of interest and selectively hybridizes to sample nucleic acids containing a particular nucleotide at that position. See for example Shuber et al. (U.S. patent number 6,214,558 and 6,300,077), the content of which is incorporated by reference herein in its entirety.

In particular embodiments, nucleic acids are sequenced in order to detect variants (i.e., mutations) in the nucleic acid compared to wild-type and/or non-mutated forms of the sequence. The nucleic acid can include a plurality of nucleic acids derived from a plurality of genetic elements. Methods of detecting sequence variants are known in the art, and sequence variants can be detected by any sequencing method known in the art e.g., ensemble sequencing or single molecule sequencing.

Sequencing may be by any method known in the art. DNA sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Sequencing of separated molecules has more recently been demonstrated by sequential or single extension reactions using polymerases or ligases as well as by single or sequential differential hybridizations with libraries of probes.

One conventional method to perform sequencing is by chain termination and gel separation, as described by Sanger et al., Proc Natl. Acad. Sci. USA, 74(12): 5463 67 (1977). Another conventional sequencing method involves chemical degradation of nucleic acid fragments. See, Maxam et al., Proc. Natl. Acad. Sci., 74: 560 564 (1977). Methods have also been developed based upon sequencing by hybridization. See, e.g., Harris et al., (U.S. patent application number 2009/0156412). The content of each reference is incorporated by reference herein in its entirety.

A sequencing technique that can be used in the methods of the provided invention includes, for example, Helicos True Single Molecule Sequencing (tSMS) (Harris T. D. et al. (2008) Science 320:106-109). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm². The flow cell is then loaded into an instrument, e.g., HeliScope.TM. sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step. Further description of tSMS is shown for example in Lapidus et al. (U.S. patent number 7,169,560), Lapidus et al. (U.S. patent application number 2009/0191565), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003), the contents of each of these references is incorporated by reference herein in its entirety.

Another example of a DNA sequencing technique that can be used in the methods of the provided invention is 454 sequencing (Roche) (Margulies, M et al. 2005, Nature, 437, 376-380). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

Another example of a DNA sequencing technique that can be used in the methods of the provided invention is SOLiD technology (Applied Biosystems). In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

Another example of a DNA sequencing technique that can be used in the methods of the provided invention is Ion Torrent sequencing (U.S. patent application numbers 2009/0026082, 2009/0127589, 2010/0035252, 2010/0137143, 2010/0188073, 2010/0197507, 2010/0282617, 2010/0300559), 2010/0300895, 2010/0301398, and 2010/0304982), the content of each of which is incorporated by reference herein in its entirety. In Ion Torrent sequencing, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to a surface and is attached at a resolution such that the fragments are individually resolvable. Addition of one or more nucleotides releases a proton (H⁺), which signal detected and recorded in a sequencing instrument. The signal strength is proportional to the number of nucleotides incorporated.

Another example of a sequencing technology that can be used in the methods of the provided invention is Illumina sequencing. Illumina sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

Another example of a sequencing technology that can be used in the methods of the provided invention includes the single molecule, real-time (SMRT) technology of Pacific Biosciences. In SMRT, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

Another example of a sequencing technique that can be used in the methods of the provided invention is nanopore sequencing (Soni G V and Meller A. (2007) Clin Chem 53: 1996-2001). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence.

Another example of a sequencing technique that can be used in the methods of the provided invention involves using a chemical-sensitive field effect transistor (chemFET) array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

Another example of a sequencing technique that can be used in the methods of the provided invention involves using a electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

If the nucleic acid from the sample is degraded or only a minimal amount of nucleic acid can be obtained from the sample, PCR can be performed on the nucleic acid in order to obtain a sufficient amount of nucleic acid for sequencing (See e.g., Mullis et al. U.S. patent number 4,683,195, the contents of which are incorporated by reference herein in its entirety).

Methods of detecting levels of gene products (e.g., RNA or protein) are known in the art. Commonly used methods known in the art for the quantification of mRNA expression in a sample include northern blotting and in situ hybridization (Parker & Barnes, Methods in Molecular Biology 106:247 283 (1999), the contents of which are incorporated by reference herein in their entirety); RNAse protection assays (Hod, Biotechniques 13:852 854 (1992), the contents of which are incorporated by reference herein in their entirety); and PCR-based methods, such as reverse transcription polymerase chain reaction (RT-PCR) (Weis et al., Trends in Genetics 8:263 264 (1992), the contents of which are incorporated by reference herein in their entirety). Alternatively, antibodies may be employed that can recognize specific duplexes, including RNA duplexes, DNA-RNA hybrid duplexes, or DNA-protein duplexes. Other methods known in the art for measuring gene expression (e.g., RNA or protein amounts) are shown in Yeatman et al. (U.S. patent application number 2006/0195269), the content of which is hereby incorporated by reference in its entirety.

A differentially expressed gene or differential gene expression refer to a gene whose expression is activated to a higher or lower level in a subject suffering from a disorder, such as infertility, relative to its expression in a normal or control subject. The terms also include genes whose expression is activated to a higher or lower level at different stages of the same disorder. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example.

Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disorder, such as infertility, or between various stages of the same disorder. Differential expression includes both quantitative, as well as qualitative, differences in the temporal or cellular expression pattern in a gene or its expression products. Differential gene expression (increases and decreases in expression) is based upon percent or fold changes over expression in normal cells. Increases may be of 1, 5, 10, 20, 30, 40, 50, 60, 70, 80, 90, 100, 120, 140, 160, 180, or 200% relative to expression levels in normal cells. Alternatively, fold increases may be of 1, 1.5, 2, 2.5, 3, 3.5, 4, 4.5, 5, 5.5, 6, 6.5, 7, 7.5, 8, 8.5, 9, 9.5, or 10 fold over expression levels in normal cells. Decreases may be of 1, 5, 10, 20, 30, 40, 50, 55, 60, 65, 70, 75, 80, 82, 84, 86, 88, 90, 92, 94, 96, 98, 99 or 100% relative to expression levels in normal cells.

In certain embodiments, reverse transcriptase PCR (RT-PCR) is used to measure gene expression. RT-PCR is a quantitative method that can be used to compare mRNA levels in different sample populations to characterize patterns of gene expression, to discriminate between closely related mRNAs, and to analyze RNA structure.

The first step is the isolation of mRNA from a target sample. The starting material is typically total RNA isolated from human tissues or fluids.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andres et al., BioTechniques 18:42044 (1995). The contents of each of theses references is incorporated by reference herein in their entirety. In particular, RNA isolation can be performed using a purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MASTERPURE Complete DNA and RNA Purification Kit (EPICENTRE, Madison, Wis.), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

The first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, Calif., USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700™ Sequence Detection System™ (Perkin-Elmer-Applied Biosystems, Foster City, Calif., USA), or Lightcycler (Roche Molecular Biochemicals, Mannheim, Germany). In certain embodiments, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Cₜ).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin. For performing analysis on pre-implantation embryos and oocytes, Chuk is a gene that is used for normalization.

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, in which internal competitor for each target sequence is used for normalization, and with quantitative comparative PCR using a normalization gene contained within the sample, or a housekeeping gene for RT-PCR. For further details see, e.g. Held et al., Genome Research 6:986 994 (1996), the contents of which are incorporated by reference herein in their entirety.

In another embodiment, a MassARRAY-based gene expression profiling method is used to measure gene expression. In the MassARRAY-based gene expression profiling method, developed by Sequenom, Inc. (San Diego, Calif.) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059 3064 (2003).

Further PCR-based techniques include, for example, differential display (Liang and Pardee, Science 257:967 971 (1992)); amplified fragment length polymorphism (iAFLP) (Kawamoto et al., Genome Res. 12:1305 1312 (1999)); BeadArray™ technology (Illumina, San Diego, Calif.; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, Tex.) in a rapid assay for gene expression (Yang et al., Genome Res. 11:1888 1898 (2001)); and high coverage expression profiling (HiCEP) analysis (Fukumura et al., Nucl. Acids. Res. 31(16) e94 (2003)). The contents of each of which are incorporated by reference herein in their entirety.

In certain embodiments, differential gene expression can also be identified, or confirmed using a microarray technique. In this method, polynucleotide sequences of interest (including cDNAs and oligonucleotides) are plated, or arrayed, on a microchip substrate. The arrayed sequences are then hybridized with specific DNA probes from cells or tissues of interest. Methods for making microarrays and determining gene product expression (e.g., RNA or protein) are shown in Yeatman et al. (U.S. patent application number 2006/0195269), the content of which is incorporated by reference herein in its entirety.

In a specific embodiment of the microarray technique, PCR amplified inserts of cDNA clones are applied to a substrate in a dense array, for example, at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pair-wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2): 106 149 (1996), the contents of which are incorporated by reference herein in their entirety). Microarray analysis can be performed by commercially available equipment, following manufacturer's protocols, such as by using the Affymetrix GenChip technology, or Incyte's microarray technology.

Alternatively, protein levels can be determined by constructing an antibody microarray in which binding sites comprise immobilized, preferably monoclonal, antibodies specific to a plurality of protein species encoded by the cell genome. Preferably, antibodies are present for a substantial fraction of the proteins of interest. Methods for making monoclonal antibodies are well known (see, e.g., Harlow and Lane, 1988, ANTIBODIES: A LABORATORY MANUAL, Cold Spring Harbor, N.Y., which is incorporated in its entirety for all purposes). In one embodiment, monoclonal antibodies are raised against synthetic peptide fragments designed based on genomic sequence of the cell. With such an antibody array, proteins from the cell are contacted to the array, and their binding is assayed with assays known in the art. Generally, the expression, and the level of expression, of proteins of diagnostic or prognostic interest can be detected through immunohistochemical staining of tissue slices or sections.

Finally, levels of transcripts of marker genes in a number of tissue specimens may be characterized using a "tissue array" (Kononen et al., Nat. Med 4(7):844-7 (1998)). In a tissue array, multiple tissue samples are assessed on the same microarray. The arrays allow in situ detection of RNA and protein levels; consecutive sections allow the analysis of multiple samples simultaneously.

In other embodiments, Serial Analysis of Gene Expression (SAGE) is used to measure gene expression. Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484 487 (1995); and Velculescu et al., Cell 88:243 51 (1997, the contents of each of which are incorporated by reference herein in their entirety).

In other embodiments Massively Parallel Signature Sequencing (MPSS) is used to measure gene expression. This method, described by Brenner et al., Nature Biotechnology 18:630 634 (2000), is a sequencing approach that combines non-gel-based signature sequencing with in vitro cloning of millions of templates on separate 5 µm diameter microbeads. First, a microbead library of DNA templates is constructed by in vitro cloning. This is followed by the assembly of a planar array of the template-containing microbeads in a flow cell at a high density (typically greater than 3 x 10⁶ microbeads/cm²). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

Immunohistochemistry methods are also suitable for detecting the expression levels of the gene products of the present invention. Thus, antibodies (monoclonal or polyclonal) or antisera, such as polyclonal antisera, specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

In certain embodiments, a proteomics approach is used to measure gene expression. A proteome refers to the totality of the proteins present in a sample (e.g. tissue, organism, or cell culture) at a certain point of time. Proteomics includes, among other things, study of the global changes of protein expression in a sample (also referred to as expression proteomics). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

In some embodiments, mass spectrometry (MS) analysis can be used alone or in combination with other methods (e.g., immunoassays or RNA measuring assays) to determine the presence and/or quantity of the one or more biomarkers disclosed herein in a biological sample. In some embodiments, the MS analysis includes matrix-assisted laser desorption/ionization (MALDI) time-of-flight (TOF) MS analysis, such as for example direct-spot MALDI-TOF or liquid chromatography MALDI-TOF mass spectrometry analysis. In some embodiments, the MS analysis comprises electrospray ionization (ESI) MS, such as for example liquid chromatography (LC) ESI-MS. Mass analysis can be accomplished using commercially-available spectrometers. Methods for utilizing MS analysis, including MALDI-TOF MS and ESI-MS, to detect the presence and quantity of biomarker peptides in biological samples are known in the art. See for example U.S. Pat. Nos. 6,925,389; 6,989,100; and 6,890,763 for further guidance, each of which is incorporated by reference herein in their entirety.

### Phenotypic traits

In certain embodiments, methods of the invention assess risk to a putative offspring of developing a condition by correlating assay results with an analysis of a phenotypic trait or environmental exposure that may be associated with infertility. Exemplary phenotypic traits or environmental exposures are shown in Table 4.

**Table 4 - Phenotypic and environmental variables impacting fertility success**

| |
|---|
| Cholesterol levels on different days of the menstrual cycle |
| Age of first menses for patient and female blood relatives (e.g. sisters, mother, grandmothers) |
| Age of menopause for female blood relatives (e.g. sisters, mother, grandmothers) |
| Number of previous pregnancies (biochemical/ectopic/clinical/fetal heart beat detected, live birth outcomes), age at the time, and outcome for patient and female blood relatives (e.g. sisters, mother, grandmothers) |
| Diagnosis of Polycystic Ovarian Syndrome |
| History of hydrosalpinx or tubal occlusion |
| History of endometriosis, pelvic pain, or painful periods |
| Cancer history/type of cancer/treatment/outcome for patient and female blood relatives (e.g. sisters, mother, grandmothers) |
| Age that sexual activity began, current level of sexual activity |
| Smoking history for patient and blood relatives |
| Travel schedule/number of flying hours a year/time difference changes of more than 3 hours (Jetlag and Flight-associated Radiation Exposure) |
| Nature of periods (length of menses, length of cycle) |
| Biological age (number of years since first menses) |
| Birth control use |
| Drug use (illegal or legal) |
| Body mass index (current, lowest ever, highest ever) |
| History of polyps |
| History of hormonal imbalance |
| History of amenorrhoea |
| History of eating disorders |
| Alcohol consumption by patient or blood relatives |
| Details of mother's pregnancy with patient (i.e. measures of uterine environment): any drugs taken, smoking, alcohol, stress levels, exposure to plastics (i.e. Tupperware), composition of diet (see below) |
| Sleep patterns: number of hours a night, continuous/overall |
| Diet: meat, organic produce, vegetables, vitamin or other supplement consumption, dairy (full fat or reduced fat), coffee/tea consumption, folic acid, sugar (complex, artificial, simple), processed food versus home cooked. |
| Exposure to plastics: microwave in plastic, cook with plastic, store food in plastic, plastic water or coffee mugs. |
| Water consumption: amount per day, format: straight from the tap, bottled water (plastic or bottle), filtered (type: e.g. Britta/Pur) |
| Residence history starting with mother's pregnancy: location/duration |
| Environmental exposure to potential toxins for different regions (extracted from government monitoring databases) |
| Health metrics: autoimmune disease, chronic illness/condition |
| Pelvic surgery history |
| Life time number of pelvic X-rays |
| History of sexually transmitted infections: type/treatment/outcome |
| Reproductive hormone levels: follicle stimulating hormone, anti-Mullerian hormone, estrogen, progesterone |
| Stress |
| Thickness and type of endometrium throughout the menstrual cycle. |
| Age |
| Height |
| Fertility treatment history and details: history of hormone stimulation, brand of drugs used, basal antral follicle count, follicle count after stimulation with different protocols, number/quality/stage of retrieved oocytes/ development profile of embryos resulting from in vitro insemination (natural or ICSI), details of IVF procedure (which clinic, doctor/embryologist at clinic, assisted hatching, fresh or thawed oocytes/embryos, embryo transfer (blood on the catheter/squirt detection and direction on ultrasound), number of successful and unsuccessful IVF attempts |
| Morning sickness during pregnancy |
| Breast size before/during/after pregnancy |
| History of ovarian cysts |
| Twin or sibling from multiple birth (mono-zygotic or di-zygotic) |
| Male factor infertility for reproductive partner: Semen analysis (count, motility,morphology), Vasectomy, male cancer, smoking, alcohol, diet, STIs |
| Blood type |
| DES exposure *in utero* |
| Past and current exercise/athletic history |
| Levels of phthalates, including metabolites: MEP - monoethyl phthalate, MECPP - mono(2-ethyl-5-carboxypentyl) phthalate, MEHHP - mono(2-ethyl-5-hydroxyhexyl) phthalate, MEOHP - mono(2-ethyl-5-ox-ohexyl) phthalate, MBP - monobutyl phthalate, MBzP - monobenzyl phthalate, MEHP - mono(2-ethylhexyl) phthalate, MiBP - mono-isobutyl phthalate, MCPP - mono(3-carboxypropyl) phthalate, MCOP - monocarboxyisooctyl phthalate, MCNP - monocarboxyisononyl phthalate |
| Familial history of Premature Ovarian Failure/Insufficiency |
| Autoimmunity history - Antiadrenal antibodies (anti-21-hydroxylase antibodies), antiovarian antibodies, antithyroid anitibodies (anti-thyroid peroxidase, antithyroglobulin) |
| Hormone levels: Leutenizing hormone (using immunofluorometric assay), Δ4-Androstenedione (using radioimmunoassay), Dehydroepiandrosterone (using radioimmunoassay), and Inhibin B (commercial ELISA) |
| Number of years trying to conceive |
| Dioxin and PVC exposure |
| Hair color |
| Nevi (moles) |
| Lead, cadmium, and other heavy metal exposure |

Information regarding the fertility-associated phenotypic traits of the female, such as those listed in Table 4, can be obtained by any means known in the art. In many cases, such information can be obtained from a questionnaire completed by the subject that contains questions regarding certain fertility-associated phenotypic traits. Additional information can be obtained from a questionnaire completed by the subject's partner and blood relatives. The questionnaire includes questions regarding the subject's fertility-associated phenotypic traits, such as her age, smoking habits, or frequency of alcohol consumption. Information can also be obtained from the medical history of the subject, as well as the medical history of blood relatives and other family members. Additional information can be obtained from the medical history and family medical history of the subject's partner. Medical history information can be obtained through analysis of electronic medical records, paper medical records, a series of questions about medical history included in the questionnaire, and a combination thereof. In other cases, the information can be obtained by analyzing a sample collected from the female subject, reproductive partners of the subject, blood relatives of the subject, and a combination thereof. The sample may include human tissue or bodily fluid. Any of the assays described herein may be used to obtain the phenotypic trait.

In other embodiments, an assay specific to an environmental exposure is used to obtain the phenotypic trait of interest. Such assays are known to those of skill in the art, and may be used with methods of the invention. For example, the hormones used in birth control pills (estrogen and progesterone) may be detected from a urine or blood test. Venners et al. (Hum. Reprod. 21(9): 2272-2280, 2006) reports assays for detecting estrogen and progesterone in urine and blood samples. Venner also reports assays for detecting the chemicals used in fertility treatments.

Similarly, illicit drug use may be detected from a tissue or body fluid, such as hair, urine sweat, or blood, and there are numerous commercially available assays (LabCorp) for conducting such tests. Standard drug tests look for ten different classes of drugs, and the test is commercially known as a "10-panel urine screen". The 10-panel urine screen consists of the following: 1. Amphetamines (including Methamphetamine) 2. Barbiturates 3. Benzodiazepines 4. Cannabinoids (THC) 5. Cocaine 6. Methadone 7. Methaqualone 8. Opiates (Codeine,Morphine, Heroin,Oxycodone, Vicodin, etc.) 9. Phencyclidine (PCP) 10. Propoxyphene. Use of alcohol can also be detected by such tests.

Numerous assays can be used to tests a patient's exposure to plastics (e.g., Bisphenol A (BPA)). BPA is most commonly found as a component of polycarbonates (about 74% of total BPA produced) and in the production of epoxy resins (about 20%). As well as being found in a myriad of products including plastic food and beverage contains (including baby and water bottles), BPA is also commonly found in various household appliances, electronics, sports safety equipment, adhesives, cash register receipts, medical devices, eyeglass lenses, water supply pipes, and many other products. Assays for testing blood, sweat, or urine for presence of BPA are described, for example, in Genuis et al. (Journal of Environmental and Public Health, Volume 2012, Article ID 185731, 10 pages, 2012).

Association studies can be performed to analyze the effect of genetic mutations or abnormal gene expression on a particular trait being studied. Infertility and risk to offspring as a trait may be analyzed as a non-continuous variable in a case-control study that includes as the patients infertile females and as controls fertile females that are age and ethnically matched. Methods including logistic regression analysis and Chi square tests may be used to identify an association between genetic mutations or abnormal gene expression and infertility and risk to offspring. In addition, when using logistic regression, adjustments for covariates like age, smoking, BMI and other factors that effect infertility, such as those shown in Table 4, may be included in the analysis.

In addition, haplotype effects can be estimated using programs such as Haploscore. Alternatively, programs such as Haploview and Phase can be used to estimate haplotype frequencies and then further analysis such as Chi square test can be performed. Logistic regression analysis may be used to generate an odds ratio and relative risk for each genetic variant or variants.

The association between genetic mutations and/or abnormal gene expression and infertility and risk to offspring may be analyzed within cases only or comparing cases and controls using analysis of variance. Such analysis may include, adjustments for covariates like age, smoking, BMI and other factors that effect infertility. In addition, haplotype effects can be estimated using programs such as Haploscore.

Method of logistic regression are described, for example in, Ruczinski (Journal of Computational and Graphical Statistics 12:475-512, 2003); Agresti (An Introduction to Categorical Data Analysis, John Wiley & Sons, Inc., 1996, New York, Chapter 8); and Yeatman et al. (U.S. patent application number 2006/0195269), the content of each of which is hereby incorporated by reference in its entirety.

Other algorithms for analyzing associations are known. For example, the stochastic gradient boosting is used to generate multiple additive regression tree (MART) models to predict a range of outcome probabilities. Each tree is a recursive graph of decisions the possible consequences of which partition patient parameters; each node represents a question (e.g., is the FSH level greater than x?) and the branch taken from that node represents the decision made (e.g. yes or no). The choice of question corresponding to each node is automated. A MART model is the weighted sum of iteratively produced regression trees. At each iteration, a regression tree is fitted according to a criterion in which the samples more involved in the prediction error are given priority. This tree is added to the existing trees, the prediction error is recalculated, and the cycle continues, leading to a progressive refinement of the prediction. The strengths of this method include analysis of many variables without knowledge of their complex interactions beforehand.

A different approach called the generalized linear model, expresses the outcome as a weighted sum of functions of the predictor variables. The weights are calculated based on least squares or Bayesian methods to minimize the prediction error on the training set. A predictor's weight reveals the effect of changing that predictor, while holding the others constant, on the outcome. In cases where one or more predictors are highly correlated, in a phenomenon known as collinearity, the relative values of their weights are less meaningful; steps must be taken to remove that collinearity, such as by excluding the nearly redundant variables from the model. Thus, when properly interpreted, the weights express the relative importance of the predictors. Less general formulations of the generalized linear model include linear regression, multiple regression, and multifactor logistic regression models, and are highly used in the medical community as clinical predictors.

### Microarrays

In certain aspects, the invention provides a microarray including a plurality of oligonucleotides attached to a substrate at discrete addressable positions, in which at least one of the oligonucleotides hybridizes to a portion of a genetic region from Table 1 that includes an infertility-associated mutation.

Methods of constructing microarrays are known in the art. See for example Yeatman et al. (U.S. patent application number 2006/0195269), the content of which is hereby incorporated by reference in its entirety.

Microarrays are prepared by selecting probes that include a polynucleotide sequence, and then immobilizing such probes to a solid support or surface. For example, the probes may comprise DNA sequences, RNA sequences, or copolymer sequences of DNA and RNA. The polynucleotide sequences of the probes may also comprise DNA and/or RNA analogues, or combinations thereof. For example, the polynucleotide sequences of the probes may be full or partial fragments of genomic DNA. The polynucleotide sequences of the probes may also be synthesized nucleotide sequences, such as synthetic oligonucleotide sequences. The probe sequences can be synthesized either enzymatically in vivo, enzymatically in vitro (e.g., by PCR), or non-enzymatically in vitro.

The probe or probes used in the methods of the invention are preferably immobilized to a solid support, which may be either porous or non-porous. For example, the probes of the invention may be polynucleotide sequences, which are attached to a nitrocellulose or nylon membrane or filter covalently at either the 3' or the 5' end of the polynucleotide. Such hybridization probes are well known in the art (see, e.g., Sambrook et al., MOLECULAR CLONING--A LABORATORY MANUAL (2ND ED.), Vols. 1-3, Cold Spring Harbor Laboratory, Cold Spring Harbor, N.Y. (1989). Alternatively, the solid support or surface may be a glass or plastic surface. In a particularly preferred embodiment, hybridization levels are measured to microarrays of probes consisting of a solid phase on the surface of which are immobilized a population of polynucleotides, such as a population of DNA or DNA mimics, or, alternatively, a population of RNA or RNA mimics. The solid phase may be a nonporous or, optionally, a porous material such as a gel.

In preferred embodiments, a microarray comprises a support or surface with an ordered array of binding (e.g., hybridization) sites or "probes" each representing one of the genes described herein, particularly the genes described in Table 1. Preferably the microarrays are addressable arrays, and more preferably positionally addressable arrays. More specifically, each probe of the array is preferably located at a known, predetermined position on the solid support such that the identity (i.e., the sequence) of each probe can be determined from its position in the array (i.e., on the support or surface). In preferred embodiments, each probe is covalently attached to the solid support at a single site.

Microarrays can be made in a number of ways, of which several are described below. However produced, microarrays share certain characteristics. The arrays are reproducible, allowing multiple copies of a given array to be produced and easily compared with each other. Preferably, microarrays are made from materials that are stable under binding (e.g., nucleic acid hybridization) conditions. The microarrays are preferably small, e.g., between 1 cm² and 25 cm², between 12 cm² and 13 cm², or 3 cm². However, larger arrays are also contemplated and may be preferable, e.g., for use in screening arrays. Preferably, a given binding site or unique set of binding sites in the microarray will specifically bind (e.g., hybridize) to the product of a single gene in a cell (e.g., to a specific mRNA, or to a specific cDNA derived therefrom). However, in general, other related or similar sequences will cross hybridize to a given binding site.

The microarrays of the present invention include one or more test probes, each of which has a polynucleotide sequence that is complementary to a subsequence of RNA or DNA to be detected. Preferably, the position of each probe on the solid surface is known. Indeed, the microarrays are preferably positionally addressable arrays. Specifically, each probe of the array is preferably located at a known, predetermined position on the solid support such that the identity (i.e., the sequence) of each probe can be determined from its position on the array (i.e., on the support or surface).

According to the invention, the microarray is an array (i.e., a matrix) in which each position represents one of the biomarkers described herein. For example, each position can contain a DNA or DNA analogue based on genomic DNA to which a particular RNA or cDNA transcribed from that genetic marker can specifically hybridize. The DNA or DNA analogue can be, e.g., a synthetic oligomer or a gene fragment. In one embodiment, probes representing each of the markers is present on the array. In a preferred embodiment, the array comprises probes for each of the genes listed in Table 1.

As noted above, the probe to which a particular polynucleotide molecule specifically hybridizes according to the invention contains a complementary genomic polynucleotide sequence. The probes of the microarray preferably consist of nucleotide sequences of no more than 1,000 nucleotides. In some embodiments, the probes of the array consist of nucleotide sequences of 10 to 1,000 nucleotides. In a preferred embodiment, the nucleotide sequences of the probes are in the range of 10-200 nucleotides in length and are genomic sequences of a species of organism, such that a plurality of different probes is present, with sequences complementary and thus capable of hybridizing to the genome of such a species of organism, sequentially tiled across all or a portion of such genome. In other specific embodiments, the probes are in the range of 10-30 nucleotides in length, in the range of 10-40 nucleotides in length, in the range of 20-50 nucleotides in length, in the range of 40-80 nucleotides in length, in the range of 50-150 nucleotides in length, in the range of 80-120 nucleotides in length, and most preferably are 60 nucleotides in length.

The probes may comprise DNA or DNA "mimics" (e.g., derivatives and analogues) corresponding to a portion of an organism's genome. In another embodiment, the probes of the microarray are complementary RNA or RNA mimics. DNA mimics are polymers composed of subunits capable of specific, Watson-Crick-like hybridization with DNA, or of specific hybridization with RNA. The nucleic acids can be modified at the base moiety, at the sugar moiety, or at the phosphate backbone. Exemplary DNA mimics include, e.g., phosphorothioates.

DNA can be obtained, e.g., by polymerase chain reaction (PCR) amplification of genomic DNA or cloned sequences. PCR primers are preferably chosen based on a known sequence of the genome that will result in amplification of specific fragments of genomic DNA. Computer programs that are well known in the art are useful in the design of primers with the required specificity and optimal amplification properties, such as Oligo version 5.0 (National Biosciences). Typically each probe on the microarray will be between 10 bases and 50,000 bases, usually between 300 bases and 1,000 bases in length. PCR methods are well known in the art, and are described, for example, in Innis et al., eds., PCR PROTOCOLS: A GUIDE TO METHODS AND APPLICATIONS, Academic Press Inc., San Diego, Calif. (1990). It will be apparent to one skilled in the art that controlled robotic systems are useful for isolating and amplifying nucleic acids.

An alternative, preferred means for generating the polynucleotide probes of the microarray is by synthesis of synthetic polynucleotides or oligonucleotides, e.g., using N-phosphonate or phosphoramidite chemistries (Froehler et al., Nucleic Acid Res. 14:5399-5407 (1986); McBride et al., Tetrahedron Lett. 24:246-248 (1983)). Synthetic sequences are typically between about 10 and about 500 bases in length, more typically between about 20 and about 100 bases, and most preferably between about 40 and about 70 bases in length. In some embodiments, synthetic nucleic acids include non-natural bases, such as, but by no means limited to, inosine. As noted above, nucleic acid analogues may be used as binding sites for hybridization. An example of a suitable nucleic acid analogue is peptide nucleic acid (see, e.g., Egholm et al., Nature 363:566-568 (1993); U.S. Pat. No. 5,539,083).

Probes are preferably selected using an algorithm that takes into account binding energies, base composition, sequence complexity, cross-hybridization binding energies, and secondary structure. See Friend et al., International Patent Publication WO 01/05935, published Jan. 25, 2001; Hughes et al., Nat. Biotech. 19:342-7 (2001).

A skilled artisan will also appreciate that positive control probes, e.g., probes known to be complementary and hybridizable to sequences in the target polynucleotide molecules, and negative control probes, e.g., probes known to not be complementary and hybridizable to sequences in the target polynucleotide molecules, should be included on the array. In one embodiment, positive controls are synthesized along the perimeter of the array. In another embodiment, positive controls are synthesized in diagonal stripes across the array. In still another embodiment, the reverse complement for each probe is synthesized next to the position of the probe to serve as a negative control. In yet another embodiment, sequences from other species of organism are used as negative controls or as "spike-in" controls.

The probes are attached to a solid support or surface, which may be made, e.g., from glass, plastic (e.g., polypropylene, nylon), polyacrylamide, nitrocellulose, gel, or other porous or nonporous material. A preferred method for attaching the nucleic acids to a surface is by printing on glass plates, as is described generally by Schena et al, Science 270:467-470 (1995). This method is especially useful for preparing microarrays of cDNA (See also, DeRisi et al, Nature Genetics 14:457-460 (1996); Shalon et al., Genome Res. 6:639-645 (1996); and Schena et al., Proc. Natl. Acad. Sci. U.S.A. 93:10539-11286 (1995)).

A second preferred method for making microarrays is by making high-density oligonucleotide arrays. Techniques are known for producing arrays containing thousands of oligonucleotides complementary to defined sequences, at defined locations on a surface using photolithographic techniques for synthesis in situ (see, Fodor et al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat. Nos. 5,578,832; 5,556,752; and 5,510,270) or other methods for rapid synthesis and deposition of defined oligonucleotides (Blanchard et al., Biosensors & Bioelectronics 11:687-690). When these methods are used, oligonucleotides (e.g., 60-mers) of known sequence are synthesized directly on a surface such as a derivatized glass slide. Usually, the array produced is redundant, with several oligonucleotide molecules per RNA.

Other methods for making microarrays, e.g., by masking (Maskos and Southern, 1992, Nuc. Acids. Res. 20:1679-1684), may also be used. In principle, and as noted supra, any type of array, for example, dot blots on a nylon hybridization membrane (see Sambrook et al., MOLECULAR CLONING--A LABORATORY MANUAL (2ND ED.), Vols. 1-3, Cold Spring Harbor Laboratory, Cold Spring Harbor, N.Y. (1989)) could be used. However, as will be recognized by those skilled in the art, very small arrays will frequently be preferred because hybridization volumes will be smaller.

In one embodiment, the arrays of the present invention are prepared by synthesizing polynucleotide probes on a support. In such an embodiment, polynucleotide probes are attached to the support covalently at either the 3' or the 5' end of the polynucleotide.

In a particularly preferred embodiment, microarrays of the invention are manufactured by means of an ink jet printing device for oligonucleotide synthesis, e.g., using the methods and systems described by Blanchard in U.S. Pat. No. 6,028,189; Blanchard et al., 1996, Biosensors and Bioelectronics 11:687-690; Blanchard, 1998, in Synthetic DNA Arrays in Genetic Engineering, Vol. 20, J. K. Setlow, Ed., Plenum Press, New York at pages 111-123. Specifically, the oligonucleotide probes in such microarrays are preferably synthesized in arrays, e.g., on a glass slide, by serially depositing individual nucleotide bases in "microdroplets" of a high surface tension solvent such as propylene carbonate. The microdroplets have small volumes (e.g., 100 pL or less, more preferably 50 pL or less) and are separated from each other on the microarray (e.g., by hydrophobic domains) to form circular surface tension wells, which define the locations of the array elements (i.e., the different probes). Microarrays manufactured by this ink-jet method are typically of high density, preferably having a density of at least about 2,500 different probes per 1 cm.sup.2. The polynucleotide probes are attached to the support covalently at either the 3' or the 5' end of the polynucleotide.

The polynucleotide molecules which may be analyzed by the present invention are DNA, RNA, or protein. The target polynucleotides are detectably labeled at one or more nucleotides. Any method known in the art may be used to detectably label the target polynucleotides. Preferably, this labeling incorporates the label uniformly along the length of the DNA or RNA, and more preferably, the labeling is carried out at a high degree of efficiency.

In a preferred embodiment, the detectable label is a luminescent label. For example, fluorescent labels, bioluminescent labels, chemiluminescent labels, and colorimetric labels may be used in the present invention. In a highly preferred embodiment, the label is a fluorescent label, such as a fluorescein, a phosphor, a rhodamine, or a polymethine dye derivative. Examples of commercially available fluorescent labels include, for example, fluorescent phosphoramidites such as FluorePrime (Amersham Pharmacia, Piscataway, N.J.), Fluoredite (Millipore, Bedford, Mass.), FAM (ABI, Foster City, Calif.), and Cy3 or Cy5 (Amersham Pharmacia, Piscataway, N.J.). In another embodiment, the detectable label is a radiolabeled nucleotide.

In a further preferred embodiment, target polynucleotide molecules from a patient sample are labeled differentially from target polynucleotide molecules of a reference sample. The reference can comprise target polynucleotide molecules from normal tissue samples.

Nucleic acid hybridization and wash conditions are chosen so that the target polynucleotide molecules specifically bind or specifically hybridize to the complementary polynucleotide sequences of the array, preferably to a specific array site, wherein its complementary DNA is located.

Arrays containing double-stranded probe DNA situated thereon are preferably subjected to denaturing conditions to render the DNA single-stranded prior to contacting with the target polynucleotide molecules. Arrays containing single-stranded probe DNA (e.g., synthetic oligodeoxyribonucleic acids) may need to be denatured prior to contacting with the target polynucleotide molecules, e.g., to remove hairpins or dimers which form due to self complementary sequences.

Optimal hybridization conditions will depend on the length (e.g., oligomer versus polynucleotide greater than 200 bases) and type (e.g., RNA, or DNA) of probe and target nucleic acids. One of skill in the art will appreciate that as the oligonucleotides become shorter, it may become necessary to adjust their length to achieve a relatively uniform melting temperature for satisfactory hybridization results. General parameters for specific (i.e., stringent) hybridization conditions for nucleic acids are described in Sambrook et al., MOLECULAR CLONING--A LABORATORY MANUAL (2ND ED.), Vols. 1-3, Cold Spring Harbor Laboratory, Cold Spring Harbor, N.Y. (1989), and in Ausubel et al., CURRENT PROTOCOLS IN MOLECULAR BIOLOGY, vol. 2, Current Protocols Publishing, New York (1994). Typical hybridization conditions for the cDNA microarrays of Schena et al. are hybridization in 5 x SSC plus 0.2% SDS at 65°C for four hours, followed by washes at 25° C in low stringency wash buffer (1 x SSC plus 0.2% SDS), followed by 10 minutes at 25°C in higher stringency wash buffer (0.1 x SSC plus 0.2% SDS) (Schena et al., Proc. Natl. Acad. Sci. U.S.A. 93:10614 (1993)). Useful hybridization conditions are also provided in, e.g., Tijessen, 1993, HYBRIDIZATION WITH NUCLEIC ACID PROBES, Elsevier Science Publishers B.V.; and Kricka, 1992, NONISOTOPIC DNA PROBE TECHNIQUES, Academic Press, San Diego, Calif.

Particularly preferred hybridization conditions include hybridization at a temperature at or near the mean melting temperature of the probes (e.g., within 51°C., more preferably within 21°C.) in 1 M NaCl, 50 mM MES buffer (pH 6.5), 0.5% sodium sarcosine and 30% formamide.

When fluorescently labeled genetic regions or products of these genetic regions are used, the fluorescence emissions at each site of a microarray may be, preferably, detected by scanning confocal laser microscopy. In one embodiment, a separate scan, using the appropriate excitation line, is carried out for each of the two fluorophores used. Alternatively, a laser may be used that allows simultaneous specimen illumination at wavelengths specific to the two fluorophores and emissions from the two fluorophores can be analyzed simultaneously (see Shalon et al., 1996, "A DNA microarray system for analyzing complex DNA samples using two-color fluorescent probe hybridization," Genome Research 6:639-645, which is incorporated by reference in its entirety for all purposes). In a preferred embodiment, the arrays are scanned with a laser fluorescent scanner with a computer controlled X-Y stage and a microscope objective. Sequential excitation of the two fluorophores is achieved with a multi-line, mixed gas laser and the emitted light is split by wavelength and detected with two photomultiplier tubes. Fluorescence laser scanning devices are described in Schena et al., Genome Res. 6:639-645 (1996), and in other references cited herein. Alternatively, the fiber-optic bundle described by Ferguson et al., Nature Biotech. 14:1681-1684 (1996), may be used to monitor mRNA abundance levels at a large number of sites simultaneously.

### References

Abrams, M. T., Doheny, K. F., Mazzocco, M. M., Knight, S. J., Baumgardner, T. L., Freund, L. S., Davies, K. E. and Reiss, A. L. (1997). Cognitive, behavioral, and neuroanatomical assessment of two unrelated male children expressing FRAXE. Am. J. Med. Genet. 74, 73-81.
Blackburn, M. R., Volmer, J. B., Thrasher, J. L., Zhong, H., Crosby, J. R., Lee, J. J. and Kellems, R. E. (2000). Metabolic Consequences of Adenosine Deaminase Deficiency in Mice Are Associated with Defects in Alveogenesis, Pulmonary Inflammation, and Airway Obstruction. Journal of Experimental Medicine 192, 159-170.
Bottini, N., De Luca, D., Saccucci, P., Fiumara, A., Elia, M., Porfirio, M. C., Lucarelli, P. and Curatolo, P. (2001). Autism: evidence of association with adenosine deaminase genetic polymorphism. Neurogenetics 3, 111-113.
Bottini, N., Ronchetti, F. and Gloria-Bottini, F. (2002). Cooperative effect of adenosine deaminase and ABO-secretor genetic complex on susceptibility to childhood asthma. European Respiratory Journal 20, 1613-1615.
Bultman, S., Gebuhr, T., Yee, D., La Mantia, C., Nicholson, J., Gilliam, A., Randazzo, F., Metzger, D., Chambon, P., Crabtree, G., et al. (2000). A Brg1 null mutation in the mouse reveals functional differences among mammalian SWI/SNF complexes. Mol. Cell 6, 1287-1295.
Burns, K. H., Viveiros, M. M., Ren, Y., Wang, P., DeMayo, F. J., Frail, D. E., Eppig, J. J. and Matzuk, M. M. (2003). Roles of NPM2 in chromatin and nucleolar organization in oocytes and embryos. Science 300, 633-636.
Carlson, L. L., Page, A. W. and Bestor, T. H. (1992). Properties and localization of DNA methyltransferase in preimplantation mouse embryos: implications for genomic imprinting. Genes Dev. 6, 2536-2541.
Cenarro, A. (2003). A common variant in the ABCA1 gene is associated with a lower risk for premature coronary heart disease in familial hypercholesterolaemia. Journal of Medical Genetics 40, 163-168.
Dion, V., Lin, Y., Hubert, L., Waterland, R. A. and Wilson, J. H. (2008). Dnmt1 deficiency promotes CAG repeat expansion in the mouse germline. Human Molecular Genetics 17, 1306-1317.
Francesco Bedogni, R. D. H. G. E. E. B. R. N. R. A. M. D. R. P. B. T. K. B. J. L. R. R. R. F. H. (2010). Tbr1 regulates regional and laminar identity of postmitotic neurons in developing neocortex. Proceedings of the National Academy of Sciences 107, 13129-13134.
Fu, W., Tao, W., Zheng, P., Fu, J., Bian, M., Jiang, Q., Clarke, P. R. and Zhang, C. (2010). Clathrin recruits phosphorylated TACC3 to spindle poles for bipolar spindle assembly and chromosome alignment. J. Cell. Sci. 123, 3645-3651.
Fujimoto, V. Y., Kane, J. P., Ishida, B. Y., Bloom, M. S. and Browne, R. W. (2010). High-density lipoprotein metabolism and the human embryo. Human Reproduction Update 16, 20-38.
Gonzalo, S., Jaco, I., Fraga, M. F., Chen, T., Li, E., Esteller, M. and Blasco, M. A. (2006). DNA methyltransferases control telomere length and telomere recombination in mammalian cells. Nat. Cell Biol. 8, 416-424.
Gunter Schumann, (2011). Genome-wide association and genetic functional studies identify autism susceptibility candidate 2 gene (AUTS2) in the regulation of alcohol consumption. Proc. Natl. Acad. Sci. U.S.A. 108, 7119-7124.
Iuchi, S. and Green, H. (1999). Basonuclin, a zinc finger protein of keratinocytes and reproductive germ cells, binds to the rRNA gene promoter. Proc. Natl. Acad. Sci. U.S.A. 96, 9628-9632.
Li, L., Baibakov, B. and Dean, J. (2008). A Subcortical Maternal Complex Essential for Preimplantation Mouse Embryogenesis. Developmental Cell 15, 416-425.
Loughery, J. E. P., Dunne, P. D., O'Neill, K. M., Meehan, R. R., McDaid, J. R. and Walsh, C. P. (2011). DNMT1 deficiency triggers mismatch repair defects in human cells through depletion of repair protein levels in a process involving the DNA damage response. Human Molecular Genetics 20, 3241-3255.
Ma, J., Zeng, F., Schultz, R. M. and Tseng, H. (2006). Basonuclin: a novel mammalian maternal-effect gene. Development 133, 2053-2062.
Murray, A., Webb, J., Dennis, N., Conway, G. and Morton, N. (1999). Microdeletions in FMR2 may be a significant cause of premature ovarian failure. Journal of Medical Genetics 36, 767-770.
Nicotra, M., Bottini, N., Grasso, M., Gimelfarb, A., Lucarini, N., Cosmi, E. and Bottini, E. (1998). Adenosine deaminase and human reproduction: a comparative study of fertile women and women with recurrent spontaneous abortion. Am. J. Reprod. Immunol. 39, 266-270.
Sahoo, T., Theisen, A., Marble, M., Tervo, R., Rosenfeld, J. A., Torchia, B. S. and Shaffer, L. G. (2011). Microdeletion of Xq28 involving the AFF2 (FMR2) gene in two unrelated males with developmental delay. Am. J. Med. Genet. A 155A, 3110-3115.
Sato, S., Hayashi, T. and Kobayashi, E. (2011). Characterization of porcine autism susceptibility candidate 2 as a candidate gene for the number of corpora lutea in pigs. Animal Reproduction Science 126, 211-220.
Talkowski, M. E., Rosenfeld, J. A., Blumenthal, I., Pillalamarri, V., Chiang, C., Heilbut, A., Ernst, C., Hanscom, C., Rossin, E., Lindgren, A. M., et al. (2012). Sequencing Chromosomal Abnormalities Reveals Neurodevelopmental Loci that Confer Risk across Diagnostic Boundaries. Cell 149, 525-537.
Trudy A Christiansen-Weber, J. R. V. Y. W. K. N. B. L. R. S. N. P. A. P. W.-P. F.-L. (2000). Functional Loss of ABCA1 in Mice Causes Severe Placental Malformation, Aberrant Lipid Distribution, and Kidney Glomerulonephritis As Well As High-Density Lipoprotein Cholesterol Deficiency. The American Journal of Pathology 157, 1017.
Wang, J., Zhang, S., Schultz, R. M. and Tseng, H. (2006). Search for basonuclin target genes. Biochemical and Biophysical Research Communications 348, 1261-1271.
Yurttas, P., Morency, E. and Coonrod, S. A. (2010). Use of proteomics to identify highly abundant maternal factors that drive the egg-to-embryo transition. Reproduction 139, 809-823.
Yurttas, P., Vitale, A. M., Fitzhenry, R. J., Cohen-Gould, L., Wu, W., Gossen, J. A. and Coonrod, S. A. (2008). Role for PADI6 and the cytoplasmic lattices in ribosomal storage in oocytes and translational control in the early mouse embryo. Development 135, 2627-2636.

### Incorporation by Reference

References and citations to other documents, such as patents, patent applications, patent publications, journals, books, papers, web contents, have been made throughout this disclosure. All such documents are hereby incorporated herein by reference in their entirety for all purposes.

### Equivalents

The invention may be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes which come within the meaning and range of equivalency of the claims are therefore intended to be embraced therein.

### EXAMPLES

### Example 1 - Identification of oocyte proteins

Oocytes are collected from females, for example mice, by superovulation, and zona pellucidae are removed by treatment with acid Tyrode solution. Oocyte plasma membrane (oolemma) proteins exposed on the surface can be distinguished at this point by biotin labeling. The treated oocytes are washed in 0.01 M PBS and treated with lysis buffer (7 M urea, 2 M thiourea, 4% (w/v) 3-[(3-cholamidopropyl)dimethylammoniol-1-propanesulfonate (CHAPS), 65 mM dithiothreitol (DTT), and 1% (v/v) protease inhibitor at -80°C). Oocyte proteins are resolved by one-dimensional or two-dimensional SDS-PAGE. The gels are stained, visualized, and sliced. Proteins in the gel pieces are digested (12.5 ng/µl trypsin in 50 mM ammonium bicarbonate overnight at 37°C), and the peptides are extracted and microsequenced.

### Example 2 - Sample Population for Identification of Infertility-Related Polymorphisms

Genomic DNA is collected from 30 female subjects (15 who have failed multiple rounds of IVF versus 15 who were successful). In particular, all of the subjects are under age 38. Members of the control group succeeded in conceiving through IVF. Members of the test group have a clinical diagnosis of idiopathic infertility, and have failed three of more rounds of IVF with no prior pregnancy. The women are able to produce eggs for IVF and have a reproductively normal male partner. To focus on infertility resulting from oocyte defects (and eliminate factors such as implantation defects) women who have subsequently conceived by egg donation are favored.

### Example 3 - Sample Population for Identification of Infertility-Related Polymorphisms

In a follow-up study of a larger cohort, genomic DNA is collected from 300 female subjects (divided into groups having profiles similar to the groups described above). The DNA sequence polymorphisms to be investigated are selected based on the results of small initial studies.

### Example 4 - Sample Population for Identification of Premature Ovarian Failure (POF) and Premature Maternal Aging Polymorphisms

Genomic DNA is collected from 30 female subjects who are experiencing symptoms of premature decline in egg quality and reserve including abnormal menstrual cycles or amenorrhea. In particular, all of the subjects are between the ages of 15-40 and have follicle stimulating hormone (FSH) levels of over 20 international units (IU) and a basal antral follicle count of under 5. Members of the control group succeeded in conceiving through IVF. Members of the test group have no previous history of toxic exposure to known fertility damaging treatments such as chemotherapy. Members of this group may also have one or more female family member who experienced menopause before the age of 40.

### Example 5 - Sample Procurement and Preparation

Blood is drawn from patients at fertility clinics for standard procedures such as gauging hormone levels and many clinics bank this material after consent for future research projects. Although DNA is easily obtained from blood, wider population sampling is accomplished using home-based, noninvasive methods of DNA collection such as saliva using an Oragene DNA self collection kit (DNA Genotek).

Blood samples - Three-milliliter whole blood samples are venously collected and treated with sodium citrate anticoagulant and stored at 4 °C until DNA extraction.

Whole Saliva - Whole saliva is collected using the Oragene DNA selfcollection kit following the manufacturer's instructions. Participants are asked to rub their tongues around the inside of their mouths for about 15 sec and then deposit approximately 2 ml saliva into the collection cup. The collection cup is designed so that the solution from the vial.'s lower compartment is released and mixes with the saliva when the cap is securely fastened. This starts the initial phase of DNA isolation, and stabilizes the saliva sample for long-term storage at room temperature or in low temperature freezers. Whole saliva samples are stored and shipped, if necessary, at room temperature. Whole saliva has the potential advantage over other non-invasive DNA sampling methods, such as buccal and oral rinse, of providing large numbers of nucleated cells (eg., epithelial cells, leukocytes) per sample.

Blood clots - Clotted blood that is usually discarded after extraction through serum separation, for other laboratory tests such as for monitoring reproductive hormone levels is collected and stored at -80 °C until extraction.

Sample Preparation - Genomic DNA is prepared from patient blood or saliva for downstream sequencing applications with commercially available kits (e.g., Invitrogen.'s ChargeSwitch® gDNA Blood Kit or DNA Genotek kits, respectively). Genomic DNA from clotted is prepared by standard methods involving proteinase K digestion, salt/chloroform extraction and 90% ethanol precipitation of DNA. (see N Kanai et al., 1994, " Rapid and simple method for preparation of genomic DNA from easily obtainable clotted blood," J Clin Pathol 47:1043-1044, which is incorporated by reference in its entirety for all purposes).

### Example 6 - Manufacturing of a Customized Oligonucleotide Library

A customized oligonucleotide library can be used to enrich samples for DNAs of interest. Several methods for manufacturing customized oligonucleotide libraries are known in the art. In one example, Nimblegen sequence capture custom array design is used to create a customized target enrichment system tailored to infertility related genes. A customized library of oligonucleotides is designed to target genetic regions of Table 1. The custom DNA oligonucleotides are synthesized on a high density DNA Nimblegen Sequence Capture Array with Maskless Array Synthesizer (MAS) technology. The Nimblegen Sequence Capture Array system workflow is array based and is performed on glass slides with an XI mixer (Roche NimbleGen) and the NimbleGen Hybridization System.

In a similar example, Agilent's eArray (a web-based design tool) is used to create a customized target enrichment system tailored to infertility related genes. The SureSelect Target Enrichment System workflow is solution-based and is performed in microcentrifuge tubes or microtiter plates. A customized oligonucleotide library is used to enrich samples for DNA of interest. Agilent's eArray (a web-based design tool) is used to created a customized target enrichment system tailored to infertility related genes. A customized library is designed to target genetic regions of Table 1. The custom RNA oligonucleotides, or baits, are biotinylated for easy capture onto streptavidin-labeled magnetic beads and used in Agilent's SureSelectTarget Enrichment System. The SureSelect Target Enrichment System workflow is solution-based and is performed in microcentrifuge tubes or microtiter plates.

### Example 7 - Capture of Genomic DNA

Genomic DNA is sheared and assembled into a library format specific to the sequencing instrument utilized downstream. Size selection is performed on the sheared DNA and confirmed by electrophoresis or other size detection method.

Several methods to capture genomic DNA are known in the art. In one example, the size-selected DNA is purified and the ends are ligated to annealed oligonucleotide linkers from Illumina to prepare a DNA library. DNA-adaptor ligated fragments are hybrized to a Nimblegen Sequence Capture array using an XI mixer (Roche NimbleGen) and the Roche NimbleGen Hybridization System. After hybridization, are washed and DNA fragments bound to the array are eluted with elution buffer. The captured DNA is then dried by centrifugation, rehydrated and PCR amplified with polymerase. Enrichment of DNA can be assessed by quantitative PCR comparison to the same sample prior to hybridization.

In a similar example, the size-selected DNA is incubated with biotinylated RNA oligonucleotides "baits" for 24 hours. The RNA/DNA hybrids are immobilized to streptavidin-labeled magnetic beads, which are captured magnetically. The RNA baits are then digested, leaving only the target selected DNA of interest, which is then amplified and sequenced.

### Example 8 - Sequencing of Target Selected DNA

Target-selected DNA is sequenced by a paired end (50bp) re-sequencing procedure using Illumina.'s Genome Analyzer. The combined DNS targeting and resequencing provides 45 fold redundancy which is greater than the accepted industry standard for SNP discovery.

### Example 9 - Correlation of Infertility with an Autism Spectrum Disorder

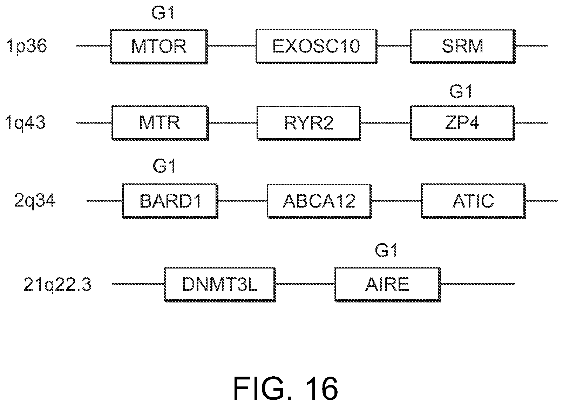

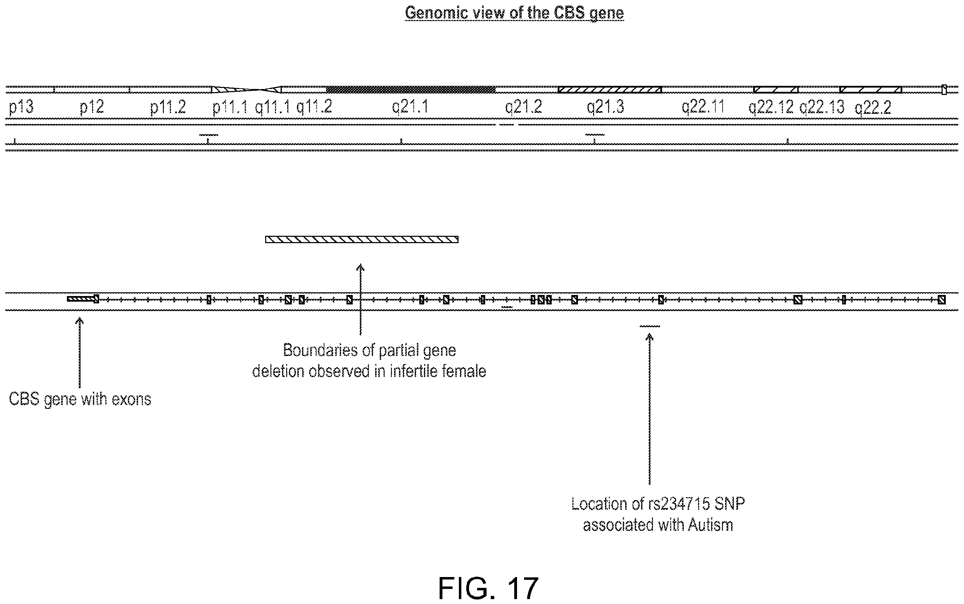

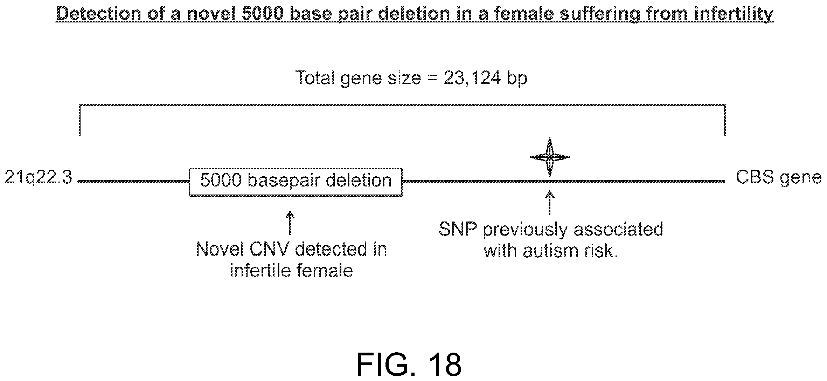

Data herein show that a number of the one carbon metabolism genes that are implicated in autism cluster in the genome close to fertility genes (Fig. 16-18). Figs. 17-18 shows a novel 5,000 bp deletion detected in a patient with infertility, who was able, with the help of IVF, to get pregnant (this person had been unsuccessful before). Data herein show that that a gene mutation can both predispose a woman for infertility and her offspring for autism or other disorders if therapy is used to help her overcome her infertility. As shown in Fig. 18, the deletion overlaps several exons and is therefore predicted to affect CBS function. Alterations in the CBS gene have been shown to be involved with autism spectrum disorder.

Embodiments of the invention may include the features of the following enumerated paragraphs ("paras").
1. A method for assessing risk to a putative offspring of developing a condition, the method comprising:
   obtaining a maternal sample;
   conducting an assay on at least one infertility-associated biomarker; and
   assessing risk to a putative offspring of developing a condition based upon results of the assay.
2. The method according to para 1, wherein the biomarker is a gene.
3. The method according to para 2, wherein the gene is a maternal effect gene.
4. The method according to para 3, wherein the assay comprises sequencing at least a portion of the gene to determine presence or absence of a mutation that is associated with infertility.
5. The method according to para 4, wherein the mutation is selected from the group consisting of: a single nucleotide polymorphism; a deletion; an insertion; a rearrangement; a copy number variation; and a combination thereof.
6. The method according to para 4, wherein sequencing is sequencing-by-synthesis.
7. The method according to para 1, wherein the biomarker is a gene product.
8. The method according to para 7, wherein the gene product is a product of a maternal effect gene.
9. The method according to para 7, wherein the gene product is RNA or protein.
10. The method according to para 7, wherein the assay comprises determining an amount of the gene product and comparing the determined amount to a reference.
11. The method according to para 1, wherein the sample is a human tissue or body fluid.
12. The method according to para 1, wherein prior to the conducting step, the method further comprises enriching the sample for the biomarker.
13. The method according to para 1, wherein assessing further comprises correlating results of the assay with at least one infertility associated phenotypic trait or environmental exposure.
14. The method according to para 1, wherein the condition is an autism spectrum disorder.
15. The method according to para 14, wherein the autism spectrum disorder is selected from the group consisting of autism (classical autism), asperger syndrome, rett syndrome, childhood disintegrative disorder, and pervasive developmental disorder not otherwise specified (PDD-NOS).
16. The method according to para 1, further comprising producing a treatment plan that reduces or eliminates the risk to the putative offspring of developing the condition based upon results of the assay.
17. The method according to para 1, wherein the treatment plan comprises administration of vitamins or drugs that reduces or eliminates the risk to the putative offspring of developing the condition.
18. A method for assessing infertility and risk to a putative offspring of developing a condition, the method comprising:
   obtaining a maternal sample;
   conducting an assay on at least one infertility-associated biomarker; and
   assessing infertility and risk to a putative offspring of developing a condition based on results of the assay.
19. The method according to para 18, wherein the biomarker is a gene.
20. The method according to para 19, wherein the assay comprises sequencing at least a portion of the gene to determine presence or absence of a mutation that is associated with infertility.
21. The method according to para 18, wherein the biomarker is a gene product.
22. The method according to para 21, wherein the assay comprises determining an amount of the gene product and comparing the determined amount to a reference.
23. The method according to para 18, wherein assessing further comprises correlating results of the assay with at least one infertility associated phenotypic trait or environmental exposure.
24. The method according to para 23, wherein the condition is an autism spectrum disorder.
25. The method according to para 24, wherein the autism spectrum disorder is selected from the group consisting of autism (classical autism), asperger syndrome, rett syndrome, childhood disintegrative disorder, and pervasive developmental disorder not otherwise specified (PDD-NOS).

## Claims

1. A method for assessing infertility in a woman and a risk to a putative offspring of the woman of developing cancer, the method comprising:
conducting an assay on a sample obtained from the woman to determine presence or absence of at least one mutation in at least one gene associated with both a) infertility and b) cancer in the putative offspring, wherein the gene is the woman's gene and is a cancer gene selected from Table 3; and
assessing risk to both the woman of infertility and to the putative offspring developing cancer based upon results of the assay.

2. The method according to claim 1, wherein the assay comprises sequencing at least a portion of the gene to determine the presence or absence of the mutation.

3. The method according to claim 1, wherein the mutation is selected from the group consisting of: a single nucleotide polymorphism; a deletion; an insertion; a rearrangement; a copy number variation; and a combination thereof.

4. The method according to claim 1, wherein sequencing is sequencing-by-synthesis.

5. The method according to claim 1, wherein the assay further comprises determining the presence or absence of an abnormal expression of a gene product, wherein the gene product is a product of a cancer gene selected from Table 3.

6. The method according to claim 5 wherein the gene product is RNA or protein.

7. The method according to claim 5, wherein the assay comprises determining an amount of the gene product and comparing the determined amount to a reference.

8. The method according to claim 5, wherein prior to the conducting step, the method further comprises enriching the sample for the gene or its gene product.

9. The method according to claim 1, wherein the sample is a human tissue or body fluid.

10. The method according to claim 1, wherein assessing further comprises correlating results of the assay with at least one infertility associated phenotypic trait or environmental exposure.

11. The method according to claim 1, wherein at least one mutation is a mutation selected from Table 2.

12. The method according to claim 1, wherein the cancer gene is selected from the group consisting of ABCA1, ACVR1, ACVR1B, ACVR2A, ADAMTS1, ADM, AFF2, AKR1C3, AKT1, ALDOB, AMBP, AMD1, ANXA1, APC, AR, AREG, ARL11, ARL3, ARL4A, ASCL2, ATF7IP, ATM, ATR, AURKA, BARD1, BAX, BBS12, BBS4, BCL2, BCL2L1, BCL2L10, BECN1, BMPR1A, BMPR1B, BOP1, BRCA1, BRCA2, BRIP1, BUB1, BUB1B, C3, CARD8, CASP1, CASP5, CASP8, CCDC28B, CCND1, CCND2, CCND3, CCNH, CD9, CDC42, CDK4, CDK6, CDK7, CDKN1B, CDKN2A, CDX2, CEBPA, CENPF, COL1A2, CSF1, CSF2, CTCF, CTGF, CTNNB1, CX3CL1, CYP1A1, DDIT3, DDX43, DICER1, DKK1, DLC1, DMC1, DNMT1, DNMT3B, EEF1A1, EEF1A2, EFNA4, EFNA5, EGR1, EGR3, EHMT1, EIF3C, EPHA1, EPHA3, EPHA7, EPHB1, EPHB2, EPHB6, ERCC1, ERCC2, ESR1, ESR2, ETV5, EZH2, FANCC, FANCG, FGF23, FGF8, FGFBP3, FGFR1, FHL2, FKBP4, FOLR1, FOXE1, FOXL2, FOXO3, FOXP3, FST, GGT1, GJA1, GPC3, GPRC5B, GRN, HAND2, HNRNPK, HOXA11, HSD17B2, HSD17B7, HSPG2, HTATIP2, ICAM1, ICAM2, IDH1, IGF1, IGF1R, IGF2BP1, IGF2R, IGFALS, IGFBP1, IGFBP2, IGFBP3, IGFBP4, IGFBP5, IGFBP7, IL17A, IL17B, IL1B, IL23A, IL6, IL6ST, IL8, INHA, INHBB, IRF1, ITGA11, ITGA2, ITGA3, ITGA9, ITGAV, ITGB1, JAG1, JAG2, KITLG, KL, KLF4, LAMC1, LAMC2, LHCGR, LIFR, LIMS1, LIMS2, LIN28B, LOXL4, LPP, MAD1L1, MAD2L1, MAF, MAP3K1, MAPK1, MAPK3, MAPK8, MAPK9, MBD2, MBD4, MDM2, MERTK, MLH1, MLH3, MOS, MPPED2, MSH2, MSH3, MSH6, MST1, MTHFR, MTOR, MUC4, MVP, MYC, NAT1, NCOA2, NCOR1, NDP, NLRP7, NNMT, NOTCH1, NOTCH2, NRIP1, NTRK1, NTRK2, PADI2, PAEP, PCNA, PDK1, PGR, PIM1, PLA2G2A, PLAG1, PLAGL1, PLCB1, PMS1, PMS2, POF1B, PPP2CB, PRDM1, PRKCD, PRKCDBP, PRKCQ, PRLR, PRMT3, PSEN2, PTGDR, PTGS2, PTN, RAD17, RGS2, RSPO1, SFRP1, SFRP2, SFRP4, SIRT1, SMAD2, SMAD3, SMAD4, SMAD7, SMAD9, SMARCA4, SOCS1, SOD3, SPARC, SPP1, STARD13, STARD3, STARD8, STAT1, STAT3, STAT4, STAT5A, STAT5B, SULT1E1, SUZ12, SYNE1, SYNE2, TACC3, TAP1, TCL1A, TCL1B, TDGF1, TERC, TERF1, TERT, TFPI, TFPI2, TG, TGFBR3, TNF, TOP2B, TP53, TP63, TSC2, UBD, UBE3A, UQCRC2, VEGFA, VEGFB, VEGFC, VHL, WISP2, WNT7A, WNT7B, WT1, XDH, YBX1, ZP2, and ZP3.
